# EUROPEAN PATENT SPECIFICATION

(11) **EP 1 392 643 B1**
(45) Date of publication and mention of the grant of the patent: **14.09.2005**
(21) Application number: 02739257.0
(22) Date of filing: 14.05.2002
(51) Int. Cl.: C07C 233/74, A61K 31/166, A61K 31/18, A61P 21/00

(54) **TRISUBSTITUTED-N- (1S)-1,2,3,4-TETRAHYDRO-1-NAPHTHALENYL] BENZAMIDES WHICH INHIBIT P2X3 AND P2X2/3 CONTAINING RECEPTORS**
TRISUBSTITUIERTE-N-((1S)-1,2,3,4-TETRAHYDRO-1-NAPHTHALENYL) BENZAMIDE WELCHE P2X3 UND P2X2/3 ENTHALTENDE REZEPTOREN INHIBIEREN
(1S)-1,2,3,4-TETRAHYDRO-1-NAPHTHALENYLE] BENZAMIDES TRISUBSTITUES EN N QUI INHIBENT DES RECEPTEURS CONTENANT DES ANTAGONISTES P2X3 ET P2X2/3

(30) Priority: 18.05.2001 US 860254; 10.05.2002 US 141989
(43) Date of publication of application: 03.03.2004
(73) Proprietor: ABBOTT LABORATORIES, Abbott Park, IL 60064-6050 (US)
(72) Inventor: LEE, Chih-Hung, Vernon Hills, IL 60061 (US); PERNER, Richard, J., Gurnee, IL 60031 (US); LARSON, Daniel, P., Highland Park, IL 60035 (US); KOENIG, John, R., Chicago, IL 60660 (US); GOMTSYAN, Arthur, R., Vernon Hills, IL 60061 (US); ZHENG, Guo Zhu, Lake Bluff, IL 60044 (US); DIDOMENICO, Stanley, Richmond, IL 60071 (US); STEWART, Andrew, O., Libertyville, IL 60048 (US); BAYBURT, Erol, K., Gurnee, IL 60031 (US)
(74) Representative: Modiano, Guido
(86) International application number: PCT/US2002/015174
(87) International publication number: WO 2002/094767

(56) References cited:
- WO-A-96/34851
- WO-A-98/47869
- COCKAYNE D A ET AL: "P2X3-DEFICIENT MICE DISPLAY URINARY BLADDER HYPOREFLEXIA AND REDUCED NOCIFENSIVE BEHAVIOR" DRUG DEVELOPMENT RESEARCH, NEW YORK, NY, US, vol. 50, 2000, page AB005 XP008011159 ISSN: 0272-4391 cited in the application
- VIRGINIO C ET AL: "TRINITROPHENYL-SUBSTITUTED NUCLEOTIDES ARE POTENT ANTAGONISTS SELECTIVE FOR P2X1, P2X3, AND HETEROMERIC P2X2/3 RECEPTORS" MOLECULAR PHARMACOLOGY, BALTIMORE, MD, US, vol. 53, no. 6, June 1998 (1998-06), pages 969-973, XP002919965 ISSN: 0026-895X

## Description

### TECHNICAL FIELD

The present invention relates to compounds of formula (I), which are useful for treating diseases or conditions caused by or exacerbated by P2X receptor activity, pharmaceutical compositions containing compounds of formula (I) and methods of treatment using compounds of formula (I).

### BACKGROUND OF THE INVENTION

P2X receptors function as homomultimeric cation-permeable ion channels and, in some cases, as heteromeric channels consisting of two different P2X receptor subtypes ((Lewis et al., Nature 377:432-435 (1995); Le et al., The Journal of Neuroscience, 18 (1998) 7152-7159, Torres et al., Molecular Pharmacology, 54 (1998) 989-993). At least one pair of P2X receptor subtypes, P2X₂ and P2X₃, functions as a heteromeric channel in rat nodose ganglion neurons where it exhibits distinct pharmacological and electrophysiological properties (Lewis et al., Nature 377:432-435 1995).

With respect to individual receptors, the rat P2X₂ containing receptor is expressed in the spinal cord, and in the nodose and dorsal root ganglia (Brake et al., Nature 371:519-523 (1994)), while rat P2X₃ containing receptor expression is found primarily in a subset of neurons of the sensory ganglia (Chen et al., Nature 377:428-430 (1995); Vulchanova et al., Neuropharmacol. 36:1229-1242 (1997)). The distribution of both receptors is consistent with a role in pain transmission. The P2X₂ and P2X₃ subunits form functional channels when expressed alone, and can also form a functional heteromultimeric channel that has properties similar to currents seen in native sensory channels when co-expressed (Lewis et al., Nature 377:432-435 (1995)). Evidence from studies in rat nodose ganglia indicate that both P2X₂/P2X₃ heteromeric channels and P2X₂ homomeric channels contribute to adenosine triphosphate-induced currents (Virginio et al., J Physiol (Lond) 510:27-35 (1998); Thomas et al., J Physiol (Lond) 509 (Pt 2):411-417 (1998)); Vulchanova et al., Proc Natl Acad Sci U S A 93:8063-8067 (1996);; Simon et al., Mol Pharmacol 52:237-248 (1997)).

ATP, which activates P2X₂, P2X₃, and P2X₂/P2X₃ containing receptors, functions as an excitatory neurotransmitter in the spinal cord dorsal horn and in primary afferents from sensory ganglia (Holton and Holton, J. Physiol. (Lond.) 126:124-140 (1954)). ATP-induced activation of P2X receptors on dorsal root ganglion nerve terminals in the spinal cord stimulates the release of glutamate, a key neurotransmitter involved in nociceptive signaling (Gu and MacDermott, Nature 389:749-753 (1997)). Thus, ATP released from damaged cells can evoke pain by activating P2X₂, P2X₃, or P2X₂/P2X₃ containing receptors on nociceptive nerve endings of sensory nerves. This is consistent with the induction of pain by intradermally applied ATP in the human blister-base model (Bleehen, Br J Pharmacol 62:573-577 (1978)); the identification of P2X₃ containing receptors on nociceptive neurons in the tooth pulp (Cook et al., Nature 387:505-508 (1997)); and with reports that P2X antagonists are analgesic in animal models (Driessen and Starke, Naunyn Schmiedebergs Arch Pharmacol 350:618-625 (1994)). This evidence suggests that P2X₂ and P2X₃ function in nociception, and that modulators of these human P2X receptors are useful as analgesics.

It has been recently demonstrated that P2X₃ receptor gene disruption results in a diminished sensitivity to noxious chemical stimuli and reduced pain (Cesare et al., Drug Dev. Res. 50: S01-02 (2000); Cockayne et al., Drug Dev. Res. 50: 005 (2000)). P2X₃ containing receptor knock-out mice also exhibited a marked urinary bladder hyporeflexia upon cystometric evaluation, suggesting that P2X₃ antagonists have utility for treating bladder overactivity. P2X₃ knock-out mice had decreased voiding frequency, increased voiding volume, but normal bladder pressure. It has been proposed that ATP acts as a physiological regulator of sensory neurotransmission in visceral hollow organs such as bladder (Namasivayam et al., Brit. J. Urol. Int. 84L 854-860. (1999), and P2X₃ containing receptors localized on the basal surface of the urothelium. The urology data on the P2X₃ knock-out mice suggest that P2X₃ plays a major role in modulating the volume threshold for activation of micturition and that P2X₃ antagonists have therapeutic utility for urinary incontinence.

The nociceptive effects of exogenously administered ATP and P2X containing receptor agonists have also been demonstrated in laboratory animals (Bland-Ward and Humphrey, 1997; Hamilton et al., 1999). The peripheral nociceptive actions of P2X activation and stimulation of spinal P2X containing receptors also contribute to nociception as indicated by the ability of intrathecally (i.t.) administered P2 receptor agonists to increase sensitivity to acute and persistent noxious stimuli in rodents (Driessen et al., 1994; Tsuda et al., 1999a; 1999b).

The utility of available purinergic ligands to evaluate the role of individual P2 receptor subtypes in mammalian physiology has been complicated by the susceptibility of P2 receptor agonists to undergo enzymatic degradation, and by the lack of P2 receptor subtype-selective agonists and antagonists (King et al., 1999; Ralevic and Burnstock, 1998).

Since subtype-selective ligands for the individual P2 receptors have yet to be identified, efforts to elucidate the specific P2X containing receptor subtypes involved in the transmission of nociceptive signals has been largely based on receptor localization and functional studies using immunohistochemical techniques. These studies have shown that both the homomeric P2X₃ and heteromeric P2X_{2/3} containing receptor subtypes are selectively localized to the central and peripheral terminals of small diameter sensory neurons (Chen et al., 1995; Lewis et al., 1995; Vulchanova et al., 1997; 1998). Further, recent data has shown that P2X₃ specific immunoreactivity is significantly increased in both the injured dorsal root ganglion and in the ipsalateral spinal dorsal horn following chronic constriction injury of the rat sciatic nerve (Novakovic et al., 1999).

- Several trinitrophenyl analogs of ATP have been shown to be potent antagonists at those P2X receptors that can be activated by αβmeATP, i.e. at the P2X₁, P2X₃, and P2X_{2/3} receptors (Virginio *et al.,* "Trinitrophenyl-substituted Nucleotides are Potent Antagonists Selective for P2X₁, P2X₃, and Heteromeric P2X_{2/3} Receptors" Mol. Pharmacology 53:9690973 (1998)-.

The functional and immunohistochemical localization of P2X₃ and/or P2X_{2/3} containing receptors on sensory nerves indicates that these P2X containing receptors have a primary role in mediating the nociceptive effects of exogenous ATP. Thus, compounds which block or inhibit activation of P2X₃ containing receptors serve to block the pain stimulus. Antagonists of the P2X₃ homomeric channel and/or the P2X₂/P2X₃ heteromeric channel could successfully block the transmission of pain.

The compounds of the present invention are novel P2X₃ and P2X_{2/3} antagonists, having utility in treating pain as well as in treating bladder overactivity and urinary incontinence.

### SUMMARY OF THE INVENTION

The present invention discloses trisubstituted-N-[(1S)-1,2,3,4-tetrahydro-1-naphthalenyl]benzamides compounds, a method for controlling pain in mammals, and pharmaceutical compositions including those compounds. More particularly, the present invention is directed to compounds of formula (I) 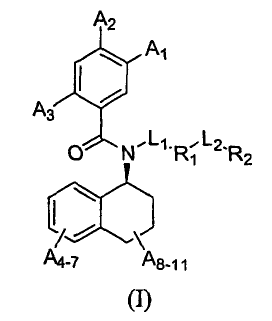 or pharmaceutically acceptable salts or prodrugs thereof, wherein
A₁ and A₂ are each independently selected from alkoxycarbonyl, alkylcarbonyloxy, carboxy, hydroxy, hydroxyalkyl, (NR_{A}R_{B})carbonyl, (NR_{C}S(O)₂R_{D})carbonyl, -S(O)₂OH, or tetrazolyl;
or
A₁ and A₂ together with the carbon atoms to which they are attached form a five membered heterocycle containing a sulfur atom wherein the five membered heterocycle is optionally substituted with 1 or 2 substituents selected from mercapto or oxo;
A₃ is selected from alkoxycarbonyl, alkylcarbonyloxy, carboxy, hydroxy, hydroxyalkyl, (NR_{A}R_{B})carbonyl, (NR_{C}S(O)₂R_{D})carbonyl, -S(O)₂OH, or tetrazolyl;
A₄, A₅, A₆ and A₇ are each independently selected from hydrogen, alkoxy, alkoxycarbonyl, alkenyl, alkyl, alkylcarbonyl, alkylcarbonyloxy, alkynyl, carboxy, cyano, haloalkoxy, haloalkyl, halogen, hydroxy, hydroxyalkyl, nitro, -NR_{E}R_{F}, or (NR_{E}R_{F})carbonyl;
A₈, A₉, A₁₀ and A₁₁ are each independently selected from hydrogen, alkoxy, alkoxycarbonyl, alkenyl, alkyl, alkylcarbonyl, alkylcarbonyloxy, alkynyl, carboxy, haloalkoxy, haloalkyl, halogen, hydroxy, hydroxyalkyl, -NR_{E}R_{F}, (NR_{E}R_{F})carbonyl, or oxo;
R_{A} and R_{B} are each independently selected from hydrogen, alkyl, or cyano;
R_{C} is selected from hydrogen or alkyl;
R_{D} is selected from alkoxy, alkyl, aryl, arylalkoxy, arylalkyl, haloalkoxy, or haloalkyl;
R_{E} and R_{F} are each independently selected from hydrogen, alkyl, alkylcarbonyl, formyl, or hydroxyalkyl;
L₁ is selected from alkenylene, alkylene, alkynylene, -(CH₂)ₘO(CH₂)ₙ-, -(CH₂)ₘS(CH₂)ₙ-, or -(CH₂)ₚC(O)(CH₂)_{q}- wherein each group is drawn with the left end attached to N and the right end attached to R₁;
m is an integer 0-10;
n is an integer 0-10;
R₁ is selected from aryl, cycloalkenyl, cycloalkyl, or heterocycle; wherein the aryl group is substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}Rc, and -CH=NOR_{EE} wherein R_{EE} is selected from hydrogen and alkyl, as defined herein; the cycloalkenyl and cycloalkyl groups are substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo,-NR_{E}R_{F}, (NR_{E}R_{F})carbonyl and -CH=NOR_{EE} wherein R_{EE} is selected from hydrogen and alkyl, as defined herein; and the heterocycle group is substituted with 0, 1, 2, or 3 substituents independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, arylalkoxycarbonyl, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(RA)C(O)NR_{B}R_{C}, and -CH=NOR_{EE} wherein R_{EE} is selected from hydrogen and alkyl, as defined herein;
L₂ is absent or selected from a covalent bond, alkenylene, alkylene, alkynylene, -(CH₂)ₚO(CH₂)_{q}-, -(CH₂)ₚS(CH₂)_{q}-, -(CH₂)ₚC(O)(CH₂)_{q}-, -(CH₂)ₚC(OH)(CH₂)_{q}-, or -(CH₂)ₚCH=NO(CH₂)_{q}- wherein each group is drawn with the left end attached to R₁ and the right end attached to R₂;
p is an integer 0-10;
q is an integer 0-10; and
R₂ is absent or selected from aryl, cycloalkenyl, cycloalkyl, or heterocycle. wherein the aryl group is substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, allcoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}Rc, and -CH=NOR_{EE} wherein R_{EE} is selected from hydrogen and alkyl, as defined herein; the cycloalkenyl and cycloalkyl groups are substituted with 0, 1, 2, 3, 4 or 5 substituents each independently selected from alkenyl, alkenyloxy, alkoxy, alkoxyalkoxy, allcoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo,-NR_{E}R_{F}, (NR_{E}R_{F})carbonyl and -CH=NOR_{EE} wherein R_{EE} is selected from hydrogen and alkyl, as defined herein; and the heterocycle group is substituted with 0, 1, 2, or 3 substituents independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, arylalkoxycarbonyl, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(RA)C(O)NR_{B}Rc, and -CH=NOR_{EE} wherein R_{EE} is selected from hydrogen and alkyl, as defined herein;

### DETAILED DESCRIPTION OF THE INVENTION

The principle embodiment of the present invention is directed to compounds of formula (I) 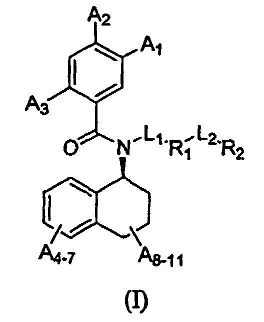 or pharmaceutically acceptable salts or prodrugs thereof, wherein
A₁ and A₂ are each independently selected from alkoxycarbonyl, alkylcarbonyloxy, carboxy, hydroxy, hydroxyalkyl, (NR_{A}R_{B})carbonyl, (NR_{C}S(O)₂R_{D})carbonyl, -S(O)₂OH, or tetrazolyl;
or
A₁ and A₂ together with the carbon atoms to which they are attached form a five membered heterocycle containing a sulfur atom wherein the five membered heterocycle is optionally substituted with 1 or 2 substituents selected from mercapto or oxo;
A₃ is selected from alkoxycarbonyl, alkylcarbonyloxy, carboxy, hydroxy, hydroxyalkyl, (NR_{A}R_{B})carbonyl, (NR_{C}S(O)₂R_{D})carbonyl, -S(O)₂OH, or tetrazolyl;
A₄, A₅, A₆ and A₇ are each independently selected from hydrogen, alkoxy, alkoxycarbonyl, alkenyl, alkyl, alkylcarbonyl, alkylcarbonyloxy, alkynyl, carboxy, cyano, haloalkoxy, haloalkyl, halogen, hydroxy, hydroxyalkyl, nitro, -NR_{E}R_{F}, or (NR_{E}R_{F})carbonyl;
A₈, A₉, A₁₀ and A₁₁ are each independently selected from hydrogen, alkoxy, alkoxycarbonyl, alkenyl, alkyl, alkylcarbonyl, alkylcarbonyloxy, alkynyl, carboxy, haloalkoxy, haloalkyl, halogen, hydroxy, hydroxyalkyl, -NR_{E}R_{F}, (NR_{E}R_{F})carbonyl, or oxo;
R_{A} and R_{B} are each independently selected from hydrogen, alkyl, or cyano;
R_{C} is selected from hydrogen or alkyl;
R_{D} is selected from alkoxy, alkyl, aryl, arylalkoxy, arylalkyl, haloalkoxy, or haloalkyl;
R_{E} and R_{F} are each independently selected from hydrogen, alkyl, alkylcarbonyl, formyl, or hydroxyalkyl;
L₁ is selected from alkenylene, alkylene, alkynylene, -(CH₂)ₘO(CH₂)ₙ-, -(CH₂)ₘS(CH₂)ₙ-, or -(CH₂)ₚC(O)(CH₂)_{q}- wherein each group is drawn with the left end attached to N and the right end attached to R₁;
m is an integer 0-10;
n is an integer 0-10;
R₁ is selected from aryl, cycloalkenyl, cycloalkyl, or heterocycle; wherein the aryl group is substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}Rc, and -CH=NOR_{EE} wherein R_{EE} is selected from hydrogen and alkyl, as defined herein; the cycloalkenyl and cycloalkyl groups are substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo,-NR_{E}R_{F}, (NR_{E}R_{F})carbonyl and -CH=NOR_{EE} wherein R_{EE} is selected from hydrogen and alkyl, as defined herein; and the heterocycle group is substituted with 0, 1, 2, or 3 substituents independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, arylalkoxycarbonyl, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(RA)C(O)NR_{B}R_{C}, and -CH=NOR_{EE} wherein R_{EE} is selected from hydrogen and alkyl, as defined herein;
L₂ is absent or selected from a covalent bond, alkenylene, alkylene, alkynylene, -(CH₂)ₚO(CH₂)_{q}-, -(CH₂)ₚS(CH₂)_{q}-, -(CH₂)ₚC(O)(CH₂)_{q}-, -(CH₂)ₚC(OH)(CH₂)_{q}-, or -(CH₂)ₚCH=NO(CH₂)_{q}- wherein each group is drawn with the left end attached to R₁ and the right end attached to R₂;
p is an integer 0-10;
q is an integer 0-10; and
R₂ is absent or selected from aryl, cycloalkenyl, cycloalkyl, or heterocycle; wherein the aryl group is substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkytthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}Rc, and -CH=NOR_{EE} wherein R_{EE} is selected from hydrogen and alkyl, as defined herein; the cycloalkenyl and cycloalkyl groups are substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo,-NR_{E}R_{F}, (NR_{E}R_{F})carbonyl and -CH=NOR_{EE} wherein R_{EE} is selected from hydrogen and alkyl, as defined herein; and the heterocycle group is substituted with 0, 1, 2, or 3 substituents independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, arylalkoxycarbonyl, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(RA)C(O)NR_{B}R_{C}, and -CH=NOR_{EE} wherein R_{EE} is selected from hydrogen and alkyl, as defined herein.

In another embodiment, compounds of the present invention have formula (I) wherein A₁ and A₂ are each independently selected from alkoxycarbonyl, carboxy, hydroxy, (NR_{A}R_{B})carbonyl, (NR_{C}S(O)₂R_{D})carbonyl, and -S(O)₂OH; A₃ is carboxy; A₄, A₅, A₆, A₇, A₈, A₉, A₁₀ and A₁₁ are each hydrogen; R_{A} and R_{B} are each independently selected from hydrogen, alkyl, and cyano; L₁ is selected from alkylene and -(CH₂)ₘO(CH₂)ₙ-; R₁ is selected from aryl, cycloalkyl and heterocycle; L₂ is absent or selected from a covalent bond, alkylene, -(CH₂)ₚO(CH₂)_{q}-, -(CH₂)ₚC(O)(CH₂)_{q}-, -(CH₂)ₚC(OH)(CH₂)_{q}-, -(CH₂)ₚS(CH₂)_{q}-, and -(CH₂)ₚCH=NO(CH₂)_{q}-; p is 0; q is an integer 0-1; R₂ is absent or selected from aryl, cycloalkenyl, cycloalkyl and heterocycle; and R_{C} and R_{D} are as defined in formula (I).

In another embodiment, compounds of the present invention have formula (I) wherein A₁ and A₂ together with the carbon atoms to which they are attached form a five membered heterocycle containing a sulfur atom wherein the five membered heterocycle is optionally substituted with 1 or 2 substituents selected from mercapto and oxo; and A₃, A₄, A₅, A₆, A₇, A₈, A₉, A₁₀, A₁₁, L₁, R₁, L₂ and R₂ are as defined in formula (I).

In another embodiment, compounds of the present invention have formula (I) wherein L₁ is alkylene; R₁ is aryl; L₂ is absent; R₂ is absent; and A₁, A₂, A₃, A₄, A₅, A₆, A₇, A₈, A₉, A₁₀ and A₁₁ are as defined in formula (I).

In another embodiment, compounds of the present invention have formula (I) wherein L₁ is alkylene; R₁ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; L₂ is absent; R₂ is absent; and R_{A}, R_{B}, R_{C}, R_{E}, R_{F}, R_{EE}, A₁, A₂, A₃, A₄, A₅, A₆, A₇, A₈, A₉, A₁₀ and A₁₁ are as defined in formula (I).

In another embodiment, compounds of the present invention have formula (I) wherein A₁, A₂ and A₃ are each carboxy; A₄, A₅, A₆, A₇, A₈, A₉, A₁₀ and A₁₁ are each hydrogen; L₁ is alkylene wherein said is alkylene selected from -CH₂-, -CH₂CH₂- and -CH₂CH(CH₃)-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is absent; and R₂ is absent; and R_{E}, R_{F}, and R_{EE} are as defined as in formula (I).

In another embodiment, compounds of the present invention have formula (I) wherein L₁ is alkylene; R₁ is aryl wherein said aryl is fluorenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxy, hydroxyalkyl, nitro, oxo, or -NR_{E}R_{F}; L₂ is absent; R₂ is absent; and R_{E}, R_{F}, A₁, A₂, A₃, A₄, A₅, A₆, A₇, A₈, A₉, A₁₀ and A₁₁ are as defined in formula (I).

In another embodiment, compounds of the present invention have formula (I) wherein A₁, A₂ and A₃ are each carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is fluorenyl substituted with 0 or 1 substituent selected from hydroxy and oxo; L₂ is absent; and R₂ is absent.

In another embodiment, compounds of the present invention have formula (I) wherein L₁ is alkylene; R₁ is aryl; L₂ is a covalent bond; R₂ is aryl; and A₁, A₂, A₃, A₄, A₅, A₆, A₇, A₈, A₉, A₁₀ and A₁₁ are as defined in formula (I).

In another embodiment, compounds of the present invention have formula (I) wherein L₁ is alkylene; R₁ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; L₂ is a covalent bond; R₂ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; and R_{A}, R_{B}, R_{C}, R_{E}, R_{F}, R_{EE}, A₁, A₂, A₃, A₄, A₅, A₆, A₇, A₈, A₉, A₁₀ and A₁₁ are as defined in formula (I).

In another embodiment, compounds of the present invention have formula (I) wherein A₁, A₂ and A₃ are each carboxy; A₄, A₅, A₆, A₇, A₈, A₉, A₁₀ and An are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is a covalent bond; and R₂ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; and R_{E}, R_{F}, and R_{EE} are as defined as in formula (I).

In another embodiment, compounds of the present invention have formula (I) wherein A₁ and A₂ together with the carbon atoms to which they are attached form a five membered heterocycle containing a sulfur atom wherein the five membered heterocycle is substituted with 0,1, or 2 substituents selected from mercapto and oxo; A₃ is carboxy; A₄, A₅, A₆, A₇, A₈, A₉, A₁₀, and A₁₁ are each hydrogen; L₁ is alkylene wherein said alkylene is -CH₂-; R₁ is aryl wherein said aryl is phenyl substituted with 0,1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is a covalent bond; R₂ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; and R_{E}, R_{F}, and R_{EE} are as defined as in formula (I).

In another embodiment, compounds of the present invention have formula (I) wherein L₁ is alkylene; R₁ is aryl; L₂ is a covalent bond; R₂ is heterocycle; and A₁, A₂, A₃, A₄, A₅, A₆, A₇, A₈, A₉, A₁₀ and A₁₁ are as defined in formula (I).

In another embodiment, compounds of the present invention have formula (I) wherein L₁ is alkylene; R₁ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, akylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; L₂ is a covalent bond; R₂ is heterocycle wherein said heterocycle is selected from azetidinyl, azepanyl, aziridinyl, furyl, morpholinyl, piperazinyl, piperidinyl, pyrazinyl, pyridazinyl, pyridinyl, pyrimidinyl, pyrrolidinyl and thienyl wherein said heterocycle is substituted with 0, 1, 2,or 3 substituents independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, arylalkoxycarbonyl, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NRER_{F}, (NR_{E}R_{F})carbonyl, -N(R_{A})C(O)NR_{B}R_{C}, or -CH=NOR_{EE}; and R_{A}, R_{B}, R_{C}, R_{E}, R_{F}, R_{EE}, A₁, A₂, A₃, A₄, A₅, A₆, A₇, A₈ A₉, A₁₀ and A₁₁ are as defined in formula (I).

In another embodiment, compounds of the present invention have formula (I) wherein A₁, A₂ and A₃ are each carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is a covalent bond; and R₂ is heterocycle wherein said heterocycle is selected from pyridinyl, pyrrolidinyl, and thienyl wherein said heterocycle is substituted with 0,1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, nitro, -N(R_{A})C(O)NR_{B}R_{C}, or -NR_{E}R_{F}; and R_{A}, R_{B}, R_{C}, R_{E}, R_{F}, R_{EE} are as defined in formula (I).

In another embodiment, compounds of the present invention have formula (I) wherein L₁ is alkylene; R₁ is aryl; L₂ is alkylene; R₂ is aryl; and A₁, A₂, A₃, A₄, A₅, A₆, A₇, A₈, A₉, A₁₀ and A₁₁ are as defined in formula (I).

In another embodiment, compounds of the present invention have formula (I) wherein L₁ is alkylene; R₁ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; L₂ is alkylene; R₂ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; and R_{A}, R_{B}, R_{C}, R_{E}, R_{F}, R_{EE}, A₁, A₂, A₃, A₄, A₅, A₆, A₇, A₈, A₉, A₁₀ and A₁₁ are as defined in formula (I).

In another embodiment, compounds of the present invention have formula (I) wherein A₁, A₂ and A₃ are each carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is alkylene wherein said alkylene is -CH₂-; and R₂ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; and R_{E}, R_{F}, and R_{EE} are as defined in formula (I).

In another embodiment, compounds of the present invention have formula (I) wherein L₁ is alkylene; R₁ is aryl; L₂ is -(CH₂)ₚO(CH₂)_{q}-; R₂ is aryl; and A₁, A₂, A₃, A₄, A₅, A₆, A₇, A₈, A₉, A₁₀ A₁₁, p and q are as defined in formula (I).

In another embodiment, compounds of the present invention have formula (I) wherein L₁ is alkylene; R₁ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; L₂ is -(CH₂)ₚO(CH₂)_{q}-; R₂ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; and R_{A}, R_{B}, R_{C}, R_{E}, R_{F}, R_{EE}, A₁, A₂, A₃, A₄, A₅, A₆, A₇, A₈, A₉, A₁₀ A₁₁, p and q are as defined in formula (I).

In another embodiment, compounds of the present invention have formula (I) wherein A₁, A₂ and A₃ are each carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is -(CH₂)ₚO(CH₂)_{q}-; p is 0; q is 0; and R₂ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; and R_{E}, R_{F}, and R_{EE} are as defined in formula (I).

In another embodiment, compounds of the present invention have formula (I) wherein A₁ and A₂ are each independently selected from alkoxycarbonyl, carboxy, hydroxy, (NR_{A}R_{B})carbonyl, or -S(O)₂OH; A₃ is carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is -(CH₂)ₚO(CH₂)_{q}-; p is 0; q is 0; and R₂ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; and R_{E}, R_{F}, and R_{EE} are as defined in formula (I).

In another embodiment, compounds of the present invention have formula (I) wherein A₁ and A₂ are each independently selected from carboxy or (NR_{C}S(O)₂R_{D})carbonyl wherein one of A₁ or A₂ is (NR_{C}S(O)₂R_{D})carbonyl; A₃ is carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; L₂ is -(CH₂)ₚO(CH₂)_{q}-; p is 0; q is 0; R₂ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; and R_{A}, R_{B}, R_{C}, R_{D}, R_{E}, R_{F}, and R_{EE} are as defined in formula (I).

In another embodiment, compounds of the present invention have formula (I) wherein A₁ and A₂ are each independently selected from carboxy or (NR_{C}S(O)₂R_{D})carbonyl wherein one of A₁ or A₂ is (NR_{C}S(O)₂R_{D})carbonyl; A₃ is carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is -(CH₂)ₚO(CH₂)_{q}-; p is 0; q is 0; R₂ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; R_{D} is selected from alkyl or aryl; and R_{A}, R_{B}, R_{C}, R_{E}, R_{F}, and R_{EE} are as defined in formula (I).

In another embodiment, compounds of the present invention have formula (I) whereinA₁ and A₂ are each independently selected from carboxy or (NR_{C}S(O)₂R_{D})carbonyl wherein one of A₁ or A₂ is (NR_{C}S(O)₂R_{D})carbonyl; A₃ is carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F}; L₂ is -(CH₂)ₚO(CH₂)_{q}-; p is 0; q is 0; R₂ is aryl wherein said aryl is phenyl substituted with 0,1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F}; R_{D} is selected from alkyl or aryl wherein said aryl is phenyl substituted with 0,1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, -NR_{E}R_{F}, and -N(R_{A})C(O)NR_{B}R_{C}; and R_{A}, R_{B}, R_{C}, R_{E}, R_{F}, and R_{EE} are as defined in formula (I).

In another embodiment, compounds of the present invention have formula (I) wherein A₁ and A₂ are each independently selected from carboxy or (NR_{C}S(O)₂R_{D})carbonyl wherein one of A₁ or A₂ is (NR_{C}S(O)₂R_{D})carbonyl; A₃ is carboxy; A₄, A₅, A₆, A₇, A₈ A₉, A₁₀ and A₁₁ are each hydrogen; L₁ is alkylene wherein said alkylene is -CH₂-; R₁ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; L₂ is -(CH₂)ₚO(CH₂)_{q}-; p is 0; q is 0; R₂ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; R_{D} is heterocycle; and R_{A}, R_{B}, R_{C}, R_{E}, R_{F}, and R_{EE} are as defined in formula (I).
XX In another embodiment, compounds of the present invention have formula (I) wherein A₁ and A₂ are each independently selected from carboxy or (NR_{C}S(O)₂R_{D})carbonyl wherein one of A₁ or A₂ is (NR_{C}S(O)₂R_{D})carbonyl; A₃ is carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is -(CH₂)ₚO(CH₂)_{q}-; p is 0; q is 0; R₂ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; R_{D} is heterocycle wherein said heterocycle is pyridinyl and thienyl wherein said heterocycle is substituted with 0,1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, nitro, -N(R_{A})C(O)NR_{B}R_{C}, or -NR_{E}R_{F}; and R_{A}, R_{B}, R_{C}, R_{E}, R_{F}, and R_{EE} are as defined in formula (I).

In another embodiment, compounds of the present invention have formula (I) wherein A₁ and A₂ are each independently selected from carboxy or (NR_{C}S(O)₂R_{D})carbonyl wherein one of A₁ or A₂ is (NR_{C}S(O)₂R_{D})carbonyl; A₃ is carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloallcylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; L₂ is -(CH₂)ₚO(CH₂)_{q}-; p is 0; q is 0; R₂ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; R_{D} is arylalkyl; and R_{A}, R_{B}, R_{C}, R_{E}, R_{F}, and R_{EE} are as defined in formula (I).

In another embodiment, compounds of the present invention have formula (I) wherein A₁ and A₂ are each independently selected from carboxy or (NR_{C}S(O)₂R_{D})carbonyl wherein one of A₁ or A₂ is (NR_{C}S(O)₂R_{D})carbonyl; A₃ is carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is -(CH₂)ₚO(CH₂)_{q}-; p is 0; q is 0; R₂ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; R_{D} is arylalkyl wherein the aryl of arylalkyl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; and R_{C}, R_{E}, R_{F}, and R_{EE} are as defined in formula (I).

In another embodiment, compounds of the present invention have formula (I) wherein A₁, A₂ and A₃ are each carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is -(CH₂)ₚO(CH₂)_{q}-; p is 0; q is 1; and R₂ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; and R_{E}, R_{F}, and R_{EE} are as defined in formula (I).

In another embodiment, compounds of the present invention have formula (I) wherein L₁ is alkylene; R₁ is aryl; L₂ is -(CH₂)ₚO(CH₂)_{q}-; R₂ is cycloalkyl; and A₁, A₂, A₃, A₄, A₅, A₆, A₇, A₈, A₉, A₁₀ A₁₁, p and q are as defined in formula (I).

In another embodiment, compounds of the present invention have formula (I) wherein L₁ is alkylene; R₁ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; L₂ is -(CH₂)ₚO(CH₂)_{q}-; R₂ is cycloalkyl; and A₁, A₂, A₃, A₄, A₅, A₆, A₇, A₈, A₉, A₁₀ A₁₁, p and q are as defined in formula (I); and R_{A}, R_{B}, R_{C}, R_{E}, R_{F}, and R_{EE} are as defined in formula (I).

In another embodiment, compounds of the present invention have formula (1) wherein A₁, A₂ and A₃ are each carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0,1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is -(CH₂)ₚO(CH₂)_{q}-; p is 0; q is 0; and R₂ is cycloalkyl; and R_{E}, R_{F}, and R_{EE} are as defined in formula (I).
6 In another embodiment, compounds of the present invention have formula (I) wherein L₁ is alkylene; R₁ is aryl; L₂ is -(CH₂)ₚO(CH₂)_{q}-; R₂ is cycloalkenyl; and A₁, A₂, A₃, A₄, A₅, A₆, A₇, A₈, A₉, A₁₀ A₁₁, p and q are as defined in formula (I).

In another embodiment, compounds of the present invention have formula (I) wherein L₁ is alkylene; R₁ is aryl wherein said aryl is phenyl substituted with 0,1,2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; L₂ is -(CH₂)ₚO(CH₂)_{q}-; R₂ is cycloalkenyl; and A₁, A₂, A₃, A₄, A₅, A₆, A₇, A₈, A₉, A₁₀ A₁₁, p and q are as defined in formula (I); and R_{A}, R_{B}, R_{C}, R_{E}, R_{F}, and R_{EE} are as defined in formula (I).

In another embodiment, compounds of the present invention have formula (I) wherein A₁, A₂ and A₃ are each carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is -(CH₂)ₚO(CH₂)_{q}-; p is 0; q is 0; and R₂ is cycloalkenyl; and R_{E}, R_{F}, and R_{EE} are as defined in formula (I).

In another embodiment, compounds of the present invention have formula (I) wherein L₁ is alkylene; R₁ is aryl; L₂ is -(CH₂)ₚO(CH₂)_{q}-; R₂ is heterocycle; and A₁, A₂, A₃, A₄, A₅, A₆, A₇, A₈, A₉, A₁₀ A₁₁, p and q are as defined in formula (I).

In another embodiment, compounds of the present invention have formula (I) wherein L₁ is alkylene; R₁ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; L₂ is -(CH₂)ₚO(CH₂)_{q}-; R₂ is heterocycle wherein heterocycle is selected from furyl, pyrazinyl, pyridazinyl, pyridinyl, pyrimidinyl, and thienyl wherein said heterocycle is substituted with 0, 1, 2,or 3 substituents independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, arylalkoxycarbonyl, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; and R_{A}, R_{B}, P_{C}, R_{E}, R_{F}, R_{EE}, A₁, A₂, A₃, A₄, A₅, A₆, A₇, A₈, A₉, A₁₀ A₁₁, p, and q are as defined in formula (I)

In another embodiment, compounds of the present invention have formula (I) wherein A₁, A₂ and A₃ are each carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is -(CH₂)ₚO(CH₂)_{q}-; p is 0; q is 0; and R₂ is heterocycle wherein heterocycle is selected from pyridinyl or pyrimidinyl wherein the heterocycle is substituted with 0,1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, nitro, -N(R_{A})C(O)NR_{B}R_{C}, or -NR_{E}R_{F}; and R_{A}, R_{B}, R_{C}, R_{E}, R_{F}, and R_{EE} are as defined in formula (I).

In another embodiment, compounds of the present invention have formula (I) wherein L₁ is alkylene; R₁ is aryl; L₂ is -(CH₂)ₚS(CH₂)_{q}-; R₂ is aryl; and A₁, A₂, A₃, A₄, A₅, A₆, A₇, A₈, A₉, A₁₀ A₁₁, p and q are as defined in formula (I).

In another embodiment, compounds of the present invention have formula (I) wherein L₁ is alkylene; R₁ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; L₂ is -(CH₂)ₚS(CH₂)_{q}-; R₂ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; and R_{A}, R_{B}, R_{C}, R_{E}, R_{F}, R_{EE}, A₁, A₂, A₃, A₄, A₅, A₆, A₇, A₈, A₉, A₁₀ A₁₁, p and q are as defined in formula (I).

In another embodiment, compounds of the present invention have formula (I) wherein A₁, A₂ and A₃ are each carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is -(CH₂)ₚS(CH₂)_{q}-; p is 0; q is 0; and R₂ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; and R_{E}, R_{F}, and R_{EE} are as defined in formula (I).

In another embodiment, compounds of the present invention have formula (I) wherein L₁ is alkylene; R₁ is aryl; L₂ is -(CH₂)ₚC(O)(CH₂)_{q}-; R₂ is aryl; and A₁, A₂, A₃, A₄, A₅, A₆, A₇, A₈, A₉, A₁₀ A₁₁, p and q are as defined in formula (I).

In another embodiment, compounds of the present invention have formula (I) wherein L₁ is alkylene; R₁ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}P_{F})carbonyl, -N(R_{A})C(O)NR_{B}R_{C}, or -CH=NOR_{EE}; L₂ is -(CH₂)ₚC(O)(CH₂)_{q}-; R₂ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; and R_{A}, R_{B}, R_{C}, R_{E}, R_{F}, R_{EE}, A₁, A₂, A₃, A₄, A₅ A₆, A₇, A₈, A₉, A₁₀ A₁₁, p and q are as defined in formula (I).

In another embodiment, compounds of the present invention have formula (I) wherein A₁, A₂ and A₃ are each carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is -(CH₂)ₚC(O)(CH₂)_{q}-; p is 0; q is 0; and R₂ is aryl wherein said aryl is phenyl substituted with 0,1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; and R_{E}, R_{F}, and R_{EE} are as defined in formula (I).

In another embodiment, compounds of the present invention have formula (I) wherein L₁ is alkylene; R₁ is aryl; L₂ is -(CH₂)ₚC(OH)(CH₂)_{q}-; R₂ is aryl; and A₁, A₂, A₃, A₄, A₅, A₆, A₇, A₈, A₉, A₁₀ A₁₁, p and q are as defined in formula (I).

In another embodiment, compounds of the present invention have formula (I) wherein L₁ is alkylene; R₁ is aryl wherein said aryl is phenyl substituted with 0,1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; L₂ is -(CH₂)ₚC(OH)(CH₂)_{q}-; R₂ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; and R_{A}, R_{B}, R_{C}, R_{E}, R_{F}, R_{EE}, A₁, A₂, A₃, A₄, A₅, A₆, A₇, A₈ A₉, A₁₀ A₁₁, p, and q are as defined in formula (I).

In another embodiment, compounds of the present invention have formula (I) wherein A₁, A₂ and A₃ are each carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is -(CH₂)ₚC(OH)(CH₂)_{q}-; p is 0; q is 0; and R₂ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; and R_{E}, R_{F}, and R_{EE} are as defined in formula (I).

In another embodiment, compounds of the present invention have formula (I) wherein L₁ is alkylene; R₁ is aryl; L₂ is -(CH₂)ₚCH=NO(CH₂)_{q}-; R₂ is aryl; and A₁, A₂, A₃, A₄, A₅, A₆, A₇, A₈, A₉, A₁₀ A₁₁, p and q are as defined in formula (I).

In another embodiment, compounds of the present invention have formula (I) wherein L₁ is alkylene; R₁ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; L₂ is -(CH₂)ₚCH=NO(CH₂)_{q}-; R₂ is aryl wherein said aryl is phenyl substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; and R_{A}, R_{B}, R_{C}, R_{E}, R_{F}, R_{EE}, A₁, A₂, A₃, A₄, A₅, A₆, A₇, A₈, A₉, A₁₀ A₁₁, p, and q are as defined in formula (I).

In another embodiment, compounds of the present invention have formula (I) wherein A₁, A₂ and A₃ are each carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is -(CH₂)ₚCH=NO(CH₂)_{q}-; p is 0; q is an integer 0-1; and R₂ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; and R_{E}, R_{F}, and R_{EE} are as defined in formula (I).

In another embodiment, compounds of the present invention have formula (I) wherein L₁ is alkylene; R₁ is aryl; L₂ is -(CH₂)ₚCH=NO(CH₂)_{q}-; R₂ is heterocycle; and A₁, A₂, A₃, A₄, A₅, A₆, A₇, A₈, A₉, A₁₀ A₁₁, p and q are as defined in formula (I).

In another embodiment, compounds of the present invention have formula (I) wherein A₁, A₂ and A₃ are each carboxy; A₄, A₅, A₆, A₇, A₈, A₉, A₁₀ and A₁₁ are each hydrogen; L₁ is alkylene wherein said alkylene is -CH₂-; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is -(CH₂)ₚCH=NO(CH₂)_{q}-; p is 0; q is 0; and R₂ is heterocycle wherein said heterocycle is pyranyl substituted with 0,1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, nitro, -N(R_{A})C(O)NR_{B}R_{C}, or -NR_{E}R_{F}; and R_{A}, R_{B}, R_{C}, R_{E}, R_{F}, and R_{EE} are as defined in formula (I).

In another embodiment, compounds of the present invention have formula (I) wherein L₁ is alkylene; R₁ is cycloalkyl; L₂ is absent; R₂ is absent; and A₁, A₂, A₃, A₄, A₅, A₆, A₇, A₈, A₉, A₁₀ A₁₁, p and q are as defined in formula (I).

In another embodiment, compounds of the present invention have formula (I) wherein A₁, A₂ and A₃ are each carboxy; A₄, A₅, A₆, A₇, A₈, A₉, A₁₀ and A₁₁ are each hydrogen; L₁ is -CH₂-; R₁ is cycloalkyl; L₂ is absent; and R₂ is absent.

In another embodiment, compounds of the present invention have formula (I) wherein L₁ is -(CH₂)ₘO(CH₂)ₙ-; R₁ is aryl; L₂ is absent; R₂ is absent; and A₁, A₂, A₃, A₄, A₅, A₆, A₇, A₈, A₉, A₁₀ A₁₁, m and n are as defined in formula (I).

In another embodiment, compounds of the present invention have formula (I) wherein L₁ is -(CH₂)ₘO(CH₂)ₙ-; R₁ is aryl wherein said aryl is phenyl substituted with 0,1,2,3,4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; L₂ is absent; R₂ is absent; and R_{A}, R_{B}, R_{C}, R_{E}, R_{F}, R_{EE}, A₁, A₂, A₃, A₄, A₅, A₆, A₇, A₈, A₉, A₁₀ A₁₁, m, and n are as defined in formula (I).

In another embodiment, compounds of the present invention have formula (I) wherein A₁, A₂ and A₃ are each carboxy; A₄, A₅, A₆, A₇, A₈, A₉, A₁₀ and A₁₁ are each hydrogen; L₁ is -(CH₂)ₘO(CH₂)ₙ-; m is an integer 2-4; n is 0; R₁ is aryl wherein said aryl is phenyl substituted with 0, 1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, or -NR_{E}R_{F}; L₂ is absent; R₂ is absent; and R_{E}, R_{F}, and R_{EE} are as defined in formula (I).

In another embodiment, compounds of the present invention have formula (I) wherein L₁ is alkylene; R₁ is heterocycle; L₂ is absent; R₂ is absent; and A₁, A₂, A₃, A₄, A₅, A₆, A₇, A₈, A₉, A₁₀ and A₁₁ are as defined in formula (I).

In another embodiment, compounds of the present invention have formula (I) wherein L₁ is alkylene; R₁ is heterocycle wherein said heterocycle is selected from furyl, pyrazinyl, pyridazinyl, pyridinyl, pyrimidinyl and thienyl wherein said heterocycle is substituted with 0, 1, 2,or 3 substituents independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, arylalkoxycarbonyl, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, or -CH=NOR_{EE}; L₂ is absent; R₂ is absent; and A₁, A₂, A₃, A₄, A₅, A₆, A₇, A₈, A₉, A₁₀ and A₁₁ are as defined in formula (I); and R_{A}, R_{B}, R_{C}, R_{E}, R_{F}, and R_{EE} are as defined in formula (I).

In another embodiment, compounds of the present invention have formula (I) wherein A₁, A₂ and A₃ are each carboxy; A₄, A₅, A₆, A₇, A₈, A₉, A₁₀ and A₁₁ are each hydrogen; L₁ is alkylene wherein alkylene is -CH₂-; R₁ is heterocycle wherein said heterocycle is thienyl substituted with 0,1, or 2 substituents selected from alkoxy, alkoxycarbonyl, alkenyl, alkenyloxy, alkyl, alkylthio, cyano, formyl, haloalkoxy, haloalkyl, haloalkylthio, halogen, hydroxyalkyl, nitro, -N(R_{A})C(O)NR_{B}R_{C}, or -NR_{E}R_{F}; L₂ is absent; R₂ is absent; and R_{E}, R_{F}, and R_{EE} are as defined in formula (I).

Another embodiment of the present invention relates to pharmaceutical compositions comprising a therapeutically effective amount of a compound of formula (I) or a pharmaceutically acceptable salt thereof in combination with a pharmaceutically acceptable carrier.

Another embodiment of the present invention relates to a method for controlling pain in a mammal in need of such treatment comprising administering a therapeutically effective amount of a compound of formula (I) or a pharmaceutically acceptable salt thereof in combination with a pharmaceutically acceptable carrier.

Another embodiment of the present invention relates to a method of treating urinary incontinence in a host mammal in need of such treatment comprising administering a therapeutically effective amount of a compound of formula (I) or a pharmaceutically acceptable salt thereof in combination with a pharmaceutically acceptable carrier.

Another embodiment of the present invention relates to a method of treating bladder overactivity in a host mammal in need of such treatment comprising administering a therapeutically effective amount of a compound of formula (I) or a pharmaceutically acceptable salt thereof in combination with a pharmaceutically acceptable carrier.

### Definition of Terms

As used throughout this specification and the appended claims, the following terms have the following meanings:

The term "alkenyl" as used herein, means a straight or branched chain hydrocarbon containing from 2 to 10 carbons and containing at least one carbon-carbon double bond formed by the removal of two hydrogens. Representative examples of alkenyl include, but are not limited to, ethenyl, 2-propenyl, 2-methyl-2-propenyl, 3-butenyl, 4-pentenyl, 5-hexenyl, 2-heptenyl, 2-methyl-1-heptenyl, and 3-decenyl.

The term "alkenylene" denotes a divalent group derived from a straight or branched chain hydrocarbon of from 2 to 10 carbon atoms containing at least one double bond. Representative examples of alkenylene include, but are not limited to, -CH=CH-, -CH=CH₂CH₂-, and -CH=C(CH₃)CH₂-.

The term "alkenyloxy" as used herein, means an alkenyl group, as defined herein, is appended to the parent molecular moiety through an oxygen atom. Representative examples of alkenyloxy include, but are not limited to, allyloxy, 2-butenyloxy and 3-butenyloxy.

The term "alkoxy" as used herein, means an alkyl group, as defined herein, is appended to the parent molecular moiety through an oxygen atom. Representative examples of alkoxy include, but are not limited to, methoxy, ethoxy, propoxy, 2-propoxy, butoxy, tert-butoxy, pentyloxy, and hexyloxy.

The term "alkoxyalkoxy" as used herein, means an alkoxy group, as defined herein, is appended to the parent molecular moiety through another alkoxy group, as defined herein. Representative examples of alkoxyalkoxy include, but are not limited to, tert-butoxymethoxy, 2-ethoxyethoxy, 2-methoxyethoxy, and methoxymethoxy.

The term "alkoxyalkyl" as used herein, means an alkoxy group, as defined herein, is appended to the parent molecular moiety through an alkyl group, as defined herein. Representative examples of alkoxyalkyl include, but are not limited to, tert-butoxymethyl, 2-ethoxyethyl, 2-methoxyethyl, and methoxymethyl.

The term "alkoxycarbonyl" as used herein, means an alkoxy group, as defined herein, is appended to the parent molecular moiety through a carbonyl group, as defined herein. Representative examples of alkoxycarbonyl include, but are not limited to, methoxycarbonyl, ethoxycarbonyl, and tert-butoxycarbonyl.

The term "alkoxycarbonylalkyl" as used herein, means an alkoxycarbonyl group, as defined herein, is appended to the parent molecular moiety through an alkyl group, as defined herein. Representative examples of alkoxycarbonylalkyl include, but are not limited to, 3-methoxycarbonylpropyl, 4-ethoxycarbonylbutyl, and 2-tert-butoxycarbonylethyl.

The term "alkoxysulfonyl" as used herein, means an alkoxy group, as defined herein, is appended appended to the parent molecular moiety through a sulfonyl group, as defined herein. Representative examples of alkoxysulfonyl include, but are not limited to, methoxysulfonyl, ethoxysulfonyl and propoxysulfonyl.

The term "alkyl" as used herein, means a straight or branched chain hydrocarbon containing from 1 to 10 carbon atoms. Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, and n-decyl.

The term "alkylcarbonyl" as used herein, means an alkyl group, as defined herein, is appended to the parent molecular moiety through a carbonyl group, as defined herein. Representative examples of alkylcarbonyl include, but are not limited to, acetyl, 1-oxopropyl, 2,2-dimethyl-1-oxopropyl, 1-oxobutyl, and 1-oxopentyl.

The term "alkylcarbonylalkyl" as used herein, means an alkylcarbonyl group, as defined herein, is appended to the parent molecular moiety through an alkyl group, as defined herein. Representative examples of alkylcarbonylalkyl include, but are not limited to, 2-oxopropyl, 3,3-dimethyl-2-oxopropyl, 3-oxobutyl, and 3-oxopentyl.

The term "alkylcarbonyloxy" as used herein, means an alkylcarbonyl group, as defined herein, is appended to the parent molecular moiety through an oxygen atom. Representative examples of alkylcarbonyloxy include, but are not limited to, acetyloxy, ethylcarbonyloxy, and tert-butylcarbonyloxy.

The term "alkylene" denotes a divalent group derived from a straight or branched chain hydrocarbon of from 1 to 10 carbon atoms. Representative examples of alkylene include, but are not limited to, -CH₂-, -CH₂CH₂-, -CH₂CH(CH₃)- -CH₂CH₂CH₂-, -CH₂CH₂CH₂CH₂-, and -CH₂CH(CH₃)CH₂-.

The term "alkylsulfonyl" as used herein, means an alkyl group, as defined herein, is appended to the parent molecular moiety through a sulfonyl group, as defined herein. Representative examples of alkylsulfonyl include, but are not limited to, methylsulfonyl and ethylsulfonyl.

The term "alkylthio" as used herein, means an alkyl group, as defined herein, is appended to the parent molecular moiety through a sulfur atom. Representative examples of alkylthio include, but are not limited, methylsulfanyl, ethylsulfanyl, tert-butylsulfanyl, and hexylsulfanyl.

The term "alkylthioalkyl" as used herein, means an alkylthio group, as defined herein, is appended to the parent molecular moiety through an alkyl group, as defined herein. Representative examples of alkylthioalkyl include, but are not limited, methylsulfanylmethyl and 2-(ethylsulfanyl)ethyl.

The term "alkynyl" as used herein, means a straight or branched chain hydrocarbon group containing from 2 to 10 carbon atoms and containing at least one carbon-carbon triple bond. Representative examples of alkynyl include, but are not limited, to acetylenyl, 1-propynyl, 2-propynyl, 3-butynyl, 2-pentynyl, and 1-butynyl.

The term "alkynylene" denotes a divalent group derived from a straight or branched chain hydrocarbon of from 2 to 10 carbon atoms containing at least one triple bond. Representative examples of alkynylene include, but are not limited to, -C≡C-, -CH₂C≡C-, -CH(CH₃)CH₂C≡C-, -C≡CCH₂-, and -C≡CCH(CH₃)CH₂-.

The term "alkynyloxy," as used herein, means an alkynyl group, as defined herein, is appended to the parent molecular moiety through an oxygen atom. Representative examples of alkynyloxy include, but are not limited to, 2-propynyloxy and 2-butynyloxy.

The term "aryl" as used herein, means a phenyl group, or a bicyclic or a tricyclic fused ring system wherein one or more of the fused rings is a phenyl group. Bicyclic fused ring systems are exemplified by a phenyl group fused to a cycloalkyl group, as defined herein, or another phenyl group. Tricyclic fused ring systems are exemplified by a bicyclic fused ring system fused to a cycloalkyl group, as defined herein, or another phenyl group. Representative examples of aryl include, but are not limited to, anthracenyl, azulenyl, fluorenyl, indanyl, indenyl, naphthyl, phenyl, and tetrahydronaphthyl.

The aryl groups of this invention are substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE} wherein R_{EE} is selected from hydrogen and alkyl, as defined herein.

The term "arylalkoxy" as used herein, means an aryl group, as defined herein, is appended to the parent molecular moiety through an alkoxy group, as defined herein. Representative examples of arylalkoxy include, but are not limited to, 2-phenylethoxy, 3-naphth-2-ylpropoxy, and 5-phenylpentyloxy.

The term "arylalkoxycarbonyl" as used herein, means an arylalkoxy group, as defined herein, is appended to the parent molecular moiety through a carbonyl group, as defined herein. Representative examples of arylalkoxycarbonyl include, but are not limited to, benzyloxycarbonyl and naphth-2-ylmethoxycarbonyl.

The term "carbonyl" as used herein, means a -C(O)- group.

The term "carboxy" as used herein, means a -CO₂H group.

The term "carboxyalkyl" as used herein, means a carboxy group, as defined herein, is appended to the parent molecular moiety through an alkyl group, as defined herein. Representative examples of carboxyalkyl include, but are not limited to, carboxymethyl, 2-carboxyethyl, and 3-carboxypropyl.

The term "cyano" as used herein, means a -CN group.

The term "cyanoalkyl" as used herein, means a cyano group, as defined herein, is appended to the parent molecular moiety through an alkyl group, as defined herein. Representative examples of cyanoalkyl include, but are not limited to, cyanomethyl, 2-cyanoethyl, and 3-cyanopropyl.

The term "cycloalkyl" as used herein, means a monocyclic, bicyclic, or tricyclic ring system. Monocyclic ring systems are exemplified by a saturated cyclic hydrocarbon group containing from 3 to 8 carbon atoms. Examples of monocyclic ring systems include cyclopropyl, cyclobutyl, cyclopentyl, cyclohexyl, cycloheptyl and cyclooctyl. Bicyclic ring systems are exemplified by a bridged monocyclic ring system in which two non-adjacent carbon atoms of the monocyclic ring are linked by an alkylene bridge of between one and three additional carbon atoms. Representative examples of bicyclic ring systems include, but are not limited to, bicyclo[3.1.1]heptane, bicyclo[2.2.1]heptane, bicyclo[2.2.2]octane, bicyclo[3.2.2]nonane, bicyclo[3.3.1]nonane, and bicyclo[4.2.1]nonane. Tricyclic ring systems are exemplified by a bicyclic ring system in which two non-adjacent carbon atoms of the bicyclic ring are linked by a bond or an alkylene bridge of between one and three carbon atoms. Representative examples of tricyclic-ring systems include, but are not limited to, tricyclo[3.3.1.0^{3,7}]nonane and tricyclo[3.3.1.1^{3,7}]decane (adamantane).

The cycloalkyl groups of this invention are substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl and -CH=NOR_{EE} wherein R_{EE} is selected from hydrogen and alkyl, as defined herein.

The term "cycloalkenyl" as used herein, means a cyclic hydrocarbon containing from 3 to 8 carbons and containing at least one carbon-carbon double bond formed by the removal of two hydrogens. Representative examples of cycloalkenyl include, but are not limited to, 2-cyclohexen-1-yl, 3-cyclohexen-1-yl, 2,4-cyclohexadien-1-yl and 3-cyclopenten-1-yl.

The cycloalkenyl groups of this invention are substituted with 0,1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl and -CH=NOR_{EE} wherein R_{EE} is selected from hydrogen and alkyl, as defined herein.

The term "ethylenedioxy" as used herein, refers to a -O(CH₂)₂O- group wherein the oxygen atoms of the ethylenedioxy group are attached to the parent molecular moiety through one carbon atom forming a 5 membered ring or the oxygen atoms of the ethylenedioxy group are attached to the parent molecular moiety through two adjacent carbon atoms forming a six membered ring.

The term "formyl" as used herein, means a -C(O)H group.

The term "halo" or "halogen" as used herein, means -Cl, -Br, -I or -F.

The term "haloalkoxy" as used herein, means at least one halogen, as defined herein, is appended to the parent molecular moiety through an alkoxy group, as defined herein. Representative examples of haloalkoxy include, but are not limited to, chloromethoxy, 2-fluoroethoxy, trifluoromethoxy, and pentafluoroethoxy.

The term "haloalkyl" as used herein, means at least one halogen, as defined herein, is appended to the parent molecular moiety through an alkyl group, as defined herein. Representative examples of haloalkyl include, but are not limited to, chloromethyl, 2-fluoroethyl, trifluoromethyl, pentafluoroethyl, and 2-chloro-3-fluoropentyl.

The term "haloalkylthio" as used herein, means a haloalkyl group, as defined herein, is appended to the parent molecular moiety through a sulfur atom. Representative examples of haloalkylthio include, but are not limited to, (trifluoromethyl)sulfanyl and (pentafluoroethyl)sulfanyl.

The term "heterocycle" or "heterocyclic" as used herein, means a monocyclic, bicyclic, or tricyclic ring system. Monocyclic ring systems are exemplified by any 3- or 4-membered ring containing a heteroatom independently selected from oxygen, nitrogen and sulfur; or a 5-, 6- or 7-membered ring containing one, two or three heteroatoms wherein the heteroatoms are independently selected from nitrogen, oxygen and sulfur. The 5-membered ring has from 0-2 double bonds and the 6- and 7-membered ring have from 0-3 double bonds. Representative examples of monocyclic ring systems include, but are not limited to, azetidinyl, azepanyl, aziridinyl, diazepinyl, 1,3-dioxolanyl, dioxanyl, dithianyl, furyl, imidazolyl, imidazolinyl, imidazolidinyl, isothiazolyl, isothiazolinyl, isothiazolidinyl, isoxazolyl, isoxazolinyl, isoxazolidinyl, morpholinyl, oxadiazolyl, oxadiazolinyl, oxadiazolidinyl, oxazolyl, oxazolinyl, oxazolidinyl, piperazinyl, piperidinyl, pyranyl, pyrazinyl, pyrazolyl, pyrazolinyl, pyrazolidinyl, pyridinyl, pyrimidinyl, pyridazinyl, pyrrolyl, pyrrolinyl, pyrrolidinyl, tetrahydrofuranyl, tetrahydrothienyl, tetrazinyl, tetrazolyl, thiadiazolyl, thiadiazolinyl, thiadiazolidinyl, thiazolyl, thiazolinyl, thiazolidinyl, thienyl, thiomorpholinyl, 1,1-dioxidothiomorpholinyl (thiomorpholine sulfone), thiopyranyl, triazinyl, triazolyl, and trithianyl. Bicyclic ring systems are exemplified by any of the above monocyclic ring systems fused to an aryl group as defined herein, a cycloalkyl group as defined herein, or another monocyclic ring system. Representative examples of bicyclic ring systems include but are not limited to, for example, benzimidazolyl, benzodioxinyl, benzothiazolyl, benzothienyl, benzotriazolyl, benzoxazolyl, benzofuranyl, benzopyranyl, benzothiopyranyl, cinnolinyl, indazolyl, indolyl, 2,3-dihydroindolyl, indolizinyl, naphthyridinyl, isobenzofuranyl, isobenzothienyl, isoindolyl, isoquinolinyl, phthalazinyl, pyranopyridinyl, quinolinyl, quinolizinyl, quinoxalinyl, quinazolinyl, tetrahydroisoquinolinyl, tetrahydroquinolinyl, and thiopyranopyridinyl. Tricyclic rings systems are exemplified by any of the above bicyclic ring systems fused to an aryl group as defined herein, a cycloalkyl group as defined herein, or a monocyclic ring system. Representative examples of tricyclic ring systems include, but are not limited to, acridinyl, carbazolyl, carbolinyl, dibenzo[b,d]furanyl, dibenzo[b,d]thienyl, naphtho[2,3-b]furan, naphtho[2,3-b]thienyl, phenazinyl, phenothiazinyl, phenoxazinyl, thianthrenyl, thioxanthenyl and xanthenyl.

The heterocycles of this invention are substituted with 0, 1, 2,or 3 substituents independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, arylalkoxycarbonyl, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE} wherein R_{EE} is selected from hydrogen and alkyl, as defined herein.

The term "hydroxy," as used herein, means an -OH group.

The term "hydroxyalkyl," as used herein, means at least one hydroxy group, as defined herein, is appended to the parent molecular moiety through an alkyl group, as defined herein. Representative examples of hydroxyalkyl include, but are not limited to, 1,3-dihydroxypropyl, 1,2-dihydroxypropyl, hydroxymethyl, 2-hydroxyethyl, 3-hydroxypropyl, and 2-ethyl-4-hydroxyheptyl.

The term "mercapto" as used herein, means a -SH group.

The term "methylenedioxy" as used herein, means a -OC(Z₃)(Z₄)O- group wherein the oxygen atoms of the methylenedioxy are attached to the parent molecular moiety through two adjacent carbon atoms. Z₃ and Z₄ are each independently selected from hydrogen or alkyl or Z₃ and Z₄ together with the carbon atom to which they are attached form a 5 or 6 membered cycloalkyl group, as defined herein.

The term "nitro" as used herein, refers to a -NO₂ group.

The term "-NR_{A}R_{B}" as used herein, means two groups, R_{A} and R_{B}, are appended to the parent molecular moiety through a nitrogen atom. R_{A} and R_{B} are each independently selected from hydrogen, alkyl and cyano. Representative examples of -NR_{A}R_{B} include, but are not limited to, amino, methylamino, dimethylamino and cyanoamino.

The term "(NR_{A}R_{B})carbonyl" as used herein, means a -NR_{A}R_{B} group, as defined herein, is appended to the parent molecular moiety through a carbonyl group, as defined herein. Representative examples of (NR_{A}R_{B})carbonyl include, but are not limited to, aminocarbonyl, (methylamino)carbonyl, (dimethylamino)carbonyl, (cyanoamino)carbonyl and (ethylmethylamino)carbonyl.

The term "-NR_{E}R_{F}" as used herein, means two groups, R_{E} and R_{F}, are appended to the parent molecular moiety through a nitrogen atom. R_{E} and R_{F} are each independently selected from hydrogen, alkyl, alkylcarbonyl, formyl, and hydroxyalkyl. Representative examples of -NR_{E}R_{F} include, but are not limited to, amino, acetylamino, methylamino, dimethylamino, ethylmethylamino, (2,3-dihydoxypropyl)amino, and formylamino.

The term "(NR_{E}R_{F})carbonyl" as used herein, means a -NR_{E}R_{F} group, as defined herein, is appended to the parent molecular moiety through a carbonyl group, as defined herein. Representative examples of (NR_{E}R_{F})carbonyl include, but are not limited to, aminocarbonyl, (methylamino)carbonyl, (dimethylamino)carbonyl and (ethylmethylamino)carbonyl.

The term "-NR_{C}S(O)₂R_{D}" is used herein means R_{D} appended to the parent molecular moiety through a sulfonyl group, the sulfonyl group with is further appended to the parent molecular moiety through an amino group as defined herein. Examples of -NR_{C}S(O)₂R_{D} include, but not limited to, arylsulfonylamino wherein aryl is substituted or unsubstituted phenyl.

The term "(NR_{C}S(O)₂R_{D})carbonyl " is used herein means a -NR_{C}S(O)₂R_{D} group appended to the parent molecular moiety through a carbonyl group.

The term "oxo" as used herein, means a =O moiety.

The term "sulfonyl" as used herein, means a -SO₂- group.

### In Vitro Data

### Determination of Inhibition Potencies

Compounds of the present invention were determined to be P2X₃ and P2X_{2/3} antagonists based on their ability to inhibit increases in cytosolic Ca²⁺ concentration elicited by the P2X receptor agonist αβ-methyleneATP (αβ-meATP; Sigma, St. Louis, MO) as described in Bianchi et al. (1999). The fluorescent Ca²⁺ chelating dye fluo-4 was used as an indicator of the relative levels of intracellular Ca²⁺ in a 96-well format using a Fluorescence Imaging Plate Reader (FLIPR, Molecular Devices, Sunnyvale, CA). Cells expressing recombinant human P2X₃ or P2X_{2/3} containing receptors were grown to confluence and plated in 96-well black-walled tissue culture plates approximately 18 hours prior to the experiment. One to two hours before the assay, cells were loaded with fluo-4 AM (2.28 µM; Molecular Probes, Eugene, OR) in D-PBS and maintained in a dark environment at room temperature. Immediately before the assay, each plate was washed twice with 250 µl D-PBS per well to remove extracellular fluo-4 AM and then 100 µl D-PBS was added to the wells. Two 50 µl additions of compounds (4X concentration prepared in D-PBS) were made to the cells during each experiment. The first addition consisting of test antagonist was made and incubation continued for 3 minutes before the addition of the agonist αβ-meATP, measurement continued for 3 minutes after this final addition. Fluorescence data was collected at 1 or 5 second intervals throughout the course of each experiment and were analyzed based on the peak increase in relative fluorescence units compared with basal fluorescence. Antagonist concentration-response data, expressed as a percentage of the maximal αβ-meATP response in the absence of test antagonist, were analyzed using GraphPad Prism (San Diego, CA).

The compounds of the present invention were found to be antagonists of the P2X₃ containing receptor with potencies from 5000 nM to 0.5 nM. In a preferred range, the compounds of the present invention antagonized P2X₃ containing receptors with potencies from 500 nM to 0.5 nM. In a more preferred range, the compounds of the present invention antagonized P2X₃ containing receptors with potencies from 50 nM to 0.5 nM.

Additionally, the compounds of the present invention were found to be antagonists of the P2X_{2/3} containing receptors with potencies from 4800 nM to 0.5 nM. In a preferred range, the compounds of the present invention antagonized P2X_{2/3} containing receptors with potencies from 500 nM to 0.5 nM. In a more preferred range, the compounds of the present invention antagonized P2X_{2/3} containing receptors with potencies from 50 nM to 0.5 nM.

### In Vivo Data

### Determination of Antinociceptive Effect

Following a 30-mininute acclimation period to individual clear observation cages, 50 µl of a 5% formalin solution was injected subcutaneously (s.c.) into the dorsal aspect of the right hindpaw of rats (male Sprague-Dawley, 200-300 g) were then returned to the observation cages, which were suspended above mirrors. Rats, six per group, were observed for either a continuous period of 60 minutes or for periods of time corresponding to phase 1 and phase 2 of the formalin test (Abbott et al., Pain, 60 (1995) 91-102). Phase 1 of the formalin test was defined as the period of time immediately following injection of formalin until 10 minutes after the formalin injection. Effects on Phase 2 of the formalin test were determined by monitoring for the 20 minute period of time from 30 to 50 minutes following formalin injection. Nociceptive behaviors were recorded from animals during the session by observing each animal for one 60 second observation period during each 5 minute interval. Nociceptive behaviors recorded included flinching, licking or biting the injected paw.

The compounds of the present invention were found to have antinociceptive effects with potencies from 100 µmol/kg to 15 µmol/kg.

The in vitro and in vivo data demonstrates that compounds of the present invention antagonize the P2X₃ containing receptor, antagonize the P2X_{2/3} containing receptor, and are useful for treating pain. Compounds of the present invention are thus useful for ameliorating or preventing additional disorders that are affected by the P2X₃ and/or the P2X_{2/3} containing receptors such as bladder overactivity and urinary incontinence.

The compounds of the present invention can be used in the form of pharmaceutically acceptable salts derived from inorganic or organic acids. The phrase "pharmaceutically acceptable salt" means those salts which are, within the scope of sound medical judgement, suitable for use in contact with the tissues of humans and lower animals without undue toxicity, irritation, allergic response and the like and are commensurate with a reasonable benefit/risk ratio.

Pharmaceutically acceptable salts are well-known in the art. For example, S. M. Berge et al. describe pharmaceutically acceptable salts in detail in (J. Pharmaceutical Sciences, 1977, 66: 1 et seq). The salts can be prepared in situ during the final isolation and purification of the compounds of the invention or separately by reacting a free base function with a suitable organic acid. Representative acid addition salts include, but are not limited to acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, fumarate, hydrochloride, hydrobromide, hydroiodide, 2-hydroxyethansulfonate (isothionate), lactate, maleate, methanesulfonate, nicotinate, 2-naphthalenesulfonate, oxalate, palmitoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups can be quaternized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which can be employed to form pharmaceutically acceptable acid addition salts include such inorganic acids as hydrochloric acid, hydrobromic acid, sulfuric acid, and phosphoric acid and such organic acids as acetic acid, fumaric acid, maleic acid, 4-methylbenzenesulfonic acid, succinic acid and citric acid.

Basic addition salts can be prepared in situ during the final isolation and purification of compounds of this invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammonium, methylamine, dimethylamine, trimethylamine, triethylamine, diethylamine, ethylamine and the like. Other representative organic amines useful for the formation of base addition salts include ethylenediamine, ethanolamine, diethanolamine, piperidine, piperazine and the like.

Dosage forms for topical administration of a compound of this invention include powders, sprays, ointments and inhalants. The active compound is mixed under sterile conditions with a pharmaceutically acceptable carrier and any needed preservatives, buffers or propellants which can be required. Opthalmic formulations, eye ointments, powders and solutions are also contemplated as being within the scope of this invention.

Actual dosage levels of active ingredients in the pharmaceutical compositions of this invention can be varied so as to obtain an amount of the active compound(s) which is effective to achieve the desired therapeutic response for a particular patient, compositions and mode of administration. The selected dosage level will depend upon the activity of the particular compound, the route of administration, the severity of the condition being treated and the condition and prior medical history of the patient being treated. However, it is within the skill of the art to start doses of the compound at levels lower than required for to achieve the desired therapeutic effect and to gradually increase the dosage until the desired effect is achieved.

When used in the above or other treatments, a therapeutically effective amount of one of the compounds of the present invention can be employed in pure form or, where such forms exist, in pharmaceutically acceptable salt, ester or prodrug form. Alternatively, the compound can be administered as a pharmaceutical composition containing the compound of interest in combination with one or more pharmaceutically acceptable excipients. The phrase "therapeutically effective amount" of the compound of the invention means a sufficient amount of the compound to treat disorders, at a reasonable benefit/risk ratio applicable to any medical treatment. It will be understood, however, that the total daily usage of the compounds and compositions of the present invention will be decided by the attending physician within the scope of sound medical judgement. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. For example, it is well within the skill of the art to start doses of the compound at levels lower than required to achieve the desired therapeutic effect and to gradually increase the dosage until the desired effect is achieved.

The total daily dose of the compounds of this invention administered to a human or lower animal may range from about 0.01 to about 100 mg/kg/day. For purposes of oral administration, more preferable doses can be in the range of from about 0.1 to about 25 mg/kg/day. If desired, the effective daily dose can be divided into multiple doses for purposes of administration; consequently, single dose compositions may contain such amounts or submultiples thereof to make up the daily dose.

The present invention also provides pharmaceutical compositions that comprise compounds of the present invention formulated together with one or more non-toxic pharmaceutically acceptable carriers. The pharmaceutical compositions can be specially formulated for oral administration in solid or liquid form, for parenteral injection or for rectal administration.

The pharmaceutical compositions of this invention can be administered to humans and other mammals orally, rectally, parenterally, intracisternally, intravaginally, intraperitoneally, topically (as by powders, ointments or drops), bucally or as an oral or nasal spray. The term "parenterally," as used herein, refers to modes of administration which include intravenous, intramuscular, intraperitoneal, intrasternal, subcutaneous and intraarticular injection and infusion.

Pharmaceutical compositions of this invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), vegetable oils (such as olive oil), injectable organic esters (such as ethyl oleate) and suitable mixtures thereof. Proper fluidity can be maintained, for example, by the use of coating materials such as lecithin, by the maintenance of the required particle size in the case of dispersions and by the use of surfactants.

These compositions may also contain adjuvants such as preservatives, wetting agents, emulsifying agents and dispersing agents. Prevention of the action of microorganisms can be ensured by the inclusion of various antibacterial and antifungal agents, for example, paraben, chlorobutanol, phenol sorbic acid and the like. It may also be desirable to include isotonic agents such as sugars, sodium chloride and the like. Prolonged absorption of the injectable pharmaceutical form can be brought about by the inclusion of agents which delay absorption such as aluminum monostearate and gelatin.

In some cases, in order to prolong the effect of the drug, it is desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

Solid dosage forms for oral administration include capsules, tablets, pills, powders and granules. In such solid dosage forms, the active compound may be mixed with at least one inert, pharmaceutically acceptable excipient or carrier, such as sodium citrate or dicalcium phosphate and/or a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate, solid polyethylene glycols, sodium lauryl sulfate and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills and granules can be prepared with coatings and shells such as enteric coatings and other coatings well-known in the pharmaceutical formulating art. They may optionally contain opacifying agents and may also be of a composition such that they release the active ingredient(s) only, or preferentially, in a certain part of the intestinal tract, optionally, in a delayed manner. Examples of embedding compositions which can be used include polymeric substances and waxes.

The active compounds can also be in micro-encapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof.

Besides inert diluents, the oral compositions may also include adjuvants such as wetting agents, emulsifying and suspending agents, sweetening, flavoring and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar, tragacanth and mixtures thereof.

Compositions for rectal or vaginal administration are preferably suppositories which can be prepared by mixing the compounds of this invention with suitable non-irritating excipients or carriers such as cocoa butter, polyethylene glycol or a suppository wax which are solid at room temperature but liquid at body temperature and therefore melt in the rectum or vaginal cavity and release the active compound.

Compounds of the present invention can also be administered in the form of liposomes. As is known in the art, liposomes are generally derived from phospholipids or other lipid substances. Liposomes are formed by mono- or multi-lamellar hydrated liquid crystals which are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients and the like. The preferred lipids are natural and synthetic phospholipids and phosphatidyl cholines (lecithins) used separately or together.

Methods to form liposomes are known in the art. See, for example, Prescott, Ed., Methods in Cell Biology, Volume XIV, Academic Press, New York, N.Y. (1976), p. 33 et seq.

Compounds of the present invention that are formed by in vivo conversion of a different compound that was administered to a mammal are intended to be included within the scope of the present invention.

The compounds of the invention can exist in unsolvated as well as solvated forms, including hydrated forms, such as hemi-hydrates. In general, the solvated forms, with pharmaceutically acceptable solvents such as water and ethanol among others are equivalent to the unsolvated forms for the purposes of the invention.

The compounds of the invention, including but not limited to those specified in the examples, are P2X₃ and P2X₂/P2X₃ containing receptor antagonists in mammals. As P2X₃ and P2X₂/P2X₃ containing receptor antagonists, the compounds of the present invention are useful for the treatment and prevention of disorders such as bladder overactivity, urinary incontinence or pain.

The ability of the compounds of the present invention, including but not limited to those specified in the examples, to treat bladder overactivity or urinary incontinence is demonstrated by Namasivayam et al., Brit. J. Urol. Int. 84L 854-860. (1999).

The ability of the compounds of the present invention, including but not limited to those specified in the examples, to treat pain is demonstrated by Cesare et al., Drug Dev. Res. 50: S01-02 (2000); Cockayne et al., Drug Dev. Res. 50: 005 (2000); Bleehen, Br. J. Pharmacol. 62:573-577 (1978); Cook et al., Nature 387:505-508 (1997); and Driessen and Starke, Naunyn Schmiedebergs Arch. Pharmacol. 350:618-625 (1994).

### Abbreviations

Abbreviations which have been used in the descriptions of the Schemes and the Examples that follow are: DCC for 1,3-dicyclohexylcarbodiimide, DME for dimethoxyethane, DMF for N,N-dimethylformamide, DMSO for dimethylsulfoxide, EDCI or EDC for 1-ethyl-3-[3-(dimethylamino)propyl]-carbodiimide hydrochloride, LAH for lithium aluminum hydride, RP-HPLC for reverse phase-high pressure liquid chromatography and THF for tetrahydrofuran.

### Preparation of Compounds of the Present Invention

The compounds and processes of the present invention will be better understood in connection with the following synthetic Schemes and Examples which illustrate a means by which the compounds of the present invention can be prepared.

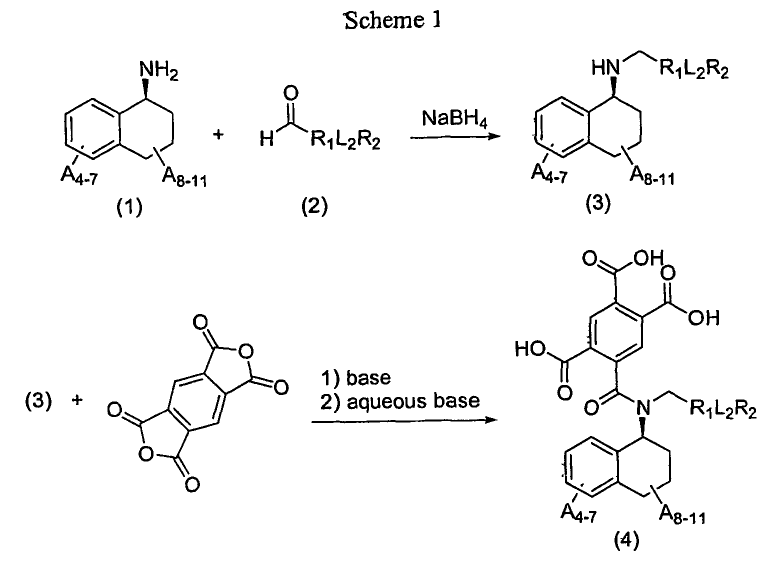

Benzenetricarboxylic acids of general formula (4), wherein A₄, A₅, A₆, A₇, A₈, A₉, A₁₀, A₁₁, R₁, R₂ and L₂ are as defined in formula (I), may be prepared as described in Scheme 1. (1S)-1,2,3,4-Tetrahydro-1-naphthalenylamines of general formula (1), purchased commercially or prepared using standard chemistry known to those in the art, may be treated with aldehydes of general formula (2) and a hydride source such as sodium borohydride in a solvent such as ethanol to provide secondary amines of general formula (3). Secondary amines of general formula (3) may be treated with 1,2,4,5-benzenetetracarboxylic dianhydride and an organic base such as triethylamine and after a period of 4-72 hours a base in water such as sodium carbonate/water may be added to the reaction mixture to provide benzenetricarboxylic acids of general formula (4).

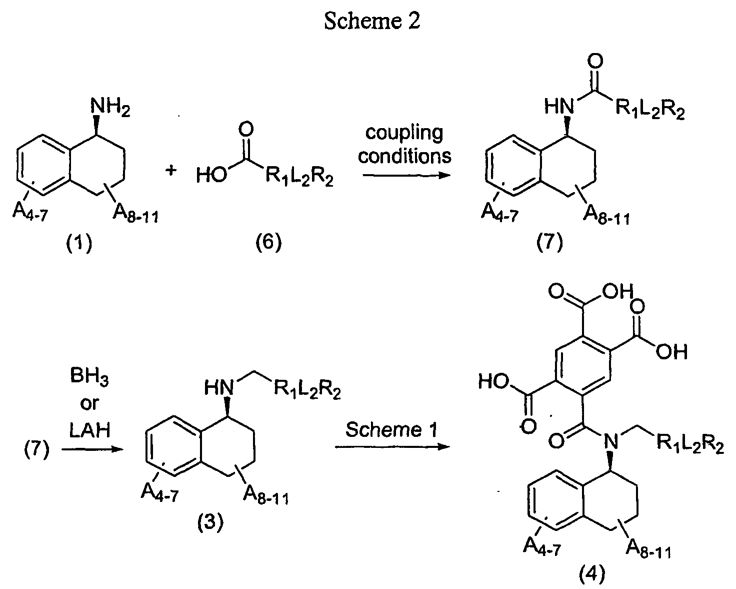

An alternate method of preparing benzenetricarboxylic acids of general formula (4), wherein A₄, A₅, A₆, A₇, A₈, A₉, A₁₀, A₁₁, R₁, R₂ and L₂ are as defined in formula (I), may be used as described in Scheme 2. (1S)-1,2,3,4-Tetrahydro-1-naphthalenylamines of general formula (I) may be coupled with acids of general formula (6) using standard coupling conditions known to those in the art such. Carbodiimides such as DCC or EDCI may be used or other coupling conditions may be used such as thionyl chloride or chloroformates to provide amides of general formula (7). Amides of general formula (7) may be treated with borane-tetrahyrofuran complex or lithium aluminum hydride to provide secondary amines of general formula (3). Secondary amines of general formula (3) may be processed as described in Scheme 1 to provide benzenetricarboxylic acids of general formula (4).

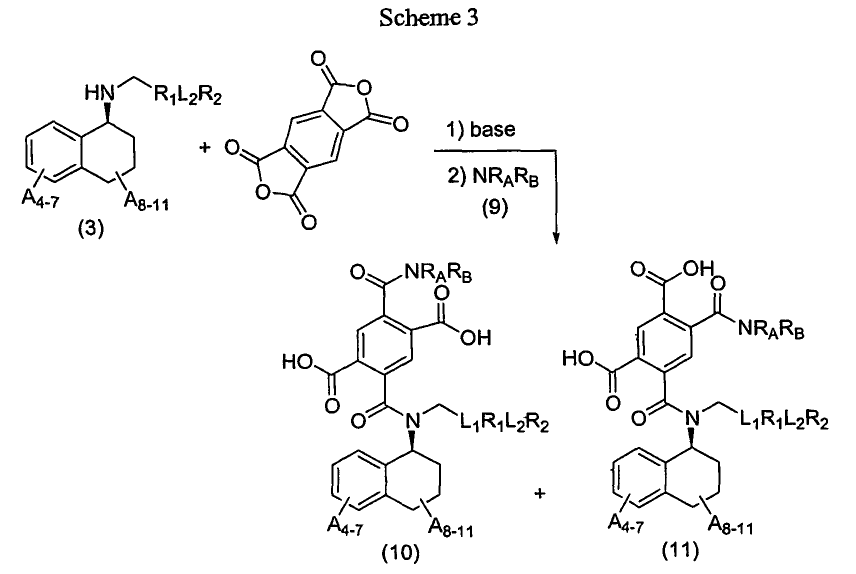

Amides of general formula (10) and (11), wherein A₄, A₅, A₆, A₇, A₈, A₉, A₁₀, A₁₁, R₁, R₂, L₁, L₂, R_{A} and R_{B} are as defined in formula (I), may be prepared as described in Scheme 2. Secondary amines of general formula (3) may be treated with 1,2,4,5-benzenetetracarboxylic dianhydride and an organic base such as triethylamine and after a period of 4-72 hours an amine of general formula (9) may be added to the reaction mixture to provide amides of general formula (10) and (11). Esters may also be prepared in similar fashion except that an alcohol is added after the 4-72 hour period instead of an amine.

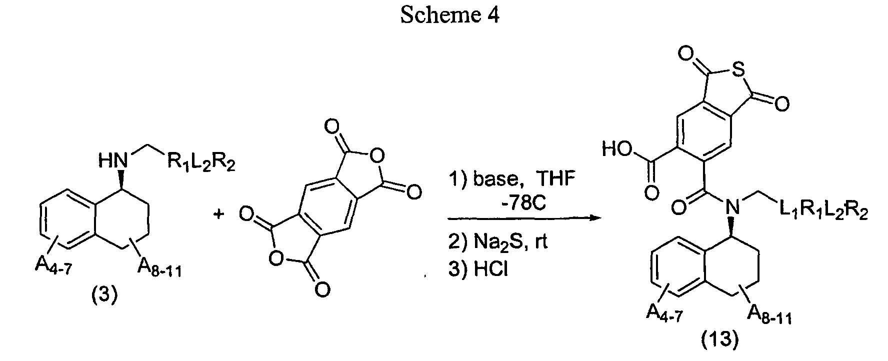

1,3-Dioxo-1,3-dihydro-2-benzothiophene-5-carboxylic acids of general formula (13), wherein A₄, A₅, A₆, A₇, A₈, A₉, A₁₀, A₁₁, R₁, R₂, L₁ and L₂ are as defined in formula (I), may be prepared as described in Scheme 4. Secondary amines of general formula (3) may be treated with 1,2,4,5-benzenetetracarboxylic dianhydride and an organic base such as triethylamine at -78 °C and allowed to warm to ambient temperature and stir for 4-72 hours. The reaction mixture may then be recooled to -78°C and treated with sodium sulfide nonahydrate followed by aqueous acid to provide 1,3-dioxo-1,3-dihydro-2-benzothiophene-5-carboxylic acids of general formula (13).

The following Examples are intended as an illustration of and not a limitation upon the scope of the invention as defined in the appended claims.

### Example 1

### 5-({[3-(4-chlorophenoxy)benzyl][(1S)-1,2,3,4-tetrahydro-1-naphthalenyl]amino}carbonyl)-1,2,4-benzenetricarboxylic acid

### Example 1A

### N-[3-(4-chlorophenoxy)benzyl]-N-[(1S)-1,2,3,4-tetrahydro-1-naphthalenyl]amine

3-(4-Chlorophenoxy)benzaldehyde (1.50 mL, 7.82 mmol, purchased from Acros Organics) in 30 mL absolute ethanol was treated with (1S)-1,2,3,4-tetrahydro-1-naphthalenylamine (1.12 mL, 7.82 mmol, purchased from Lancaster). After stirring at ambient temperature for 4 hours, the mixture was treated with NaBH₄ (0.33 g, 8.60 mmol) in one portion. After stirring an additional 18 hours, the reaction mixture was concentrated under reduced pressure and the residue dissolved in diethyl ether and quenched by addition of a solution of 1N NaOH. The phases were allowed to separate and the aqueous phase was extracted with diethyl ether. The organic phases were combined, washed with water, brine, dried (Na₂SO₄), filtered and the filtrate concentrated under reduced pressure. The residue was purified by flash chromatography (silica gel, 15% ethyl acetate/hexanes) to provide the title compound as a colorless oil (2.26 g, 79%). ¹H NMR (300 MHz, CDCl₃) δ 6.85-7.37 (m, 12H), 3.75-3.95 (m, 3H), 2.65-2.9 (m, 2H), 1.4-2.1 (m, 5H); MS (ESI+) 364 (M+H)⁺.

### Example 1B

### 5-({[3-(4-chlorophenoxy)benzyl][(1S)-1,2,3,4-tetrahydro-1-naphthalenyl]amino}carbonyl)-1,2,4-benzenetricarboxylic acid

1,2,4,5-Benzenetetracarboxylic dianhydride (2.70 g, 12.4 mmol) and triethylamine (2.15 mL, 15.5 mmol) in THF (60 mL)were treated dropwise with the product from Example 1A (2.25 g, 6.18 mmol) in THF (10 mL) at -78 °C. The reaction mixture was allowed to stir for 16 hours gradually warming to ambient temperature. The mixture was treated with saturated aqueous Na₂CO₃ solution, stirred vigorously for 30 minutes and then carefully acidified using 12M HCl. The acidified solution was extracted with ethyl acetate. The organic extracts were combined, washed with 1N HCl, brine, dried (Na₂SO₄), filtered and concentrated under reduced pressure. The residue was purified by flash chromatography (silica gel, 100:1:1 ethyl acetate:HCO₂H:H₂O) to provide the title compound as a white solid (1.92 g, 52% yield).
¹H NMR (300 MHz, DMSO-d₆) δ 13.9 (bs, 1H), 6.6-8.3 (m, 14H), 3.9-5.65 (m, 3H), 2.5-2.7 (m, 2H), 1.3-2.2 (m, 4H); MS (ESI+) 600 (M+H)⁺.

### Example 2

### 5-({([1,1'-biphenyl]-4-ylmethyl)[(1S)-1,2,3,4-tetrahydro-1-naphthalenyl]amino}carbonyl)-1,2,4-benzenetricarboxylic acid

### Example 2A

### N-([1,1'-biphenyl]-4-ylmethyl)-N-[(1S)-1,2,3,4-tetrahydro-1-naphthalenyl]amine

[1,1'-Biphenyl]-4-carbaldehyde and (1S)-1,2,3,4-tetrahydro-1-naphthalenylamine were processed as described in Example 1A to provide the title compound.
¹H NMR (CDCl₃) δ 7.61-7.05 (m, 13H), 4.02-3.81 (m, 3H), 2.78 (m, 2H), 2.10-1.90 (m, 3H), 1.76 (m, 1H), 1.50 (broad s, 1H); MS (ESI+) 314 (M+H)⁺.

### Example 2B

### 5-({([1,1'-biphenyl]-4-ylmethyl)[(1S)-1,2,3,4-tetrahydro-1-naphthalenyl]amino}carbonyl)-1,2,4-benzenetricarboxylic acid

The product from Example 2A and 1,2,4,5-benzenetetracarboxylic dianhydride were processed as described in Example 1B to provide the title compound.
¹H NMR (DMSO-d₆) δ 8.40-7.05 (m, 15H), 5.04-4.61 (m, 1H), 4.37-3.89 (m, 2H), 3.37 (broad s, 3H), 2.71-2.55 (m, 2H), 2.10-1.18 (m, 4H); MS (ESI+) 550 (M+H)⁺.

### Example 3

### 5-({[(2'-chloro[1,1'-biphenyl]-4-yl)methyl][(1S)-1,2,3,4-tetrahydro-1-naphthalenyl]amino}carbonyl)-1,2,4-benzenetricarboxylic acid

### Example 3A

### N-[(2'-chloro[1,1'-biphenyl]-4-yl)methyl]-N-[(1S)-1,2,3,4-tetrahydro-1-naphthalenyl]amine

2'-Chloro[1,1'-biphenyl]-4-carbaldehyde and (1S)-1,2,3,4-tetrahydro-1-naphthalenylamine were processed as described in Example 1A to provide the title compound.
¹H NMR (CDCl₃) δ 7.50-7.05 (m, 12H), 4.02-3.80 (m, 3H), 2.78 (m, 2H), 2.10-1.90 (m, 3H), 1.76 (m, 1H), 1.53 (broad s, 1H); MS (ESI+) 348 (M+H)⁺.

### Example 3B

### 5-({[(2'-chloro[1,1'-biphenyl]-4-yl)methyl][(1S)-1,2,3,4-tetrahydro-1-naphthalenyl]amino}carbonyl)-1,2,4-benzenetricarboxylic acid

The product from Example 3A and 1,2,4,5-benzenetetracarboxylic dianhydride were processed as described in Example 1B to provide the title compound.
¹H NMR (DMSO-d₆) δ 8.30-7.05 (m, 14H), 5.11-4.61 (m, 1H), 4.37-3.90 (m, 2H), 2.71-2.55 (m, 2H), 2.13-1.40 (m, 4H); MS (ESI-) 583 (M-H)⁻.

### Example 4

### 5-({[(3',5'-dichloro[1,1'-biphenyl]-4-yl)methyl][(1S)-1,2,3,4-tetrahydro-1-naphthalenyl]amino}carbonyl)-1,2,4-benzenetricarboxylic acid

### Example 4A

### N-[(3',5'-dichloro[1,1'-biphenyl]-4-yl)methyl]-N-[(1S)-1,2,3,4-tetrahydro-1-naphthalenyl]amine

3',5'-Dichloro[1,1'-biphenyl]-4-carbaldehyde and (1S)-1,2,3,4-tetrahydro-1-naphthalenylamine were processed as described in Example 1A to provide the title compound.
¹H NMR (CDCl₃) δ 7.50-7.05 (m, 11H), 4.02-3.80 (m, 3H), 2.80 (m, 2H), 2.10-1.90 (m, 3H), 1.76 (m, 1H), 1.56 (broad s, 1H); MS (ESI+) 382 (M+H)⁺.

### Example 4B

### 5-({[(3',5'-dichloro[1,1'-biphenyl]-4-yl)methyl][(1S)-1,2,3,4-tetrahydro-1-naphthalenyl]amino}carbonyl)-1,2,4-benzenetricarboxylic acid

The product from Example 4A and 1,2,4,5-benzenetetracarboxylic dianhydride were processed as described in Example 1B to provide the title compound.
¹H NMR (DMSO-d₆) δ 8.29-7.05 (m, 13H), 5.07-4.61 (m, 1H), 4.37-3.90 (m, 2H), 2.71-2.55 (m, 2H), 2.20-1.38 (m, 4H); MS (ESI+) 618 (M+H)⁺.

### Example 5

### 5-({[(2'-methoxy[1,1'-biphenyl]-4-yl)methyl][(1S)-1,2,3,4-tetrahydro-1-naphthalenyl]amino}carbonyl)-1,2,4-benzenetricarboxylic acid

### Example 5A

### N-[(2'-methoxy[1,1'-biphenyl]-4-yl)methyl]-N-[(1S)-1,2,3,4-tetrahydro-1-naphthalenyl]amine

2'-Methoxy[1,1'-biphenyl]-4-carbaldehyde and (1S)-1,2,3,4-tetrahydro-1-naphthalenylamine were processed as described in Example 1A to provide the title compound.
¹H NMR (CDCl₃) δ 7.51-6.95 (m, 12H), 4.00-3.82 (m, 3H), 3.80 (s, 3H), 2.80 (m, 2H), 2.10-1.90 (m, 3H), 1.76 (m, 1H), 1.53 (broad s, 1H); MS (ESI+) 344 (M+H)⁺.

### Example 5B

### 5-({[(2'-methoxy[1,1'-biphenyl]-4-yl)methyl][(1S)-1,2,3,4-tetrahydro-1-naphthalenyl]amino}carbonyl)-1,2,4-benzenetricarboxylic acid

The product from Example 5A and 1,2,4,5-benzenetetracarboxylic dianhydride were processed as described in Example 1B to provide the title compound.
¹H NMR (DMSO-d₆) δ 8.29-6.95 (m, 14H), 5.05-4.61 (m, 1H), 4.38-3.87 (m, 2H), 3.78 and 3.70 (2s, 3H), 2.73-2.55 (m, 2H), 2.20-1.36 (m, 4H); MS (ESI+) 580 (M+H)⁺.

### Example 6

### 5-({[(4'-chloro[1,1'-biphenyl]-4-yl)methyl][(1S)-1,2,3,4-tetrahydro-1-nanhthalenyl]amino}carbonyl)-1,2,4-benzenetricarboxylic acid

### Example 6A

### N-[(4'-chloro[1,1'-biphenyl]-4-yl)methyl]-N-[(1S)-1,2,3,4-tetrahydro-1-naphthalenyl]amine

4'-Chloro[1,1'-biphenyl]-4-carbaldehyde and (1S)-1,2,3,4-tetrahydro-1-naphthalenylamine were processed as described in Example 1A to provide the title compound.
¹H NMR (CDCl₃) δ 7.52-7.05 (m, 12H), 4.02-3.80 (m, 3H), 2.80 (m, 2H), 2.10-1.90 (m, 3H), 1.76 (m, 1H), 1.55 (broad s, 1H); MS (ESI+) 348 (M+H)⁺.

### Example 6B

### 5-({[(4'-chloro[1,1'-biphenyl]-4-yl)methyl][(1S)-1,2,3,4-tetrahydro-1-naphthalenyl]amino}carbonyl)-1,2,4-benzenetricarboxylic acid

The product from Example 6A and 1,2,4,5-benzenetetracarboxylic dianhydride were processed as described in Example 1B to provide the title compound.
¹H NMR (DMSO-d₆) δ 8.29-7.02 (m, 14H), 5.07-4.60 (m, 1H), 4.34-3.87 (m, 2H), 2.73-2.55 (m, 2H), 2.22-1.36 (m, 4H); MS (ESI+) 583 (M+H)⁺.

### Example 7

### 5-({[(4'-fluoro[1,1'-biphenyl]-4-yl)methyl][(1S)-1,2,3,4-tetrahydro-1-naphthalenyl]amino}carbonyl)-1,2,4-benzenetricarboxylic acid

### Example 7A

### N-[(4'-fluoro[1,1'-biphenyl]-4-yl)methyl]-N-[(1S)-1,2,3,4-tetrahydro-1-naphthalenyl]amine

4'-Fluoro[1,1'-biphenyl]-4-carbaldehyde and (1S)-1,2,3,4-tetrahydro-1-naphthalenylamine were processed as described in Example 1A to provide the title compound.
¹H NMR (CDCl₃) δ 7.60-7.00 (m, 12H), 4.02-3.80 (m, 3H), 2.80 (m, 2H), 2.12-1.90 (m, 3H), 1.76 (m, 1H), 1.52 (broad s, 1H); MS (ESI+) 332 (M+H)⁺.

### Example 7B

### 5-({[(4'-fluoro[1,1'-biphenyl]-4-yl)methyl][(1S)-1,2,3,4-tetrahydro-1-naphthalenyl]amino}carbonyl)-1,2,4-benzenetricarboxylic acid

The product from Example 7A and 1,2,4,5-benzenetetracarboxylic dianhydride were processed as described in Example 1B to provide the title compound.
¹H NMR (DMSO-d₆) δ 8.30-7.05 (m, 14H), 5.07-4.60 (m, 1H), 4.34-3.87 (m, 2H), 2.73-2.55 (m, 2H), 2.20-1.36 (m, 4H); MS (ESI+) 568 (M+H)⁺.

### Example 8

### 5-({[(4'-methoxy[1,1'-biphenyl]-4-yl)methyl][(1S)-1,2,3,4-tetrahydro-1-naphthalenyl]amino}carbonyl)-1,2,4-benzenetricarboxylic acid

### Example 8A

### N-[(4'-methoxy[1,1'-biphenyl]-4-yl)methyl]-N-[(1S)-1,2,3,4-tetrahydro-1-naphthalenyl]amine

4'-Methoxy[1,1'-biphenyl]-4-carbaldehyde and (1S)-1,2,3,4-tetrahydro-1-naphthalenylamine were processed as described in Example 1A to provide the title compound.

### Example 8B

### 5-({[(4'-methoxy[1,1'-biphenyl]-4-yl)methyl][(1S)-1,2,3,4-tetrahydro-1-naphthalenyl]amino}carbonyl)-1,2,4-benzenetricarboxylic acid

The product from Example 8A and 1,2,4,5-benzenetetracarboxylic dianhydride were processed as described in Example 1B to provide the title compound.
¹H NMR (DMSO-d₆) δ 8.30-6.93 (m, 14H), 5.04-4.61 (m, 1H), 4.33-3.87 (m, 2H), 3.79 and 3.78 (2s, 3H), 2.73-2.55 (m, 2H), 2.20-1.36 (m, 4H); MS (ESI+) 580 (M+H)⁺.

### Example 9

### 5-[((1S)-1,2,3,4-tetrahydro-1-naphthalenyl{[2'-(trifluoromethyl)[1,1'-biphenyl]-4-yl]methyl}amino)carbonyl]-1,2,4-benzenetricarboxylic acid

### Example 9A

### N-[(1S)-1,2,3,4-tetrahydro-1-naphthalenyl]-N-{[2'-(trifluoromethyl)[1,1'-biphenyl]-4-yl]methyl}amine

2'-(Trifluoromethyl)[1,1'-biphenyl]-4-carbaldehyde and (1S)-1,2,3,4-tetrahydro-1-naphthalenylamine were processed as described in Example 1A to provide the title compound.
¹H NMR (CDCl₃) δ 7.75-7.05 (m, 12H), 4.28-3.87 (m, 3H), 2.90 (m, 1H), 2.71 (m, 1H), 2.28-2.10 (m, 3H), 1.72 (m, 1H); MS (ESI+) 382 (M+H)⁺.

### Example 9B

### 5-[((1S)-1,2,3,4-tetrahydro-1-naphthalenyl{[2'-(trifluoromethyl)[1,1'-biphenyl]-4-yl]methyl}amino)carbonyl]-1,2,4-benzenetricarboxylic acid

The product from Example 9A and 1,2,4,5-benzenetetracarboxylic dianhydride were processed as described in Example 1B to provide the title compound.
¹H NMR (DMSO-d₆) δ 8.30-7.02 (m, 14H), 5.06-4.61 (m, 1H), 4.36-3.90 (m, 2H), 2.73-2.55 (m, 2H), 2.20-1.36 (m, 4H); MS (ESI+) 618 (M+H)⁺.

### Example 10

### 5-({[(2'-methyl[1,1'-biphenyl]-4-yl)methyl][(1S)-1,2,3,4-tetrahydro-1-naphthalenyl]amino}carbonyl)-1,2,4-benzenetricarboxylic acid

### Example 10A

### N-[(2'-methyl[1,1'-biphenyl]-4-yl)methyl]-N-[(1S)-1,2,3,4-tetrahydro-1-naphthalenyl]amine

2'-Methyl[1,1'-biphenyl]-4-carbaldehyde and (1S)-1,2,3,4-tetrahydro-1-naphthalenylamine were processed as described in Example 1A to provide the title compound.
¹H NMR (CDCl₃) δ 7.70-7.05 (m, 12H), 4.28-3.87 (m, 3H), 2.90 (m, 1H), 2.70 (m, 1H), 2.28-2.10 (m, 3H), 2.20 (s, 3H), 1.72 (m, 1H); MS (ESI+) 328 (M+H)⁺.

### Example 10B

### 5-({[(2'-methyl[1,1'-biphenyl]-4-yl)methyl][(1S)-1,2,3,4-tetrahydro-1-naphthalenyl]amino}carbonyl)-1,2,4-benzenetricarboxylic acid

The product from Example 10A and 1,2,4,5-benzenetetracarboxylic dianhydride were processed as described in Example 1B to provide the title compound.
¹H NMR (DMSO-d₆) δ 8.29-7.00 (m, 14H), 5.06-4.61 (m, 1H), 4.36-3.92 (m, 2H), 2.78-2.55 (m, 2H), 2.23 and 2.13 (2s, 3H), 2.18-1.36 (m, 4H); MS (ESI+) 564 (M+H)⁺.

### Example 11

### 5-[((1S)-1,2,3,4-tetrahydro-1-naphthalenyl{[4'-(trifluoromethyl)[1,1'-biphenyl]-2-yl]methyl}amino)carbonyl]-1,2,4-benzenetricarboxylic acid

### Example 11A

### N-[(1S)-1,2,3,4-tetrahydro-1-naphthalenyl]-N-{[4'-(trifluoromethyl)[1,1'-biphenyl]-2-yl]methyl}amine

4'-(Trifluoromethyl)[1,1'-biphenyl]-2-carbaldehyde and (1S)-1,2,3,4-tetrahydro-1-naphthalenylamine were processed as described in Example 1A to provide the title compound.
¹H NMR (CDCl₃) δ 8.20 (m, 1H), 7.65-6.95 (m, 11H), 4.10 (t, J=6Hz, 1H), 3.82 (s, 2H), 2.61 (m, 2H), 2.00-1.43 (m, 4H); MS (ESI+) 382 (M+H)⁺.

### Example 11B

### 5-[((1S)-1,2,3,4-tetrahydro-1-naphthalenyl{[4'-(trifluoromethyl)[1,1'-biphenyl]-2-yl]methyl}amino)carbonyl]-1,2,4-benzenetricarboxylic acid

The product from Example 11A and 1,2,4,5-benzenetetracarboxylic dianhydride were processed as described in Example 1B to provide the title compound.
¹H NMR (DMSO-d₆) δ 8.31-6.80 (m, 14H), 4.91-4.52 (m, 1H), 4.22-3.80 (m, 2H), 2.70-2.39 (m, 2H), 1.93-1.20 (m, 4H); MS (ESI+) 618 (M+H)⁺.

### Example 12

### 5-({[(4'-fluoro[1,1'-biphenyl]-2-yl)methyl][(1S)-1,2,3,4-tetrahydro-1-naphthalenyl]amino}carbonyl)-1,2,4-benzenetricarboxylic acid

### Example 12A

### N-[(4'-fluoro[1,1'-biphenyl]-2-yl)methyl]-N-[(1S)-1,2,3,4-tetrahydro-1-naphthalenyl]amine

4'-Fluoro[1,1'-biphenyl]-2-carbaldehyde and (1S)-1,2,3,4-tetrahydro-1-naphthalenylamine were processed as described in Example 1A to provide the title compound.
¹H NMR (CDCl₃) δ 8.20 (m, 1H), 7.49-7.00 (m, 11H), 4.10 (t, J=6Hz, 1H), 3.90 (s, 2H), 2.63 (m, 2H), 1.98-1.48 (m, 4H); MS (ESI+) 332 (M+H)⁺.

### Example 12B

### 5-({[(4'-fluoro[1,1'-biphenyl]-2-yl)methyl][(1S)-1,2,3,4-tetrahydro-1-naphthalenyl]amino}carbonyl)-1,2,4-benzenetricarboxylic acid

The product from Example 12A and 1,2,4,5-benzenetetracarboxylic dianhydride were processed as described in Example 1B to provide the title compound.
¹H NMR (DMSO-d₆) δ 8.28-6.80 (m, 14H), 4.86-3.87 (m, 3H), 2.70-2.39 (m, 2H), 2.02-1.20 (m, 4H); MS (ESI+) 568 (M+H)⁺.

### Example 13

### 5-({(4-chlorobenzyl)[(1S)-1,2,3,4-tetrahydro-1-naphthalenyl]amino}carbonyl)-1,2,4-benzenetricarboxylic acid

### Example 13A

### N-(4-chlorobenzyl)-N-[(1S)-1,2,3,4-tetrahydro-1-naphthalenyl]amine

4-Chlorobenzaldehyde and (1S)-1,2,3,4-tetrahydro-1-naphthalenylamine were processed as described in Example 1A to provide the title compound.
¹H NMR (CDCl₃) δ 7.68-6.95 (m, 8H), 4.18 (m, 1H), 3.80 (m, 2H), 2.95-2.61 (m, 2H), 2.20-2.05 (m, 3H), 1.70 (m, 1H); MS (ESI+) 272 (M+H)⁺.

### Example 13B

### 5-({(4-chlorobenzyl)[(1S)-1,2,3,4-tetrahydro-1-naphthalenyl]amino}carbonyl)-1,2,4-benzenetricarboxylic acid

The product from Example 13A and 1,2,4,5-benzenetetracarboxylic dianhydride were processed as described in Example 1B to provide the title compound.
¹H NMR (DMSO-d₆) δ 8.30-7.00 (m, 10H), 5.00-3.70 (m, 3H), 2.70-2.55 (m, 2H), 2.02-1.40 (m, 4H); MS (ESI+) 508 (M+H)⁺.

### Example 14

### 5-({(4-bromobenzyl)[(1S)-1,2,3,4-tetrahydro-1-naphthalenyl]amino}carbonyl)-1,2,4-benzenetricarboxylic acid

### Example 14A

### N-(4-bromobenzyl)-N-[(1S)-1,2,3,4-tetrahydro-1-naphthalenyl]amine

4-Bromobenzaldehyde and (1S)-1,2,3,4-tetrahydro-1-naphthalenylamine were processed as described in Example 1A to provide the title compound.
¹H NMR (CDCl₃) δ 7.60-7.0 (m, 8H), 4.10 (m, 1H), 3.78 (m, 2H), 2.95-2.61 (m, 2H), 2.20-2.05 (m, 3H), 1.70 (m, 1H); MS (ESI+) 316 (M+H)⁺.

### Example 14B

### 5-({(4-bromobenzyl)[(1S)-1,2,3,4-tetrahydro-1-naphthalenyl]amino}carbonyl)-1,2,4-benzenetricarboxylic acid

The product from Example 14A and 1,2,4,5-benzenetetracarboxylic dianhydride were processed as described in Example 1B to provide the title compound.
¹H NMR (DMSO-d₆) δ 8.29-7.00 (m, 10H), 5.00-3.70 (m, 3H), 2.70-2.55 (m, 2H), 2.02-1.35 (m, 4H); MS (ESI+) 552 (M+H)⁺.

### Example 15

### 5-({(3-bromobenzyl)[(1S)-1,2,3,4-tetrahydro-1-naphthalenyl]amino}carbonyl)-1,2,4-benzenetricarboxylic acid

### Example 15A

### N-(3-bromobenzyl)-N-[(1S)-1,2,3,4-tetrahydro-1-naphthalenyl]amine

3-Bromobenzaldehyde and (1S)-1,2,3,4-tetrahydro-1-naphthalenylamine were processed as described in Example 1A to provide the title compound.
¹H NMR (CDCl₃) δ 7.70-7.05 (m, 8H), 4.10 (m, 1H), 3.80 (m, 2H), 2.95-2.61 (m, 2H), 2.20-2.05 (m, 3H), 1.70 (m, 1H); MS (ESI+) 316 (M+H)⁺,

### Example 15B

### 5-({(3-bromobenzyl)[(1S)-1,2,3,4-tetrahydro-1-naphthalenyl]amino}carbonyl)-1,2,4-benzenetricarboxylic acid

The product from Example 15A and 1,2,4,5-benzenetetracarboxylic dianhydride were processed as described in Example 1B to provide the title compound.
¹H NMR (DMSO-d₆) δ 8.29-7.00 (m, 10H), 5.00-3.70 (m, 3H), 2.78-2.55 (m, 2H), 2.05-1.37 (m, 4H); MS (ESI+) 552 (M+H)⁺.

### Example 16

### 5-({(3,4-dichlorobenzyl)[(1S)-1,2,3,4-tetrahydro-1-naphthalenyl]amino}carbonyl)-1,2,4-benzenetricarboxylic acid

### Example 16A

### N-(3,4-dichlorobenzyl)-N-[(1S)-1,2,3,4-tetrahydro-1-naphthalenyl]amine

3,4-Dichlorobenzaldehyde and (1S)-1,2,3,4-tetrahydro-1-naphthalenylamine were processed as described in Example 1A to provide the title compound.
¹H NMR (CDCl₃) δ 7.68-7.10 (m, 7H), 4.20 (t, J=6Hz, 1H), 3.80 (m, 2H), 2.95-2.63 (m, 2H), 2.20-2.05 (m, 3H), 1.73 (m, 1H); MS (ESI+) 306 (M+H)⁺.

### Example 16B

### 5-({(3,4-dichlorobenzyl)[(1S)-1,2,3,4-tetrahydro-1-naphthalenyl]amino}carbonyl)-1,2,4-benzenetricarboxylic acid

The product from Example 16A and 1,2,4,5-benzenetetracarboxylic dianhydride were processed as described in Example 1B to provide the title compound.
¹H NMR (DMSO-d₆) δ 8.29-7.00 (m, 9H), 5.00-3.80 (m, 3H), 2.72-2.55 (m, 2H), 2.10-1.37 (m, 4H); MS (ESI+) 542 (M+H)⁺.

### Example 17

### 5-({(4-cyanobenzyl)[(1S)-1,2,3,4-tetrahydro-1-naphthalenyl]amino}carbonyl)-1,2,4-benzenetricarboxylic acid

### Example 17A

### 4-{[(1S)-1,2,3,4-tetrahydro-1-naphthalenylamino]methyl}benzonitrile

4-Formylbenzonitrile and (1S)-1,2,3,4-tetrahydro-1-naphthalenylamine were processed as described in Example 1A to provide the title compound.
¹H NMR (CDCl₃) δ 7.78-7.10 (m, 8H), 4.12 (t, J=6Hz, 1H), 3.90 (m, 2H), 2.95-2.67 (m, 2H), 2.20-2.05 (m, 3H), 1.70 (m, 1H); MS (ESI+) 262 (M+H)⁺.

### Example 17B

### 5-({(4-cyanobenzyl)[(1S)-1,2,3,4-tetrahydro-1-naphthalenyl]amino}carbonyl)-1,2,4-benzenetricarboxylic acid

The product from Example 17A and 1,2,4,5-benzenetetracarboxylic dianhydride were processed as described in Example 1B to provide the title compound.
¹H NMR (DMSO-d₆) δ 8.29-7.00 (m, 10H), 5.05-3.94 (m, 3H), 2.72-2.55 (m, 2H), 2.10-1.40 (m, 4H); MS (ESI+) 499 (M+H)⁺.

### Example 18

### 5-({(4-chloro-3-nitrobenzyl)[(1S)-1,2,3,4-tetrahydro-1-naphthalenyl]amino}carbonyl)-1,2,4-benzenetricarboxylic acid

### Example 18A

### N-(4-chloro-3-nitrobenzyl)-N-[(1S)-1,2,3,4-tetrahydro-1-naphthalenyl]amine

4-Chloro-3-nitrobenzaldehyde and (1S)-1,2,3,4-tetrahydro-1-naphthalenylamine were processed as described in Example 1A to provide the title compound.
¹H NMR (CDCl₃) δ 8.06 (s, 1H), 7.90 (m, 1H), 7.61-7.50 (m, 2H), 7.28-7.10 (m, 3H), 4.21 (broad s, 1H), 3.90 (m, 2H), 2.95-2.67 (m, 2H), 2.20-2.05 (m, 3H), 1.72 (m, 1H); MS (ESI+) 317 (M+H)⁺.

### Example 18B

### 5-({(4-chloro-3-nitrobenzyl)[(1S)-1,2,3,4-tetrahydro-1-naphthalenyl]amino}carbonyl)-1,2,4-benzenetricarboxylic acid

The product from Example 18A and 1,2,4,5-benzenetetracarboxylic dianhydride were processed as described in Example 1B to provide the title compound.
¹H NMR (DMSO-d₆) δ 8.29-6.87 (m, 9H), 5.00-4.00 (m, 3H), 2.80-2.55 (m, 2H), 2.10-1.40 (m, 4H); MS (ESI+) 553 (M+H)⁺.

### Example 19

### 5-({[(4'-fluoro[1,1'-biphenyl]-3-yl)methyl][(1S)-1,2,3,4-tetrahydro-1-naphthalenyl]amino}carbonyl)-1,2,4-benzenetricarboxylic acid

### Example 19A

### N-[(4'-fluoro[1,1'-biphenyl]-3-yl)methyl]-N-[(1S)-1,2,3,4-tetrahydro-1-naphthalenyl]amine

4'-Fluoro[1,1'-biphenyl]-3-carbaldehyde and (1S)-1,2,3,4-tetrahydro-1-naphthalenylamine were processed as described in Example 1A to provide the title compound.

### Example 19B

### 5-({[(4'-fluoro[1,1'-biphenyl]-3-yl)methyl][(1S)-1,2,3,4-tetrahydro-1-naphthalenyl]amino}carbonyl)-1,2,4-benzenetricarboxylic acid

The product from Example 19A and 1,2,4,5-benzenetetracarboxylic dianhydride were processed as described in Example 1B to provide the title compound.
¹H NMR (DMSO-d₆) δ 8.50-7.00 (m, 14H), 5.05-4.63 (m, 1H), 4.40-3.96 (m, 2H), 2.70-2.55 (m, 2H), 2.17-1.37 (m, 4H) MS (ESI+) 568 (M+H)⁺.

### Example 20

### 5-({[4-(dimethylamino)benzyl][(1S)-1,2,3,4-tetrahydro-1-naphthalenyl]amino}carbonyl)-1,2,4-benzenetricarboxylic acid

### Example 20A

### N-[4-(dimethylamino)benzyl]-N-[(1S)-1,2,3,4-tetrahydro-1-naphthalenyl]amine

4-(Dimethylamino)benzaldehyde and (1S)-1,2,3,4-tetrahydro-1-naphthalenylamine were processed as described in Example 1A to provide the title compound.
¹H NMR (CDCl₃) δ 7.35-7.03 (m, 6H), 6.72 (m, 2H), 3.88-3.70 (m, 3H), 2.91 (s, 6H), 2.78 (m, 2H), 2.08-1.69 (m, 4H), 1.50 (broad s, 1H); MS (ESI+) 281 (M+H)⁺.

### Example 20B

### 5-({[4-(dimethylamino)benzyl][(1S)-1,2,3,4-tetrahydro-1-naphthalenyl]amino}carbonyl)-1,2,4-benzenetricarboxylic acid

The product from Example 20A and 1,2,4,5-benzenetetracarboxylic dianhydride were processed as described in Example 1B to provide the title compound.
¹H NMR (DMSO-d₆) δ 8.25-6.60 (m, 10H), 5.00-3.70 (m, 3H), 3.00 and 2.87 (2s, 6H), 2.70-2.55 (m, 2H), 2.20-1.38 (m, 4H); MS (ESI+) 517 (M+H)⁺.

### Example 21

### 5-({(3-chlorobenzyl)[(1S)-1,2,3,4-tetrahydro-1-naphthalenyl]amino}carbonyl)-1,2,4-benzenetricarboxylic acid

### Example 21A

### N-(3-chlorobenzyl)-N-[(1S)-1,2,3,4-tetrahydro-1-naphthalenyl]amine

3-Chlorobenzaldehyde and (1S)-1,2,3,4-tetrahydro-1-naphthalenylamine were processed as described in Example 1A to provide the title compound.
¹H NMR (CDCl₃) δ 7.60-7.03 (m, 8H), 4.18 (t, J=6Hz, 1H), 3.75 (m, 2H), 2.95-2.62 (m, 2H), 2.20-2.05 (m, 3H), 1.70 (m, 1H); MS (ESI+) 272 (M+H)⁺.

### Example 21B

### 5-({(3-chlorobenzyl)[(1S)-1,2,3,4-tetrahydro-1-naphthalenyl]amino}carbonyl)-1,2,4-benzenetricarboxylic acid

The product from Example 21A and 1,2,4,5-benzenetetracarboxylic dianhydride were processed as described in Example 1B to provide the title compound.
¹H NMR (DMSO-d₆) δ 8.28-7.00 (m, 10H), 5.02-3.82 (m, 3H), 2.70-2.55 (m, 2H), 2.20-1.40 (m, 4H); MS (ESI+) 508 (M+H)⁺.

### Example 22

### 5-({(3-cyanobenzyl)[(1S)-1,2,3,4-tetrahydro-1-naphthalenyl]amino}carbonyl)-1,2,4-benzenetricarboxylic acid

### Example 22A

### 3-{[(1S)-1,2,3,4-tetrahydro-1-naphthalenylamino]methyl}benzonitrile

3-Formylbenzonitrile and (1S)-1,2,3,4-tetrahydro-1-naphthalenylamine were processed as described in Example 1 A to provide the title compound.
¹H NMR (CDCl₃) δ 8.02 (d, J=7.5Hz,1H), 7.80 (s, 1H), 7.65-7.10 (m, 6H), 4.18 (broad s, 1H), 3.90 (m, 2H), 2.95-2.62 (m, 2H), 2.20-2.05 (m, 3H), 1.80-1.52 (m, 2H); MS (ESI+) 263 (M+H)⁺.

### Example 22B

### 5-({(3-cyanobenzyl)[(1S)-1,2,3,4-tetrahydro-1-naphthalenyl]amino}carbonyl)-1,2,4-benzenetricarboxylic acid

The product from Example 22A and 1,2,4,5-benzenetetracarboxylic dianhydride were processed as described in Example 1B to provide the title compound.
¹H NMR (DMSO-d₆) δ 8.30-7.00 (m, 10H), 5.02-3.91 (m, 3H), 2.70-2.55 (m, 2H), 2.20-1.40 (m, 4H); MS (ESI+) 499 (M+H)⁺.

### Example 23

### 5-({[4-(1-pyrrolidinyl)benzyl][(1S)-1,2,3,4-tetrahydro-1-naphthalenyl]amino}carbonyl)-1,2,4-benzenetricarboxylic acid

### Example 23A

### N-[4-(1-pyrrolidinyl)benzyl]-N-[(1S)-1,2,3,4-tetrahydro-1-naphthalenyl]amine

4-(1-Pyrrolidinyl)benzaldehyde and (1S)-1,2,3,4-tetrahydro-1-naphthalenylamine were processed as described in Example 1A to provide the title compound.
¹H NMR (CDCl₃) δ 7.58-7.05 (m, 6H), 6.49 (m, 2H), 4.12 (t, J=6Hz, 1H), 3.75 (m, 2H), 3.30-3.10 (m, 5H), 2.95-2.61 (m, 2H), 2.18-1.90 (m, 6H), 1.72 (m, 1H); MS (ESI+) 307 (M+H)⁺.

### Example 23B

### 5-({[4-(1-pyrrolidinyl)benzyl][(1S)-1,2,3,4-tetrahydro-1-naphthalenyl]amino}carbonyl)-1,2,4-benzenetricarboxylic acid

The product from Example 23A and 1,2,4,5-benzenetetracarboxylic dianhydride were processed as described in Example 1B to provide the title compound.
¹H NMR (DMSO-d₆) δ 8.26-6.30 (m, 10H), 5.00-3.91 (m, 3H), 3.27-3.10 (m, 4H), 2.70-2.55 (m, 2H), 2.20-1.32 (m, 8H); MS (ESI+) 543 (M+H)⁺.

### Example 24

### 5-[((1S)-1,2,3,4-tetrahydro-1-naphthalenyl{[4'-(trifluoromethyl)[1,1'-biphenyl]-4-yl]methyl}amino)carbonyl]-1,2,4-benzenetricarboxylic acid

### Example 24A

### N-[(1S)-1,2,3,4-tetrahydro-1-naphthalenyl]-N-{[4'-(trifluoromethyl)[1,1'-biphenyl]-4-yl]methyl}amine

4'-(Trifluoromethyl)[1,1'-biphenyl]-4-carbaldehyde and (1S)-1,2,3,4-tetrahydro-1-naphthalenylamine were processed as described in Example 1A to provide the title compound.
¹H NMR (CDCl₃) δ 7.72-7.08 (m, 12H), 4.22 (broad s, 1H), 3.85 (broad s, 2H), 2.95-2.60 (m, 2H), 2.15 (m, 3H), 1.70 (m, 1H); MS (ESI+) 382 (M+H)⁺.

### Example 24B

### 5-[((1S)-1,2,3,4-tetrahydro-1-naphthalenyl{[4'-(trifluoromethyl)[1,1'-biphenyl]-4-yl]methyl}amino)carbonyl]-1,2,4-benzenetricarboxylic acid

The product from Example 24A and 1,2,4,5-benzenetetracarboxylic dianhydride were processed as described in Example 1B to provide the title compound.
¹H NMR (DMSO-d₆) δ 8.30-7.00 (m, 14H), 5.10-3.91 (m, 3H), 2.78-2.55 (m, 2H), 2.23-1.40 (m, 4H); MS (ESI+) 618 (M+H)⁺.

### Example 25

### 5-({[4-(3-pyridinyl)benzyl][(1S)-1,2,3,4-tetrahydro-1-naphthalenyl]amino}carbonyl)-1,2,4-benzenetricarboxylic acid

### Example 25A

### N-[4-(3-pyridinyl)benzyl]-N-[(1S)-1,2,3,4-tetrahydro-1-naphthalenyl]amine

4-(3-Pyridinyl)benzaldehyde and (1S)-1,2,3,4-tetrahydro-1-naphthalenylamine were processed as described in Example 1A to provide the title compound.
¹H NMR (CDCl₃) δ 8.35 (d, J=1.5Hz, 1H), 8.09 (m, 1H), 7.86 (m, 1H), 7.60-7.08 (m, 9H), 4.05-3.70 (m, 3H), 2.80 (m, 2H), 2.10-1.70 (m, 4H); MS (ESI+) 315 (M+H)⁺.

### Example 25B

### 5-({[4-(3-pyridinyl)benzyl][(1S)-1,2,3,4-tetrahydro-1-naphthalenyl]amino}carbonyl)-1,2,4-benzenetricarboxylic acid

The product from Example 25A and 1,2,4,5-benzenetetracarboxylic dianhydride were processed as described in Example 1B to provide the title compound.
¹H NMR (DMSO-d₆) δ 9.01-7.03 (m, 14H), 5.10-3.70 (m, 3H), 2.75-2.55 (m, 2H), 2.30-1.40 (m, 4H); MS (ESI+) 551 (M+H)⁺.

### Example 26

### 5-({([1,1'-biphenyl]-2-ylmethyl)[(1S)-1,2,3,4-tetrahydro-1-naphthalenyl]amino}carbonyl)-1,2,4-benzenetricarboxylic acid

### Example 26A

### N-([1,1'-biphenyl]-2-ylmethyl)-N-[(1S)-1,2,3,4-tetrahydro-1-naphthalenyl]amine

[1,1'-Biphenyl]-2-carbaldehyde and (1S)-1,2,3,4-tetrahydro-1-naphthalenylamine were processed as described in Example 1A to provide the title compound.

### Example 26B

### 5-({([1,1'-biphenyl]-2-ylmethyl)[(1S)-1,2,3,4-tetrahydro-1-naphthalenyl]amino}carbonyl)-1,2,4-benzenetricarboxylic acid

The product from Example 26A and 1,2,4,5-benzenetetracarboxylic dianhydride were processed as described in Example 1B to provide the title compound.
¹H NMR (DMSO-d₆) δ 8.30-6.80 (m, 15H), 4.82-3.80 (m, 3H), 2.75-2.45 (m, 2H), 2.05-1.33 (m, 4H); MS (ESI+) 550 (M+H)⁺.

### Example 27

### 5-({([1,1'-biphenyl]-3-ylmethyl)[(1S)-1,2,3,4-tetrahydro-1-naphthalenyl]amino}carbonyl)-1,2,4-benzenetricarboxylic acid

### Example 27A

### N-([1,1'-biphenyl]-3-ylmethyl)-N-[(1S)-1,2,3,4-tetrahydro-1-naphthalenyl]amine

[1,1'-Biphenyl]-3-carbaldehyde and (1S)-1,2,3,4-tetrahydro-1-naphthalenylamine were processed as described in Example 1A to provide the title compound.

### Example 27B

### 5-({([1,1'-biphenyl]-3-ylmethyl)[(1S)-1,2,3,4-tetrahydro-1-naphthalenyl]amino}carbonyl)-1,2,4-benzenetricarboxylic acid

The product from Example 27A and 1,2,4,5-benzenetetracarboxylic dianhydride were processed as described in Example 1B to provide the title compound.
¹H NMR (DMSO-d₆) δ 8.38-6.90 (m, 15H), 4.90-4.64 (m, 4H), 4.27-4.18 (m, 1H), 4.00-3.93 (m, 1H), 2.90-2.55 (m, 2H), 2.20-1.4, (m, 4H); MS (ESI+) 550 (M+H)⁺.

### Example 28

### 5-({[(2-methyl[1,1'-biphenyl]-3-yl)methyl][(1S)-1,2,3,4-tetrahydro-1-naphthalenyl]amino}carbonyl)-1,2,4-benzenetricarboxylic acid

### Example 28A

### N-[(2-methyl[1,1'-biphenyl]-3-yl)methyl]-N-[(1S)-1,2,3,4-tetrahydro-1-naphthalenyl]amine

2-Methyl[1,1'-biphenyl]-3-carbaldehyde and (1S)-1,2,3,4-tetrahydro-1-naphthalenylamine were processed as described in Example 1A to provide the title compound.
¹H NMR (CDCl₃) δ 7.44-7.06 (m, 12H), 4.02-3.85 (m, 3H), 2.90-2.69 (m, 2H), 2.28 (s, 3H), 2.10-1.70 (m, 4H); MS (DCI+) 238 (M+H)⁺.

### Example 28B

### 5-({[(2-methyl[1,1'-binhenyl]-3-yl)methyl][(1S)-1,2,3,4-tetrahydro-1-naphthalenyl]amino}carbonyl)-1,2,4-benzenetricarboxylic acid

The product from Example 28A and 1,2,4,5-benzenetetracarboxylic dianhydride were processed as described in Example 1B to provide the title compound.
¹H NMR (DMSO-d₆) δ 8.70-6.95 (m, 14H), 4.96 (d, 1H), 4.72 (m, 1H), 4.00-3.73 (m, 1H), 3.19 (s, 3H), 2.63-2.38 (m, 2H), 2.00-1.20 (m, 4H); MS (ESI+) 564 (M+H)⁺.

### Example 29

### 5-[((1S)-1,2,3,4-tetrahydro-1-naphthalenyl{[4'-(trifluoromethyl)[1,1'-biphenyl]-3-yl]methyl}amino)carbonyl]-1,2,4-benzenetricarboxylic acid

### Example 29A

### N-[(1S)-1,2,3,4-tetrahydro-1-naphthalenyl]-N-{[4'-(trifluoromethyl)[1,1'-biphenyl]-3-yl]methyl}amine

4'-(Trifluoromethyl)[1,1'-biphenyl]-3-carbaldehyde and (1S)-1,2,3,4-tetrahydro-1-naphthalenylamine were processed as described in Example 1A to provide the title compound.
¹H NMR (CDC1₃) δ 7.60-7.51 (m, 3H), 7.44-7.35 (m, 4H), 7.16-7.07 (m, 5H), 4.01 (d, 1H, J=13.5 Hz) 3.91 (d, 1H, J=13.5 Hz), 3.86 (t, 1H, J=5 Hz), 2.90-2.67 (m, 2H), 2.10-1.90 (m, 3H), 1.82-1.70 (m, 1H); MS (DCI+) 332 (M+H)⁺.

### Example 29B

### 5-[((1S)-1,2,3,4-tetrahydro-1-naphthalenyl{[4'-(trifluoromethyl)[1,1'-biphenyl]-3-yl]methyl}amino)carbonyl]-1,2,4-benzenetricarboxylic acid

The product from Example 29A and 1,2,4,5-benzenetetracarboxylic dianhydride were processed as described in Example 1B to provide the title compound.
¹H NMR (DMSO-d₆) δ 8.74-7.00 (m, 14H), 5.04-4.86 (m, 1H), 4.80-4.65 (m, 1H), 3.96-3.83 (m, 1H), 2.70-2.37 (m, 2H), 2.08-1.13 (m, 4H); MS (ESI+) 618 (M+H)⁺.

### Example 30

### 5-({(cyclohexylmethyl)[(1S)-1,2,3,4-tetrahydro-1-naphthalenyl]amino}carbonyl)-1,2,4-benzenetricarboxylic acid

### Example 30A

### N-(cyclohexylmethyl)-N-[(1S)-1,2,3,4-tetrahydro-1-naphthalenyl]amine

Cyclohexanecarbaldehyde and (1S)-1,2,3,4-tetrahydro-1-naphthalenylamine were processed as described in Example 1A to provide the title compound.
¹H NMR (300 MHz, DMSO-d₆) δ 8.8-9.1 (bs, 2H), 7.60-7.67 (m, 1H), 7.15-7.35 (m, 3H), 4.45-4.55 (m, 1H), 2.55-2.9 (m ,4H), 1.55-2.15 (m, 10H), 0.8-1.3 (m, 5H); MS (ESI+) 244 (M+H)⁺.

### Example 30B

### 5-({(cyclohexylmethyl)[(1S)-1,2,3,4-tetrahydro-1-naphthalenyl]amino}carbonyl)-1,2,4-benzenetricarboxylic acid

The product from Example 30A and 1,2,4,5-benzenetetracarboxylic dianhydride were processed as described in Example 1B to provide the title compound.
¹H NMR (300 MHz, DMSO-d₆) δ 7.0-8.3 (m, 6H), 4.4-4.65 (m, 1H), 2.4-2.85 (m, 3H), 0.5-2.3 (m, 16H); MS (ESI+) 480 (M+H)⁺.

### Example 31

### 5-({(2-phenylpropyl)[(1S)-1,2,3,4-tetrahydro-1-naphthalenyl]amino}carbonyl)-1,2,4-benzenetricarboxylic acid

### Example 31A

### N-(2-phenylpropyl)-N-[(1S)-1,2,3,4-tetrahydro-1-naphthalenyl]amine

Hydratropaldehyde and (1S)-1,2,3,4-tetrahydro-1-naphthalenylamine were processed as described in Example 1A to provide the title compound.
¹H NMR (300 MHz, DMSO-d₆) δ 8.4-9.3 (m, 2H), 7.15-7.7 (m, 9H), 4.37-4.6 (m, 1H), 2.9-3.4 (m, 3H+H₂O), 2.6-2.9 (m, 2H), 1.4-2.15 (m, 4H), 1.25-1.35 (m, 3H); MS (ESI+) 266 (M+H)⁺.

### Example 31B

### 5-({(2-phenylpropyl)[(1S)-1,2,3,4-tetrahydro-1-naphthalenyl]amino}carbonyl)-1,2,4-benzenetricarboxylic acid

The product from Example 31A and 1,2,4,5-benzenetetracarboxylic dianhydride were processed as described in Example 1B to provide the title compound.
¹H NMR (300 MHz, CD₃OD) δ 6.95-8.5 (m, 11H), 4.35-4.7 (m, 1H), 3.5-3.7 (m, 1H), 2.45-3.2 (m, 3H), 1.0-2.1 (m, 8H); MS (ESI+) 502 (M+H)⁺.

### Example 32

### 5-({(2-phenylethyl)[(1S)-1,2,3,4-tetrahydro-1-naphthalenyl]amino}carbonyl)-1,2,4-benzenetricarboxylic acid

### Example 32A

### N-(2-phenylethyl)-N-[(1S)-1,2,3,4-tetrahydro-1-naphthalenyl]amine

Phenylacetaldehyde and (1S)-1,2,3,4-tetrahydro-1-naphthalenylamine were processed as described in Example 1A to provide the title compound.
¹H NMR (300 MHz, DMSO-d₆) δ 9.0-9.3 (m, 2H), 7.15-7.65 (m, 9H), 4.45-4.55 (m, 1H), 2.65-3.25 (m, 6H), 1.65-2.25 (m, 4H); MS (ESI+) 252 (M+H)⁺.

### Example 32B

### 5-({(2-phenylethyl)[(1S)-1,2,3,4-tetrahydro-1-naphthalenyl]amino}carbonyl)-1,2,4-benzenetricarboxylic acid

The product from Example 32A and 1,2,4,5-benzenetetracarboxylic dianhydride were processed as described in Example 1B to provide the title compound.
¹H NMR (300 MHz, CD₃OD) δ 8.47 (m, 1H), 7.0-7.9 (m, 10H), 4.55-4.65 (m, 1H), 3.35-3.75 (m, 1H), 1.4-3.25 (m, 9H); MS (ESI+) 488 (M+H)⁺.

### Example 33

### 5-({benzyl[(1S)-1,2,3,4-tetrahydro-1-naphthalenyl]amino}carbonyl)-1,2,4-benzenetricarboxylic acid

### Example 33A

### N-benzyl-N-[(1S)-1,2,3,4-tetrahydro-1-naphthalenyl]amine

Benzaldehyde and (1S)-1,2,3,4-tetrahydro-1-naphthalenylamine were processed as described in Example 1A to provide the title compound.
¹H NMR (300 MHz, DMSO-d₆) δ 9.3-9.6 (m, 2H), 7.15-7.65 (m, 9H), 4.35-4.5 (m, 1H), 4.13-4.23 (m, 2H), 2.65-2.95 (m, 2H), 1.65-2.3 (m, 4H); MS (ESI+) 238 (M+H)⁺.

### Example 33B

### 5-({benzyl[(1S)-1,2,3,4-tetrahydro-1-naphthalenyl]amino}carbonyl)-1,2,4-benzenetricarboxylic acid

The product from Example 33A and 1,2,4,5-benzenetetracarboxylic dianhydride were processed as described in Example 1B to provide the title compound.
¹H NMR (300 MHz, DMSO-d₆) δ 7.0-8.4 (m, 11H), 3.3-5.5 (m, 3H), 2.5-2.7 (m, 2H), 1.3-2.2 (m, 4H); MS (ESI+) 474 (M+H)⁺.

### Example 34

### 5-({(3-methoxybenzyl)[(1S)-1,2,3,4-tetrahydro-1-naphthalenyl]amino}carbonyl)-1,2,4-benzenetricarboxylic acid

### Example 34A

### N-(3-methoxybenzyl)-N-[(1S)-1,2,3,4-tetrahydro-1-naphthalenyl]amine

3-Methoxybenzaldehyde and (1S)-1,2,3,4-tetrahydro-1-naphthalenylamine were processed as described in Example 1A to provide the title compound.
¹H NMR (300 MHz, DMSO-d₆) δ 9.3-9.5 (m, 2H), 6.95-7.57 (m, 8H), 4.35-4.45 (m, 1H), 4.07-4.15 (m, 2H), 3.78 (s, 3H), 2.6-2.95 (m, 2H), 1.65-2.3 (m, 4H); MS (ESI+) 268 (M+H)⁺.

### Example 34B

### 5-({(3-methoxybenzyl)[(1S)-1,2,3,4-tetrahydro-1-naphthalenyl]amino}carbonyl)-1,2,4-benzenetricarboxylic acid

The product from Example 34A and 1,2,4,5-benzenetetracarboxylic dianhydride were processed as described in Example 1B to provide the title compound.
¹H NMR (300 MHz, DMSO-d₆) δ 6.6-8.3 (m, 10H), 4.05-5.5 (m, 3H), 3.65-3.85 (m, 3H), 2.5-2.7 (m, 2H), 1.2-2.2 (4H); MS (ESI+) 504 (M+H)⁺.

### Example 35

### 5-[((1S)-1,2,3,4-tetrahydro-1-naphthalenyl{3-[3-(trifluoromethyl)pbenoxy]benzyl}amino)carbonyl]-1,2,4-benzenetricarboxylic acid

### Example 35A

### N-[(1S)-1,2,3,4-tetrahydro-1-naphthalenyl]-N-{3-[3-(trifluoromethyl)phenoxy]benzyl}amine

3-[3-(Trifluoromethyl)phenoxy]benzaldehyde and (1S)-1,2,3,4-tetrahydro-1-naphthalenylamine were processed as described in Example 1A to provide the title compound.
¹H NMR (300 MHz, CDCl₃) δ 6.85-7.5 (m, 12H), 3.8-3.97 (m, 3H), 2.65-2.95 (m, 2H), 1.6-2.1 (m, 5H); MS (ESI+) 398 (M+H)⁺.

### Example 35B

### 5-[((1S)-1,2,3,4-tetrahydro-1-naphthalenyl{3-[3-(trifluoromethyl)phenoxy]benzyl}amino)carbonyl]-1,2,4-benzenetricarboxylic acid

The product from Example 35A and 1,2,4,5-benzenetetracarboxylic dianhydride were processed as described in Example 1B to provide the title compound.
¹H NMR (300 MHz, DMSO-d₆) d 13.9 (bs, 1H), 6.65-8.3 (m, 14H), 3.9-5.65 (m, 3H), 2.5-2.7 (m, 2H), 1.3-2.1 (m, 4H); MS (ESI+) 634 (M+H)⁺.

### Example 36

### 5-({(3-phenoxybenzyl)[(1S)-1,2,3,4-tetrahydro-1-naphthalenyl]amino}carbonyl)-1,2,4-benzenetricarboxylic acid

### Example 36A

### N-(3-phenoxybenzyl)-N-[(1S)-1,2,3,4-tetrahydro-1-naphthalenyl]amine

3-Phenoxybenzaldehyde and (1S)-1,2,3,4-tetrahydro-1-naphthalenylamine were processed as described in Example 1A to provide the title compound.
¹H NMR (300 MHz, CDCl₃) δ 6.8-7.53 (13H), 3.77-4.1 (m, 3H), 2.6-2.95 (m, 2H), 1.4-2.2 (m, 5H); MS (ESI+) 330 (M+H)⁺.

### Example 36B

### 5-({(3-phenoxybenzyl)[(1S)-1,2,3,4-tetrahydro-1-naphthalenyl]amino}carbonyl)-1,2,4-benzenetricarboxylic acid

The product from Example 36A and 1,2,4,5-benzenetetracarboxylic dianhydride were processed as described in Example 1B to provide the title compound.
¹H NMR (300 MHz, DMSO-d₆) δ 6.5-8.3 (m, 15H), 3.8-5.6 (m, 3H), 2.5-2.7 (m, 2H), 1.6-2.1 (m, 4H); MS (ESI+) 566 (M+H)⁺.

### Example 37

### 5-({[3-(4-methoxyphenoxy)benzyl][(1S)-1,2,3,4-tetrahydro-1-naphthalenyl]amino}carbonyl)-1,2,4-benzenetricarboxylic acid

### Example 37A

### N-[3-(4-methoxyphenoxy)benzyl]-N-[(1S)-1,2,3,4-tetrahydro-1-naphthalenyl]amine

3-(4-Methoxyphenoxy)benzaldehyde and (1S)-1,2,3,4-tetrahydro-1-naphthalenylamine were processed as described in Example 1A to provide the title compound.
¹H NMR (300 MHz, CDCl₃) δ 6.8-7.53 (12H), 3.77-4.1 (m, 6H), 2.6-2.95 (m, 2H), 1.4-2.2 (m, 5H); MS (ESI+) 360 (M+H)⁺.

### Example 37B

### 5-({[3-(4-methoxyphenoxy)benzyl][(1S)-1,2,3,4-tetrahydro-1-naphthalenyl]amino}carbonyl)-1,2,4-benzenetricarboxylic acid

The product from Example 37A and 1,2,4,5-benzenetetracarboxylic dianhydride were processed as described in Example 1B to provide the title compound.
¹H NMR (300 MHz, DMSO-d₆) δ 6.5-8.3 (m, 14H), 3.8-5.6 (m, 3H), 3.7-3.8 (m, 3H), 2.5-2.7 (m, 2H), 1.6-2.1 (m, 4H); MS (ESI+) 596 (M+H)⁺.

### Example 38

### 5-({[3-(3,4-dichlorophenoxy)benzyl][(1S)-1,2,3,4-tetrahydro-1-naphthalenyl]amino}carbonyl)-1,2,4-benzenetricarboxylic acid

### Example 38A

### N-[3-(3,4-dichlorophenoxy)benzyl]-N-[(1S)-1,2,3,4-tetrahydro-1-naphthalenyl)amine

3-(3,4-Dichlorophenoxy)benzaldehyde and (1S)-1,2,3,4-tetrahydro-1-naphthalenylamine were processed as described in Example 1A to provide the title compound.
¹H NMR (300 MHz, CDCl₃) δ 6.8-7.4 (m, 11H), 3.75-3.97 (m, 3H), 2.65-2.9 (m, 2H), 1.4-2.1 (m, 5H); MS (ESI+) 398 (M+H)⁺.

### Example 38B

### 5-({[3-(3,4-dichlorophenoxy)benzyl][(1S)-1,2,3,4-tetrahydro-1-naphthalenyl]amino}carbonyl)-1,2,4-benzenetricarboxylic acid

The product from Example 38A and 1,2,4,5-benzenetetracarboxylic dianhydride were processed as described in Example 1B to provide the title compound.
¹H NMR (300 MHz, DMSO-d₆) δ 13.9 (bs, 1H), 6.65-8.3 (m, 13H), 3.85-5.66 (m, 3H), 2.5-2.7 (m, 2H), 1.3-2.2 (m, 4H); MS (ESI+) 634 (M+H)⁺.

### Example 39

### 5-({[3-(4-chlorophenoxy)benzyl][(1S)-1,2,3,4-tetrahydro-1-naphthalenyl]amino}carbonyl)-1,2,4-benzenetricarboxylic acid

### Example 39A

### N-[3-(4-chlorophenoxy)benzyl]-N-[(1S)-1,2,3,4-tetrahydro-1-naphthalenyl]amine

3-(4-Chlorophenoxy)benzaldehyde and (1S)-1,2,3,4-tetrahydro-1-naphthalenylamine were processed as described in Example 1A to provide the title compound.
¹H NMR (300 MHz, CDCl₃) δ 6.85-7.37 (m, 12H), 3.75-3.95 (m, 3H), 2.65-2.9 (m, 2H), 1.4-2.1 (m, 5H); MS (ESI+) 364 (M+H)⁺.

### Example 39B

### 5-({[3-(4-chlorophenoxy)benzyl][(1S)-1,2,3,4-tetrahydro-1-naphthalenyl]amino}carbonyl)-1,2,4-benzenetricarboxylic acid

The product from Example 39A and 1,2,4,5-benzenetetracarboxylic dianhydride were processed as described in Example 1B to provide the title compound.
¹H NMR (300 MHz, DMSO-d₆) δ 13.9 (bs, 1H), 6.6-8.3 (m, 14H), 3.9-5.65 (m, 3H), 2.5-2.7 (m, 2H), 1.3-2.2 (m, 4H); MS (ESI+) 600 (M+H)⁺.

### Example 40

### 5-({[3-(4-tert-butylphenoxy)benzyl][(1S)-1,2,3,4-tetrahydro-1-naphthalenyl]amino}carbonyl)-1,2,4-benzenetricarboxylic acid

### Example 40A

### N-[3-(4-tert-butylphenoxy)benzyl]-N-[(1S)-1,2,3,4-tetrahydro-1-naphthalenyl]amine

3-(4-tert-Butylphenoxy)benzaldehyde and (1S)-1,2,3,4-tetrahydro-1-naphthalenylamine were processed as described in Example 1A to provide the title compound.
¹H NMR (300 MHz, CDCl₃) δ 6.85-7.37 (m, 12H), 3.75-3.95 (m, 3H), 2.65-2.9 (m, 2H), 1.45-2.15 (m, 5H), 1.32 (s, 9H); MS (ESI+) 386 (M+H)⁺.

### Example 40B

### 5-({[3-(4-tert-butylphenoxy)benzyl][(1S)-1,2,3,4-tetrahydro-1-naphthalenyl]amino}carbonyl)-1,2,4-benzenetricarboxylic acid

The product from Example 40A and 1,2,4,5-benzenetetracarboxylic dianhydride were processed as described in Example 1B to provide the title compound.
¹H NMR (300 MHz, DMSO-d₆) δ 6.65-8.3 (m, 14H), 3.75-5.6 (m, 3H), 2.5-2.7 (m, 2H), 1.35-2.1 (m, 4H), 1.25-1.35 (m, 9H); MS (ESI+) 622 (M+H)⁺.

### Example 41

### 5-({[3-(4-methylphenoxy)benzyl][(1S)-1,2,3,4-tetrahydro-1-naphthalenyl]amino}carbonyl)-1,2,4-benzenetricarboxylic acid

### Example 41A

### N-[3-(4-methylphenoxy)benzyl]-N-[(1S)-1,2,3,4-tetrahydro-1-naphthalenyl]amine

3-(4-Methylphenoxy)benzaldehyde and (1S)-1,2,3,4-tetrahydro-1-naphthalenylamine were processed as described in Example 1A to provide the title compound.
¹H NMR (300 MHz, CDCl₃) δ 6.82-7.47 (m, 12H), 3.77-3.95 (m, 3H), 2.65-2.9 (m, 2H), 2.35 (s, 3H), 1.4-2.1 (m, 5H); MS (ESI+) 344 (M+H)⁺.

### Example 41B

### 5-({[3-(4-methylphenoxy)benzyl][(1S)-1,2,3,4-tetrahydro-1-naphthalenyl]amino}carbonyl)-1,2,4-benzenetricarboxylic acid

The product from Example 41A and 1,2,4,5-benzenetetracarboxylic dianhydride were processed as described in Example 1B to provide the title compound.
¹H NMR (300 MHz, DMSO-d₆) δ 13.9 (bs, 1H), 6.55-8.3 (m, 14H), 3.35-5.6 (m, 3H), 2.55-2.7 (m, 2H), 2.25-2.35 (m, 3H), 1.3-2.1 (m, 4H); MS (ESI+) 580 (M+H)⁺.

### Example 42

### 5-({[3-(3,5-dichlorophenoxy)benzyl][(1S)-1,2,3,4-tetrahydro-1-naphthalenyl]amino}carbonyl)-1,2,4-benzenetricarboxylic acid

### Example 42A

### N-[3-(3,5-dichlorophenoxy)benzyl]-N-[(1S)-1,2,3,4-tetrahydro-1-naphthalenyl]amine

3-(3,5-Dichlorophenoxy)benzaldehyde and (1S)-1,2,3,4-tetrahydro-1-naphthalenylamine were processed as described in Example 1A to provide the title compound.
MS (ESI+) 398 (M+H)⁺.

### Example 42B

### 5-({[3-(3,5-dichlorophenoxy)benzyl][(1S)-1,2,3,4-tetrahydro-1-naphthalenyl]amino}carbonyl)-1,2,4-benzenetricarboxylic acid

The product from Example 42A and 1,2,4,5-benzenetetracarboxylic dianhydride were processed as described in Example 1B to provide the title compound.
¹H NMR (300 MHz, DMSO-d₆) δ 13.9 (bs, 1H), 6.75-8.3 (m, 13H), 3.9-5.65 (m, 3H), 2.55-2.7 (m, 2H), 1.3-2.15 (m, 4H); MS (ESI+) 634 (M+H)⁺.

### Example 43

### 5-({(3-benzylbenzyl)[(1S)-1,2,3,4-tetrahydro-1-naphthalenyl]amino}carbonyl)-1,2,4-benzenetricarboxylic acid

### Example 43A

### N-(3-benzylbenzyl)-N-[(1S)-1,2,3,4-tetrahydro-1-naphthalenyl]amine

The product from Example 45C (759 mg, 2.21 mmol) in triethylsilane (5 mL) was treated with trifluoroacetic acid (5 mL, xs). After stirring for 30 minutes at room temperature, the reaction mixture was diluted with ethyl acetate and basified with concentrated NH₄OH. The separated organic phase was washed with brine, dried (Na₂SO₄), filtered and the filtrate concentrated under reduced pressure to provide the title compound as an oil (715 mg).
¹H NMR (300 MHz, CDCl₃) δ 7.0-7.35 (m, 13H), 3.75-4.0 (m, 5H), 2.6-2.9 (m, 2H), 1.4-2.1 (m, 5H), 0.85-1.0 (m, EtSiH), 0.45-0.65 (m, EtSiH); MS (DCI/NH₃) 328 (M+H)⁺.

### Example 43B

### 5-({(3-benzylbenzyl)[(1S)-1,2,3,4-tetrahydro-1-naphthalenyl]amino}carbonyl)-1,2,4-benzenetricarboxylic acid

The product from Example 43A and 1,2,4,5-benzenetetracarboxylic dianhydride were processed as described in Example 1B to provide the title compound.
¹H NMR (300MHz, DMSO-d₆) δ 6.75-8.3 (m, 15H), 3.7-5.5 (m, 5H), 2.5-2.65 (m, 2H), 1.25-2.1 (m, 4H); MS (ESI+) 564 (M+H)⁺.

### Example 44

### 5-({(9H-fluoren-2-ylmethyl)[(1S)-1,2,3,4-tetrahydro-1-naphthalenyl]amino}carbonyl)-1,2,4-benzenetricarboxylic acid

### Example 44A

### N-(9H-fluoren-2-ylmethyl)-N-[(1S)-1,2,3,4-tetrahydro-1-naphthalenyl]amine

The product from Example 46C was processed as described in Example 43A to provide the title compound.
¹H NMR (300 MHz, CDCl₃) δ 7.05-7.85 (m, 11H), 3.85-4.07 (m, 5H), 2.67-2.93 (m, 2H), 1.4-2.15 (m, 5H); MS (DCI/NH₃) 326 (M+H)⁺.

### Example 44B

### 5-({(9H-fluoren-2-ylmethyl)[(1S)-1,2,3,4-tetrahydro-1-naphthalenyl]amino}carbonyl)-1,2,4-benzenetricarboxylic acid

The product from Example 44A and 1,2,4,5-benzenetetracarboxylic dianhydride were processed as described in Example 1B to provide the title compound.
¹H NMR (300MHz, DMSO-d₆) δ 13.9 (bs, 1H), 7.0-8.3 (m, 13H), 3.4-5.6 (5H), 2.5-2.7 (2H), 1.3-2.2 (4H); MS (ESI+) 562 (M+H)⁺.

### Example 45

### 5-({{3-[hydroxy(phenyl)methyl]benzyl}[(1S)-1,2,3,4-tetrahydro-1-naphthalenyl]amino}carbonyl)-1,2,4-benzenetricarboxylic acid

### Example 45A

### [3-(hydroxymethyl)phenyl](phenyl)methanone

3-benzoylbenzoic acid (6.26 g, 27.7 mmol) was carefully added in several portions to borane-N,N-diethylaniline complex (7.38 mL, 41.5 mmol) in anhydrous THF (60 mL) at ambient temperature. After 15 minutes, the reaction mixture was heated at reflux for 5 hours and then cooled to ambient temperature. The reaction mixture was diluted with diethyl ether, quenched with 1N HCl and the separated aqueous phase was extracted with diethyl ether. The organic layers were combined, washed with 1N HCl, brine, dried (Na₂SO₄), filtered and the filtrate concentrated under reduced pressure. The residue was purified by flash chromatography (silica gel, 40% ethyl acetate/hexanes) to provide a mixture of the title compound and the diol, [3-(hydroxymethyl)phenyl](phenyl)methanol, as a colorless oil (3.59 g).

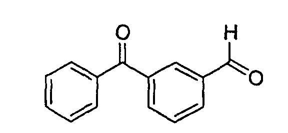

### Example 45B

### 3-benzoylbenzaldehyde

The mixture from Example 45A (3.59 g) and celite (diatomaceous earth) in anhydrous CH₂Cl₂ (100 mL) were treated with and pyridinium chlorochromate (12.6 g, 58.6 mmol). After stirring for 18 hours, the reaction mixture was filtered and the filtrate concentrated under reduced pressure. The residue was taken up in ethyl acetate, washed with 1N HCl, saturated NaHCO₃, brine, dried (Na₂SO₄), filtered and the filtrate concentrated under reduced pressure. The residue was purified by flash chromatography (silca gel, 30% ethyl acetate/hexanes) to provide the title compound as a colorless oil (2.05 g, 35%yield).
¹H NMR (300 MHz, CDCl₃) δ 10.1(s, 1H), 8.25-8.3 (m, 1H), 8.05-8.15 (m 2H), 7.75-7.85 (m, 2H), 7.6-7.72 (m, 2H), 7.47-7.55 (m, 2H); MS (DCI/NH₃) 228 (M+NH₄)⁺.

### Example 45C

### phenyl(3-{[(1S)-1,2,3,4-tetrahydro-1-naphthalenylamino]methyl}phenyl)methanol

The product from Example 45B (2.04 g, 9.70 mmol) in absolute ethanol (25 mL) was treated with (1S)-1,2,3,4-tetrahydro-1-naphthalenylamine (1.39 mL, 9.70 mmol). After 3 hours, the mixture was treated with sodium borohydride and the reaction mixture was allowed to stir at room temperature for 16 hours. The reaction mixture was quenched with 1N NaOH, diluted with ethyl acetate and the separated aqueous phase was extracted with ethyl acetate. The organic layers were combined, washed with water, brine, dried (Na₂SO₄), filtered and the filtrate concentrated under reduced pressure. The residue was purified by flash chromatography (silica gel, 7% methanol/CH₂Cl₂) to provide the title compound as a colorless oil (2.85 g, 86% yield).
¹H NMR (300 MHz, CDCl₃) δ 7.05-7.5 (m, 13H), 5.85 (s, 1H), 3.8-3.95 (m, 3H), 2.65-2.9 (m, 2H), 1.5-2.1 (m, 6H); MS (DCI/NH₃) 344 (M+H)⁺.

### Example 45D

### 5-({{3-[hydroxy(phenyl)methyl]benzyl}[(1S)-1,2,3,4-tetrahydro-1-naphthalenyl]amino}carbonyl)-1,2,4-benzenetricarboxylic acid

The product from Example 45C and 1,2,4,5-benzenetetracarboxylic dianhydride were processed as described in Example 1B to provide the title compound.
¹H NMR (300MHz, DMSO-d₆) δ 13.85 (bs, 1H), 6.8-8.3 (m, 15H), 5.55-5.75 (m, 1H), 3.6-5.3 (m, 3H), 2.5-2.6 (m, 2H), 1.2-2.05 (m, 5H); MS (ESI-) 578 (M-H)⁻.

### Example 46

### 5-({[(9-hydroxy-9H-fluoren-2-yl)methyl][(1S)-1,2,3,4-tetrahydro-1-naphthalenyl]amino}carbonyl)-1,2,4-benzenetricarboxylic acid

### Example 46A

### 2-(hydroxymethyl)-9H-fluoren-9-one

9-Oxo-9H-fluorene-2-carboxylic acid, purchased from Aldrich, was processed as described in Example 45A to provide the title compound.

### Example 46B

### 9-oxo-9H-fluorene-2-carbaldehyde

The product from Example 46A was processed as described in Example 45B to provide the title compound.

### Example 46C

### 2-{[(1S)-1,2,3,4-tetrahydro-1-naphthalenylamino]methyl}-9H-fluoren-9-ol

The product from Example 46B and (1S)-1,2,3,4-tetrahydro-1-naphthalenylamine were processed as described in Example 45C to provide the title compound.
¹H NMR (300 MHz, CDCl₃) δ 7.05-7.73 (m, 11H), 5.57 (s, 1H), 3.84-4.03 (m, 3H), 2.67-2.91 (m, 2H), 1.6-2.1 (m, 6H); MS (DCI/NH₃) 342 (M+H)⁺.

### Example 46D

### 5-({[(9-hydroxy-9H-fluoren-2-yl)methyl][(1S)-1,2,3,4-tetrahydro-1-naphthalenyl]amino}carbonyl)-1,2,4-benzenetricarboxylic acid

The product from Example 46C and 1,2,4,5-benzenetetracarboxylic dianhydride were processed as described in Example 1B to provide the title compound.
¹H NMR (300MHz, DMSO-d₆) δ 7.0-8.3 (m, 13H), 3.85-5.65 (m, 4H), 2.55-2.7 (m, 2H), 1.25-2.2 (m, 5H); MS (ESI-) 576 (M-H)⁻.

### Example 47

### 5-({(3-benzoylbenzyl)[(1S)-1,2,3,4-tetrahydro-1-naphthalenyl]amino}carbonyl)-1,2,4-benzenetricarboxylic acid

### Example 47A

### phenyl(3-{[(1S)-1,2,3,4-tetrahydro-1-naphthalenylamino]methyl}phenyl)methanone

The product from Example 45C (319 mg, 0.84 mmol) and triethylamine (468 µL, 3.36 mmol) in DMSO (10 mL) were treated with sulfur trioxide pyridine complex (535 mg, 3.36 mmol). After stirring for 2 hours at room temperature, the reaction mixture was partitioned between water and diethyl ether. The aqueous phase was extracted with diethyl ether. The organic extracts were combined, washed with brine, dried (Na₂SO₄), filtered, and the filtrate concentrated under reduced pressure. The residue was purified by flash chromatography (silica gel, 20% ethyl acetate/hexanes) to provide a colorless oil (106 mg, 37% yield).
¹H NMR (300 MHz, CDCl₃) δ 7.05-7.85 (m, 13H), 3.85-4.05 (m, 3H), 2.65-2.9 (m, 2H), 1.6-2.2 (m, 5H); MS (DCI/NH₃) 342 (M+H)⁺.

### Example 47B

### 5-({(3-benzoylbenzyl)[(1S)-1,2,3,4-tetrahydro-1-naphthalenyl]amino}carbonyl)-1,2,4-benzenetricarboxylic acid

The product from Example 47A and 1,2,4,5-benzenetetracarboxylic dianhydride were processed as described in Example 1B to provide the title compound.
¹H NMR (300MHz, DMSO-d₆) δ 7.0-8.3 (m, 15H), 4.0-5.7 (m, 3H), 2.55-2.8 (m, 2H), 1.3-2.2 (m, 4H); MS (ESI-) 576 (M-H)⁻.

### Example 48

### 5-({[3-(4-nitrophenoxy)benzyl][(1S)-1,2,3,4-tetrahydro-1-naphthalenyl]amino}carbonyl)-1,2,4-benzenetricarboxylic acid

### Example 48A

### 3-(4-Nitrophenoxy)benzaldehyde

3-Hydroxybenzaldehyde (1.15 g, 9.4 mmol), 1-fluoro-4-nitrobenzene (1.0 mL, 9.4 mmol) and K₂CO₃ (2.61 g, 19 mmol) were combined in DMF (20 mL) and heated at 80 °C. After 6 hours, the reaction mixture was cooled to room temperature and partitioned between water and diethyl ether. The separated aqueous phase was extracted with diethyl ether. The organic layers were combined, washed with 1N NaOH, brine, dried (MgSO₄), filtered and the filtrate concentrated under reduced pressure to provide the title compound as a yellow solid (1.98 g).
¹H NMR (300 MHz, CDCl₃) δ 10.0 (s, 1H), 8.2-8.27 (m, 2H), 7.75-7.78 (m, 1H), 7.55-7.65 (m, 2H), 7.35-7.4 (m, 1H), 7.05-7.1 (m, 2H); MS (DCI/NH₃) 261 (M+NH₄)⁺.

### Example 48B

### N-[3-(4-nitrophenoxy)benzyl]-N-[(1S)-1,2,3,4-tetrahydro-1-naphthalenyl]amine

The product from Example 48A and (1S)-1,2,3,4-tetrahydro-1-naphthalenylamine were processed as described in Example 1A to provide the title compound.
¹H NMR (300 MHz, CDCl₃) δ 6.95-8.3 (m, 12H), 3.82-4.02 (m, 3H), 2.65-2.9 (m, 2H), 1.4-2.2 (m, 5H); MS (DCI/NH₃) 375 (M+H)⁺.

### Example 48C

### 5-({[3-(4-nitrophenoxy)benzyl][(1S)-1,2,3,4-tetrahydro-1-naphthalenyl]amino}carbonyl)-1,2,4-benzenetricarboxylic acid

The product from Example 48B and 1,2,4,5-benzenetetracarboxylic dianhydride were processed as described in Example 1B to provide the title compound.
¹H NMR (300MHz, DMSO-d₆) δ 6.75-8.3 (m, 14H), 3.85-5.7 (m, 3H), 2.5-2.75 (m, 2H), 1.3-2.2 (m, 4H); MS (ESI+) 611 (M+H)⁺.

### Example 49

### 5-({[(9-oxo-9H-fluoren-2-yl)methyl][(1S)-1,2,3,4-tetrahydro-1-naphthalenyl]amino}carbonyl)-1,2,4-benzenetricarboxylic acid

### Example 49A

### 2-{[(1S)-1,2,3,4-tetrahydro-1-naphthalenylamino]methyl}-9H-fluoren-9-one

The product from Example 46C and sulfur trioxide pryridine were processed as described in Example 47A to provide the title compound.

### Example 49B

### 5-({[(9-oxo-9H-fluoren-2-yl)methyl][(1S)-1,2,3,4-tetrahydro-1-naphthalenyl]amino}carbonyl)-1,2,4-benzenetricarboxylic acid

The product from Example 49A and 1,2,4,5-benzenetetracarboxylic dianhydride were processed as described in Example 1B to provide the title compound.
¹H NMR (300MHz, DMSO-d₆) δ 7.0-8.35 (m, 13H), 3.9-5.6 (m ,3H), 2.55-2.75 (m, 2H), 1.35-2.2 (m, 4H); MS (ESI-) 574 (M-H)⁻.

### Example 50

### 5-({[3-(4-cyanophenoxy)benzyl][(1S)-1,2,3,4-tetrahydro-1-naphthalenyl]amino}carbonyl)-1,2,4-benzenetricarboxylic acid

### Example 50A

### 4-(3-Formylphenoxy)benzonitrile

4-Fluorobenzonitrile and 3-hydroxybenzaldehyde were processed as described in Example 48A to provide the title compound.

### Example 50B

### 4-(3-{[(1S)-1,2,3,4-tetrahydro-1-naphthalenylamino]methyl}phenoxy)benzonitrile

The product from Example 50A and (1S)-1,2,3,4-tetrahydro-1-naphthalenylamine were processed as described in Example 1A to provide the title compound.
¹H NMR (300 MHz, CDCl₃) δ 6.9-7.65 (m, 12H), 3.8-4.0 (m, 3H), 2.65-2.92 (m, 2H), 1.3-2.2 (m, 5H); MS (DCI/NH₃) 355 (M+H)⁺.

### Example 50C

### 5-({[3-(4-cyanophenoxy)benzyl][(1S)-1,2,3,4-tetrahydro-1-naphthalenyl]amino}carbonyl)-1,2,4-benzenetricarboxylic acid

The product from Example 50B and 1,2,4,5-benzenetetracarboxylic dianhydride were processed as described in Example 1B to provide the title compound.
¹H NMR (300MHz, DMSO-d₆) δ 13.9 (bs, 1H), 6.7-8.3 (m, 14H), 3.9-5.7 (m, 3H), 2.55-2.75 (m, 2H), 1.3-2.2 (m, 4H); MS (ESI-) 589 (M-H)⁻.

### Example 51

### 5-({[3-(benzyloxy)benzyl][(1S)-1,2,3,4-tetrahydro-1-naphthalenyl]amino}carbonyl)-1,2,4-benzenetricarboxylic acid

### Example 51A

### N-[3-(benzyloxy)benzyl]-N-[(1S)-1,2,3,4-tetrahydro-1-naphthalenyl]amine

3-(Benzyloxy)benzaldehyde and (1S)-1,2,3,4-tetrahydro-1-naphthalenylamine were processed as described in Example 1A to provide the title compound.
¹H NMR (300 MHz, DMSO-d₆) δ 9.35-9.65 (m, 2H), 7.03-7.58 (m, 13H), 5.13 (s, 2H), 4.35-4.45 (m, 1H), 4.1-4.18 (m, 2H), 2.65-2.93 (m, 2H), 1.65-2.3 (m, 4H); MS (DCI/NH₃) 344 (M+H)⁺.

### Example 51B

### 5-({[3-(benzyloxy)benzyl][(1S)-1,2,3,4-tetrahydro-1-naphthalenyl]amino}carbonyl)-1,2,4-benzenetricarboxylic acid

The product from Example 51A and 1,2,4,5-benzenetetracarboxylic dianhydride were processed as described in Example 1B to provide the title compound.
¹H NMR (300MHz, DMSO-d₆) δ 6.55-8.3 (m, 15H), 3.75-5.55 (m, 5H), 2.5-2.7 (m, 2H), 1.3-2.1 (m, 4H); MS (ESI-) 578 (M-H)⁻.

### Example 52

### 5-({(4-phenoxybutyl)[(1S)-1,2,3,4-tetrahydro-1-naphthalenyl]amino}carbonyl)-1,2,4-benzenetricarboxylic acid

### Example 52A

### 4-phenoxy-N-[(1S)-1,2,3,4-tetrahydro-1-naphthalenyl]butanamide

4-Phenoxybutanoic acid (1.05 g, 5.8 mmol, Lancaster) and triethylamine (2.4 mL, 17.4 mmol) in anhydrous CH₂Cl₂ (25 mL) were treated with pivaloyl chloride (0.75 mL, 6.1 mmol) at 0 °C. After stirring for 1 hour, the reactiom mixture was treated with (1S)-1,2,3,4-tetrahydro-1-naphthalenylamine (1.0 mL, 6.9 mmol) and the reaction mixture was allowed to warm to room temperature and stir for 16 hours. The reaction mixture was diluted with CH₂Cl₂, washed with 1N HCl, saturated NaHCO₃, brine, dried (Na₂SO₄), filtered and the filtrate concentrated under reduced pressure. The residue was purified by flash chromatography (silica gel, 40% ethyl acetate/hexanes) to provide the title compound as a colorless oil (2.07 g).

### Example 52B

### N-(4-phenoxybutyl)-N-[(1S)-1,2,3,4-tetrahydro-1-naphthalenyl]amine

The product from Example 52A (2.07 g) in THF (20 mL) was treated with boranetetrahydrofuran complex (12 mL, 12 mmol) and heated at reflux for 1.5 hours. The mixture was allowed to cool to room temperature and quenched with 1N NaOH. The mixture was stirred 30 for minutes and diluted with diethyl ether. The separated organic phase was washed with brine, dried (Na₂SO₄), filtered and the filtrate concentrated. The residue was purified by flash chromatography (silica gel, 30% ethyl acetate/hexanes) to provide the title compound as a pale brown oil (0.48 g, 28% yield).
¹H NMR (300 MHz, CDCl₃) δ 6.75-7.55 (m, 9H), 3.8-4.05 (m, 3H), 2.65-2.9 (m, 4H), 1.6-2.1 (m, 9H); MS (DCI/NH₃) 296 (M+H)⁺.

### Example 52C

### 5-({(4-phenoxybutyl)[(1S)-1,2,3,4-tetrahydro-1-naphthalenyl]amino}carbonyl)-1,2,4-benzenetricarboxylic acid

The product from Example 52B and 1,2,4,5-benzenetetracarboxylic dianhydride were processed as described in Example 1B to provide the title compound.
¹H NMR (300MHz, DMSO-d₆) δ 13.7 (bs, 1H), 6.65-8.3 (m, 11H), 2.6-5.6 (m, 7H), 1.2-2.2 (m, 8H); MS (ESI-) 530 (M-H)⁻.

### Example 53

### 5-({(3-nitrobenzyl)[(1S)-1,2,3,4-tetrahydro-1-naphthalenyl]amino}carbonyl)-1,2,4-benzenetricarboxylic acid

### Example 53A

### N-(3-nitrobenzyl)-N-[(1S)-1,2,3,4-tetrahydro-1-naphthalenyl]amine

3-Nitrobenzaldehyde and (1S)-1,2,3,4-tetrahydro-1-naphthalenylamine were processed as described in Example 1A to provide the title compound.
¹H NMR (300 MHz, CDCl₃) δ 8.28-8.31 (m, 1H), 8.08-8.13 (m, 1H), 7.73-7.8 (m, 1H), 7.35-7.53 (m, 2H), 7.05-7.23 (m, 3H), 3.8-4.08 (m, 2H), 3.78-3.85 (m, 1H), 2.65-2.9 (m, 2H), 1.65-2.1 (m, 4H); MS (DCI/NH₃) 283 (M+H)⁺.

### Example 53B

### 5-({(3-nitrobenzyl)[(1S)-1,2,3,4-tetrahydro-1-naphthalenyl]amino}carbonyl)-1,2,4-benzenetricarboxylic acid

The product from Example 53A and 1,2,4,5-benzenetetracarboxylic dianhydride were processed as described in Example 1B to provide the title compound.
¹H NMR (300MHz, DMSO-d₆) δ 6.85-8.4 (m, 10H), 4.1-5.85 (m, 3H), 2.55-2.75 (m, 2H), 1.35-2.25 (m, 4H); MS (ESI-) 517 (M-H)⁻.

### Example 54

### 5-({[3-(cyclohexyloxy)benzyl][(1S)-1,2,3,4-tetrahydro-1-naphthalenyl]amino}carbonyl)-1,2,4-benzenetricarboxylic acid

### Example 54A

### 3-(cyclohexyloxy)benzaldehyde

3-Hydroxybenzaldehyde, purchased from Acros, and cyclohexanol were processed under Mitsunobu conditions, as described in Saccomano, et al., J. Med. Chem. 34, (1991) 291-298, to provide the title compound.

### Example 54B

### N-[3-(cyclohexyloxy)benzyl]-N-[(1S)-1,2,3,4-tetrahydro-1-naphthalenyl]amine

The product from Example 54A and (1S)-1,2,3,4-tetrahydro-1-naphthalenylamine were processed as described in Example 1A to provide the title compound.
¹H NMR (300 MHz, CDCl₃) δ 6.7-7.4 (m, 8H+Ph₃PO), 4.1-4.23 (m, 1H), 3.77-3.95 (m, 3H), 2.65-2.9 (m, 2H), 1.35-2.1 (m, 15H); MS (DCI/NH₃) 336 (M+H)⁺.

### Example 54C

### 5-({[3-(cyclohexyloxy)benzyl][(1S)-1,2,3,4-tetrahydro-1-naphthalenyl]amino}carbonyl)-1,2,4-benzenetricarboxylic acid

The product from Example 54B and 1,2,4,5-benzenetetracarboxylic dianhydride were processed as described in Example 1B to provide the title compound.
¹H NMR (300MHz, DMSO-d₆) δ 6.45-8.3 (m, 10H), 3.7-5.5 (m, 4H), 2.55-2.7 (m, 2H), 1.15-2.2 (m, 14H); MS (ESI+) 572 (M+H)⁺.

### Example 55

### 5-({{3-[exo-bicyclo[2.2.1]hept-2-yloxy]benzyl}[(1S)-1,2,3,4-tetrahydro-1-naphthalenyl]amino}carbonyl)-1,2,4-benzenetricarboxylic acid

### Example 55A

### 3-(exo-bicyclo[2.2.1]hept-2-yloxy)benzaldehyde

3-Hydroxybenzaldehyde, purchased from Acros, and endo-bicyclo[2.2.1]heptan-2-ol, purchased from Aldrich, were processed under Mitsunobu conditions, as described in Saccomano, et al., J. Med. Chem. 34, (1991) 291-298, to provide the title compound.

### Example 55B

### N-[3-(exo-bicyclo[2.2.1]hept-2-yloxy)benzyl]-N-[(1S)-1,2,3,4-tetrahydro-1-naphthalenyl]amine

The product from Example 55A and (1S)-1,2,3,4-tetrahydro-1-naphthalenylamine were processed as described in Example 1A to provide the title compound.
¹H NMR (300 MHz, CDCl₃) δ 6.7-7.4 (m, 8H+Ph₃PO), 4.15-4.2 (m, 1H), 3.75-3.95 (m, 3H), 2.65-2.9 (m, 2H), 2.43-2.48 (m, 1H), 2.28-2.35 (m, 1H), 1.1-2.1 (m, 13H); MS (DCI/NH₃) 348 (M+H)⁺.

### Example 55C

### 5-({{3-[exo-bicyclo[2.2.1]hept-2-yloxy]benzyl}[(1S)-1,2,3,4-tetrahydro-1-naphthalenyl]amino}carbonyl)-1,2,4-benzenetricarboxylic acid

The product from Example 55B and 1,2,4,5-benzenetetracarboxylic dianhydride were processed as described in Example 1B to provide the title compound.
¹H NMR (300MHz, DMSO-d₆) δ 6.4-8.3 (m, 10H), 3.7-5.5 (m, 4H), 2.55-2.7 (m, 2H), 1.0-2.4 (m, 14H); MS (ESI+) 584 (M+H)⁺.

### Example 56

### 5-({[spiro[1,3-benzodioxol-5-yl-2,1'-cyclohexane]methyl][(1S)-1,2,3,4-tetrahydro-1-naphthalenyl]amino}carbonyl)-1,2,4-benzenetricarboxylic acid

### Example 56A

### spiro[5-bromo-1,3-benzodioxole-2,1'-cyclohexane]

A vigorously stirred solution of spiro[1,3-benzodioxole-2,1'-cyclohexane] (2.49 g, 13.1 mmol), prepared according to Boeckmann, J. and Schill, G., Chem Ber. 110 (1977) 703, in CHCl₃ (100 mL) and saturated NaHCO₃ (100 mL) was treated with bromine (0.67 mL, 13.1 mmol) in CHCl₃ (10 mL) dropwise. After stirring for 3 hours, the organic phase was separated and washed with 10% Na₂S₂O₅ solution, water, brine, dried (Na₂SO₄), filtered and the filtrate concentrated under reduced pressure. The residue was purified by flash chromatography (silica gel, eluting with hexanes) to provide the title compound as a white solid (1.98 g).

### Example 56B

### spiro[5-formyl-1,3-benzodioxole-2,1'-cyclohexane]

The product from Example 56A (1.17 g, 4.35 mmol) in THF (40 mL) was treated with tert-butyllithium (5.80 mL of a 1.5 M solution in pentane, 8.70 mmol) dropwise at -78 °C. After 15 minutes, the reaction mixture was treated with DMF (3.37 mL, 43.5 mmol) and stirred at -78 °C for 2 hours. The reaction mixture was diluted with diethyl ether and quenched with 1N HCl. The separated organic phase was washed with 1N NaOH, water, brine, dried (Na₂SO₄), filtered and the filtrate concentrated under reduced pressure. The residue was purified by flash chromatography (silica gel, 2% ethyl acetate/hexanes) to provide the title compound as a colorless oil (415 mg, 15% yield).
¹H NMR (300 MHz, CDCl₃) δ 9.78 (s, 1H), 7.36 (dd, 1H), 7.23 (d, 1H), 6.84 (d, 1H), 1.9-1.97 (m, 4H), 1.7-1.8 (m, 4H), 1.47-1.57 (m, 2H); MS (DCI/NH₃) 219 (M+H)⁺, 236 (M+NH₄)⁺.

### Example 56C

### N-[spiro[1,3-benzodioxol-5-yl-2,1'-cyclohexane]methyl]-N-[(1S)-1,2,3,4-tetrahydro-1-naphthalenyl]amine

The product from Example 56B and (1S)-1,2,3,4-tetrahydro-1-naphthalenylamine were processed as described in Example 1A to provide the title compound.
¹H NMR (300 MHz, CDCl₃) δ 6.65-7.42 (m, 7H), 3.7-3.88 (m, 3H), 2.65-2.9 (m, 2H), 1.4-2.1 (m, 15H); MS (DCI/NH₃) 350 (M+H)⁺.

### Example 56D

### 5-({[spiro[1,3-benzodioxol-5-yl-2,1'-cyclohexane]methyl][(1S)-1,2,3,4-tetrahydro-1-naphthalenyl]amino}carbonyl)-1,2,4-benzenetricarboxylic acid

The product from Example 56C and 1,2,4,5-benzenetetracarboxylic dianhydride were processed as described in Example 1B to provide the title compound.
¹H NMR (300MHz, DMSO-d₆) δ 6.25-8.45 (m, 9H), 3.7-5.6 (m, 3H), 2.55-2.75 (m, 2H), 1.3-2.2 (m, 14H); MS (ESI+) 586 (M+H)⁺.

### Example 57

### 5-({[3-(4-fluorophenoxy)benzyl][(1S)-1,2,3,4-tetrahydro-1-naphthalenyl]amino}carbonyl)-1,2,4-benzenetricarboxylic acid

### Example 57A

### N-[3-(4-fluorophenoxy)benzyl]-N-[(1S)-1,2,3,4-tetrahydro-1-naphthalenyl]amine

3-(4-Fluorophenoxy)benzaldehyde, prepared as described in Tanaka, et al., J. Med. Chem., 41 (1998) 4408-4420, and (1S)-1,2,3,4-tetrahydro-1-naphthalenylamine were processed as described in Example 1A to provide the title compound.
¹H NMR (300 MHz, CDCl₃) δ 6.8-7.35 (m, 12H), 3.75-3.95 (m, 3H), 2.65-2.9 (m, 2H), 1.4-2.1 (m, 5H); MS (DCI/NH₃) 348 (M+H)⁺.

### Example 57B

### 5-({[3-(4-fluorophenoxy)benzyl][(1S)-1,2,3,4-tetrahydro-1-naphthalenyl]amino}carbonyl)-1,2,4-benzenetricarboxylic acid

The product from Example 57A and 1,2,4,5-benzenetetracarboxylic dianhydride were processed as described in Example 1B to provide the title compound.
¹H NMR (300MHz, DMSO-d₆) δ 6.5-8.45 (m, 14H), 3.7-5.6 (m, 3H), 2.55-2.75 (m, 2H), 1.3-2.15 (m, 4H); MS (ESI+) 584 (M+H)⁺.

### Example 58

### 5-({[3-(allyloxy)benzyl][(1S)-1,2,3,4-tetrahydro-1-naphthalenyl]amino}carbonyl)-1,2,4-benzenetricarboxylic acid

### Example 58A

### 3-(allyloxy)benzaldehyde

3-Hydroxybenzaldehyde (1.00 g, 8.19 mmol) and allyl bromide (0.780 mL, 9.01 mmol) in N,N-dimethylacetamide (35 mL) was treated with Cs₂CO₃ (4.01 g, 12.3 mmol) and allowed to stir at room temperature for 12 hours. The reaction mixture was diluted with diethyl ether (150 mL), washed with 1N HCl, saturated NaHCO₃, brine, dried (MgSO₄), filtered and the filtrate concentrated. The residue was purified by flash chromatography (silica gel, using 7% ethyl acetate/hexanes as eluent) to provide the title compound as a clear oil (1.19 g, 89%).
¹H NMR (CDCl₃, 300 MHz) δ 4.82 (m, 2H), 5.25-5.5.50 (m, 2H), 6.03 (m, 1H), 7.18-7.52 (m, 4H), 9.95 (s, 1H); MS (DCI+) 163 (M+H)⁺.

### Example 58B

### N-[3-(allyloxy)benzyl]-N-[(1S)-1,2,3,4-tetrahydro-1-naphthalenyl]amine

The product from Example 58A and (1S)-1,2,3,4-tetrahydro-1-naphthalenylamine were processed as described in Example 1A to provide the title compound.
¹H NMR (CDCl₃, 300 MHz) δ 1.42-2.05 (m, 4H), 2.79 (m, 2H), 3.78-3.95 (m, 3H), 4.50 (d, 2H), 5.26 (m, 1H), 5.42 (m, 1H), 6.05 (m, 1H), 6.78-7.37 (m, 8H); MS (DCI+) 294 (M+H)⁺.

### Example 58C

### 5-({[3-(allyloxy)benzyl][(1S)-1,2,3,4-tetrahydro-1-naphthalenyl]amino}carbonyl)-1,2,4-benzenetricarboxylic acid

The product from Example 58B and 1,2,4,5-benzenetetracarboxylic dianhydride were processed as described in Example 1B to provide the title compound.
¹H NMR (DMSO-d₆, 400 MHz) δ 1.18-2.17 (m, 4H), 2.55-2.70 (m, 2H), 3.65-5.02 (m, 5H), 5.22-5.50 (m, 2H), 6.05 (m, 1H), 6.55-8.30 (m, 10H); HRMS (FAB) calculated for C₃₀H₂₈NO₈ 530.1815 (M+H)⁺. Found 530.1811 (M+H)⁺.

### Example 59

### 5-({[3-(2-cyclohexen-1-yloxy)benzyl][(1S)-1,2,3,4-tetrahydro-1-naphthalenyl]amino}carbonyl)-1,2,4-benzenetricarboxylic acid

### Example 59A

### 3-(2-cyclohexen-1-yloxy)benzaldehyde

3-Bromo-1-cyclohexene and 3-hydroxybenzaldehyde were processed as described in Example 58A to provide the title compound.
¹H NMR (CDCl₃, 300 MHz) δ 1.62-2.20 (m, 6H), 4.87 (m, 1H), 5.91 (m, 1H), 6.02 (m, 1H), 7.18-7.45 (m, 4H), 9.98 (s, 1H); MS (DCI+) 203 (M+H)⁺.

### Example 59B

### N-[3-(2-cyclohexen-1-yloxy)benzyl]-N-[(1S)-1,2,3,4-tetrahydro-1-naphthalenyl]amine

The product from Example 59A and (1S)-1,2,3,4-tetrahydro-1-naphthalenylamine were processed as described in Example 1A to provide the title compound.
¹H NMR (CDCl₃, 300 MHz) δ 1.41-2.22 (m, 10H), 2.65-2.91 (m, 2H), 3.78-3.95 (m, 3H), 4.80 (m, 1H), 5.86-6.02 (m, 2H), 6.78-7.42 (m, 8H); MS (DCI+) 334 (M+H)⁺.

### Example 59C

### 5-({[3-(2-cyclohexen-1-yloxy)benzyl][(1S)-1,2,3,4-tetrahydro-1-naphthalenyl]amino}carbonyl)-1,2,4-benzenetricarboxylic acid

The product from Example 59B and 1,2,4,5-benzenetetracarboxylic dianhydride were processed as described in Example 1B to provide the title compound.
¹H NMR (DMSO-d₆, 400 MHz) δ 1.22-2.15 (m, 10H), 2.50-2.68 (m, 2H), 3.83-4.28 (m, 2H), 4.65-4.95 (m, 2H), 5.23-6.05 (m, 2H), 6.45-8.25 (m, 10H); HRMS (FAB): calculatedd for C₃₃H₃₂NO₈ 570.2128 (M+H)⁺. Found 570.2123 (M+H)⁺.

### Example 60

### 5-({[3,5-bis(trifluoromethyl)benzyl][(1S)-1,2,3,4-tetrahydro-1-naphthalenyl]amino}carbonyl)-1,2,4-benzenetricarboxylic acid

### Example 60A

### N-[3,5-bis(trifluoromethyl)benzyl]-N-[(1S)-1,2,3,4-tetrahydro-1-naphthalenyl]amine

3,5-bis(Trifluoromethyl)benzaldehyde and (1S)-1,2,3,4-tetrahydro-1-naphthalenylamine were processed as described in Example 1A to provide the title compound.
¹H NMR (CDCl₃, 300 MHz) δ 1.67-2.11 (m, 4H), 2.64-2.91 (m, 2H), 3.82 (t, 1H), 3.96-4.02 (m, 2H), 7.05-7.92 (m, 7H); MS (ESI+) 374 (M+H)⁺.

### Example 60B

### 5-({[3,5-bis(trifluoromethyl)benzyl][(1S)-1,2,3,4-tetrahydro-1-naphthalenyl]amino}carbonyl)-1,2,4-benzenetricarboxylic acid

The product from Example 60A and 1,2,4,5-benzenetetracarboxylic dianhydride were processed as described in Example 1B to provide the title compound.
¹H NMR (DMSO-d₆, 400 MHz) δ 1.47-2.15 (m, 4H), 2.52-2.80 (m, 2H), 4.23-5.82 (m, 3H), 6.73-8.32 (m, 9H); HRMS (FAB): calculated for C₂₉H₂₂NO₇F₆ 610.1300 (M+H)⁺. Found 610.1287 (M+H)⁺.

### Example 61

### 5-({(1S)-1,2,3,4-tetrahydro-1-naphthalenyl[3-(trifluoromethyl)benzyl]amino}carbonyl)-1,2,4-benzenetricarboxylic acid

### Example 61A

### N-[(1S)-1,2,3,4-tetrahydro-1-naphthalenyl]-N-[3-(trifluoromethyl)benzyl]amine

3-(Trifluoromethyl)benzaldehyde and (1S)-1,2,3,4-tetrahydro-1-naphthalenylamine were processed as described in Example 1A to provide the title compound.
¹H NMR (CDCl₃, 300 MHz) δ 1.71-2.10 (m, 4H), 2.63-2.90 (m, 2H), 3.80-4.01 (m, 3H), 7.08-7.75 (m, 8H); MS (ESI+) 306 (M+H)⁺.

### Example 61B

### 5-({(1S)-1,2,3,4-tetrahydro-1-naphthalenyl[3-(trifluoromethyl)benzyl]amino}carbonyl)-1,2,4-benzenetricarboxylic acid

The product from Example 61A and 1,2,4,5-benzenetetracarboxylic dianhydride were processed as described in Example 1B to provide the title compound.
¹H NMR (DMSO-d₆, 400 MHz) δ 1.40-2.15 (m, 4H), 2.55-2.65 (m, 2H), 4.05-5.68 (m, 3H), 6.95-8.38 (m, 10H); HRMS (FAB): calculated for C₂₈H₂₃NO₇F₃ 542.1427 (M+H)⁺. Found 542.1439 (M+H)⁺.

### Example 62

### 5-({(3,5-difluorobenzyl)[(1S)-1,2,3,4-tetrahydro-1-naphthalenyl]amino}carbonyl)-1,2,4-benzenetricarboxylic acid

### Example 62A

### N-(3,5-difluorobenzyl)-N-[(1S)-1,2,3,4-tetrahydro-1-naphthalenyl]amine

3,5-Difluorobenzaldehyde and (1S)-1,2,3,4-tetrahydro-1-naphthalenylamine were processed as described in Example 1A to provide the title compound.
¹H NMR (CDCl₃, 300 MHz) δ 1.72-2.25 (m, 4H), 2.72-2.93 (m, 2H), 3.80-4.01 (m, 3H), 6.63-7.44 (m, 7H); MS (ESI+) 274 (M+H)⁺.

### Example 62B

### 5-({(3,5-difluorobenzyl)[(1S)-1,2,3,4-tetrahydro-1-naphthalenyl]amino}carbonyl)-1,2,4-benzenetricarboxylic acid

The product from Example 62A and 1,2,4,5-benzenetetracarboxylic dianhydride were processed as described in Example 1B to provide the title compound.
¹H NMR (DMSO-d₆,400 MHz) δ 1.45-2.08 (m, 4H), 2.53-2.80 (m, 2H), 3.88-5.65 (m, 3H), 6.75-8.33 (m, 9H); HRMS (FAB): calculated for C₂₇H₂₂NO₇F₂ 510.1364 (M+H)⁺. Found 510.1368 (M+H)⁺.

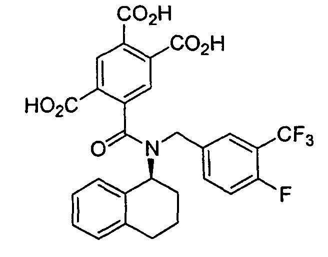

### Example 63

### 5-({[4-fluoro-3-(trifluoromethyl)benzyl][(1S)-1,2,3,4-tetrahydro-1-naphthalenyl]amino}carbonyl)-1,2,4-benzenetricarboxylic acid

### Example 63A

### N-[4-fluoro-3-(trifluoromethyl)benzyl]-N-[(1S)-1,2,3,4-tetrahydro-1-naphthalenyl]amine

4-Fluoro-3-(trifluoromethyl)benzaldehyde and (1S)-1,2,3,4-tetrahydro-1-naphthalenylamine were processed as described in Example 1A to provide the title compound.
¹H NMR (CDCl₃, 300 MHz) δ 1.65-2.11 (m, 4H), 2.71-2.92 (m, 2H), 3.82-3.98 (m, 3H), 7.11-7.75 (m, 7H); MS (ESI+) 324 (M+H)⁺.

### Example 63B

### 5-({[4-fluoro-3-(trifluoromethyl)benzyl][(1S)-1,2,3,4-tetrahydro-1-naphthalenyl]amino}carbonyl)-1,2,4-benzenetricarboxylic acid

The product from Example 63A and 1,2,4,5-benzenetetracarboxylic dianhydride were processed as described in Example 1B to provide the title compound.
¹H NMR (DMSO-d₆, 400 MHz) δ 1.42-2.15 (m, 4H), 2.55-2.80 (m, 2H), 4.08-5.75 (m, 3H), 6.87-8.42 (m, 9H); HRMS (FAB): calculated for C₂₈H₂₂NO₇F₄ 560.1332 (M+H)⁺. Found 560.1326 (M+H)⁺.

### Example 64

### 5-({(3,5-dibromobenzyl)[(1S)-1,2,3,4-tetrahydro-1-naphthalenyl]amino}carbonyl)-1,2,4-benzenetricarboxylic acid

### Example 64A

### N-(3,5-dibromobenzyl)-N-[(1S)-1,2,3,4-tetrahydro-1-naphthalenyl]amine

3,5-Dibromobenzaldehyde and (1S)-1,2,3,4-tetrahydro-1-naphthalenylamine were processed as described in Example 1A to provide the title compound.
¹H NMR (CDCl₃, 300 MHz) δ 1.70-2.07 (m, 4H), 2.66-2.95 (m, 2H), 3.79-3.94 (m, 3H), 7.02-7.62 (m, 7H); MS (ESI+) 396 (M+H)⁺.

### Example 64B

### 5-({(3,5-dibromobenzyl)[(1S)-1,2,3,4-tetrahydro-1-naphthalenyl]amino}carbonyl)-1,2,4-benzenetricarboxylic acid

The product from Example 64A and 1,2,4,5-benzenetetracarboxylic dianhydride were processed as described in Example 1B to provide the title compound.
¹H NMR (DMSO-d₆, 400 MHz) δ 1.40-2.18 (m, 4H), 2.53-2.80 (m, 2H), 3.93-5.68 (m, 3H), 6.87-8.32 (m, 9H); HRMS (FAB): calculated for C₂₇H₂₂NO₇Br₂ 629.9763 (M+H)⁺. Found 629.9766 (M+H)⁺.

### Example 65

### 5-({[3-fluoro-5-(trifluoromethyl)benzyl][(1S)-1,2,3,4-tetrahydro-1-naphthalenyl]amino}carbonyl)-1,2,4-benzenetricarboxylic acid

### Example 65A

### N-[3-fluoro-5-(trifluoromethyl)benzyl]-N-[(1S)-1,2,3,4-tetrahydro-1-naphthalenyl]amine

3-Fluoro-5-(trifluoromethyl)benzaldehyde and (1S)-1,2,3,4-tetrahydro-1-naphthalenylamine were processed as described in Example 1A to provide the title compound.
¹H NMR (CDCl₃, 300 MHz) δ 1.68-2.04 (m, 4H), 2.72-2.91 (m, 2H), 3.85 (t, 1H), 3.91-4.04 (m, 2H), 7.02-7.44 (m, 7H); MS (ESI+) 324 (M+H)⁺.

### Example 65B

### 5-({[3-fluoro-5-(trifluoromethyl)benzyl][(1S)-1,2,3,4-tetrahydro-1-naphthalenyl]amino}carbonyl)-1,2,4-benzenetricarboxylic acid

The product from Example 65A and 1,2,4,5-benzenetetracarboxylic dianhydride were processed as described in Example 1B to provide the title compound.
¹H NMR (DMSO-d₆, 400 MHz) δ 1.40-2.25 (m, 4H), 2.50-2.85 (m, 2H), 3.98-5.78 (m, 3H), 6.85-8.35 (m, 9H); HRMS (FAB): calculated for C₂₈H₂₂NO₇F₄ 560.1332 (M+H)⁺. Found 560.1326 (M+H)⁺.

### Example 66

### 5-({(3,5-dimethoxybenzyl)[(1S)-1,2,3,4-tetrahydro-1-naphthalenyl]amino}carbonyl)-1,2,4-benzenetricarboxylic acid

### Example 66A

### N-(3,5-dimethoxybenzyl)-N-[(1S)-1,2,3,4-tetrahydro-1-naphthalenyl]amine

3,5-Dimethoxybenzaldehyde and (1S)-1,2,3,4-tetrahydro-1-naphthalenylamine were processed as described in Example 1A to provide the title compound.
¹H NMR (CDCl₃, 400 MHz) δ 1.75 (m, 1H), 1.81-1.90 (m, 2H), 2.03 (m, 1H), 2.65-2.91 (m, 2H), 3.79-3.89 (m, 9H), 6.39 (t, 1H), 6.60 (m, 2H), 7.04-7.18 (m, 3H), 7.18 (m, 1H); MS (ESI+) 298 (M+H)⁺.

### Example 66B

### 5-({(3,5-dimethoxybenzyl)[(1S)-1,2,3,4-tetrahydro-1-naphthalenyl]amino}carbonyl)-1,2,4-benzenetricarboxylic acid

The product from Example 66A and 1,2,4,5-benzenetetracarboxylic dianhydride were processed as described in Example 1B to provide the title compound.
¹H NMR (DMSO-d₆, 400 MHz) δ 1.35-2.15 (m, 4H), 2.55-2.70 (m, 2H), 3.63-3.73 (m, 6H), 4.05-5.50 (m, 3H), 6.17-8.28 (m, 9H); HRMS (FAB): calculated for C₂₉H₂₈NO₉ 534.1764 (M+H)⁺. Found 534.1758 (M+H)⁺.

### Example 67

### 5-({(2,3-dichlorobenzyl)[(1S)-1,2,3,4-tetrahydro-1-naphthalenyl]amino}carbonyl)-1,2,4-benzenetricarboxylic acid

### Example 67A

### N-(2,3-dichlorobenzyl)-N-[(1S)-1,2,3,4-tetrahydro-1-naphthalenyl]amine

2,3-Dichlorobenzaldehyde and (1S)-1,2,3,4-tetrahydro-1-naphthalenylamine were processed as described in Example 1A to provide the title compound.
¹H NMR (CDCl₃, 300 MHz) δ 1.72-2.11 (m, 4H), 2.66-2.92 (m, 2H), 3.82 (t, 1H), 3.94-4.06 (m, 2H), 7.04-7.45 (m, 7H); MS (ESI+) 306 (M+H)⁺.

### Example 67B

### 5-({(2,3-dichlorobenzyl)[(1S)-1,2,3,4-tetrahydro-1-naphthalenyl]amino}carbonyl)-1,2,4-benzenetricarboxylic acid

The product from Example 67A and 1,2,4,5-benzenetetracarboxylic dianhydride were processed as described in Example 1B to provide the title compound.
¹H NMR (DMSO-d₆, 400 MHz) δ 1.45-2.25 (m, 4H), 2.51-2.70 (m, 2H), 3.95-5.77 (m, 3H), 7.05-8.32 (m, 9H); HRMS (FAB): calculated for C₂₇H₂₂NO₇Cl₂ 542.0773 (M+H)⁺. Found 542.0770 (M+H)⁺.

### Example 68

### 5-({(2,4-dichlorobenzyl)[(1S)-1,2,3,4-tetrahydro-1-naphthalenyl]amino}carbonyl)-1,2,4-benzenetricarboxylic acid

### Example 68A

### N-(2,4-dichlorobenzyl)-N-[(1S)-1,2,3,4-tetrahydro-1-naphthalenyl]amine

2,4-Dichlorobenzaldehyde and (1S)-1,2,3,4-tetrahydro-1-naphthalenylamine were processed as described in Example 1A to provide the title compound.
¹H NMR (CDCl₃, 300 MHz) δ 1.64-2.10 (m, 4H), 2.65-2.90 (m, 2H), 3.79 (t, 1H), 3.85-4.02 (m, 2H), 7.03-7.48 (m, 7H); MS (ESI+) 306 (M+H)⁺.

### Example 68B

### 5-({(2,4-dichlorobenzyl)[(1S)-1,2,3,4-tetrahydro-1-naphthalenyl]amino}carbonyl)-1,2,4-benzenetricarboxylic acid

The product from Example 68A and 1,2,4,5-benzenetetracarboxylic dianhydride were processed as described in Example 1B to provide the title compound.
¹H NMR (DMSO-d₆, 400 MHz) δ 1.40-2.25 (m, 4H), 2.53-2.70 (m, 2H), 3.95-5.75 (m, 3H), 7.05-8.32 (m, 9H); HRMS (FAB): calculated for C₂₇H₂₂NO₇Cl₂ 542.0773 (M+H)⁺. Found 542.0767 (M+H)⁺.

### Example 69

### 5-({(3,5-dimethylbenzyl)[(1S)-1,2,3,4-tetrahydro-1-naphthalenyl]amino}carbonyl)-1,2,4-benzenetricarboxylic acid

### Example 69A

### N-(3,5-dimethylbenzyl)-N-[(1S)-1,2,3,4-tetrahydro-1-naphthalenyl]amine

3,5-Dimethylbenzaldehyde and (1S)-1,2,3,4-tetrahydro-1-naphthalenylamine were processed as described in Example 1A to provide the title compound.
¹H NMR (CDCl₃, 300 MHz) δ 1.62-2.08 (m, 4H), 2.30 (s, 6H), 2.83-2.95 (m, 2H), 3.71-3.90 (m, 3H), 6.93-7.18 (m, 7H); MS (ESI+) 266 (M+H)⁺.

### Example 69B

### 5-({(3,5-dimethylbenzyl)[(1S)-1,2,3,4-tetrahydro-1-naphthalenyl]amino}carbonyl)-1,2,4-benzenetricarboxylic acid

The product from Example 69A and 1,2,4,5-benzenetetracarboxylic dianhydride were processed as described in Example 1B to provide the title compound.
¹H NMR (DMSO-d₆, 400 MHz) δ 1.85-2.05 (m, 4H), 2.15-2.23 (m, 6H), 2.50-2.75 (m, 2H), 3.75-5.83 (m, 3H), 6.50-8.30 (m, 9H); HRMS (FAB): calculated for C₂₉H₂₈NO₇ 502.1866 (M+H)⁺, Found 502.1874 (M+H)⁺.

### Example 70

### 5-[((1S)-1,2,3,4-tetrahydro-1-naphthalenyl{3-[(trifluoromethyl)sulfanyl]benzyl}amino)carbonyl]-1,2,4-benzenetricarboxylic acid

### Example 70A

### N-[(1S)-1,2,3,4-tetrahydro-1-naphthalenyl]-N-{3-[(trifluoromethyl)sulfanyl]benzyl}amine

3-[(Trifluoromethyl)sulfanyl]benzaldehyde and (1S)-1,2,3,4-tetrahydro-1-naphthalenylamine were processed as described in Example 1A to provide the title compound.
¹H NMR (CDCl₃, 300 MHz) δ 1.65-2.04 (m, 4H), 2.63-2.90 (m, 2H), 3.80 (t, 1H), 3.84-4.02 (m, 2H), 7.03-7.78 (m, 8H); MS (ESI+) 338 (M+H)⁺.

### Example 70B

### 5-[((1S)-1,2,3,4-tetrahydro-1-naphthalenyl{3-[(trifluoromethyl)sulfanyl]benzyl}amino)carbonyl]-1,2,4-benzenetricarboxylic acid

The product from Example 70A and 1,2,4,5-benzenetetracarboxylic dianhydride were processed as described in Example 1B to provide the title compound.
¹H NMR (DMSO-d₆, 400 MHz) δ 1.40-2.15 (m, 4H), 2.52-2.80 (m, 2H), 3.95-5.65 (m, 3H), 6.95-8.15 (m, 10H); HRMS (FAB): calculated for C₂₈H₂₃NO₇F₃S 574.1147 (M+H)⁺. Found 574.1128 (M+H)⁺.

### Example 71

### 5-({[3-(phenlsulfanyl)benzyl][(1S)-1,2,3,4-tetrahydro-1-naphthalenyl]amino}carbonyl)-1,2,4-benzenetricarboxylic acid

### Example 71A

### 3-(phenylsulfanyl)benzaldehyde

2-(3-Bromophenyl)-1,3-dioxolane (1.00 mL, 6.61 mmol) in THF (10 mL) was treated with 1.6M n-BuLi in hexanes (4.34 mL, 6.94 mmol) at -78 °C. After stirring for 1.0 minutes, the mixture was treated with diphenyl disulfide (1.44 g, 6.61 mmol) and the reaction mixture was stirred overnight without replenishing the cooling bath. The reaction mixture was quenched with 20 mL of water and extracted with diethyl ether (3X 50 mL.) The organic layers were combined, dried over MgSO₄, filtered and the filtrate concentrated. The crude oil was stirred in 25 mL of a 5:1 solution of CH₃CN/1N HCl overnight. The reaction mixture was diluted with 50 mL of brine and extracted with diethyl ether .(3X 50mL.) The organic layers were combined, dried over MgSO₄, filtered and the filtrate concentrated to provide the title compound which was used without further purification.

### Example 71B

### N-[3-(phenylsulfanyl)benzyl]-N-[(1S)-1,2,3,4-tetrahydro-1-naphthalenyl]amine

The product from Example 71A and (1S)-1,2,3,4-tetrahydro-1-naphthalenylamine were processed as described in Example 1A to provide the title compound as a clear oil. (1.06 g, 46%).
¹H NMR (CDCl₃, 300 MHz) δ 1.75-2.04 (m, 4H), 2.61-2.83 (m, 2H), 3.72-3.97 (m, 3H), 7.02-7.41 (m, 13H); MS (ESI+) 346 (M+H)⁺.

### Example 71C

### 5-({[3-(phenylsulfanyl)benzyl][(1S)-1,2,3,4-tetrahydro-1-naphthalenyl]amino}carbonyl)-1,2,4-benzenetricarboxylic acid

The product from Example 71B and 1,2,4,5-benzenetetracarboxylic dianhydride were processed as described in Example 1B to provide the title compound.
¹H NMR (DMSO-d₆, 400 MHz) δ 1.35-2.05 (m, 4H), 2.53-2.65 (m, 2H), 3.80-5.55 (m, 3H), 6.85-8.37 (m, 15H); HRMS (FAB): calculated for C₃₃H₂₈NO₇S 582.1586 (M+H)⁺. Found 582.1575 (M+H)⁺.

### Example 72

### 5-({{3-[(4-methoxyphenyl)sulfanyl]benzyl}[(1S)-1,2,3,4-tetrahydro-1-naphthalenyl]amino}carbonyl)-1,2,4-benzenetricarboxylic acid

### Example 72A

### 3-[(4-methoxyphenyl)sulfanyl]benzaldehyde

2-(3-Bromophenyl)-1,3-dioxolane and bis(4-methoxyphenyl) disulfide were processed as described in Example 71A to provide the title compound.

### Example 72B

### N-{3-[(4-methoxyphenyl)sulfanyl]benzyl}-N-[(1S)-1,2,3,4-tetrahydro-1-naphthalenyl]amine

The product from Example 72A and (1S)-1,2,3,4-tetrahydro-1-naphthalenylamine were processed as described in Example 1A to provide the title compound.
¹H NMR (CDCl₃, 300 MHz) δ 1.62-2.04 (m, 4H), 2.62-2.84 (m, 6H), 6.90-7.43 (m, 12H); MS (ESI+) 376 (M+H)⁺.

### Example 72C

### 5-({{3-[(4-methoxyphenyl)sulfanyl]benzyl}[(1S)-1,2,3,4-tetrahydro-1-naphthalenyl]amino}carbonyl)-1,2,4-benzenetricarboxylic acid

The product from Example 72B and 1,2,4,5-benzenetetracarboxylic dianhydride were processed as described in Example 1B to provide the title compound.
¹H NMR (DMSO-d₆, 400 MHz) δ 1.40-2.03 (m, 4H), 2.43-2.65 (m, 2H), 3.86-5.52 (m, 6H), 6.71-8.31 (m, 14H); HRMS (FAB): calculated for C₃₄H₃₀NO₈S 611.1614 (M+H)⁺. Found 611.1628. (M+H)⁺.

### Example 73

### 5-({{3-[(4-nitrophenyl)sulfanyl]benzyl}[(1S)-1,2,3,4-tetrahydro-1-naphthalenyl]amino}carbonyl)-1,2,4-benzenetricarboxylic acid

### Example 73A

### 3-[(4-nitrophenyl)sulfanyl]benzaldehyde

2-(3-Bromophenyl)-1,3-dioxolane and bis(4-nitrophenyl) disulfide were processed as described in Example 71A to provide the title compound.

### Example 73B

### N-{3-[(4-nitrophenyl)sulfanyl]benzyl}-N-[(1S)-1,2,3,4-tetrahydro-1-naphthalenyl]amine

The product from Example 73A and (1S)-1,2,3,4-tetrahydro-1-naphthalenylamine were processed as described in Example 1A to provide the title compound.
¹H NMR (CDCl₃, 300 MHz) δ 1.62-2.02 (m, 4H), 2.82-2.91 (m, 2H), 3.81 (t, 1H), 3.84-4.00 (m, 2H), 7.06-8.09 (m, 12H); MS (ESI+) 391 (M+H)⁺.

### Example 73C

### 5-({{3-[(4-nitrophenyl)sulfanyl]benzyl}[(1S)-1,2,3,4-tetrahydro-1-naphthalenyl]amino}carbonyl)-1,2,4-benzenetricarboxylic acid

The product from Example 73B and 1,2,4,5-benzenetetracarboxylic dianhydride were processed as described in Example 1B to provide the title compound.
¹H NMR (DMSO-d₆, 400 MHz) δ 1.60-2.08 (m, 4H), 2.41-2.69 (m, 2H), 3.98-5.63 (m, 3H), 7.02-8.39 (m, 14H); HRMS (FAB): calculated for C₃₃H₂₇N₂O₉S 627.1437 (M+H)⁺. Found 627.1451 (M+H)⁺.

### Example 74

### 5-({[3-(methylsulfanyl)benzyl][(1S)-1,2,3,4-tetrahydro-1-naphthalenyl]amino}carbonyl)-1,2,4-benzenetricarboxylic acid

### Example 74A

### 3-(methylsulfanyl)benzaldehyde

2-(3-Bromophenyl)-1,3-dioxolane and dimethyl disulfide were processed as described in Example 71 A to provide the title compound.

### Example 74B

### N-[3-(methylsulfanyl)benzyl]-N-[(1S)-1,2,3,4-tetrahydro-1-naphthalenyl]amine

The product from Example 74A and (1S)-1,2,3,4-tetrahydro-1-naphthalenylamine were processed as described in Example 1A to provide the title compound.
¹H NMR (CDCl₃, 300 MHz) δ 1.61-2.04 (m, 4H), 2.45 (s, 3H), 2.65-2.82 (m, 2H), 3.79-3.97 (m, 2H), 7.02-7.39 (m, 8H); MS (ESI+) 284 (M+H)⁺.

### Example 74C

### 5-({[3-(methylsulfanyl)benzyl][(1S)-1,2,3,4-tetrahydro-1-naphthalenyl]amino}carbonyl)-1,2,4-benzenetricarboxylic acid

The product from Example 74B and 1,2,4,5-benzenetetracarboxylic dianhydride were processed as described in Example 1B to provide the title compound.
¹H NMR (DMSO-d₆, 400 MHz) δ 1.45-2.09 (m, 4H), 2.50-2.68 (m, 2H), 3.87-5.50 (m, 3H), 6.81-8.27 (m, 10H); HRMS (FAB): calculated for C₂₈H₂₆NO₇S 520.1430 (M+H)⁺. Found 5420.1409 (M+H)⁺.

### Example 75

### 5-({{3-[(4-chlorophenyl)sulfanyl]benzyl}[(1S)-1,2,3,4-tetrahydro-1-naphthalenyl]amino}carbonyl)-1,2,4-benzenetricarboxylic acid

### Example 75A

### 3-[(4-chlorophenyl)sulfanyl]benzaldehyde

2-(3-Bromophenyl)-1,3-dioxolane and bis(4-chlorophenyl) disulfide were processed as described in Example 71A to provide the title compound.

### Example 75B

### N-{3-[(4-chlorophenyl)sulfanyl]benzyl}-N-[(1S)-1,2,3,4-tetrahydro-1-naphthalenyl]amine

The product from Example 75A and (1S)-1,2,3,4-tetrahydro-1-naphthalenylamine were processed as described in Example 1A to provide the title compound.
¹H NMR (CDCl₃, 300 MHz) δ 1.65-2.07 (m, 4H), 2.69-2.83 (m, 2H), 3.75-3.96 (m, 3H), 7.02-7.41 (m, 12H); MS (ESI+) 380 (M+H)⁺.

### Example 75C

### 5-({{3-[(4-chlorophenyl)sulfanyl]benzyl}[(1S)-1,2,3,4-tetrahydro-1-naphthalenyl]amino}carbonyl)-1,2,4-benzenetricarboxylic acid

The product from Example 75B and 1,2,4,5-benzenetetracarboxylic dianhydride were processed as described in Example 1B to provide the title compound.
¹H NMR (DMSO-d₆, 400 MHz) δ 1.42-1.99 (m, 4H), 2.50-2.64 (m, 2H), 3.90-5.53 (m, 3H), 6.91-8.28 (m, 14H); HRMS (FAB): calculated for C₃₃H₂₇NO₇C1S 616.1197 (M+H)⁺. Found 616.1181 (M+H)⁺.

### Example 76

### 5-({{3-[(methoxyimino)methyl]benzyl}[(1S)-1,2,3,4-tetrahydro-1-naphthalenyl]amino}carbonyl)-1,2,4-benzenetricarboxylic acid

### Example 76A

### methyl 3-formylbenzoate

3-Formylbenzoic acid (3.11 g, 20.7 mmol, Aldrich) in 10:1 1 methanol:water (110 mL) was treated with Cs₂CO₃ (3.11 g, 10.4 mmol). After stirring 18 hours, the solvent was evaporated under reduced pressure and the residue was dried under reduced pressure at 60 °C. The residue was suspended in DMF (40 mL) and treated with iodomethane (2.58 mL, 41.4 mmol). After stirring at ambient temperature 2 hours, the mixture was poured into water and extracted with diethyl ether. The ether extracts were combined, washed with water, saturated NaHCO₃, dried (MgSO₄), filtered and the filtrate concentrated under reduced pressure to provide the title compound as a white solid (2.81 g).
¹H NMR (300 MHz, CDCl₃) δ 10.09 (s, 1H), 8.52-8.55 (m, 1H), 8.28-8.33 (m, 1H), 8.05-8.12 (m, 1H), 7.6-7.67 (m, 1H), 3.98 (s, 3H); MS (DCI/NH₃) 182 (M+NH₄)⁺.

### Example 76B

### methyl 3- {[(1S)-1,2,3,4-tetrahydro-1-naphthalenylamino]methyl}benzoate

The product from Example 76A (2.80 g, 17.1 mmol) in methanol (75 mL) was treated with (1S)-1,2,3,4-tetrahydro-1-naphthalenylamine (2.45 mL, 17.1 mmol, Lancaster). After stirring for 18 hours, the mixture was treated with sodium borohydride and the mixture was allowed to stir an additional 4 hours. The volatiles were evaporated under reduced pressure and the residue was dissolved in diethyl ether and quenched with 1N NaOH solution. The separated organic phase was washed with water, brine, dried (Na₂SO₄), filtered and the filtrate concentrated under reduced pressure to provide the title compound as a colorless oil (4.99 g).
¹H NMR (300 MHz, CDCl₃) δ 8.05-8.08 (m, 1H), 7.9-7.95 (m, 1H), 7.6-7.65 (m, 1H), 7.3-7.45 (m, 2H), 7.05-7.2 (m, 3H), 3.78-4.03 (m, 6H), 2.65-2.9 (m, 2H), 1.65-2.1 (m, 4H); MS (DCI/NH₃) 296 (M+H)⁺.

### Example 76C

### (3-{[(1S)-1,2,3,4-tetrahydro-1-naphthalenylamino]methyl}phenyl)methanol

The product from Example 76B (4.99 g, 16.9 mmol) in anhydrous THF (100 mL) was treated with lithium aluminum hydride (16.9 mL, 16.9 mmol, 1M solution in THF) and heated at reflux for 1.5 hours. The reaction mixture was cooled to 0 °C and quenched by careful addition of Na₂SO₄.10H₂O. The mixture was stirred for 30 minutes and then treated with MgSO₄, filtered through celite and the filtrate was concentrated under reduced pressure to provide the title compound as a colorless oil (4.23 g).
¹H NMR (300 MHz, CDCl₃) δ 7.05-7.45 (m, 8H), 4.7 (s, 2H), 3.68-4.0 (m, 3H), 2.65-2.9 (m, 2H), 1.4-2.1 (m, 6H); MS (DCI/NH₃) 268 (M+H)⁺.

### Example 76D

### 5-({[3-(hydroxymethyl)benzyl][(1S)-1,2,3,4-tetrahydro-1-naphthalenyl]amino}carbonyl)-1,2,4-benzenetricarboxylic acid

The product from Example 76C (1.27 g, 4.75 mmol) and diisopropylethylamine (2.41 mL, 14.2 mmol) in anhydrous THF (40 mL) was treated with 1,2,4,5-benzenetetracarboxylic dianhydride (3.11 g, 14.2 mmol) in one portion at -78 °C. The mixture was allowed to gradually warm to room temperature over 16 hours. The mixture was treated with 1M LiOH solution (40 mL) and stirred vigorously 24 hours. The mixture was acidified and extracted with ethyl acetate. The organic extracts were combined, washed with 1N HCl, brine, dried (Na₂SO₄), filtered and the filtrate concentrated under reduced pressure. The residue was purified by flash chromatography (silica gel, 100:1:1 ethyl acetate:formic acid:water) to provide the title compound as a white solid (2.31 g).

### Example 76E

### 5-({(3-formylbenzyl)[(1S)-1,2,3,4-tetrahydro-1-naphthalenyl]amino}carbonyl)-1,2,4-benzenetricarboxylic acid

The product from Example 76D (2.3 g, 4.6 mmol) and triethylamine (4.48 mL, 32.1 mmol) in DMSO (25 mL) was treated with sulfur trioxide pyridine complex (2.92 g, 18.4 mmol). After 5 hours, the mixture was partitioned between ethyl acetate and 1N HCl. The separated organic phase was washed with 1N HCl, brine, and concentrated under reduced pressure. The residue was dissolved in THF (30 mL) and saturated aqueous Na₂CO₃ solution (30 mL) was added. After stirring vigorously for 1 hour, the separated aqueous layer was acidified and extracted with ethyl acetate. The organic extracts were combined, washed with brine, dried (Na₂SO₄), filtered and the filtrate concentrated under reduced pressure to provide the title compound as a pale yellow solid (1.73 g).

### Example 76F

### 5-({{3-[(methoxyimino)methyl]benzyl}[(1S)-1,2,3,4-tetrahydro-1-naphthalenyl]amino}carbonyl)-1,2,4-benzenetricarboxylic acid

The product from Example 76E (132 mg, 0.26 mmol) in pyridine (5 mL) was treated with O-methylhydroxylamine hydrochloride (26 mg, 0.32 mmol). After stirring for 1 hour at room temperature, the volatiles were evaporated under reduced pressure. The residue was dissolved in ethyl acetate and washed with 1N HCl, water, brine, dried (Na₂SO₄), filtered and the filtrate concentrated under reduced pressure. The residue was purified by flash chromatography (silica gel, 100:1:1 ethyl acetate:formic acid:water) to provide the title compound as a white solid (43 mg, 31%).
¹H NMR (300 MHz, DMSO-d₆) δ 7.0-8.35 (m, 11H), 3.8-5.6 (m, 6H), 2.55-2.75 (m, 2H), 1.3-2.1 (4H); MS (ESI-) 529 (M-H)⁻.

### Example 77

### 5-({{3-[(E)-(tert-butoxyimino)methyl]benzyl}[(1S)-1,2,3,4-tetrahydro-1-naphthalenyl]amino}carbonyl)-1,2,4-benzenetricarboxylic acid

The product from Example 76E and O-(tert-butyl)hydroxylamine were processed as described in Example 76F to provide the title compound.
¹H NMR (300MHz, DMSO-d₆) δ 7.0-8.3 (m, 11H), 3.85-5.6 (m, 3H), 2.55-2.7 (m, 2H), 1.35-2.1 (4H), 1.3 (s, 9H); MS (ESI+)573 (M+H)⁺.

### Example 78

### 5-({{3-[(isouropoxyimino)methyl]benzyl}[(1S)-1,2,3,4-tetrahydro-1-naphthalenyl]amino}carbonyl)-1,2,4-benzenetricarboxylic acid

The product from Example 76E and O-isopropylhydroxylamine were processed as described in Example 76F to provide the title compound.
¹H NMR (300MHz, DMSO-d₆) δ 7.0-8.3 (m, 11H), 3.8-5.6 (m, 5H), 2.55-2.7 (m, 2H), 1.3-2.15 (m, 5H), 0.92, (d, 6H); MS (ESI+) 573 (M+H)⁺.

### Example 79

### 5-{[(1S)-1,2,3,4-tetrahydro-1-naphthalenyl(3-{[(tetrahydro-2H-pryran-2-yloxy)imino]methyl}benzyl)amino]carbonyl}-1,2,4-benzenetricarboxylic acid

The product from Example 76E and O-tetrahydro-2H-pyran-2-ylhydroxylamine were processed as described in Example 76F to provide the title compound.
¹H NMR (300MHz, DMSO-d₆) δ 7.0-8.35 (m, 11H), 3.4-5.6 (m, 6H), 2.55-2.75 (m, 2H), 1.3-2.1 (m, 10H); MS (ESI-) 599 (M-H)⁻.

### Example 80

### 5-({{3-[(phenoxyimino)methyl]benzyl}[(1S)-1,2,3,4-tetrahydro-1-naphthalenyl]amino}carbonyl)-1,2,4-benzenetricarboxylic acid

The product from Example 76E and O-phenylhydroxylamine were processed as described in Example 76F to provide the title compound.
¹H NMR (300MHz, DMSO-d₆) δ 7.0-8.7 (m, 16H), 3.9-5.7 (m, 3H), 2.55-2.75 (m, 2H), 1.35-2.15 (m, 4H); MS (ESI-) 591 (M-H)⁻.

### Example 81

### 5-({(3-{[(benzyloxy)imino]methyl}benzyl)[(1S)-1,2,3,4-tetrahydro-1-naphthalenyl]amino}carbonyl)-1,2,4-benzenetricarboxylic acid

The product from Example 76E and O-benzylhydroxylamine were processed as described in Example 76F to provide the title compound.
¹H NMR (300MHz, DMSO-d₆) δ 7.0-8.3 (m, 16H), 3.85-5.6 (m, 5H), 2.55-2.75 (m, 2H), 1.3-2.15 (m, 4H); MS (ESI-) 605 (M-H)⁻.

### Example 82

### 5-({[3-({[(4-nitrobenzyl)oxy]imino}methyl)benzyl][(1S)-1,2,3,4-tetrahydro-1-naphthalenyl]amino}carbonyl)-1,2,4-benzenetricarboxylic acid

The product from Example 76E and O-(4-nitrobenzyl)hydroxylamine were processed as described in Example 76F to provide the title compound.
¹H NMR (300MHz, DMSO-d₆) δ 7.0-8.4 (m, 15H), 3.85-5.6 (m, 5H), 2.5-2.7 (m, 2H), 1.3-2.1 (m, 4H); MS (ESI-) 650 (M-H)⁻.

### Example 83

### 5-({[3-({[(2,3,4,5,6-pentafluorobenzyl)oxy]imino}methyl)benzyl][(1S)-1,2,3,4-tetrahydro-1-naphthalenyl]amino}carbonyl)-1,2,4-benzenetricarboxylic acid

The product from Example 76E and O-(2,3,4,5,6-pentafluorobenzyl)hydroxylamine were processed as described in Example 76F to provide the title compound.
¹H NMR (300MHz, DMSO-d₆) δ 7.0-8.3 (m, 11H), 3.8-5.7 (m, 5H), 2.5-2.7 (m, 2H), 1.35-2.2 (m, 4H); MS (ESI+) 697 (M+H)⁺.

### Example 84

### 5-({{[5-(ethoxycarbonyl)-2-thienyl]methyl}[(1S)-1,2,3,4-tetrahydro-1-naphthalenyl]amino}carbonyl)-1,2,4-benzenetricarboxylic acid

### Example 84A

### methyl 5-formyl-2-thiophenecarboxylate

5-Formyl-2-thiophenecarboxylic acid, purchased from Lancaster, was processed as described in Example 76A to provide the title compound.

### Example 84B

### ethyl 5- {[(1S)-1,2,3,4-tetrahydro-1-naphthalenylamino]methyl}-2-thiophenecarboxylate

The product from Example 84A in ethanol and (1S)-1,2,3,4-tetrahydro-1-naphthalenylamine were processed as described in Example 76B to provide the title compound.
¹H NMR (DMSO-d₆) δ 7.65 (d,1H); 7.45 (m, 1H); 7.15 (m, 2H); 7.05 (m, 2H); 4.25 (q, 2H); 4.0 (d, 2H); 3.7 (m, 1H); 2.55-2.8 (m, 2H); 1.55-2.0 (m, 4H); 1.28 (t, 3H); MS (ESI+) 316 (M+H)⁺.

### Example 84C

### 5-({{[5-(ethoxycarbonyl)-2-thienyl]methyl}[(1S)-1,2,3,4-tetrahydro-1-naphthalenyl]amino}carbonyl)-1,2,4-benzenetricarboxylic acid

The product from Example 84B and 1,2,4,5-benzenetetracarboxylic dianhydride were processed as described in Example 1B to provide the title compound.
¹H NMR (DMSO-d₆) δ 6.99-8.3 (m, 8H); 4.25 (q, 2H); 4.1-4.99 (m, 3H); 2.6-2.8 (m, 2H); 1.7-1.85 (m, 2H); 1.4-1.55 (m, 2H); 1.3 (t, 3H); MS (ESI-) 550 (M-H)⁻, (ESI+) 552 (M+H)⁺.

### Example 85

### 5-({[(5'-fluoro-2'-methoxy[1,1'-biphenyl]-3-yl)methyl][(1S)-1,2,3,4-tetrahydro-1-naphthalenyl]amino}carbonyl)-1,2,4-benzenetricarboxylic acid

The product from Example 15B (165 mg, 0.3 mmol), 5-fluoro-2-methoxyphenylboronic acid (68 mg, 0.4 mmol) and tetrakis(triphenylphosphine)palladium(0) (17 mg, 0.015 mmol) in THF (10 ml) were treated with 3M aqueous sodium carbonate (0.4 mL) and heated at reflux for 16 hours. After cooling to room temperature, the reaction mixture was diluted with water, acidified with 1N hydrochloric acid and extracted with diethyl ether. The organic layers were combined, dried with magnesium sulfate, filtered and the filtrate concentrated. The residue was purified by a Prep Nova Pak (HRC18) column using 0.1% trifluoroacetic acid:acetonitrile (10:90 to 95:5) as eluant to provide the title compound (50 mg).
¹H NMR (DMSO-d₆) δ 8.27-6.96 (m, 13H), 5.00-3.82 (m, 3H), 3.75, 3.70 (2 s, 3H), 2.74-2.53 (m, 2H), 2.20-1.39 (m, 4H); MS (ESI+) 598 (M+H)⁺.

### Example 86

### 5-({[(5'-chloro-2'-methoxy[1,1'-biphenyl]-3-yl)methyl][(1S)-1,2,3,4-tetrahydro-1-naphthalenyl]amino}carbonyl)-1,2,4-benzenetricarboxylic acid

The product from Example 15B (165 mg, 0.3 mmol) and 5-chloro-2-methoxyphenylboronic acid were processed as described in Example 85 to provide the title compound.
¹H NMR (DMSO-d₆) 8.28-7.03 (m, 13H), 5.00-3.80 (m, 3H), 3.76 and 3.50 (2s, 3H), 2.75-2.55 (m, 2H), 2.10-1.33 (m, 4H); MS (ESI+) 614 (M+H)⁺.

### Example 87

### 5-({[(3',5'-dichloro[1,1'-biphenyl]-3-yl)methyl][(1S)-1,2,3,4-tetrahydro-1-naphthalenyl]amino}carbonyl)-1,2,4-benzenetricarboxylic acid

The product from Example 15B (165 mg, 0.3 mmol) and 3,5-dichlorophenylboronic acid were processed as described in Example 85 to provide the title compound.
¹H NMR (DMSO-d₆) δ 8.28-7.03 (m, 13H), 5.10-3.80 (m, 3H), 2.75-2.55 (m, 2H), 2.05-1.33 (m, 4H); MS (ESI+) 618 (M+H)⁺.

### Example 88

### 5-({[(2'-methoxy[1,1'-biphenyl]-3-yl)methyl][(1S)-1,2,3,4-tetrahydro-1-naphthalenyl]amino}carbonyl)-1,2,4-benzenetricarboxylic acid

The product from Example 15B (165 mg, 0.3 mmol) and 2-methoxyphenylboronic acid were processed as described in Example 85 to provide the title compound.
¹H NMR (DMSO-d₆) δ 8.28-6.95 (m, 14H), 5.04-3.80 (m, 3H), 3.76 and 3.72 (2s, 3H), 2.70-2.55 (m, 2H), 2.20-1.35 (m, 4H); MS (ESI+) 580 (M+H)⁺.

### Example 89

### 5-({[(2'-chloro[1,1'-biphenyl]-3-yl)methyl][(1S)-1,2,3,4-tetrahydro-1-naphthalenyl]amino}carbonyl)-1,2,4-benzenetricarboxylic acid

The product from Example 15B (165 mg, 0.3 mmol) and 2-chlorophenylboronic acid were processed as described in Example 85 to provide the title compound.
¹H NMR (DMSO-d₆) 8.28-7.00 (m, 14H), 5.04-3.90 (m, 3H), 2.76-2.55 (m, 2H), 2.20-1.35 (m, 4H); MS (ESI+) 584 (M+H)⁺.

### Example 90

### 5-({[(2',5'-dimethoxy[1,1'-biphenyl]-3-yl)methyl][(1S)-1,2,3,4-tetrahydro-1-naphthalenyl]amino}carbonyl)-1,2,4-benzenetricarboxylic acid

The product from Example 15B (165 mg, 0.3 mmol) and 2,5-dimethoxyphenylboronic acid were processed as described in Example 85 to provide the title compound.
¹H NMR (DMSO-d₆) δ 8.28-6.75 (m, 13H), 5.00-3.80 (m, 3H), 3.72, 3.68 and 3.63 (3s, 6H), 2.70-2.55 (m, 2H), 2.10-1.40 (m, 4H); MS (ESI+) 610 (M+H)⁺.

### Example 91

### 5-({[(3'-chloro[1,1'-biphenyl]-3-yl)methyl][(1S)-1,2,3,4-tetrahydro-1-naphthalenyl]amino}carbonyl)-1,2,4-benzenetricarboxylic acid

The product from Example 15B (165 mg, 0.3 mmol) and 3-chlorophenylboronic acid were processed as described in Example 85 to provide the title compound.
¹H NMR (DMSO-d₆) δ 8.65-7.00 (m, 14H), 5.00-4.90 (m, 1H), 4.78-4.67 (m, 1H), 4.00-3.80 (m, 1H), 2.65-2.37 (m, 2H), 2.00-1.40 (m, 4H); MS (ESI+) 584 (M+H)⁺.

### Example 92

### 5-({[(3',4'-dichloro[1,1'-biphenyl]-3-yl)methyl][(1S)-1,2,3,4-tetrahydro-1-naphthalenyl]amino}carbonyl)-1,2,4-benzenetricarboxylic acid

The product from Example 15B (165 mg, 0.3 mmol) and 3,4-dichlorophenylboronic acid were processed as described in Example 85 to provide the title compound.
¹H NMR (DMSO-d₆) δ 8.70-7.00 (m, 13H), 5.02-4.66 (m, 2H), 3.94-3.88 (m, 1H), 2.64-2.38 (m, 2H), 2.00-1.35 (m, 4H); MS (ESI+) 618 (M+H)⁺.

### Example 93

### 5-({[(3'-chloro-4'-fluoro[1,1'-biphenyl]-3-yl)methyl][(1S)-1,2,3,4-tetrahydro-1-naphthalenyl]amino}carbonyl)-1,2,4-benzenetricarboxylic acid

The product from Example 15B (165 mg, 0.3 mmol) and 3-chloro-4-fluorophenylboronic acid were processed as described in Example 85 to provide the title compound.
¹H NMR (DMSO-d₆) δ 8.71-6.99 (m, 13H), 5.04-4.67 (m, 2H), 3.82-3.93 (m, 1H), 2.62-2.36 (m, 2H), 2.05-1.22 (m, 4H); MS (ESI+) 602 (M+H)⁺.

### Example 94

### 5-({[(3'-nitro[1,1'-biphenyl]-3-yl)methyl][(1S)-1,2,3,4-tetrahydro-1-naphthalenyl]amino}carbonyl)-1,2,4-benzenetricarboxylic acid

The product from Example 15B (165 mg, 0.3 mmol) and 3-nitrophenylboronic acid were processed as described in Example 85 to provide the title compound.
¹H NMR (DMSO-d₆) δ 8.75-7.00 (m, 14H), 5.05-4.85 (m, 1H), 4.70-4.80 (m, 1H), 3.90-4.00 (m, 1H), 2.70-2.34 (m, 2H), 2.10-1.20 (m, 4H); MS (ESI+) 595 (M+H)⁺.

### Example 95

### 5-({[(3'-methoxy[1,1'-biphenyl]-3-yl)methyl][(1S)-1,2,3,4-tetrahydro-1-naphthalenyl]amino}carbonyl)-1,2,4-benzenetricarboxylic acid

The product from Example 15B (165 mg, 0.3 mmol) and 3-methoxyphenylboronic acid were processed as described in Example 85 to provide the title compound.
¹H NMR (DMSO-d₆) δ 8.70-6.85 (m, 14H), 5.004.92 (t, 1H), 4.66-4.80 (m, 1H), 4.00-3.70 (m, 4H), 2.69-2.40 (m, 2H), 2.04-1.23 (m, 4H); MS (ESI+)580 (M+H)⁺.

### Example 96

### 5-({(1S)-1,2,3,4-tetrahydro-1-naphthalenyl[3-(3-thienyl)benzyl]amino}carbonyl)-1,2,4-benzenetricarboxylic acid

The product from Example 15B (165 mg, 0.3 mmol) and 3-(3-thienyl)phenylboronic acid were processed as described in Example 85 to provide the title compound.
¹H NMR (DMSO-d₆) δ 8.32-6.95 (m, 13H), 5.55-4.60 (m, 2H), 4.41-4.22 (m, 1H), 2.70-2.50 (m, 2H), 2.18-1.39 (m, 4H); MS (ESI+) 556 (M+H)⁺.

### Example 97

### 5-({[(4'-chloro[1,1'-biphenyl]-3-yl)methyl][(1S)-1,2,3,4-tetrahydro-1-naphthalenyl]amino}carbonyl)-1,2,4-benzenetricarboxylic acid

The product from Example 15B (165 mg, 0.3 mmol) and 4-chlorophenylboronic acid were processed as described in Example 85 to provide the title compound.
¹H NMR (DMSO-d₆) δ 8.34-6.99 (m, 14H)5.62-4.60 (m, 2H), 4.33 (dd, 1H), 4.00 (br s, 3H), 2.75-2.50 (m, 2H), 2.20-1.12 (m, 4H); MS (ESI+) 584 (M+H)⁺.

### Example 98

### 5-({(1S)-1,2,3,4-tetrahydro-1-naphthalenyl[3-(2-thienyl)benzyl]amino}carbonyl)-1,2,4-benzenetricarboxylic acid

The product from Example 15B (165 mg, 0.3 mmol) and 3-(2-thienyl)phenylboronic acid were processed as described in Example 85 to provide the title compound.
¹H NMR (DMSO-d₆) δ 8.29-6.96 (m, 13H), 5.57-4.62 (m, 2H), 4.40-3.93 (m, 1H), 2.74-2.55 (m, 2H), 2.20-1.40 (m, 4H); MS (ESI+) 556 (M+H)⁺.

### Example 99

### 5-({(1S)-1,2,3,4-tetrahydro-1-naphthalenyl[(3',4',5'-trimethoxy[1,1'-biphenyl]-3-yl)methyl]amino}carbonyl)-1,2,4-benzenetricarboxylic acid

The product from Example 15B (165 mg, 0.3 mmol) and 3,4,5-trimethoxyphenylboronic acid were processed as described in Example 85 to provide the title compound.
¹H NMR (DMSO-d₆) δ 8.27-6.73 (m, 12H), 5.61-4.62 (m, 2H), 4.40-3.95 (m, 1H), 3.85 (s, 6H), 3.68 (s, 3H), 2.75-2.57 (m, 2H), 2.22-1.40 (m, 4H); MS (ESI+) 640 (M+H)⁺.

### Example 100

### 2-[(methylamino)carbonyl]-5-({(3-phenoxybenzyl)[(1S)-1,2,3,4-tetrahydro-1-naphthalenyl]amino}carbonyl)terephthalic acid and 4-[(methylamino)carbonyl]-6-({(3-phenoxybenzyl)[(1S)-1,2,3,4-tetrahydro-1-naphthalenyl]amino}carbonyl)isophthalic acid

1,2,4,5-Benzenetetracarboxylic dianhydride (1.1 g, 5 mmol) in THF (25 mL) was treated with diisopropylethyl amine (1.6 mL, 9.2 mmol) dropwise at -78 °C and then treated with N-(3-phenoxybenzyl)-N-[(1S)-1,2,3,4-tetrahydro-1-naphthalenyl]amine (9 mL, 4.5 mmol, 0.5M solution in ClCH₂CH₂Cl) dropwise. After allowing the reaction mixture to gradually warm to room temperature overnight, the mixture was recooled to -78°C and treated with methylamine (2.5 mL, 5 mmol, 2M in MeOH) dropwise. The reaction mixture was again allowed to slowly warm to room temperature. The organics were removed under reduced pressure and the residue was dissolved in ethyl acetate (25 mL). The ethyl acetate solution was washed with 1N HCl (2x 5 mL), brine (5 mL), dried over Na₂SO₄, filtered and the filtrate concentrated under reduced pressure. The residue was purified by flash chromatographed (silica gel, 5:95 MeOH:CH₂Cl₂ to 1:9 MeOH:CH₂Cl₂) to provide a solid. The solid was further purified by RP-HPLC to provide the title compound as an off-white solid (319 mg, 12%).
¹H NMR (300 MHz, DMSO-d₆) δ 8.34, 8.15, 7.95, and 7.89 (4 s, 2H); 6.66-7.73 (m, 13H); 4.91 and 5.58 (2 m, 1H); 3.91 and 4.68 (2 m, 2H); 3.80 and 4.13 (2 br d, 3H); 2.59 (m, 2H); 1.39-2.06 (m, 4H); HRMS (FAB) calculated for C₃₄H₃₁N₂O₇ 579.2131 (M+H)⁺. Found: 579.2159 (M+H)⁺.

### Example 101

### 2-[(cyanoamino)carbonyl]-5-({(3-phenoxybenzyl)[(1S)-1,2,3,4-tetrahydro-1-naphthalenyl]amino}carbonyl)terephthalic acid and 4-[(cyanoamino)carbonyl]-6-({(3-phenoxybenzyl)[(1S)-1,2,3,4-tetrahydro-1-naphthalenyl]amino}carbonyl)isophthalic acid

1,2,4,5-Benzenetetracarboxylic dianhydride (1 mmol), diisopropylethyl amine, N-(3-phenoxybenzyl)-N-[(1S)-1,2,3,4-tetrahydro-1-naphthalenyl]amine and cyanamide were processed as described in Example 100 to provide the title compounds as a white solid (52 mg, 9% yield).
¹H NMR (500 MHz, d₆-DMSO) δ 8.16 and 8.25 (2 s, 2H); 6.92-7.59 (m, 13H); 4.67 and 5.53 (2 br t, 1H); 4.19 and 4.82 (2 m, 2H); 2.62 (m, 2H); 1.34-2.09 (m, 4H); MS (ESI-) (M-H) 588.

### Example 102

### 2-(aminocarbonyl)-5-({(3-phenoxybenzyl)[(1S)-1,2,3,4-tetrahydro-1-naphthalenyl]amino}carbonyl)terephthalic acid and 4-(aminocarbonyl)-6-({(3-phenoxybenzyl)[(1S)-1,2,3,4-tetrahydro-1-naphthalenyl]amino}carbonyl)isophthalic acid

1,2,4,5-Benzenetetracarboxylic dianhydride (1 mmol), diisopropylethyl amine, N-(3-phenoxybenzyl)-N-[(1S)-1,2,3,4-tetrahydro-1-naphthalenyl]amine and ammonium hydroxide were processed as described in Example 100 to provide the title compounds as a white solid (120 mg, 21% yield).
¹H NMR (500 MHz, d₆-DMSO) δ 8.31, 8.22, 8.01, and 7.95 (4 s, 2H); 6.69-7.66 (m, 13H); 4.66 and 5.49 (2 m, 1H); 4.27 and 4.83 (2 m, 2H); 2.62 (m, 2H); 1.37-2.13 (m, 4H); HRMS (FAB) calculated for C₃₃H₂₉N₂O₇ 565.1975 (M+H)⁺. Found: 565.1973 (M+H)⁺.

### Example 103

### 2-(methoxycarbonyl)-5-({(3-phenoxybenzyl)[(1S)-1,2,3,4-tetrahydro-1-naphthalenyl]amino}carbonyl)terephthalic acid and 4-(methoxycarbonyl)-6-({(3-phenoxybenzyl)[(1S)-1,2,3,4-tetrahydro-1-naphthalenyl]amino}carbonyl)isophthalic acid

1,2,4,5-Benzenetetracarboxylic dianhydride (13.8 mmol), diisopropylethyl amine, N-(3-phenoxybenzyl)-N-[(1S)-1,2,3,4-tetrahydro-1-naphthalenyl]amine and methanol were processed as described in Example 100. The residue was purified by flash chromatography (silica gel, 95:5 CH₂Cl₂:methanol to 8:2 CH₂Cl₂:methanol) to provide the title compounds as an off-white solid (120 mg, 21% yield).
¹H NMR (500 MHz, d₆-DMSO) δ 8.23 and 8.33 (2s, 1H); 6.58-7.53 (m, 14H); 4.65 and 5.59 (2 br t, 1H); 4.17 and 4.81 (2m, 2H); 3.68, 3.71, and 3.73 (3s, 3H); 2.53 (m, 2H); 1.39-2.02 (m, 4H); MS (ESI+) m/z (M+H)⁺ 580.

### Example 104

### 2-hydroxy-5-({(3-phenoxybenzyl)[(1S)-1,2,3,4-tetrahydro-1-naphthalenyl]amino}carbonyl)terephthalic acid and 4-hydroxy-6-({(3-phenoxybenzyl)[(1S)-1,2,3,4-tetrahydro-1-naphthalenyl]amino}carbonyl)isophthalic acid

### Example 104A

### 6-(acetyloxy)-1,3-dioxo-1,3-dihydro-2-benzofuran-5-carboxylic acid

4-Hydroxy-benzene-1,2,5-tricarboxylic acid (4.1 g, 18.1 mmol), prepared according to J. Am. Chem. Soc. (1949) 71, 11, was treated with acetic anhydride (50 mL) and heated at reflux for 5 hours. The mixture was allowed to cool to room temperature and then concentrated under reduced pressure. The orange-pink residue was washed with diethyl ether (3x30 mL) and dried under reduced pressure at room temperature overnight to provide the title compound (1.71 g, 38%).

### Example 104B

### 2-(acetyloxy)-5-({[3-(4-chlorophenoxy)benzyl][(1S)-1,2,3,4-tetrahydro-1-naphthalenyl]amino}carbonyl)terephthalic acid and 4-(acetyloxy)-6-({[3-(4-chlorophenoxy)benzyl][(1S)-1,2,3,4-tetrahydro-1-naphthalenyl]amino}carbonyl)isophthalic acid

The product from Example 104A (1.7 g, 6.8 mmol) and diisopropylethyl amine (4 mL, 23 mmol) in THF (60 mL) were treated with N-(3-phenoxybenzyl)-N-[(1S)-1,2,3,4-tetrahydro-1-naphthalenyl]amine (14 mL, 0.5M solution in ClCH₂CH₂Cl, 7 mmol). After stirring at room temperature overnight, the organic solvents were removed under reduced pressure and the residue partitioned between ethyl acetate (100 mL) and 1N HCl (20 mL). The phases were separated and the organic layer was washed with 1N HCl (20 mL), water (20 mL), brine (20 mL), dried over Na₂SO₄, filtered and the filtrate concentrated under reduced pressure. The residue was purified by flash chromatography (silica gel, 6:4 hexanes/ethyl acetate, then 9:1 CH₂Cl₂:methanol) to provide the title compound as a pale beige foamy solid (0.97 g, 25%).
¹H NMR (500 MHz, DMSO-d₆) δ 8.50, 8.43, 8.31, 7.75, 7.64, 7.55 (6 s, 2H); 6.56-7.43 (m, 13H); 5.60 and 4.66 (2 m, 1H); 4.79 and 4.15 (2 m, 2H); 2.61 (m, 2H); 2.28, 2.26, 2.07 (3 s, 3H); 1.44-2.09 (m, 4H); MS (ESI+) 580 (M+H)⁺.

### Example 104C

### 2-hydroxy-5-({(3-phenoxybenzyl)[(1S)-1,2,3,4-tetrahydro-1-naphthalenyl]amino}carbonyl)terephthalic acid and 4-hydroxy-6-({(3-phenoxybenzyl)[(1S)-1,2,3,4-tetrahydro-1-naphthalenyl]amino}carbonyl)isophthalic acid

The products from Example 104B (0.97 g, 1.68 mmol) in methanol (60 mL) were treated with NaHCO₃ (3.9 g, 46.4 mmol) in water (30 mL) and the mixture was stirred at room temperature for 24 hours. The methanol was removed under reduced pressure and the remaining aqueous solution was acidified to pH 1 with 1N HCl. The acidified solution was extracted with ethyl acetate (5x15 mL). The organic extracts were combined, washed with brine (10 mL), dried over Na₂SO₄, filtered and the filtrate concentrated under reduced pressure. The residue was purified by flash chromatography (silica gel, 9:1 CH₂Cl₂:methanol to 8:2 CH₂Cl₂:methanol) to provide the title compounds as a foamy white solid (208 mg, 23%).
¹H NMR (500 MHz, DMSO-d₆) δ 8.36, 8.31, 7.79, 7.39 (4 s, 2H); 6.45-7.44 (m, 13H); 4.83 (m, 2H); 4.72 (m, 1H); 2.60 (m, 2H); 1.43-2.07 (m, 4H); HRMS (FAB) calculated for C₃₂H₂₈NO₇ 538.1866 (M+H)⁺. Found: 538.1862 (M+H)⁺.

### Example 105

### 2-({(3-phenoxybenzyl)[(1S)-1,2,3,4-tetrahydro-1-naphthalenyl]amino}carbonyl)-5-sulfoterephthalic acid and 4-({(3-phenoxybenzyl)[(1S)-1,2,3,4-tetrahydro-1-naphthalenyl]amino}carbonyl)-6-sulfoisophthalic acid

### Example 105A

### 1,3-dioxo-6-sulfo-1,3-dihydro-2-benzofuran-5-carboxylic acid

5-Sulfo-1,2,4-benzenetricarboxylic acid (9.63 g, 33.2 mmol), prepared according to J. Am. Chem. Soc. (1949) 71, 11, was treated with acetic anhydride (150 mL) and refluxed for 5 hours. After cooling to room temperature, the acetic anhydride was removed under reduced pressure. The residue was washed with diethyl ether (3 x 30 mL) and then dried under reduced pressure at room temperature to provide the title compound as a tan solid.

### Example 105B

### 2-({(3-phenoxybenzyl)[(1S)-1,2,3,4-tetrahydro-1-naphthalenyl]amino}carbonyl)-5-sulfoterephthalic acid and 4-({(3-phenoxybenzyl)[(1S)-1,2,3,4-tetrahydro-1-naphthalenyl]amino}carbonyl)-6-sulfoisophthalic acid

The product from Example 105A (2 g, 7.35 mmol) and diisopropylethyl amine (2.5 mL,14.6 mmol) in THF (40 mL) were treated with N-(3-phenoxybenzyl)-N-[(1S)-1,2,3,4-tetrahydro-1-naphthalenyl]amine (10 mL, 0.5M solution in ClCH₂CH₂Cl, 5 mmol). After stirring at room temperature overnight, the organics were removed under reduced pressure and the residue was dissolved in ethyl acetate. The ethyl acetate solution was washed with 1N HCl, brine, dried over Na₂SO₄, filtered and the filtrate concentrated under reduced pressure. The residue was purified by several rounds of flash chromatography followed by a Prep Nova Pak (HRC18) column using 0.1% trifluoroacetic acid:acetonitrile (10:90 to 95:5) as eluant to provide the title compounds.
¹H NMR (500 MHz, DMSO-d₆) δ 8.36, 8.31, 8.15, and 7.71 (4s, 2H); 6.77-7.94 (m, 13 H); 4.77 and 5.41 (2m, 1H); 4.16 and 4.79 (2m, 2H); 2.60 (m, 2H); 1.33-1.98 (m, 4H); MS (ESI⁻) 600 (M-H)⁻.

### Example 106

### 5-({[3-(4-pyridinyloxy)benzyl][(1S)-1,2,3,4-tetrahydro-1-naphthalenyl]amino}carbonyl)-1,2,4-benzenetricarboxylic acid

### Example 106A

### N-[3-(4-pyridinyloxy)benzyl]-N-[(1S)-1,2,3,4-tetrahydro-1-naphthalenyl]amine

3-(4-Pyridinyloxy)benzaldehyde (0.85 g, 4.27 mmol), prepared according to J. Chem. Soc. Perkin Trans 2 (1987) 1867, and (1S)-1,2,3,4-tetrahydro-1-naphthalenylamine (0.63 g, 4.29 mmol) were combined in ethanol (10 mL), briefly heated and the solvent removed under reduced pressure. This process was repeated twice more. The residual oil was dissolved in ethanol (5 mL) and stirred with sodium borohydride (0.16 g, 4.23 mmol) overnight at room temperature. The reaction mixture was quenched with water (10 mL) and diluted with diethyl ether (50 mL). The diethyl ether was washed with saturated NaHCO₃ solution (3x10 mL), brine (10 mL), dried over Na₂SO₄, filtered and the filtrate concentrated under reduced pressure to provide the title compound as a thick orange liquid (1.19 g, 85% yield).
¹H NMR (300 MHz, CDCl₃) δ 8.52 and 8.46 (2 dd, 2H), 6.91-7.38 (m, 8H); 6.71 and 6.86 (2 dd, 2H), 3.82 (m, 3H), 2.78 (m, 2H), 2.03 and 1.77 (2m, 2H), 1.92 (q, 2H); MS (ESI+) 331 (M+H)⁺.

### Example 106B

### 5-({[3-(4-pyridinyloxy)benzyl][(1S)-1,2,3,4-tetrahydro-1-naphthalenyl]amino}carbonyl)-1,2,4-benzenetricarboxylic acid

The product from Example 106A (1.17 g), diisopropylethyl amine (2.5 mL, 14.4 mmol) and 1,2,4,5-benzenetetracarboxylic dianhydride (0.78 g, 3.58 mmol) in THF (30 mL) were processed as described in Example 1B. The reaction mixture was treated with saturated Na₂CO₃ solution (30 mL), and stirred vigorously at room temperature for 1 hour. The layers were separated and the aqueous layer was acidified with 1N HCl. The acidified aqueous layer was evaporated under reduced pressure to afford off-white plates. Soxhlet extraction (ethanol) of the crude solid, followed by RP-HPLC, provided the title compound (20 mg).
¹H NMR (500 MHz, DMSO-d₆) δ 8.63 and 8.68 (2m, 2H); 8.18 and 8.26 (2s, 2H); 7.44 and 7.52 (2m, 2H); 6.46-7.82 (m, 8H); 4.68 and 5.37 (2m, 1H); 4.14 and 4.88 (2m, 2H); 2.61 (m, 2H); 1.37-2.12 (m, 4H); MS (ESI+) 567 (M+H)⁺.

### Example 107

### 5-({[3-(2-pyrimidinyloxy)benzyl][(1S)-1,2,3,4-tetrahydro-1-naphthalenyl]amino}carbonyl)-1,2,4-benzenetricarboxylic acid

### Example 107A

### N-[3-(2-pyrimidinyloxy)benzyl]-N-[(1S)-1,2,3,4-tetrahydro-1-naphthalenyl]amine

3-(2-Pyrimidinyloxy)benzaldehyde (0.67 g, 3.35 mmol) and (1S)-1,2,3,4-tetrahydro-1-naphthalenylamine (0.49 g, 3.33 mmol) were processed as described in Example 106A. The residue was purified by flash chromatography (silica gel, 6:4 ethyl acetate:hexanes to 1:1 ethyl acetate:hexanes) to provide the title compound (190 mg, 19% yield).
¹H NMR (300 MHz, CDCl₃) δ 8.55 (d, 2H), 7.01-7.42 (m, 9H), 3.94 (m, 2H), 3.83 (t, 1H), 2.78 (m, 2H), 1.68-2.07 (m, 4H); MS (ESI+) 332 (M+H)⁺.

### Example 107B

### 5-({[3-(2-pyrimidinyloxy)benzyl][(1S)-1,2,3,4-tetrahydro-1-naphthalenyl]amino}carbonyl)-1,2,4-benzenetricarboxylic acid

The product from Example 107A, 1,2,4,5-benzenetetracarboxylic dianhydride (0.125 g, 0.57 mmol) and diisopropylethyl amine (0.4 mL, 2.3 mmol) were processed as described in Example 1B. The reaction mixture was treated with saturated Na₂CO₃ solution (30 mL) and stirred vigorously at room temperature for 1 hour. The layers were separated and the aqueous layer was acidified with 1N HCl. The acidified aqueous layer was evaporated under reduced pressure to afford a solid. Soxhlet extraction (ethanol) of the crude solid, followed by RP-HPLC, provided the title compound (4 mg).
HRMS (FAB) calculated for C₃₁H₂₆N₃O₈ 568.1720 (M+H)⁺. Found 568.1713 (M+H)⁺.

### Example 108

### 4-({[(4-methoxyphenyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(4-methoxyphenyl)sulfonyl]amino}carbonyl)-5-({(3-phenoxybenzyl)[(1S)-1,2,3,4-tetrahydro-1-naphthalenyl]amino}carbonyl)terephthalic acid

The product from Example 36B (114 mg, 0.2 mmol), 4-methoxybenzenesulfonamide (37 mg, 0.2 mmol), DMAP (25 mg, 0.2 mmol), and EDCI (78 mg, 0.4 mmol) were combined in THF (10mL) at room temperature. After stirring overnight, the mixture was concentrated under reduced pressure and CH₂Cl₂ (20 mL) was added. The mixture was washed with water and the organic layer concentrated under reduced pressure. The residue was purified via HPLC to provide the title compounds. ¹H (500 MHz; DMSO-d₆) δ 6.4-8.6 (m, br., 20H) 5.0 (m, 1H) 4.7 (m, 1H) 4.1 (m, 1H) 3.5 (br, 3H) 1.4-2.7 (m, 6H); MS (M+H)⁺ 735.

### Example 109

### 4-({[(3-chloro-4-methylphenyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(3-chloro-4-methylphenyl)sulfonyl]amino}carbonyl)-5-({(3-phenoxybenzyl)[(1S)-1,2,3,4-tetrahydro-1-naphthalenyl]amino}carbonyl)terephthalic acid

3-Chloro-4-methylbenzenesulfonamide was processed as described in Example 108 to provide the title compounds. ¹H (500 MHz; DMSO-d₆) δ 6.4-8.6 (m, 19H) 5.1 (m, 1H) 4.7 (m, 1H) 4.2 (m, 1H) 2.4 (br, 3H) 1.4-2.7 (m, 6H); MS (M+H)⁺ 754.

### Example 110

### 4-({[(2-chlorophenyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(2-chlorophenyl)sulfonyl]amino}carbonyl)-5-({(3-phenoxybenzyl)[(1S)-1,2,3,4-tetrahydro-1-naphthalenyl]amino}carbonyl)terephthalic acid

2-Chlorobenzenesulfonamide was processed as described in Example 108 to provide the title compounds. ¹H (500 MHz; DMSO-d₆) δ 6.4-8.6 (m, 20H) 5.1 (m, 1H) 4.7 (m, 1H) 4.1 (m, 1H) 1.4-2.7 (m, 6H) MS (M+H)⁺ 740.

### Example 111

### 4-({[(3-chlorophenyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(3-chlorophenyl)sulfonyl]amino}carbonyl)-5-({(3-phenoxybenzyl)[(1S)-1,2,3,4-tetrahydro-1-naphthalenyl]amino}carbonyl)terephthalic acid

3-Chlorobenzenesulfonamide was processed as described in Example 108 to provide the title compounds. ¹H (500 MHz; DMSO-d₆) δ 6.4-8.6 (m, 20H) 5.1 (m, 1H) 4.7 (m, 1H) 4.1 (m, 1H) 1.4-2.7 (m, 6H) MS (M+H)⁺ 740.

### Example 112

### 4-({[(4-chlorophenyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(4-chlorophenyl)sulfonyl]amino}carbonyl)-5-({(3-phenoxybenzyl)[(1S)-1,2,3,4-tetrahydro-1-naphthalenyl]amino}carbonyl)terephthalic acid

4-Chlorobenzenesulfonamide was processed as described in Example 108 to provide the title compounds. ¹H (500 MHz; DMSO-d₆) δ 6.4-8.6 (m, 20H) 5.0 (d, 1H) 4.7 (m, 1H) 4.2 (m, 1H) 1.4-2.7 (m, 6H); MS (M+H)⁺ 740.

### Example 114

### 4-({[(4-nitrophenyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(4-nitrophenyl)sulfonyl]amino}carbonyl)-5-({(3-phenoxybenzyl)[(1S)-1,2,3,4-tetrahydro-1-naphthalenyl]amino}carbonyl)terephthalic acid

4-Nitrobenzenesulfonamide was processed as described in Example 108 to provide the title compounds. ¹H (500 MHz; DMSO-d₆) δ 6.4-8.6 (m, 20H); 5.0 (m, 1H); 4.7 (m, 1H); 4.1 (m, 1H); 1.4-2.7 (m, 6H). MS (M+H)⁺ 750.

### Example 115

### 4-({[(4-bromophenyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(4-bromophenyl)sulfonyl]amino}carbonyl)-5-({(3-phenoxybenzyl)[(1S)-1,2,3,4-tetrahydro-1-naphthalenyl]amino}carbonyl)terephthalic acid

4-Bromobenzenesulfonamide was processed as described in Example 108 to provide the title compounds. ¹H (500 MHz; DMSO-d₆) δ 6.4-8.6 (m, 20H); 5.0 (d, 1H); 4.7 (m, 1H); 4.2 (m, 1H); 1.4-2.7 (m, 6H). MS (M+H)⁺ 784.

### Example 116

### 4-({[(3-nitrophenyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(3-nitrophenyl)sulfonyl]amino}carbonyl)-5-({(3-phenoxybenzyl)[(1S)-1,2,3,4-tetrahydro-1-naphthalenyl]amino}carbonyl)terephthalic acid

3-Nitrobenzenesulfonamide was processed as described in Example 108 to provide the title compounds. ¹H (500 MHz; DMSO-d₆) δ 6.4-8.6 (m, 20H); 5.0 (m, 1H); 4.7 (m, 1H); 4.2 (m, 1H); 1.4-2.7 (m, 6H). MS (M+H)⁺ 750.

### Example 117

### 4-({[(phenyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(phenyl)sulfonyl]amino}carbonyl)-5-({(3-phenoxybenzyl)[(1S)-1,2,3,4-tetrahydro-1-naphthalenyl]amino}carbonyl)terephthalic acid

Benzenesulfonamide was processed as described in Example 108 to provide the title compounds. ¹H (500 MHz; DMSO-d₆) δ 6.4-8.6 (m, 21H); 5.0 (m, 1H); 4.7 (m, 1H); 4.2 (m, 1H); 1.4-2.7 (m, 6H). MS (M+H)⁺ 705.

### Example 118

### 4-({[(2-nitrophenyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(2-nitrophenyl)sulfonyl]amino}carbonyl)-5-({(3-phenoxybenzyl)[(1S)-1,2,3,4-tetrahydro-1-naphthalenyl]amino}carbonyl)terephthalic acid

2-Nitrobenzenesulfonamide was processed as described in Example 108 to provide the title compounds. ¹H (500 MHz; DMSO-d₆) δ 6.4-8.6 (m, 20H); 5.0 (m, 1H); 4.7 (m, 1H); 4.2 (m, 1H); 1.4-2.7 (m, 6H). MS (M+H)⁺ 750.

### Example 119

### 4-({[(2-methylphenyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(2-methylphenyl)sulfonyl]amino}carbonyl)-5-({(3-phenoxybenzyl)[(1S)-1,2,3,4-tetrahydro-1-naphthalenyl]amino}carbonyl)terephthalic acid

2-Methylbenzenesulfonamide was processed as described in Example 108 to provide the title compounds. ¹H (500 MHz; DMSO-d₆) δ 6.4-8.6 (m, 20H); 5.0 (d, 1H); 4.7 (m, 1H); 4.2 (m, 1H); 2.7 (br., 3H); 1.4-2.7 (m, 6H). MS (M+H)⁺ 719.

### Example 120

### 4-{[({4-[(2,3-dihydroxypropyl)amino]phenyl}sulfonyl)amino]carbonyl}-6-({(3-phenoxybenzyl)[(1S)-1,2,3,4-tetrahydro-1-naphthalenyl]amino}carbonyl)isophthalic acid and 2- {[({4-[(2,3-dihydroxypropyl)amino]phenyl}sulfonyl)amino]carbonyl}-5-({(3-phenoxybenzyl)[(1S)-1,2,3,4-tetrahydro-1-naphthalenyl]amino}carbonyl)terephthalic acid

4-[(2,3-Dihydroxypropyl)amino]benzenesulfonamide was processed as described in Example 108 to provide the title compounds. ¹H (500 MHz; DMSO-d₆) δ 6.4-8.6 (m, 20H); 5.0 (m, 1H); 4.1-4.7 (m, 8H); 1.4-2.7 (m, 6H). MS (M+H)⁺ 794.

### Example 121

### 2-({[(4-{[(methylamino)carbonyl]amino}phenyl)sulfonyl]amino}carbonyl)-5-({(3-phenoxybenzyl)[(1S)-1,2,3,4-tetrahydro-1-naphthalenyl]amino}carbonyl)terephthalic acid and 4-({[(4-{[(methylamino)carbonyl]amino}phenyl)sulfonyl]amino}carbonyl)-6-({(3-phenoxybenzyl)[(1S)-1,2,3,4-tetrahydro-1-naphthalenyl]amino}carbonyl)isophthalic acid

4-{[(Methylamino)carbonyl]amino}benzenesulfonamide was processed as described in Example 108 to provide the title compounds. ¹H (500 MHz; DMSO-d₆) δ 6.4-8.6 (m, 22H); 5.0 (m,1H); 4.7 (m,1H); 4.2 (m, 1H); 2.8 (m, 3H); 1.4-2.7 (m, 6H). MS (M+H)⁺ 777.

### Example 122

### 4-[({[4-(butyrylamino)phenyl]sulfonyl}amino)carbonyl]-6-({(3-phenoxybenzyl)[(1S)-1,2,3,4-tetrahydro-1-naphthalenyl]amino}carbonyl)isophthalic acid and 2-[({[4-(butyrylamino)phenyl]sulfonyl}amino)carbonyl]-5-({(3-phenoxybenzyl)[(1S)-1,2,3,4-tetrahydro-1-naphthalenyl]amino}carbonyl)terephthalic acid

N-[4-(Aminosulfonyl)phenyl]butanamide was processed as described in Example 108 to provide the title compounds. ¹H (500 MHz; DMSO-d₆) δ 6.4-8.6 (m, 21H); 5.0 (m, 1H); 4.7 (m, 1H); 4.2 (m, 1H); 1.4-2.7 (m, 10H); 1.0 (m, 3H). MS (M+H)⁺ 790.

### Example 123

### 4-({[(3,4-difluorophenyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(3,4-difluorophenyl)sulfonyl]amino}carbonyl)-5-({(3-phenoxybenzyl)[(1S)-1,2,3,4-tetrahydro-1-naphthalenyl]amino}carbonyl)terephthalic acid

3,4-Difluorobenzenesulfonamide was processed as described in Example 108 to provide the title compounds. ¹H (500 MHz; CD₂Cl₂) δ 6.6-8.1 (m, 19H); 4.9 (d, 1H); 4.7 (m, 1H); 4.1 (m, 1H); 1.3-3.0 (m, 6H). MS (M+H)⁺ 741.

### Example 124

### 4-({[(2,5-difluorophenyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(2,5-difluorophenyl)sulfonyl]amino}carbonyl)-5-({(3-phenoxybenzyl)[(1S)-1,2,3,4-tetrahydro-1-naphthalenyl]amino}carbonyl)terephthalic acid

2,5-Difluorobenzenesulfonamide was processed as described in Example 108 to provide the title compounds. ¹H (500 MHz; CD₂Cl₂) δ 6.8-8.1 (m, 19H); 5.0 (d, 1H); 4.7 (m, 1H); 4.2 (m, 1H); 1.4-2.8 (m, 6H). MS (M+H)⁺ 741.

### Example 125

### 4-({[(2-methoxy-4-methylphenyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(2-methoxy-4-methylphenyl)sulfonyl]amino}carbonyl)-5-({(3-phenoxybenzyl)[(1S)-1,2,3,4-tetrahydro-1-naphthalenyl]amino}carbonyl)terephthalic acid

2-Methoxy-4-methylbenzenesulfonamide was processed as described in Example 108 to provide the title compounds. ¹H (500 MHz; CD₂Cl₂) δ 6.9-8.7 (m, 19H); 5.1 (m, 1H); 4.8 (m, 1H); 4.3 (m, 1H); 3.3 (br, 3H); 2.5 (m, 3H); 1.4-2.8 (m, 6H). MS (M+H)⁺ 749.

### Example 126

### 4-({[(5-methyl-2-pyridinyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(5-methyl-2-pyridinyl)sulfonyl]amino}carbonyl)-5-({(3-phenoxybenzyl)[(1S)-1,2,3,4-tetrahydro-1-naphthalenyl]amino}carbonyl)terephthalic acid

5-Methyl-2-pyridinesulfonamide was processed as described in Example 108 to provide the title compounds. ¹H (500 MHz; CD₂Cl₂) δ 6.9-8.7 (m, 19H); 4.9 (m, 1H); 4.7 (m, 1H); 4.2 (m, 1H); 2.4 (m, 3H); 1.4-2.8 (m, 6H). MS (M+H)⁺ 720.

### Example 127

### 4-{[(methylsulfonyl)amino]carbonyl}-6-({(3-phenoxybenzyl)[(1S)-1,2,3,4-tetrahydro-1-naphthalenyl]amino}carbonyl)isophthalic acid and 2-{[(methylsulfonyl)amino]carbonyl}-5-({(3-phenoxybenzyl)[(1S)-1,2,3,4-tetrahydro-1-naphthalenyl]amino}carbonyl)terephthalic acid

Methanesulfonamide was processed as described in Example 108 to provide the title compounds. ¹H (500 MHz; CD₂Cl₂) δ 6.9-8.7 (m, 16H); 4.9 (d, 1H); 4.7 (m, 1H); 4.2 (m, 1H); 2.0 (s, 3H); 1.4-2.8 (m, 6H). MS (M+H)⁺ 643.

### Example 128

### 4-({[(5-nitro-2-thienyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(5-nitro-2-thienyl)sulfonyl]amino}carbonyl)-5-({(3-phenoxybenzyl)[(1S)-1,2,3,4-tetrahydro-1-naphthalenyl]amino}carbonyl)terephthalic acid

5-Nitro-2-thiophenesulfonamide was processed as described in Example 108 to provide the title compounds. ¹H (500 MHz; CD2Cl2) δ 6.9-8.8 (m, 18H); 4.9-(m, 1H); 4.7 (m, 1H); 4.2 (m, 1H); 1.4-2.8 (m, 6H). MS (M+H)⁺ 756.

### Example 130

### 4-({[(4-methylphenyl)sulfonyl]amino}carbonyl)-6-({(3-phenoxybenzyl)[(1S)-1,2,3,4-tetrahydro-1-naphthalenyl]amino}carbonyl)isophthalic acid and 2-({[(4-methylphenyl)sulfonyl]amino}carbonyl)-5-({(3-phenoxybenzyl)[(1S)-1,2,3,4-tetrahydro-1-naphthalenyl]amino}carbonyl)terephthalic acid

4-Methylbenzenesulfonamide was processed as described in Example 108 to provide the title compounds. ¹H (500 MHz; CD₂Cl₂) δ 6.9-8.6 (m, 20H); 4.9 (m, 1H); 4.7 (m, 1H); 4.1 (m, 1H); 2.4 (m, 3H); 1.4-2.8 (m, 6H). MS (M+H)⁺ 719.

### Example 131

### 4-{[(benzylsulfonyl)amino]carbonyl}-6-({(3-phenoxybenzyl)[(1S)-1,2,3,4-tetrahydro-1-naphthalenyl]amino}carbonyl)isophthalic acid and 2- {[(benzylsulfonyl)amino]carbonyl}-5-({(3-phenoxybenzyl)[(1S)-1,2,3,4-tetrahydro-1-naphthalenyl]amino}carbonyl)terephthalic acid

1-Phenylmethanesulfonamide was processed as described in Example 108 to provide the title compounds. ¹H (500 MHz; CD₂Cl₂) δ 6.9-8.8 (m, 20H); 4.9 (d, 1H); 4.7 (m, 3H); 4.1 (m, 1H); 1.4-2.8 (m, 6H). MS (M+H)⁺ 719.

### Example 132

### 2-({[(2-fluorophenyl)sulfonyl]amino}carbonyl)-5-({(3-phenoxybenzyl)[(1S)-1,2,3,4-tetrahydro-1-naphthalenyl]amino}carbonyl)terephthalic acid and 4-({[(2-fluorophenyl)sulfonyl]amino}carbonyl)-6-({(3-phenoxybenzyl)[(1S)-1,2,3,4-tetrahydro-1-naphthalenyl]amino}carbonyl)isophthalic acid

2-Fluorobenzenesulfonamide was processed as described in Example 108 to provide the title compounds. ¹H (500 MHz; CD₂Cl₂) δ 6.9-8.8 (m, 20H); 4.9 (m, 1H); 4.7 (m, 1H); 4.1 (m, 1H); 1.4-2.8 (m, 6H). MS (M+H)⁺ 723.

### Example 133

### 2-({[(5-chloro-2-thienyl)sulfonyl]amino}carbonyl)-5-({(3-phenoxybenzyl)[(1S)-1,2,3,4-tetrahydro-1-naphthalenyl]amino}carbonyl)terephthalic acid and 4-({[(5-chloro-2-thienyl)sulfonyl]amino}carbonyl)-6-({(3-phenoxybenzyl)[(1S)-1,2,3,4-tetrahydro-1-naphthalenyl]amino}carbonyl)isophthalic acid

5-Chloro-2-thiophenesulfonamide was processed as described in Example 108 to provide the title compounds. ¹H (500 MHz; CD₂Cl₂) δ 6.9-8.8 (m,18H); 4.9 (d,1H); 4.7 (m, 1H); 4.2 (m, 1H); 1.4-2.8 (m, 6H). MS (M+H)⁺ 746.

### Example 134

### 2-({[(4-hydroxyphenyl)sulfonyl]amino}carbonyl)-5-({(3-phenoxybenzyl)[(1S)-1,2,3,4-tetrahydro-1-naphthalenyl]amino}carbonyl)terephthalic acid and 4-({[(4-hydroxyphenyl)sulfonyl]amino}carbonyl)-6-({(3-phenoxybenzyl)[(1S)-1,2,3,4-tetrahydro-1-naphthalenyl]amino}carbonyl)isophthalic acid

4-Hydroxybenzenesulfonamide was processed as described in Example 108 to provide the title compounds. ¹H (500 MHz; CD₂Cl₂) δ 6.9-8.8 (m, 20H); 4.9 (m, 1H); 4.7 (m, 1H); 4.2 (m, 1H); 1.4-2.8 (m, 6H). MS (M+H)⁺ 721.

### Example 135

### 6-({[(3',4'-dichloro-1,1'-biphenyl-3-yl)methyl][(1S)-1,2,3,4-tetrahydro-1-naphthalenyl]amino}carbonyl)-1,3-dioxo-1,3-dihydro-2-benzothiophene-5-carboxylic acid

### Example 135A

### 3',4'-dichloro-1,1'-biphenyl-3-carbaldehyde

3-Bromobenzaldehyde (3.16 g, 17 mmol) and 3,4-dichloroboronic acid (4.3 g, 22 mmol) in DME (200 mL) was treated with 3M Na₂CO₃ (28 mL, 85 mmol) and tetrakis(triphenylphosphine)palladium (II) (1.0 g, 0.85 mmol). The mixture was refluxed for 15 hours, allowed to cool to ambient temperature, diluted with ethyl acetate, and washed with 1N HCl and water. The organic layer was separated, concentrated under reduced pressure, and the residue purified by column chromatography (ethylacetate:hexane, 5:95) to provide the title compound. MS (DCI/NH₃) 250 (M+H)⁺.

### Example 135B

### N-[(3',4'-dichloro-1,1'-biphenyl-3-yl)methyl]-N-[(1S)-1,2,3,4-tetrahydro-1-naphthalenyl]amine

The product from Example 135A (2.1 g, 8.8 mmol) and (1S)-1,2,3,4-tetrahydro-1-naphthalenamine (1.43 g, 9.7 mmol) in ethanol (100 mL) were combined and stirred for 15 hours at ambient temperature. The mixture was treated with sodium borohydride (1.7 g, 44 mmol) and stirred an additional 3 hours. The mixture was treated with 3N HCl (4 mL) and diluted with ethylacetate. The organic layer was separated and concentrated to provide the title compound. MS (DCI/NH₃) 382 (M+H)⁺.

### Example 135C

### 6-({[(3',4'-dichloro-1,1'-biphenyl-3-yl)methyl][(1S)-1,2,3,4-tetrahydro-1-naphthalenyl]amino}carbonyl)-1,3-dioxo-1,3-dihydro-2-benzothiophene-5-carboxylic acid

1,2,4,5-Benzenetetracarboxylic dianhydride (8.0 g, 37.5 mmol) and triethylamine (14 mL, 102 mmol) in THF (300 mL) at -78 °C was treated with the product from Example 135B (13.0 g, 34.1 mmol) in ethanol (20 mL) over the period of 10 minutes. The mixture was allowed to gradually warm to room temperature and stir over night: The mixture was concentrated under reduced pressure and the residue was dissolved in THF (200 mL) and water (30 mL). The mixture was treated with sodium sulfide (1.33 g, 17 mmol) and stirred at ambient temperature for 1.5 hours. The mixture was diluted with ethyl acetate and washed in succession with water, 1N HCl (X2), and water. The organic phase was separated and concentrated under reduced pressure to provide the title compound. MS (DCI/NH₃) 616 (M+H)⁺.

## Claims

1. A compound of formula (I) 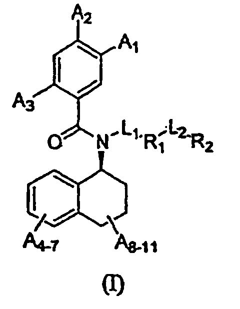 or a pharmaceutically acceptable salt thereof, wherein
A₁ and A₂ are each independently selected from the group consisting of alkoxycarbonyl alkylcarbonyloxy, carboxy, hydroxy, hydroxyalkyl, (NR_{A}R_{B})carbonyl, (NR_{C}S(O)₂R_{D}) carbonyl, -S (O)₂OH and tetrazolyl; or
A₁ and A₂ together with the carbon atoms to which they are attached form a five membered heterocycle containing a sulfur atom wherein the five membered heterocycle is optionally substituted with 1 or 2 substituents selected from the group consisting of mercapto and oxo;
A₃ is selected from the group consisting of alkoxycarbonyl, alkylcarbonyloxy, carboxy, hydroxy, hydroxyalkyl, (NR_{A}R_{B}) carbonyl, (NR_{C}S (O)₂R_{D}) carbonyl, -S(O)₂OH and tetrazolyl;
A₄, A₅, A₆ and A₇ are each independently selected from the group consisting of hydrogen, alkoxy, alkoxycarbonyl, alkenyl, alkyl, alkylcarbonyl, alkylcarbonyloxy, alkynyl, carboxy, cyano, haloalkoxy, haloalkyl, halogen, hydroxy, hydroxyalkyl, nitro, -NR_{E}R_{F} and (NR_{E}R_{F}) carbonyl;
A₈, A₉, A₁₀ and A₁₁ are each independently selected from the group consisting of hydrogen, alkoxy, alkoxycarbonyl alkenyl, alkyl, alkylcarbonyl, alkylcarbonyloxy, alkynyl, carboxy, haloalkoxy, haloalkyl, halogen, hydroxy, hydroxyalkyl, -NR_{E}R_{F} and (NR_{E}R_{F}) carbony and oxo;
R_{A} and R_{B} are each independently selected from the group consisting of hydrogen, alkyl and cyano;
R_{C} is selected from the group consisting of hydrogen and alkyl;
R_{D} is selected from the group consisting of alkoxy, alkyl, aryl, arylalkoxy, arylalkyl, haloalkoxy and haloalkyl;
R_{E} and R_{F} are each independently selected from the group consisting of hydrogen, alkyl alkylcarbonyl, formyl, and hydroxyalkyl;
L₁ is selected from the group consisting of alkenylene, alkylene, alkynylene, -(CH₂)ₘO (CH₂)ₙ-, -(CH₂)ₘS (CH₂)ₙ- and -(CH₂)ₚC(O)(CH₂)_{q}- wherein each group is drawn with the left end attached to N and the right end attached to R₁ ;
m is an integer 0-10;
n is an integer 0-10;
R₁ is selected from the group consisting of aryl, cycloalkenyl, cycloalkyl and heterocycle; wherein the aryl group is substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}Rc, and -CH=NOR_{EE} wherein R_{EE} is selected from hydrogen and alkyl, as defined herein; the cycloalkenyl and cycloalkyl groups are substituted with 0, 1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo,-NR_{E}R_{F}, (NR_{E}R_{F})carbonyl and -CH=NOR_{EE} wherein R_{EE} is selected from hydrogen and alkyl, as defined herein; and the heterocycle group is substituted with 0, 1, 2, or 3 substituents independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, arylalkoxycarbonyl, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(RA)C(O)NR_{B}Rc, and -CH=NOR_{EE} wherein R_{EE} is selected from hydrogen and alkyl, as defined herein;
L₂ is absent or selected from the group consisting of a covalent bond, alkenylene, alkylene, alkynylene, -(CH₂)ₚO(CH₂)_{q}, -(CH₂)ₚS(CH₂)_{q}, -(CH₂)ₚC(O)(CH₂)_{q}, -(CH₂)ₚC(OH)(CH₂)_{q} and -(CH₂)ₚCH=N(CH₂)_{q}- wherein each group is drawn with the left end attached to R1 and the right end attached to R2 ;
p is an integer 0-10;
q is an integer 0-10; and
R₂ is absent or selected from the group consisting of aryl, cycloalkenyl, cycloalkyl and heterocycle;
wherein at any occurence the aryl group is substituted with 0, 1, 2, 3 , 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F}) carbonyl, -N (R_{A}) C(O) NR_{B}R_{C}, and -CH=NOR_{EE} wherein R_{EE} is selected from hydrogen and alkyl, as defined herein; the cycloalkenyl and the cycloalkyl groups are substituted with 0, 1, 2, 3 ,4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F}) carbonyl and-CH=NOR_{EE} wherein R_{EE} is selected from hydrogen and alkyl, as defined herein;the heterocycles group can be substituted with 0, 1, 2, or 3 substituents independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, arylalkoxycarbonyl, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F}) carbonyl, -N(R_{A})C(O)NR_{B}R_{C}, and -CH=NOR_{EE} wherein R_{EE} is selected from hydrogen and alkyl, as defined herein.

2. A compound according to claim 1 wherein
A₁ and A₂ are each independently selected from the group consisting of alkoxycarbonyl, carboxy, hydroxy, (NR_{A}R_{B})carbonyl, -(NR_{C}S(O)₂R_{D}) carbonyl,-S (O)₂OH;
A₃ is carboxy;
A₄, A₅, A₆, A₇, A₈, A₉, A₁₀ and A₁₁ are each hydrogen;
R_{A} and R_{B} are each independently selected from the group consisting of hydrogen, alkyl and cyano;
L₁ is selected from the group consisting of alkylene and -(CH₂)ₘO(CH₂)ₙ-;
R₁ is selected from the group consisting of aryl, cycloalkyl and heterocycle;
L₂ is absent or selected from the group consisting of a covalent bond, alkylene, -(CH₂)ₚO(CH₂)_{q}-, -(CH₂)ₚC(O)(CH₂)_{q}-, -(CH₂)ₚC(OH)(CH₂)_{q}-, -(CH₂)ₚS(CH₂)_{q}-, and -(CH₂)ₚCH=NO(CH₂)_{q}-;
p is 0;
q is an integer 0-1; and
R₂ is absent or selected from the group consisting of aryl, cycloalkenyl, cycloalkyl and heterocycle.

3. A compound according to claim 1 wherein A₁ and A₂ together with the carbon atoms to which they are attached form a five membered heterocycle containing a sulfur atom wherein the five membered heterocycle is substituted with 0, 1, or 2 substituents selected from the group consisting of mercapto and oxo.

4. A compound according to claim 1 wherein
L₁ is alkylene;
R₁ is aryl;
L₂ is absent; and
R₂ is absent:

5. A compound according to claim 1 wherein
L₁ is alkylene;
R₁ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyallcyl, alkoxycarbonyl, alkoxycarbonylalkyl, alkoxysulfonyl, alkyl; alkylcarbonyl, alkylcarbonylalkyl, alkylcarbonyloxy, alkylsulfonyl, alkylthio, alkylthioallcyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE};
L₂ is absent; and
R₂ is absent,
preferably wherein
A₁, A₂ and A₃ are each carboxy;
A₄, A₅, A₆, A₇, A₈, A₉, A₁₀ and A₁₁ are each hydrogen;
L₁ is alkylene wherein said alkylene is selected from the group consisting of -CH₂-, -CH₂CH₂- and -CH₂CH(CH₃)-;
R₁ is aryl wherein said aryl is phenyl substituted with 0, 1, or 2 substituents selected from the group consisting of alkoxy, alkoxycarbonyl, alkenyl, alkenyloxy, alkyl, alkylthio, cyario, formyl, haloalkoxy, haloalkyl, haloalkylthio, halogen, hydroxyalkyl, methylenedioxy, nitro, -CH=NOR_{EE}, and -NR_{E}R_{F};
L₂ is absent; and
R₂ is absent.

6. A compound according to claim 1 wherein
L₁ is alkylene;
R₁ is aryl wherein said aryl is fluorenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxy, hydroxyalkyl, nitro, oxo, and -NR_{E}R_{F};
L₂ is absent; and
R₂ is absent,
preferably wherein
A₁, A₂ and A₃ are each carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is fluorenyl substituted with 0 or 1 substituents selected from the group consisting of hydroxy and oxo;
L₂ is absent; and
R₂ is absent.

7. A compound according to claim 1 wherein
L₁ is alkylene;
R₁ is aryl;
L₂ is a covalent bond; and
R₂ is aryl,
preferably wherein
L₁ is alkylene;
R₁ is aryl wherein said aryl is phenyl substituted with 0,1,2,3,4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE};
L₂ is a covalent bond; and
R₂ is aryl wherein said aryl is phenyl substituted with 0,1, 2, 3, 4 or 5 substituents each independently selected from alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE}, more preferably wherein
A₁, A₂ and A₃ are each carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0,1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
L₂ is a covalent bond; and
R₂ is aryl wherein said aryl is phenyl substituted with 0,1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyallcyl, methylenedioxy, nitro, -CH=NOR_{EE}, and -NR_{E}R_{F}.

8. A compound according to claim 1 wherein
A₁ and A₂ together with the carbon atoms to which they are attached form a five membered heterocycle containing a sulfur atom wherein the five membered heterocycle is substituted with 0,1, or 2 substituents selected from the group consisting of mercapto and oxo;
A₃ is carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
L₂ is a covalent bond; and
R₂ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F}.

9. A compound according to claim 1 wherein
L₁ is alkylene;
R₁ is aryl;
L₂ is a covalent bond; and
R₂ is heterocycle,
preferably wherein
L₁ is alkylene;
R₁ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylakyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE};
L₂ is a covalent bond; and
R₂ is heterocycle wherein said heterocycle is selected from the group consisting of azetidinyl, azepanyl, aziridinyl, furyl, morpholinyl, piperazinyl, piperidinyl, pyrazinyl, pyridazinyl, pyridinyl, pyrimidinyl, pyrrolidinyl and thienyl wherein said heterocycle is substituted with 0, 1, 2,or 3 substituents independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, arylalkoxycarbonyl, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE},
more preferably wherein
A₁, A₂ and A₃ are each carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
L₂ is a covalent bond; and
R₂ is heterocycle wherein said heterocycle is selected from the group consisting of pyridinyl, pyrrolidinyl and thienyl wherein said heterocycle is substituted with 0,1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, nitro, and -NR_{E}R_{F}.

10. A compound according to claim 1 wherein
L₁ is alkylene;
R₁ is aryl;
L₂ is alkylene; and
R₂ is aryl,
preferably wherein
L₁ is alkylene;
R₁ is aryl wherein said aryl is phenyl substituted with 0, 1, 2, 3, 4 or 5 substituents each independently selected from the group consisting of alkeriyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloallcylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE},
L₂ is alkylene; and
R₂ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, NR_{E}R_{F}, (NR_{E}R_{F})carbonyl, -N(R_{A})C(O)NR_{B}R_{C}, and -CH=NOR_{EE},
more preferably wherein
A₁, A₂ and A₃ are each carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
L₂ is alkylene wherein said alkylene is -CH₂-; and
R₂ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F}.

11. A compound according to claim 1 wherein
L₁ is alkylene;
R₁ is aryl;
L₂ is -(CH₂)ₚO(CH₂)_{q}-; and
R₂ is aryl,
preferably wherein
L₁ is alkylene;
R₁ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE},
L₂ is -(CH₂)ₚO(CH₂)_{q}-; and
R₂ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE},
or wherein
A₁, A₂ and A₃ are each carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
L₂ is -(CH₂)ₚO(CH₂)_{q}-;
p is 0;
q is 0; and
R₂ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F},
or wherein
A₁ and A₂ are each independently selected from the group consisting of alkoxycarbonyl, carboxy, hydroxy, (NR_{A}R_{B})carbonyl, and -S(O)₂OH;
A₃ is carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
L₂ is -(CH₂)ₚO(CH₂)_{q}-;
p is 0;
q is 0; and
R₂ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F}.

12. A compound according to claim 1 wherein
A₁ and A₂ are each independently selected from the group consisting of carboxy and -(NR_{C}S(O)₂R_{D}) carbonyl, wherein one of A1 or A2 is -(NR_{C}S (O)₂R_{D}) carbonyl;
A₃ is carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0,1,2,3,4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{c}, and -CH=NOR_{EE};
L₂ is -(CH₂)ₚO(CH₂)_{q}-;
p is 0;
q is 0; and
R₂ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE};
preferably wherein
R₁ is aryl wherein said aryl is phenyl substituted with 0,1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
R₂ is aryl wherein said aryl is phenyl substituted with 0,1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F}; and
R_{D} is selected from the group consisting of alkyl and aryl, wherein preferably said aryl is phenyl substituted with 0,1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, -NR_{E}R_{F}, and -N(R_{A})C(O)NR_{B}R_{C}.

13. A compound according to claim 1 wherein
A₁ and A₂ are each independently selected from the group consisting of carboxy and -(NR_{C}S (O)₂R_{D}) carbonyl, wherein one of A1 or A2 is -(NR_{C}S(O)₂R_{D}) carbonyl;
A₃ is carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE};
L₂ is -(CH₂)ₚO(CH₂)_{q}-;
p is 0;
q is 0;
R₂ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE}; and
R_{D} is heterocycle,
preferably wherein
R₁ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
R₂ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F}; and
R_{D} is heterocycle wherein said heterocycle is pyridinyl and thienyl wherein said heterocycle is substituted with 0,1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, nitro, -N(R_{A})C(O)NR_{B}R_{C}, and -NR_{E}R_{F}.

14. A compound according to claim 1 wherein
A₁ and A₂ are each independently selected from the group consisting of carboxy and -(NR_{C}S(O)₂R_{D}) carbonyl, wherein one of A1 or A2 is -(NR_{C}S(O)₂R_{D}) carbonyl;
A₃ is carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE};
L₂ is -(CH₂)ₚO(CH₂)_{q}-;
p is 0;
q is 0;
R₂ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE}; and
R_{D} is arylalkyl,
preferably wherein
R₁ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
R₂ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F}; and
R_{D} is arylalkyl wherein the aryl of arylalkyl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F}.

15. A compound according to claim 1 wherein
A₁, A₂ and A₃ are each carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
L₂ is -(CH₂)ₚO(CH₂)_{q}-;
p is 0;
q is 1; and
R₂ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F}.

16. A compound according to claim 1 wherein
L₁ is alkylene;
R₁ is aryl;
L₂ is -(CH₂)ₚO(CH₂)_{q}-; and
R₂ is cycloalkyl,
preferably wherein
L₁ is alkylene;
R₁ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carhonyl, -N(R_{A})C(O)NR_{B}R_{C}, and -CH=NOR_{EE};
L₂ is -(CH₂)ₚO(CH₂)_{q}-; and
R₂ is cycloalkyl,
more preferably wherein
A₁, A₂ and A₃ are each carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
L₂ is -(CH₂)ₚO(CH₂)_{q}-;
p is 0;
q is 0; and
R₂ is cycloalkyl.

17. A compound according to claim 1 wherein
L₁ is alkylene;
R₁ is aryl;
L₂ is -(CH₂)ₚO(CH₂)_{q}-; and
R₂ is cycloalkenyl,
preferably wherein
L₁ is alkylene;
R₁ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE};
L₂ is -(CH₂)ₚO(CH₂)_{q}-; and
R₂ is cycloalkenyl,
more preferably wherein
A₁, A₂ and A₃ are each carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0,1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
L₂ is -(CH₂)ₚO(CH₂)_{q}-;
p is 0;
q is 0; and
R₂ is cycloalkenyl.

18. A compound according to claim 1 wherein
L₁ is alkylene;
R₁ is aryl;
L₂ is -(CH₂)ₚO(CH₂)_{q}-; and
R₂ is heterocycle,
preferably wherein
L₁ is alkylene;
R₁ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE};
L₂ is -(CH₂)ₚO(CH₂)_{q}-; and
R₂ is heterocycle wherein said heterocycle is selected from the group consisting of furyl, pyrazinyl, pyridazinyl, pyridinyl, pyrimidinyl, and thienyl wherein said heterocycle is substituted with 0, 1, 2,or 3 substituents independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, arylalkoxycarbonyl, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE},
more preferably wherein
A₁, A₂ and A₃ are each carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
L₂ is -(CH₂)ₚO(CH₂)_{q}-;
p is 0;
q is 0; and
R₂ is heterocycle wherein said heterocycle is selected from the group consisting of pyridinyl and pyrimidinyl wherein said heterocycle is substituted with 0,1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, nitro, -N(R_{A})C(O)NR_{B}R_{C}, and -NR_{E}R_{F}.

19. A compound according to claim 1 wherein
L₁ is alkylene;
R₁ is aryl;
L₂ is -(CH₂)ₚS(CH₂)_{q}-; and
R₂ is aryl,
preferably wherein
L₁ is alkylene;
R₁ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE};
L₂ is -(CH₂)ₚS(CH₂)_{q}-; and
R₂ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloallcylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE},
more preferably wherein
A₁, A₂ and A₃ are each carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0,1, or 2 substituents selected from the group consisting of alkoxy, allcoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
L₂ is -(CH₂)ₚS(CH₂)_{q}-;
p is 0;
q is 0; and
R₂ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F}.

20. A compound according to claim 1 wherein
L₁ is alkylene;
R₁ is aryl;
L₂ is -(CH₂)ₚC(O)(CH₂)_{q}-; and
R₂ is aryl,
preferably wherein
L₁ is alkylene;
R₁ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE};
L₂ is -(CH₂)ₚC(O)(CH₂)_{q}-; and
R₂ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE},
more preferably wherein
A₁, A₂ and A₃ are each carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
L₂ is -(CH₂)ₚC(O)(CH₂)_{q}-;
p is 0;
q is 0; and
R₂ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F}.

21. A compound according to claim 1 wherein
L₁ is alkylene;
R₁ is aryl;
L₂ is -(CH₂)ₚC(OH)(CH₂)_{q}-; and
R₂ is aryl,
preferably wherein
L₁ is alkylene;
R₁ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE};
L₂ is -(CH₂)ₚC(OH)(CH₂)_{q}-; and
R₂ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE},
more preferably wherein
A₁, A₂ and A₃ are each carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
L₂ is -(CH₂)ₚC(OH)(CH₂)_{q}-;
p is 0;
q is 0; and
R₂ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F}.

22. A compound according to claim 1 wherein
L₁ is alkylene;
R₁ is aryl;
L₂ is -(CH₂)ₚCH=NO(CH₂)_{q}-; and
R₂ is aryl,
preferably wherein
L₁ is alkylene;
R₁ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE};
L₂ is -(CH₂)ₚCH=NO(CH₂)_{q}-; and
R₂ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE},
more preferably wherein
A₁, A₂ and A₃ are each carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0,1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
L₂ is -(CH₂)ₚCH=NO(CH₂)_{q}-;
p is 0;
q is an integer 0-1; and
R₂ is aryl wherein said aryl is phenyl substituted with 0,1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F}.

23. A compound according to claim 1 wherein
L₁ is alkylene;
R₁ is aryl;
L₂ is -(CH₂)ₚCH=NO(CH₂)_{q}-; and
R₂ is heterocycle,
preferably wherein
A₁, A₂ and A₃ are each carboxy;
A₄, A₅, A₆, A₇, A₈, A₉, A₁₀ and A₁₁ are each hydrogen;
L₁ is alkylene wherein said alkylene is -CH₂-;
R₁ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
L₂ is -(CH₂)ₚCH=NO(CH₂)_{q}-;
p is 0;
q is 0; and
R₂ is heterocycle wherein said heterocycle is pyranyl substituted with 0,1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, nitro, -N(R_{A})C(O)NR_{B}R_{C}, and -NR_{E}R_{F}.

24. A compound according to claim 1 wherein
L₁ is alkylene;
R₁ is cycloalkyl;
L₂ is absent; and
R₂ is absent,
preferably wherein
A₁, A₂ and A₃ are each carboxy;
A₄, A₅, A₆, A₇, A₈, A₉, A₁₀ and A₁₁ are each hydrogen;
L₁ is -CH₂-;
R₁ is cycloalkyl;
L₂ is absent; and
R₂ is absent.

25. A compound according to claim 1 wherein
L₁ is -(CH₂)ₘO(CH₂)ₙ-;
R₁ is aryl;
L₂ is absent; and
R₂ is absent,
preferably wherein
L₁ is -(CH₂)ₘO(CH₂)ₙ-;
R₁ is aryl wherein said aryl is phenyl substituted with 0, 1, 2, 3, 4 or 5 substituents each independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE};
L₂ is absent; and
R₂ is absent,
more preferably wherein
A₁, A₂ and A₃ are each carboxy;
A₄, A₅, A₆, A₇, A₈, A₉, A₁₀ and A₁₁ are each hydrogen;
L₁ is -(CH₂)ₘO(CH₂)ₙ-;
m is an integer 2-4;
n is 0;
R₁ is aryl wherein said aryl is phenyl substituted with 0, 1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, methylenedioxy, nitro, -CH=NOR_{EE}, and -NR_{E}R_{F};
L₂ is absent; and
R₂ is absent.

26. A compound according to claim 1 wherein
L₁ is alkylene;
R₁ is heterocycle;
L₂ is absent; and
R₂ is absent,
preferably wherein
L₁ is alkylene;
R₁ is heterocycle wherein said heterocycle is selected from the group consisting of furyl, pyrazinyl, pyridazinyl, pyridinyl, pyrimidinyl, and thienyl wherein said heterocycle is substituted with 0, 1, 2,or 3 substituents independently selected from the group consisting of alkenyl, alkenyloxy, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, alkynyloxy, arylalkoxycarbonyl, carboxy, carboxyalkyl, cyano, cyanoalkyl, ethylenedioxy, formyl, haloalkoxy, haloalkyl, haloalkylthio, halogen, hydroxy, hydroxyalkyl, mercapto, methylenedioxy, nitro, oxo, -NR_{E}R_{F}, (NR_{E}R_{F})carbonyl, -N(R_{A})C(O)NR_{B}R_{C}, and -CH=NOR_{EE};
L₂ is absent; and
R₂ is absent,
more preferably wherein
A₁, A₂ and A₃ are each carboxy;
A₄, A₅, A₆, A₇, A₈, A₉, A₁₀ and A₁₁ are each hydrogen;
L₁ is alkylene wherein alkylene is -CH₂-;
R₁ is heterocycle wherein said heterocycle is thienyl substituted with 0,1, or 2 substituents selected from the group consisting of alkoxy, alkoxycarbonyl, alkenyl, alkenyloxy, alkyl, alkylthio, cyano, formyl, haloalkoxy, haloalkyl, haloalkylthio, halogen, hydroxyalkyl, nitro, -N(R_{A})C(O)NR_{B}R_{C}, and -NR_{E}R_{F};
L₂ is absent; and
R₂ is absent.

27. A compound according to claim 6 selected from the group consisting of
5-({(4-chlorobenzyl)[(1S)-1,2,3,4-tetrahydro-1-naphthalenyl]amino}carbonyl)-1,2,4-benzenetricarboxylic acid;
5-({(4-bromobenzyl)[(1S)-1,2,3,4-tetrahydro-1-naphthalenyl]amino}carbonyl)-1,2,4-benzenetricarboxylic acid;
5-({(3-bromobenzyl)[(1S)-1,2,3,4-tetrahydro-1-naphthalenyl]amino}carbonyl)-1,2,4-benzenetricarboxylic acid;
5-({(3,4-dichlorobenzyl)[(1S)-1,2,3,4-tetrahydro-1-naphthalenyl]amino}carbonyl)-1,2,4-benzenetricarboxylic acid;
5-({(4-cyanobenzyl)[(1S)-1,2,3,4-tetrahydro-1-naphthalenyl]amino}carbonyl)-1,2,4-benzenetricarboxylic acid;
5-({(4-chloro-3-nitrobenzyl)[(1S)-1,2,3,4-tetrahydro-1-naptithalenyl]amino}carbonyl)-1,2,4-benzenetricarboxylic acid;
5-({[4-(dimethylamino)benzyl][(1S)-1,2,3,4-tetrahydro-1-naphthalenyl]amino}carbonyl)-1,2,4-benzenetricarboxylic acid;
5-({(3-chlorobenzyl)[(1S)-1,2,3,4-tetrahydro-1-naphthalenyl]amino}carbonyl)-1,2,4-benzenetricarboxylic acid;
5-({(3-cyanobenzyl)[(1S)-1,2,3,4-tetrahydro-1-naphthalenyl]amino}carbonyl)-1,2,4-benzenetricarboxylic acid;
5-({(2-phenylpropyl)[(1S)-1,2,3,4-tetrahydro-1-naphthalenyl]amino}carbonyl)-1,2,4-benzenetricarboxylic acid;
5-({(2-phenylethyl)[(1S)-1,2,3,4-tetrahydro-1-naphthalenyl]amino}carbonyl)-1,2,4-benzenetricarboxylic acid;
5-({benzyl[(1S)-1,2,3,4-tetrahydro-1-naphthalenyl]amino}carbonyl)-1,2,4-benzenetricarboxylic acid;
5-({(3-methoxybenzyl)[(1S)-1,2,3,4-tetrahydro-1-naphthalenyl]amino}carbonyl)-1,2,4-benzenetricarboxylic acid;
5-({(3-nitrobenzyl)[(1S)-1,2,3,4-tetrahydro-1-naphthalenyl]amino}carbonyl)-1,2,4-benzenetricarboxylic acid;
5-({[spiro[1,3-benzodioxol-5-yl-2,1'-cyclohexane]methyl][(1S)-1,2,3,4-tetrahydro-1-naphthalenyl]amino}carbonyl)-1,2,4-benzenetricarboxylic acid;
5-({[3-(allyloxy)benzyl][(1S)-1,2,3,4-tetrahydro-1-naphthalenyl]amino}carbonyl)-1,2,4-benzenetricarboxylic acid;
5-({[3,5-bis(trifluoromethyl)benzyl][(1S)-1,2,3,4-tetrahydro-1-naphthalenyl]amino}carbonyl)-1,2,4-benzenetricarboxylic acid;
5-({(1S)-1,2,3,4-tetrahydro-1-naphthalenyl[3-(trifluoromethyl)benzyl]amino}carbonyl)-1,2,4-benzenetricarboxylic acid;
5-({(3,5-difluorobenzyl)[(1S)-1,2,3,4-tetrahydro-1-naphthalenyl]amino}carbonyl)-1,2,4-benzenetricarboxylic acid;
5-({[4-fluoro-3-(trifluoromethyl)benzyl][(1S)-1,2,3,4-tetrahydro-1-naphthalenyl]amino}carbonyl)-1,2,4-benzenetricarboxylic acid;
5-({(3,5-dibromobenzyl)[(1S)-1,2,3,4-tetrahydro-1-naphthalenyl]amino}carbonyl)-1,2,4-benzenetricarboxylic acid;
5-({[3-fluoro-5-(trifluoromethyl)benzyl][(1S)-1,2,3,4-tetrahydro-1-naphthalenyl]amino}carbonyl)-1,2,4-benzenetricarboxylic acid;
5-({(3,5-dimethoxybenzyl)[(1S)-1,2,3,4-tetrahydro-1-naphthalenyl]amino}carbonyl)-1,2,4-benzenetricarboxylic acid;
5-({(2,3-dichlorobenzyl)[(1S)-1,2,3,4-tetrahydro-1-naphthalenylJamino}carbonyl)-1,2,4-benzenetricarboxylic acid;
5-({(2,4-dichlorobenzyl)[(1S)-1,2,3,4-tetrahydro-1-naphthalenyl]amino}carbonyl)-1,2,4-benzenetricarboxylic acid;
5-({(3,5-dimethylbenzyl)[(1S)-1,2,3,4-tetrahydro-1-naphthalenyl]amino}carbonyl)-1,2,4-benzenetricarboxylic acid;
5-[((1S)-1,2,3,4-tetrahydro-1-naphthalenyl{3-[(trifluoromethyl)sulfanyl]benzyl}amino)carbonyl]-1,2,4-benzenetricarboxylic acid;
5-({[3-(methylsulfanyl)benzyl][(1S)-1,2,3,4-tetrahydro-1-naphthalenyl]amino}carbonyl)-1,2,4-benzenetricarboxylic acid;
5-({{3-[(methoxyimino)methyl]benzyl}[(1S)-1,2,3,4-tetrahydro-1-naphthalenyl]amino}carbonyl)-1,2,4-benzenetricarboxylic acid;
5-({[3-(hydroxymethyl)benzyl][(1S)-1,2,3,4-tetrahydro-1-naphthalenyl]amino}carbonyl)-1,2,4-benzenetricarboxylic acid;
5-({(3-formylbenzyl)[(1S)-1,2,3,4-tetrahydro-1-naphthalenyl]amino}carbonyl)-1,2,4-benzenetricarboxylic acid;
5-({{3-[(tert-butoxyimino)methyl]benzyl}[(1S)-1,2,3,4-tetrahydro-1-naphthalenyl]amino}carbonyl)-1,2,4-henzenetricarboxylic acid; and
5-({{3-[isopropoxyimino)methyl]benzyl}[(1S)-1,2,3,4-tetrahydro-1-naphthalenyl]amino}carbonyl)-1,2,4-benzenetricarboxylic acid;
5-({(9H-fluoren-2-ylmethyl)[(1S)-1,2,3,4-tetrahydro-1-naphthalenyl]amino}carbonyl)-1,2,4-benzenetricarboxylic acid;
5-({[(9-hydroxy-9H-fluoren-2-yl)methyl][(1S)-1,2,3,4-tetrahydro-1-naphthalenyl]amino}carbonyl)-1,2,4-benzenetricarboxylic acid; and
5-({[(9-oxo-9H-fluoren-2-yl)methyl][(1S)-1,2,3,4-tetrahydro-1-naphthalenyl]amino}carbonyl)-1,2,4-benzenetricarboxylic acid;
5-({([1,1'-biphenyl]-4-ylmethyl)[(1S)-1,2,3,4-tetrahydro-1-naphthalenyl]amino}carbonyl)-1,2,4-benzenetricarboxylic acid;
5-({[(2'-chloro[1,1'-biphenyl]-4-yl)methyl][(1S)-1,2,3,4-tetrahydro-1-naphthalenyl]amino}carbonyl)-1,2,4-benzenetricarboxylic acid;
5-({[(3',5'-dichloro[1,1'-biphenyl]-4-yl)methyl][(1S)-1,2,3,4-tetrahydro-1-naphthalenyl]amino}carbonyl)-1,2,4-benzenetricarboxylic acid;
5-({[(2'-methoxy[1,1'-biphenyl]-4-yl)methyl][(1S)-1,2,3,4-tetrahydro-1-naphthalenyl]amino}carbonyl)-1,2,4-benzenetricarboxylic acid;
5-({[(4'-chloro[1,1'-biphenyl]-4-yl)methyl][(1S)-1,2,3,4-tetrahydro-1-naphthalenyl]amino}carbonyl)-1,2,4-benzenetricarboxylic acid;
5-({[(4'-fluoro[1,1'-biphenyl]-4-yl)methyl][(1S)-1,2,3,4-tetrahydro-1-naphthalenyl]amino}carbonyl)-1,2,4-benzenetricarboxylic acid;
5-({[(4'-methoxy[1,1'-biphenyl]-4-yl)methyl][(1S)-1,2,3,4-tetrahydro-1-naphthalenyl]amino}carbonyl)-1,2,4-benzenetricarboxylic acid;
5-[((1S)-1,2,3,4-tetrahydro-1-naphthalenyl{[2'-(trifluoromethyl)[1,1'-biphenyl]-4-yl]methyl}amino)carbonyl]-1,2,4-benzenetricarboxylic acid;
5-({[(2'-methyl[1,1'-biphenyl]-4-yl)methyl][(1S)-1,2,3,4-tetrahydro-1-naphthalenyl]amino}carbonyl)-1,2,4-benzenetricarboxylic acid;
5-[((1S)-1,2,3,4-tetrahydro-1-naphthalenyl{[4'-(trifluoromethyl)[1,1'-biphenyl]-2-yl]methyl}amino)carbonyl]-1,2,4-benzenetricarboxylic acid;
5-({[(4'-fluoro[1,1'-biphenyl]-2-yl)methyl][(1S)-1,2,3,4-tetrahydro-1-naphthalenyl]amino}carbonyl)-1,2,4-benzenetricarboxylic acid;
5-({[(4'-fluoro[1,1'-biphenyl]-3-yl)methyl][(1S)-1,2,3,4-tetrahydro-1-naphthalenyl]amino}carbonyl)-1,2,4-benzenetricarboxylic acid;
5-[((1S)-1,2,3,4-tetrahydro-1-naphthalenyl{[4'-(trifluoromethyl)[1,1'-biphenyl]-4-yl]methyl}amino)carbonyl]-1,2,4-benzenetricarboxylic acid;
5-({([1,1'-biphenyl]-2-ylmethyl)[(1S)-1,2,3,4-tetrahydro-1-naphthalenyl]amino}carbonyl)-1,2,4-benzenetricarboxylic acid;
5-({([1,1'-biphenyl]-3-ylmethyl)[(1S)-1,2,3,4-tetrahydro-1-naphthalenyl]amino}carbonyl)-1,2,4-benzenetricarboxylic acid;
5-({[(2-methyl[1,1'-biphenyl]-3-yl)methyl][(1S)-1,2,3,4-tetrahydro-1-naphthalenyl]amino}carbonyl)-1,2,4-benzenetricarboxylic acid;
5-[((1S)-1,2,3,4-tetrahydro-1-naphthalenyl{[4'-(trifluoromethyl)[1,1'-biphenyl]-3-yl]methyl}amino)carbonyl]-1,2,4-benzenetricarboxylic acid;
5-({[(5'-fluoro-2'-methoxy[1,1'-biphenyl]-3-yl)methyl][(1S)-1,2,3,4-tetrahydro-1-naphthalenyl]amino}carbonyl)-1,2,4-benzenetricarboacylic acid;
5-({[(5'-chloro-2'-methoxy[1,1'-biphenyl]-3-yl)methyl][(1S)-1,2,3,4-tetrahydro-1-naphthalenyl]amino}carbonyl)-1,2,4-benzenetricarboxylic acid;
5-({[(3',5'-dichloro[1,1'-biphenyl]-3-yl)methyl][(1S)-1,2,3,4-tetrahydro-1-naphthalenyl]amino}carbonyl)-1,2,4-benzenetricarboxylic acid;
5-({[(2'-methoxy[1,1'-biphenyl]-3-yl)methyl][(1S)-1,2,3,4-tetrahydro-1-naphthalenyl]amino}carbonyl)-1,2,4-benzenetricarboxylic acid;
5-({[(2'-chloro[1,1'-biphenyl]-3-yl)methyl][(1S)-1,2,3,4-tetrahydro-1-naphthalenyl]amino}carbonyl)-1,2,4-benzenetricarboxylic acid;
5-({[(2',5'-dimethoxy[1,1'-biphenyl]-3-yl)methyl][(1S)-1,2,3,4-tetrahydro-1-naphthalenyl]amino}carbonyl)-1,2,4-benzenetricarboxylic acid;
5-({[(3'-chloro[1,1'-biphenyl]-3-yl)methyl][(1S)-1,2,3,4-tetrahydro-1-naphthalenyl]amino}carbonyl)-1,2,4-benzenetricarboxylic acid;
5-({[(3',4'-dichloro[1,1'-biphenyl]-3-yl)methyl][(1S)-1,2,3,4-tetrahydro-1-naphthalenyl]amino}carbonyl)-1,2,4-benzenetricarboxylic acid;
5-({[(3'-chloro-4'-fluoro[1,1'-biphenyl]-3-yl)methyl][(1S)-1,2,3,4-tetrahydro-1-naphthalenyl]amino}carbonyl)-1,2,4-benzenetricarboxylic acid;
5-({[(3'-nitro[1,1'-biphenyl]-3-yl)methyl][(1S)-1,2,3,4-tetrahydro-1-naphthalenyl]amino}carbonyl)-1,2,4-benzenetricarboxylic acid;
5-({[(3'-methoxy[1,1'-biphenyl]-3-yl)methyl][(1S)-1,2,3,4-tetrahydro-1-naphthalenyl]amino}carbonyl)-1,2,4-benzenetricarboxylic acid;
5-({[(4'-chloro[1,1'-biphenyl]-3-yl)methyl][(1S)-1,2,3,4-tetrahydro-1-naphthalenyl]amino}carbonyl)-1,2,4-benzenetricarboxylic acid; and
5-({(1S)-1,2,3,4-tetrahydro-1-naphthalenyl[(3',4',5'-trimethoxy[1,1'-biphenyl]-3-yl)methyl]amino}carbonyl)-1,2,4-benzenetricarboxylic acid;
6-({[(3',4'-dichloro-1,1'-biphenyl-3-yl)methyl][(1S)-1,2,3,4-tetrahydro-1-naphthalenyl]amino}carbonyl)-1,3-dioxo-1,3-dihydro-2-benzothiophene-5-carboxylic acid;
5-({[4-(1-pyrrolidinyl)benzyl][(1S)-1,2,3,4-tetrahydro-1-naphthalenyl]amino}carbonyl)-1,2,4-benzenetricarboxylic acid;
5-({[4-(3-pyridinyl)benzyl][(1S)-1,2,3,4-tetrahydro-1-naphthalenyl]amino}carbonyl)-1,2,4-benzenetricarboxylic acid;
5-({(1S)-1,2,3,4-tetrahydro-1-naphthalenyl[3-(3-thienyl)benzyl]amino}carbonyl)-1,2,4-benzenetricarboxylic acid; and
5-({(1S)-1,2,3,4-tetrahydro-1-naphthalenyl[3-(2-thienyl)benzyl]amino}carbonyl)-1,2,4-benzenetricarboxylic acid;
5-({(3-benzylbenzyl)[(1S)-1,2,3,4-tetrahydro-1-naphthalenyl]amino}carbonyl)-1,2,4-benzenetricarboxylic acid;
5-({[3-(4-chlorophenoxy)benzyl][(1S)-1,2,3,4-tetrahydro-1-naphthalenyl]amino}carbonyl)-1,2,4-benzenetricarboxylic acid;
5-[((1S)-1,2,3,4-tetrahydro-1-naphthalenyl{3-[3-(trifluoromethyl)phenoxy]benzyl}amino)carbonyl]-1,2,4-benzenetricarboxylic acid;
5-({[3-(4-methoxyphenoxy)benzyl][(1S)-1,2,3,4-tetrahydro-1-naphthalenyl]amino}carbonyl)-1,2,4-benzenetricarboxylic acid;
5-({[3-(3,4-dichlorophenoxy)benzyl][(1S)-1,2,3,4-tetrahydro-1-naphthalenyl]amino}carbonyl)-1,2,4-benzenetricarboxylic acid;
5-({[3-(4-chlorophenoxy)benzyl][(1S)-1,2,3,4-tetrahydro-1-naphthalenyl]amino}carbonyl)-1,2,4-benzenetricarboxylic acid;
5-({[3-(4-tert-butylphenoxy)benzyl][(1S)-1,2,3,4-tetrahydro-1-naphthalenyl]amino}carbonyl)-1,2,4-benzenetricarboxylic acid;
5-({[3-(4-methylphenoxy)benzyl][(1S)-1,2,3,4-tetrahydro-1-naphthalenyl]amino}carbonyl)-1,2,4-benzenetricarboxylic acid;
5-({[3-(3,5-dichlorophenoxy)benzyl][(1S)-1,2,3,4-tetrahydro-1-naphthalenyl]amino}carbonyl)-1,2,4-benzenetricarboxylic acid;
5-({[3-(4-nitrophenoxy)benzyl][(1S)-1,2,3,4-tetrahydro-1-naphthalenyl]amino}carbonyl)-1,2,4-benzenetricarboxylic acid;
5-({[3-(4-cyanophenoxy)benzyl][(1S)-1,2,3,4-tetrahydro-1-naphthalenyl]amino}carbonyl)-1,2,4-benzenetricarboxylic acid; and
5-({[3-(4-fluorophenoxy)benzyl][(1S)-1,2,3,4-tetrahydro-1-naphthalenyl]amino}carbonyl)-1,2,4-benzenetricarboxylic acid;
5-({(3-phenoxybenzyl)[(1S)-1,2,3,4-tetrahydro-1-naphthalenyl]amino}carbonyl)-1,2,4-benzenetricarboxylic acid.
2-[(methylamino)carbonyl]-5-({(3-phenoxybenzyl)[(1S)-1,2,3,4-tetrahydro-1-naphthalenyl]amino}carbonyl)terephthalic acid;
4-[(methylamino)carbonyl]-6-({(3-phenoxybenzyl)[(1S)-1,2,3,4-tetrahydro-1-naphthalenyl]amino}carbonyl)isophthalic acid;
2-[(cyanoamino)carbonyl]-5-({(3-phenoxybenzyl)[(1S)-1,2,3,4-tetrahydro-1-naphthalenyl]amino}carbonyl)terephthalic acid;
4-[(cyanoamino)carbonyl]-6-({(3-phenoxybenzyl)[(1S)-1,2,3,4-tetrahydro-1-naphthalenyl]amino}carbonyl)isophthalic acid;
2-(aminocarbonyl)-5-({(3-phenoxybenzyl)[(1S)-1,2,3,4-tetrahydro-1-naphthalenyl]amino}carbonyl)terephthalic acid;
4-(aminocarbonyl)-6-({(3-phenoxybenzyl)[(1S)-1,2,3,4-tetrahydro-1-naphthalenyl]amino}carbonyl)isophthalic acid;
2-(methoxycarbonyl)-5-({(3-phenoxybenzyl)[(1S)-1,2,3,4-tetrahydro-1-naphthalenyl]amino}carbonyl)terephthalic acid;
4-(methoxycarbonyl)-6-({(3-phenoxybenzyl)[(1S)-1,2,3,4-tetrahydro-1-naphthalenyl]amino}carbonyl)isophthalic acid;
2-hydroxy-5-({(3-phenoxybenzyl)[(1S)-1,2,3,4-tetrahydro-1-naphthalenyl]amino}carbonyl)terephthalic acid;
4-hydroxy-6-({(3-phenoxybenzyl)[(1S)-1,2,3,4-tetrahydro-1-naphthalenyl]amino}carbonyl)isophthalic acid;
2-({(3-phenoxybenzyl)[(1S)-1,2,3,4-tetrahydro-1-naphthalenyl]amino}carbonyl)-5-sulfoterephthalic acid; and
4-({(3-phenoxybenzyl)[(1S)-1,2,3,4-tetrahydro-1-naphthalenyl]amino}carbonyl)-6-sulfoisophthalic acid;
4-({[(4-methoxyphenyl)sulfonyl]amino}carbonyl)-6-({(3-phenoxybenzyl)[(1S)-1,2,3,4-tetrahydro-1-naphthalenyl]amino}carbonyl)isophthalic acid;
2-({[(4-methoxyphenyl)sulfonyl]amino}carbonyl)-5-({(3-phenoxybenzyl)[(1S)-1,2,3,4-tetrahydro-1-naphthalenyl]amino}carbonyl)terephthalic acid;
4-({[(3-chloro-4-methylphenyl)sulfonyl]amino}carbonyl)-6-({(3-phenoxybenzyl)[(1S)-1,2,3,4-tetrahydro-1-naphthalenyl]amino}carbonyl)isophthalic acid;
2-({[(3-chloro-4-methylphenyl)sulfonyl]amino}carbonyl)-5-({(3-phenoxybenzyl)[(1S)-1,2,3,4-tetrahydro-1-naphthalenyl]amino}carbonyl)terephthalic acid;
4-({[(2-chlorophenyl)sulfonyl]amino}carbonyl)-6-({(3-phenoxybenzyl)[(1S)-1,2,3,4-tetrahydro-1-naphthalenyl]amino}carbonyl)isophthalic acid;
2-({[(2-chlorophenyl)sulfonyl]amino}carbonyl)-5-({(3-phenoxybenzyl)[(1S)-1,2,3,4-tetrahydro-1-naphthalenyl]amino}carbonyl)terephthalic acid;
4-({[(3-chlorophenyl)sulfonyl]amino}carbonyl)-6-({(3-phenoxybenzyl)[(1S)-1,2,3,4-tetrahydro-1-naphthalenyl]amino}carbonyl)isophthalic acid;
2-({[(3-chlorophenyl)sulfonyl]amino}carbonyl)-5-({(3-phenoxybenzyl)[(1S)-1,2,3,4-tetrahydro-1-naphthalenyl]amino}carbonyl)terephthalic acid;
4-({[(4-chlorophenyl)sulfonyl]amino}carbonyl)-6-({(3-phenoxybenzyl)[(1S)-1,2,3,4-tetrahydro-1-naphthalenyl]amino}carbonyl)isophthalic acid;
2-({[(4-chlorophenyl)sulfonyl]amino}carbonyl)-5-({(3-phenoxybenzyl)[(1S)-1,2,3,4-tetrahydro-1-naphthalenyl]amino}carbonyl)terephthalic acid;
4-({[(4-nitrophenyl)sulfonyl]amino}carbonyl)-6-({(3-phenoxybenzyl)[(1S)-1,2,3,4-tetrahydro-1-naphthalenyl]amino}carbonyl)isophthalic acid;
2-({[(4-nitrophenyl)sulfonyl]amino}carbonyl)-5-({(3-phenoxybenzyl)[(1S)-1,2,3,4-tetrahydro-1-naphthalenyl]amino}carbonyl)terephthalic acid;
4-({[(4-bromophenyl)sulfonyl]amino}carbonyl)-6-({(3-phenoxybenzyl)[(1S)-1,2,3,4-tetrahydro-1-naplithalenyl]amino}carbonyl)isophthalic acid;
2-({[(4-bromophenyl)sulfonyl]amino}carbonyl)-5-({(3-phenoxybenzyl)[(1S)-1,2,3,4-tetrahydro-1-naphthalenyl]amino}carbonyl)terephthalic acid;
4-({[(3-nitrophenyl)sulfonyl]amino}carbonyl)-6-({(3-phenoxybenzyl)[(1S)-1,2,3,4-tetrahydro-1-naphthalenyl]amino}carbohyl)isophthalic acid;
2-({[(3-nitrophenyl)sulfonyl]amino}carbonyl)-5-({(3-phenoxybenzyl)[(1S)-1,2,3,4-tetrahydro-1-naphthalenyl]amino}carbonyl)terephthalic acid;
4-({[(phenyl)sulfonyl]amino}carbonyl)-6-({(3-pbenoxybenzyl)[(1S)-1,2,3,4-tetrahydro-1-naphthalenyl]amino}carbonyl)isophthalic acid;
2-({[(phenyl)sulfonyl]amino}carbonyl)-5-({(3-phenoxybenzyl)[(1S)-1,2,3,4-tetrahydro-1-naphthalenyl]amiao}carbonyl)terephthalic acid;
4-({[(2-nitrophenyl)sulfonyl]amino}carbonyl)-6-({(3-phenoxybenzyl)[(1S)-1,2,3,4-tetrahydro-1-naphthalenyl]amino}carbonyl)isophthalic acid;
2-({[(2-nitrophenyl)sulfonyl]amino}carbonyl)-5-({(3-phenoxybenzyl)[(1S)-1,2,3,4-tetrahydro-1-naphthalenyl]amino}carbonyl)terephthalic acid;
4-({[(2-methylphenyl)sulfonyl]amino}carbonyl)-6-({(3-phenoxybenzyl)[(1S)-1,2,3,4-tetrahydro-1-naphthalenyl]amino}carbonyl)isophthalic acid;
2-({[(2-methylphenyl)sulfonyl]amino}carbonyl)-5-({(3-phenoxybenzyl)[(1S)-1,2,3,4-tetrahydro-1-naphthalenyl]amino}carbonyl)terephthalic acid;
4-{[({4-[(2,3-dihydroxypropyl)amino]phenyl}sulfonyl)amino]carbonyl}-6-({(3-phenoxybenzyl)[(1S)-1,2,3,4-tetrahydro-1-naphthalenyl]amino}carbonyl)isophthalic acid;
2-{[({4-[(2,3-dihydroxypropyl)amino]phenyl}sulfonyl)amino]carbonyl}-5-({(3-phenoxybenzyl) [(1S)-1,2,3,4-tetrahydro-1-naphthalenyl]amino}carbonyl)terephthalic acid;
2-({[(4-{[(methylamino)carbonyl]amino}phenyl)sulfonyl]amino}carbonyl)-5-({(3-phenoxybenzyl)[(1S)-1,2,3,4-tetrahydro-1-naphthalenyl]amino}carbonyl)terephthalic acid;
4-({[(4-{[(methylamino)carbonyl]amino}phenyl)sulfonyl]amino}carbonyl)-6-({(3-phenoxybenzyl)[(1S)-1,2,3,4-tetrahydro-1-naphthalenyl]amino}carbonyl)isophthalic acid;
4-[({[4-(butyrylamino)phenyl]sulfonyl}amino)carbonyl]-6-({(3-phenoxybenzyl)[(1S)-1,2,3,4-tetrahydro-1-naphthalenyl]amino}carbonyl)isophthalic acid;
2-[({[4-(butyrylamino)phenyl]sulfonyl}amino)carbonyl]-5-({(3-phenoxybenzyl)[(1S)-1,2,3,4-tetrahydro-1-naphthalenyl]amino}carbonyl)terephthalic acid;
4-({[(3,4-difluorophenyl)sulfonyl]amino}carbonyl)-6-({(3-phenoxybenzyl)[(1S)-1,2,3,4-tetrahydro-1-naphthalenyl]amino}carbopyl)isophthalic acid;
2-({[(3,4-difluorophenyl)sulfonyl]amino}carbonyl)-5-({(3-phenoxybenzyl)[(1S)-1,2,3,4-tetrahydro-1-naphthalenyl]amino}carbonyl)terephthalic acid;
4-({[(2,5-difluorophenyl)sulfonyl]amino}carbonyl)-6-({(3-phenoxybenzyl)[(1S)-1,2,3,4-tetrahydro-1-naphthalenyl]amino}cacbonyl)isophthalic acid;
2-({[(2,5-difluorophenyl)sulfonyl]amino}carbonyl)-5-({(3-phenoxybenzyl)[(1S)-1,2,3,4-tetrahydro-1-naphthalenyl]amino}carbonyl)terephthalic acid;
4-({[(2-methoxy-4-methylphenyl)sulfonyl]amino}carbonyl)-6-({(3-phenoxybenzyl)[(1S)-1,2,3,4-tetrahydro-1-naphthalenyl]amino}carbonyl)isophthalic acid;
2-({[(2-methoxy-4-methylphenyl)sulfonyl]amino}carbonyl)-5-({(3-phenoxybeozyl)[(1S)-1,2,3,4-tetrahydro-1-naphthalenyl]amino}carbonyl)terephthalic acid;
4-({[(4-methylphenyl)sulfonyl]amino}carbonyl)-6-({(3-phenoxybenzyl)[(1S)-1,2,3,4-tetrahydro-1-naphthalenyl]amino}carbonyl)isophthalic acid;
2-({[(4-methylphenyl)sulfonyl]amino}carbonyl)-5-({(3-phenoxybenzyl)[(1S)-1,2,3,4-tetrahydro-1-naphthaleayl]amino}carbonyl)terephthalic acid;
2-({[(2-fluorophenyl)sulfonyl]amino}carbonyl)-5-({(3-phenoxybenzyl)[(1S)-1,2,3,4-tetrahydro-1-naphthalenyl]amino}carbonyl)terephthalic acid;
4-({[(2-fluorophenyl)sulfonyl]amino}carbonyl)-6-({(3-phenoxybenzyl)[(1S)-1,2,3,4-tetrahydro-1-naphthalenyl]amino}carbonyl)isophthalic acid;
2-({[(4-hydroxyphenyl)sulfonyl]amino}carbonyl)-5-({(3-phenoxybenzyl)[(1S)-1,2,3,4-tetrahydro-1-naphthalenyl]amino}carbonyl)terephthalic acid; and
4-({[(4-hydroxyphenyl)sulfonyl]amino}carbonyl)-6-({(3-phenoxybenzyl)[(1S)-1,2,3,4-tetrahydro-1-naphthalenyl]amino}carbonyl)isophthalic acid;
4-({[(5-methyl-2-pyridinyl)sulfonyl]amino}carbonyl)-6-({(3-phenoxybenzyl)[(1S)-1,2,3,4-tetrahydro-1-naphthalenyl]amino}carbonyl)isophthalic acid;
2-({[(5-methyl-2-pyridinyl)sulfonyl]amino}carbonyl)-5-({(3-phenoxyberizyl)[(1S)-1,2,3,4-tetrahydro-1-naphthalenyl]amino}carbonyl)terephthalic acid;
4-({[(5-nitro-2-thienyl)sulfonyl]amino}carbonyl)-6-({(3-phenoxybenzyl)[(1S)-1,2,3,4-tetrahydro-1-naphthalenyl]amino}carbonyl)isophthalic acid;
2-({[(5-nitro-2-thienyl)sulfonyl]amino}carbonyl)-5-({(3-phenoxybenzyl)[(1S)-1,2,3,4-tetrahydro-1-naphthalenyl]amino}carbonyl)terephthalic acid;
2-({[(5-chloro-2-thienyl)sulfonyl]amino}carhonyl)-5-({(3-phenoxybenzyl)[(1S)-1,2,3,4-tetrahydro-1-naphthalenyl]amino}carbonyl)terephthalic acid; and
4-({[(5-chloro-2-thienyl)sulfonyl]amino}carbonyl)-6-({(3-phenoxybenzyl)[(1S)-1,2,3,4-tetrahydro-1-naphthalenyl]amino}carbonyl)isophthalic acid;
4-{ [(benzylsulfonyl)amino]carbonyl}-6-({(3-phenoxybenzyl)[(1S)-1,2,3,4-tetrahydro-1-naphthalenyl]amino}carbonyl)isophthalic acid; and
2-{[(benzylsulfonyl)amino]carbonyl}-5-({(3-phenoxybenzyl)[(1S)-1,2,3,4-tetrahydro-1-naphthalenyl]amino}carbonyl)terephthalic acid;
5-({[3-(benzyloxy)benzyl][(1S)-1,2,3,4-tetrahydro-1-naphthalenyl]amino}carbonyl)-1,2,4-benzenetricarboxylic acid,
5-({[3-(cyclohexyloxy)benzyl][(1S)-1,2,3,4-tetrahydro-1-naphthalenyl]amino}carbonyl)-1,2,4-benzenetricarboxylic acid;
5-({{3-[exo-bicyclo[2.2.1]hept-2-yloxy]benzyl}[(1S)-1,2,3,4-tetrahydro-1-naphthalenyl]amino}carbonyl)-1,2,4-benzenetricarboxylic acid;
5-({[3-(2-cyclohexen-1-yloxy)benzyl][(1S)-1,2,3,4-tetrahydio-1-naphthalenyl]amino}carbonyl)-1,2,4-benzenetricarboxylic acid;
5-({[3-(4-pyridinyloxy)benzyl][(1S)-1,2,3,4-tetrahydro-1-naphthalenyl]amino}carbonyl)-1,2,4-benzenetricarboxylic acid; and
5-({[3-(2-pyrinudinyloxy)benzyl][(1S)-1,2,3,4-tetrahydro-1-naphthalenyl]amino}carbonyl)-1,2,4-benzenetricarboxylic acid
5-({ [3-(phenylsulfanyl)benzyl][(1S)-1,2,3,4-tetrahydro-1-naphthalenyl]amino}carbonyl)-1,2,4-benzenetricarboxylic acid;
5-({{3-[(4-methoxyphenyl)sulfanyl]benzyl}[(1S)-1,2,3,4-tetrahydro-1-naphthalenyl]amino}carbonyl)-1,2,4-benzenetricarboxylic acid;
5-({{3-[(4-nitrophenyl)sulfanyl]benzyl}[(1S)-1,2,3,4-tetrahydro-1-naphthalenyl]amino}carbonyl)-1,2,4-benzenetricarboxylic acid; and
5-({{3-[(4-chlorophenyl)sulfanyl]benzyl}[(1S)-1,2,3,4-tetrahydro-1-naphthalenyl]amino}carbonyl)-1,2,4-benzenetricarboxylic acid;
5-({(3-benzoylbenzyl)[(1S)-1,2,3,4-tetrahydro-1-naphthalenyl]amino}carbonyl)-1,2,4-benzenetricarboxylic acid,
5-({{3-[hydroxy(phenyl)methyl]benzyl}[(1S)-1,2,3,4-tetrahydro-1-naphthalenyl]amino}carbonyl)-1,2,4-benzenetricarboxylic acid;
5-({{3-[(phenoxyimino)methyl]benzyl}[(1S)-1,2,3,4-tetrahydro-1-naphthalenyl]amino}carbonyl)-1,2,4-benzenetricarboxylic acid;
5-({(3-{[(henzyloxy)imino]methyl}benzyl)[(1S)-1,2,3,4-tetrahydro-1-naphthalenyl]amino}carbonyl)-1,2,4-benzenetricarboxylic acid;
5-({[3-({[(4-nitrobenzyl)oxy]imino}methyl)benzyl][(1S)-1,2,3,4-tetrahydro-1-naphthalenyl]amino}carbonyl)-1,2,4-benzenetricarboxylic acid; and
5-({[3-({[(2,3,4,5,6-pentafluorobenzyl)oxy]imino}methyl)benzyl][(1S)-1,2,3,4-tetrahydro-1-naphthalenyl]amino}carbonyl)-1,2,4-benzenetricarboxylic acid.
5-{[(1S)-1,2,3,4-tetrahydro-1-naphthalenyl(3-{[(tetrahydro-2H-pyran-2-yloxy)imino]methyl}benzyl)amino]carbonyl}-1,2,4-benzenetricarboxylic acid.
5-({(cyclohexylmethyl)[(1S)-1,2,3,4-tetrahydro-1-naphthalenyl]amino}carbonyl)-1,2,4-benzenetricarboxylic acid;
5-({(4-phenoxybutyl)[(1S)-1,2,3,4-tetrahydro-1-naphthalenyl]amino}carbonyl)-1,2,4-benzenetricarboxylic acid;
5-({{[5-(ethoxycarbonyl)-2-thienyl]methyl}[(1S)-1,2,3,4-tetrahydro-1-naphthalenyl]amino}carbonyl)-1,2,4-benzenetricarboxylic acid;

28. A pharmaceutical composition comprising a therapeutically effective amount of a compound according to claim 1 or a pharmaceutically acceptable salt thereof in combination with a pharmaceutically acceptable carrier.

29. Use of a compound according to claim 1 or a pharmaceutically acceptable salt thereof for manufacturing a medicament for treating bladder over-activity or urinary incontinence or pain in a host mammal in need of such treatment.

## Patentansprüche

1. Eine Verbindung von Formel (I) 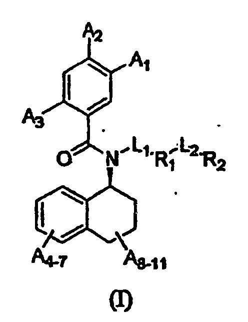 oder ein pharmazeutisch verträgliches Salz davon, worin
A₁ und A₂ jeweils unabhängig gewählt sind aus der Gruppe bestehend aus Alkoxycarbonyl, Alkylcarbonyloxy, Carboxy, Hydroxy, Hydroxyalkyl, (NR_{A}R_{B})Carbonyl, (NR_{C}S(O)₂R_{D})Carbonyl, -S(O)₂OH und Tetrazolyl; oder
A₁ und A₂ zusammengenommen mit den Kohlenstoffatomen, an welche sie gebunden sind, bilden einen fünf-gliedrigen Heterocyclus, enthaltend ein Schwefelatom, worin der fünf-gliedrige Heterocyclus wahlweise substituiert ist mit 1 oder 2 Substituenten, gewählt aus der Gruppe bestehend aus Mercapto und Oxo;
A₃ ist gewählt aus der Gruppe bestehend aus Alkoxycarbonyl, Alkylcarbonyloxy, Carboxy, Hydroxy, Hydroxyalkyl, (NR_{A}R_{B}) Carbonyl, (NR_{C}S(O)₂RD)carbonyl, -S (O)₂OH und Tetrazolyl;
A₄, A₅, A₆ und A₇ sind jeweils unabhängig gewählt aus der Gruppe bestehend aus Wasserstoff, Alkoxy, Alkoxycarbonyl, Alkenyl, Alkyl, Alkylcarbonyl, Alkylcarbonyloxy, Alkinyl, Carboxy, Cyano, Haloalkoxy, Haloalkyl, Halogen, Hydroxy, Hydroxyalkyl, Nitro, -NR_{E}R_{F} und (NR_{E}R_{F})Carbonyl;
A₈, A₉, A₁₀ und A₁₁ sind jeweils unabhängig gewählt aus der Gruppe bestehend aus Wasserstoff, Alkoxy, Alkoxycarbonyl, Alkenyl, Alkyl, Alkylcarbonyl, Alkylcarbonyloxy, Alkinyl, Carboxy, Haloalkoxy, Haloalkyl, Halogen, Hydroxy, Hydroxyalkyl, -NR_{E}R_{F} und (NR_{E}R_{F})Carbonyl und Oxo;
R_{A} und R_{B} sind jeweils unabhängig gewählt aus der Gruppe bestehend aus Wasserstoff, Alkyl und Cyano;
R_{C} ist gewählt aus der Gruppe bestehend aus Wasserstoff und Alkyl;
R_{D} ist gewählt aus der Gruppe bestehend aus Alkoxy, Alkyl, Aryl, Arylalkoxy, Arylalkyl, Haloalkoxy und Haloalkyl;
R_{E} und R_{F} sind jeweils unabhängig gewählt aus der Gruppe bestehend aus Wasserstoff, Alkyl, Alkylcarbonyl, Formyl und Hydroxyalkyl;
L₁ ist gewählt aus der Gruppe bestehend aus Alkenylen, Alkylen, Alkinylen, -(CH₂)ₘO (CH₂)ₙ-, -(CH₂)ₘS(CH₂)ₙ- und -(CH₂)ₚC(O) (CH₂)_{q}-, worin jede Gruppe gezeichnet ist mit dem linken Ende gebunden an N und das rechte Ende gebunden an R₁;
m ist eine ganze Zahl von 0-10;
n ist eine ganze Zahl von 0-10;
R₁ ist gewählt aus der Gruppe bestehend aus Aryl, Cycloalkenyl, Cycloalkyl und Heterocyclus;
L₂ ist nicht vorhanden oder gewählt aus der Gruppe bestehend aus einer kovalenten Bindung, Alkenylen, Alkylen, Alkinylen, -(CH₂)ₚO(CH₂)_{q}, -(CH₂)ₚS(CH₂)_{q}, -(CH₂)ₚC(O)(CH₂)_{q}, -(CH₂)ₚC (OH)(CH₂)_{q} und -(CH₂)ₚCH=N(CH₂)_{q}-, worin jede Gruppe gezeichnet ist mit dem linken Ende gebunden an R₁ und das rechte Ende gebunden an R₂;
p ist eine ganze Zahl von 0-10;
q ist eine ganze Zahl von 0-10; und
R₂ ist nicht vorhanden oder gewählt aus der Gruppe bestehend aus Aryl, Cycloalkenyl, Cycloalkyl und Heterocyclus;
worin bei jedem Vorkommen die Arylgruppe substituiert ist mit 0, 1, 2, 3, 4 oder 5 Substituenten, jeweils unabhängig gewählt aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F}) Carbonyl, -N(R_{A}) C(O) NR_{B}R_{C} und -CH=NOR_{EE}, worin R_{EE} gewählt ist aus Wasserstoff und Alkyl, wie hierin definiert; Die Cycloalkenyl- und die Cycloalkylgruppen sind substituiert mit 0, 1, 2, 3, oder 5 Substituenten, jeweils unabhängig gewählt aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F}) Carbonyl und -CH=NOR_{EE}, worin R_{EE} gewählt ist aus Wasserstoff und Alkyl, wie hierin definiert; die Heterocyclusgruppe kann substituiert sein mit 0, 1, 2 oder 3 Substituenten unabhängig gewählt aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Arylalkoxycarbonyl, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F}) Carbonyl, -N(R_{A})C(O) NR_{B}R_{C} und -CH=NOR_{EE}, worin R_{EE} gewählt ist aus Wasserstoff und Alkyl, wie hierin definiert.

2. Eine Verbindung gemäß Anspruch 1, worin
A₁ und A₂ jeweils unabhängig gewählt sind aus der Gruppe bestehend aus Alkoxycarbonyl, Carboxy, Hydroxy, (NR_{A}R_{B})carbonyl, - (NR_{C}S(O)₂R_{D})Carbonyl, -S (O)₂OH;
A₃ ist Carboxy;
A₄, A₅, A₆, A₇, A₈, A₉, A₁₀ und A₁₁ sind jeweils Wasserstoff;
R_{A} und R_{B} sind jeweils unabhängig gewählt aus der Gruppe bestehend aus Wasserstoff, Alkyl und Cyano;
L₁ ist gewählt aus der Gruppe bestehend aus Alkylen und -(CH₂)ₘO(CH₂)ₙ-;
R₁ ist gewählt aus der Gruppe bestehend aus Aryl, Cycloalkyl und Heterocyclus;
L₂ ist nicht vorhanden oder gewählt aus der Gruppe bestehend aus einer kovalenten Bindung, Alkylen, -(CH₂)ₚO(CH₂)_{q}-, - (CH₂)ₚC(O) (CH₂)_{q}-, - (CH₂)ₚC(OH)(CH₂)_{q}-, - (CH₂)ₚS(CH₂)_{q}- und - (CH₂)ₚCH=NO(CH₂)_{q}-;
p ist 0;
q ist eine ganze Zahl von 0-1; und
R₂ ist nicht vorhanden oder gewählt aus der Gruppe bestehend aus Aryl, Cycloalkenyl, Cycloalkyl und Heterocyclus.

3. Eine Verbindung gemäß Anspruch 1, worin A₁ und A₂ zusammengenommen mit den Kohlenstoffatomen, an welche sie gebunden sind, einen fünf-gliedrigen Heterocyclus bilden, enthaltend ein Schwefelatom, worin der fünf-gliedrige Heterocyclus substituiert ist mit 0, 1 oder 2 Substituenten gewählt aus der Gruppe bestehend aus Mercapto und Oxo.

4. Eine Verbindung gemäß Anspruch 1, worin
L₁ Alkylen ist;
R₁ Aryl ist,
L₂ nicht vorhanden ist; und
R₂ nicht vorhanden ist.

5. Eine Verbindung gemäß Anspruch 1, worin
L₁ Alkylen ist;
R₁ Aryl ist, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F})carbonyl, -N(R_{A})C(O)N_{R}BR_{C} und -CH=NOR_{EE};
L₂ ist nicht vorhanden; und
R₂ ist nicht vorhanden,
vorzugsweise worin
A₁, A₂ und A₃ jeweils Carboxy sind;
A₄, A₅, A₆, A₇, A₈, A₉, A₁₀ und A₁₁ jeweils Wasserstoff sind;
L₁ ist Alkylen, worin das Alkylen gewählt ist aus der Gruppe bestehend aus -CH₂-, -CH₂CH₂- und -CH₂CH(CH₃)-;
R₁ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ ist nicht vorhanden; und
R₂ ist nicht vorhanden.

6. Eine Verbindung gemäß Anspruch 1, worin
L₁ Alkylen ist;
R₁ ist Aryl, worin das Aryl Fluorenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxy, Hydroxyalkyl, Nitro, Oxo und -NR_{E}R_{F};
L₂ ist nicht vorhanden; und
R₂ ist nicht vorhanden,
vorzugsweise worin
A₁, A₂ und A₃ jeweils Carboxy sind;
A₄, A₅, A₆, A₇, A₈, A₉, A₁₀ und A₁₁ jeweils Wasserstoff sind;
L₁ ist Alkylen, worin das Alkylen -CH₂- ist;
R₁ ist Aryl, worin das Aryl Fluorenyl ist, substituiert mit 0 oder 1 Substituenten gewählt aus der Gruppe bestehend aus Hydroxy und Oxo;
L₂ ist nicht vorhanden; und
R₂ ist nicht vorhanden.

7. Eine Verbindung gemäß Anspruch 1, worin
L₁ Alkylen ist;
R₁ Aryl ist;
L₂ eine kovalente Bindung ist; und
R₂ Aryl ist;
vorzugsweise worin
L₁ Alkylen ist;
R₁ ist Aryl, worin Aryl Phenyl ist, substituiert mit 0, 1, 2, 3, 4 oder 5 Substituenten, jeweils unabhängig gewählt aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F}) Carbonyl, -N(R_{A})C(O)NR_{B}R_{C} und -CH=NOR_{EE};
L₂ ist eine kovalente Bindung; und
R₂ ist Aryl, worin das Aryl Phenyl ist, substituiert mit 0, 1, 2, 3, 4 oder 5 Substituenten, jeweils unabhängig gewählt aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F}) Carbonyl, -N (R_{A}) C (O) NR_{B}R_{C} und -CH=NOR_{EE},
noch bevorzugter worin
A₁, A₂ und A₃ jeweils Carboxy ist;
A₄, A₅, A₆, A₇, A₈, A₉, A₁₀ und A₁₁ jeweils Wasserstoff sind;
L₁ ist Alkylen, worin das Alkylen -CH₂- ist;
R₁ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ ist eine kovalente Bindung; und
R₂ ist Aryl, worin Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F}.

8. Eine Verbindung gemäß Anspruch 1, worin
A₁ und A₂ bilden zusammengenommen mit den Kohlenstoffatomen, an welche sie gebunden sind, einen fünf-gliedrigen Heterocyclus, enthaltend ein Schwefelatom, worin der fünf-gliedrige Heterocyclus substituiert ist mit 0, 1 oder 2 Substituenten gewählt aus der Gruppe bestehend aus Mercapto und Oxo;
A₃ ist Carboxy;
A₄, A₅, A₆, A₇, A₈, A₉, A₁₀ und A₁₁ sind jeweils Wasserstoff;
L₁ ist Alkylen, worin das Alkylen -CH₂- ist;
R₁ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ ist eine kovalente Bindung; und
R₂ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, h Hydroxyalkyl, Methylendioxy, Nitro, -CH=NOR_{EE} und -NR_{E}R_{F}.

9. Eine Verbindung gemäß Anspruch 1, worin
L₁ Alkylen ist;
R₁ ist Aryl;
L₂ ist eine kovalente Bindung; und
R₂ ist Heterocyclus,
vorzugsweise, worin
L₁ Alkylen ist;
R₁ Aryl ist, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro,
Oxo, -NR_{E}R_{F}, (NR_{E}R_{F})Carbonyl, -N(R_{A})C(O)NR_{B}R_{C} und -CH=NOR_{EE};
L₂ ist eine kovalente Bindung; und
R₂ ist Heterocyclus, worin der Heterocyclus gewählt ist aus der Gruppe bestehend aus Azetidinyl, Azepanyl, Aziridinyl, Furyl, Morpholinyl, Piperazinyl, Piperidinyl, Pyrazinyl, Pyridazinyl, Pyridinyl, Pyrimidinyl, Pyrrolidinyl und Thienyl, worin der Heterocyclus substituiert ist mit 0, 1, 2 oder 3 Substituenten unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Arylalkoxycarbonyl, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F})Carbonyl, -N(R_{A})C(O)NR_{B}R_{C} und -CH=NOR_{EE}, noch bevorzugter worin
A₁, A₂ und A₃ jeweils Carboxy sind;
A₄, A₅, A₆, A₇, A₈, A₉, A₁₀ und A₁₁ sind jeweils Wasserstoff;
L₁ ist Alkylen, worin das Alkylen -CH₂- ist;
R₁ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ ist eine kovalente Bindung; und
R₂ ist Heterocyclus, worin der Heterocyclus gewählt ist aus der Gruppe bestehend aus Pyridinyl, Pyrrolidinyl und Thienyl, worin der Heterocyclus substituiert ist mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Nitro und -NR_{E}R_{F}.

10. Eine Verbindung gemäß Anspruch 1, worin
L₁ ist Alkylen,
R₁ ist Aryl;
L₂ ist Alkylen; und
R₂ ist Aryl,
vorzugsweise, worin
L₁ Alkylen ist;
R₁ Aryl ist, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F}) Carbonyl, -N(R_{A})C(O)NR_{B}R_{C} und -CH=NOR_{EE},
L₂ ist Alkylen; und
R₂ ist Aryl, worin das Aryl Phenyl ist substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F}) Carbonyl, -N(R_{A})C(O)NR_{B}R_{C} und -CH=NOR_{EE}, noch bevorzugter worin
A₁, A₂ und A₃ jeweils Carboxy sind;
A₄, A₅, A₆, A₇, A₈, A₉, A₁₀ und A₁₁ sind jeweils Wasserstoff;
L₁ ist Alkylen, worin das Alkylen -CH₂- ist;
R₁ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ ist Alkylen, worin das Alkylen -CH₂- ist; und
R₂ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F}.

11. Eine Verbindung gemäß Anspruch 1, worin
L₁ Alkylen ist;
R₁ Aryl ist;
L₂ -(CH₂)ₚO(CH₂)_{q}- ist; und
R₂ Aryl ist,
vorzugsweise, worin
L₁ Alkylen ist,
R₁ Aryl ist, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F})carbonyl, -N(R_{A})C(O)NR_{B}R_{C} und -CH=NOR_{EE},
L₂ ist -(CH₂)ₚO(CH₂)_{q}-; und
R₂ ist Aryl, worin das Aryl Phenyl ist, substituiert mit 0, 1, 2, 3, 4 oder 5 substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F})carbonyl, -N(R_{A})C(O)NR_{B}R_{C} und -CH=NOR_{EE}, oder worin
A₁, A₂ und A₃ sind jeweils Carboxy;
A₄, A₅, A₆, A₇, A₈, A₉, A₁₀ und A₁₁ sind jeweils Wasserstoff;
L₁ ist Alkylen, worin das Alkylen -CH₂- ist;
R₁ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ ist -(CH₂)ₚO(CH₂)_{q}-;
p ist 0;
q ist 0; und
R₂ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F},
oder worin
A₁ und A₂ jeweils unabhängig gewählt ist aus der Gruppe bestehend aus Alkoxycarbonyl, Carboxy, Hydroxy, (NR_{A}R_{B})carbonyl und -S(O)₂OH;
A₃ Carboxy ist;
A₄, A₅, A₆, A₇, A₈, A₉, A₁₀ und A₁₁ jeweils Wasserstoff ist;
L₁ Alkylen ist, worin das Alkylen -CH₂- ist;
R₁ Aryl ist, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ -(CH₂)ₚO(CH₂)_{q}- ist;
p 0 ist;
q 0 ist; und
R₂ Aryl ist, worin Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F}.

12. Eine Verbindung gemäß Anspruch 1, worin
A₁ und A₂ jeweils unabhängig gewählt ist aus der Gruppe bestehend aus Carboxy und -(NR_{C}S(O)₂R_{D})carbonyl, worin eines von A₁ oder A₂ -(NR_{C}S(O)₂R_{D})carbonyl ist;
A₃ ist Carboxy;
A₄, A₅, A₆, A₇, A₈, A₉, A₁₀ und A₁₁ sind jeweils Wasserstoff;
L₁ ist Alkylen, worin das Alkylen -CH₂- ist;
R₁ ist Aryl, worin Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F}) carbonyl, -N(R_{A})C(O)NR_{B}R_{C} und -CH=NOR_{EE};
L₂ ist -(CH₂)ₚO(CH₂)_{q}-;
p ist 0;
q ist 0; und
R₂ ist Aryl, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F})carbonyl, -N(R_{A})C(O)NR_{B}R_{C} und -CH=NOR_{EE};
vorzugsweise, worin
R₁ Aryl ist, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
R₂ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F}; und
R_{D} ist gewählt aus der Gruppe bestehend aus Alkyl und Aryl, worin vorzugsweise das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE}, -NR_{E}R_{F} und -N(R_{A})C (O)NR_{B}R_{C}.

13. Eine Verbindung gemäß Anspruch 1, worin
A₁ und A₂ jeweils unabhängig gewählt ist aus der Gruppe bestehend aus Carboxy und -(NR_{C}S(O)₂R_{D})carbonyl, worin eins von A₁ oder A₂ -(NR_{C}S(O)₂R_{D})carbonyl ist;
A₃ ist Carboxy;
A₄, A₅, A₆, A₇, A₈, A₉, A₁₀ und A₁₁ sind jeweils Wasserstoff;
L₁ ist Alkylen, worin das Alkylen -CH₂- ist,
R₁ ist Aryl, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F}) carbonyl, -N(R_{A})C(O)NR_{B}R_{C} und -CH=NOR_{EE};
L₂ ist -(CH₂)ₚO(CH₂)_{q}-;
p ist 0;
q ist 0;
R₂ ist Aryl, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F})carbonyl, -N (R_{A})C(O)NR_{B}R_{C} und -CH=NOR_{EE}; und
R_{D} ist Heterocyclus,
vorzugsweise, worin
R₁ Aryl ist, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
R₂ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F}; und
R_{D} ist Heterocyclus, worin der Heterocyclus Pyridinyl und Thienyl ist, worin der Heterocyclus substituiert ist mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Nitro, -N(R_{A})C(O)NR_{B}R_{C} und -NR_{E}R_{F}.

14. Eine Verbindung gemäß Anspruch 1, worin
A₁ und A₂ jeweils unabhängig gewählt sind aus der Gruppe bestehend aus Carboxy und -(NR_{C}S(O)₂R_{D})carbonyl, worin eines von A₁ oder A₂ -(NR_{C}S(O)₂R_{D})carbonyl ist;
A₃ ist Carboxy;
A₄, A₅, A₆, A₇, A₈, A₉, A₁₀ und A₁₁ jeweils Wasserstoff ist;
L₁ ist Alkylen, worin das Alkylen -CH₂- ist;
R₁ ist Aryl, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F})carbonyl, -N(R_{A})C(O)NR_{B}R_{C} und -CH=NOR_{EE};
L₂ ist -(CH₂)ₚO(CH₂)_{q};
p ist 0;
q ist 0;
R₂ Aryl ist, worin das Aryl Phenyl ist, substituiert mit 0, 1, 2, 3, 4 oder 5 Substituenten jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl; Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F})carbonyl, -N(R_{A})C(O)NR_{B}R_{C} und -CH=NOR_{EE}; und
R_{D} ist Arylalkyl,
vorzugsweise, worin
R₁ Aryl ist, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
R₂ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F}; und
R_{D} ist Arylalkyl, worin das Aryl von Arylalkyl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F}.

15. Eine Verbindung gemäß Anspruch 1, worin
A₁, A₂ und A₃ jeweils Carboxy sind;
A₄, A₅, A₆, A₇, A₈, A₉, A₁₀ und A₁₁ jeweils Wasserstoff sind;
L₁ ist Alkylen, worin das Alkylen -CH₂- ist;
R₁ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ ist -(CH₂)ₚO(CH)₂)_{q}-;
p ist 0;
q ist 1; und
R₂ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F}.

16. Eine Verbindung gemäß Anspruch 1, worin
L₁ ist Alkylen;
R₁ ist Aryl;
L₂ ist -(CH₂)ₚO(CH₂)_{q}-; und
R₂ ist Cycloalkyl,
vorzugsweise worin
L₁ Alkylen ist;
R₁ ist Aryl, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F}) carbonyl, -N(R_{A})C(O)NR_{B}R_{C} und -CHOR_{EE};
L₂ ist -(CH₂)ₚO(CH₂)_{q}-; und
R₂ ist Cycloalkyl,
noch bevorzugter worin
A₁, A₂ und A₃ jeweils Carboxy sind;
A₄, A₅, A₆, A₇, A₈, A₉, A₁₀ und A₁₁ jeweils Wasserstoff sind;
L₁ ist Alkylen, worin das Alkylen -CH₂- ist;
R₁ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ ist -(CH₂)ₚO(CH₂)_{q}-;
p ist 0;
q ist 0; und
R₂ ist Cycloalkyl.

17. Eine Verbindung gemäß Anspruch 1, worin
L₁ Alkylen ist;
R₁ ist Aryl;
L₂ ist -(CH₂)ₚO(CH₂)_{q}-; und
R₂ ist Cycloalkenyl,
vorzugsweise, worin
L₁ Alkylen ist;
R₁ Aryl ist, worin das Aryl Phenyl ist, substituiert mit 0, 1, 2, 3, 4, oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro,
Oxo, -NR_{E}R_{F}, (NR_{E}R_{F})carbonyl, -N(R_{A})C(O)NR_{B}R_{C} und -CH=NOR_{EE};
L₂ ist -(CH₂)ₚO(CH₂)_{q}-; und
R₂ ist Cycloalkyl,
noch bevorzugter worin
A₁, A₂ und A₃ jeweils Carboxy sind;
A₄, A₅, A₆, A₇, A₈, A₉, A₁₀ und A₁₁ sind jeweils Wasserstoff;
L₁ ist Alkylen, worin das Alkylen -CH₂- ist;
R₁ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ -(CH₂)ₚO(CH₂)_{q}- ist; und
p ist 0;
q ist 0; und
R₂ ist Cycloalkenyl.

18. Eine Verbindung gemäß Anspruch 1, worin
L₁ Alkylen ist;
R₁ Aryl ist;
L₂ -(CH₂)ₚO(CH₂)_{q}- ist; und
R₂ Heterocyclus ist,
vorzugsweise, worin
L₁ Alkylen ist;
R₁ ist Aryl, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkylsulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F})carbonyl, -N(R_{A})C(O)NR_{B}R_{C} und -CH=NOR_{EE};
L₂ ist -(CH₂)ₚO(CH₂)_{q}-; und
R₂ ist Heterocyclus, worin der Heterocyclus gewählt ist aus der Gruppe bestehend aus Furyl, Pyrazinyl, Pyrazidinyl, Pyridinyl, Pyrimidinyl und Thienyl, worin der Heterocyclus substituiert ist mit 0, 1, 2, oder 3 Substituenten unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkylsulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Arylalkoxycarbonyl, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F}) carbonyl, -N(R_{A})C(O)NR_{B}R_{C} und -CH=NOR_{EE};
noch bevorzugter worin
A₁, A₂ und A₃ jeweils Carboxy sind;
A₄, A₅, A₆, A₇, A₈, A₉, A₁₀ und A₁₁ sind jeweils Wasserstoff;
L₁ ist Alkylen, worin das Alkylen -CH₂- ist;
R₁ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ ist -(CH₂)ₚO(CH₂)_{q}-;
p ist 0;
q ist 0; und
R₂ ist Heterocyclus, worin der Heterocyclus gewählt ist aus der Gruppe bestehend aus Pyridinyl und Pyrimidinyl, worin der Heterocyclus substituiert ist mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Nitro, -N(R_{A})C(O)NR_{B}R_{C} und -NR_{E}R_{F}.

19. Eine Verbindung gemäß Anspruch 1, worin
L₁ Alkylen ist;
R₁ Aryl ist;
L₂ ist -(CH₂)ₚO(CH₂)_{q}-; und
R₂ ist Aryl,
vorzugsweise worin
L₁ Alkylen ist;
R₁ Aryl ist, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F}) carbonyl, -N (R_{A})C(O) NR_{B}R_{C} und -CH=NOR_{EE};
L₂ ist -(CH₂)ₚS(CH₂)_{q}-; und
R₂ ist Aryl, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F})carbonyl, -N(R_{A})C(O)NR_{B}R_{C} und -CH=NOR_{EE};
noch bevorzugter worin
A₁, A₂ und A₃ jeweils Carboxy sind;
A₄, A₅, A₆, A₇, A₈, A₉, A₁₀ und A₁₁ sind jeweils Wasserstoff;
L₁ ist Alkylen, worin das Alkylen -CH₂- ist;
R₁ ist Aryl, worin das Aryl Phenyl ist, substituiert mit 0, 1 oder 2 Substituenten gewählt aus der Gruppe bestehend aus Alkoxy, Alkoxycarbonyl, Alkinyl, Alkinyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ ist -(CH₂)ₚS(CH₂)_{q}-; und
p ist 0;
q ist 0; und
R₂ ist Aryl, worin das Aryl Phenyl ist, substituiert mit 0, 1 oder 2 substituenten, gewählt aus der Gruppe bestehend aus Alkoxy, Alkoxycarbonyl, Alkinyl, Alkinyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F}.

20. Eine Verbindung gemäß Anspruch 1, worin
L₁ Alkylen ist;
R₁ Aryl ist;
L₂ -(CH₂)ₚC(O)(CH₂)_{q}-; und
R₂ Aryl ist,
vorzugsweise, worin
L₁ Alkylen ist;
R₁ ist Aryl, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F}) carbonyl, -N(R_{A})C(O)NR_{B}R_{C} und -CH=NOR_{EE};
L₂ ist -(CH₂)ₚC(O)(CH₂)_{q}-; und
R₂ ist Aryl, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F})carbonyl, -N(R_{A})C(O)NR_{B}R_{C} und -CH=NOR_{EE};
noch bevorzugter, worin
A₁, A₂ und A₃ jeweils Carboxy sind;
A₄, A₅, A₆, A₇, A₈, A₉, A₁₀ und A₁₁ sind jeweils Wasserstoff;
L₁ ist Alkylen, worin das Alkylen -CH₂- ist;
R₁ ist Aryl, worin das Aryl Phenyl ist, substituiert mit 0, 1 oder 2 Substituenten gewählt aus der Gruppe bestehend aus Alkoxy, Alkoxycarbonyl, Alkinyl, Alkinyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ ist -(CH₂)ₚC(O)(CH₂)_{q}-; und
p ist 0;
q ist 0; und
R₂ ist Aryl, worin das Aryl Phenyl ist, substituiert mit 0, 1 oder 2 substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F}.

21. Eine Verbindung gemäß Anspruch 1, worin
L₁ Alkylen ist;
R₁ ist Aryl;
L₂ ist -(CH₂)ₚC(OH)(CH₂)_{q}-; und
R₂ ist Aryl,
vorzugsweise, worin
L₁ Alkylen ist;
R₁ ist Aryl, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F})carbonyl, -N(R_{A})C(O)NR_{B}R_{C} und -CH=NOR_{EE};
L₂ ist -(CH₂)ₚC(OH)(CH₂)_{q}-; und
R₂ ist Aryl, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F})carbonyl, -N(R_{A})C(O)NR_{B}R_{C} und -CH=NOR_{EE};
noch bevorzugter, worin
A₁, A₂ und A₃ jeweils Carboxy sind;
A₄, A₅, A₆, A₇, A₈, A₉, A₁₀ und A₁₁ sind jeweils Wasserstoff;
L₁ ist Alkylen, worin das Alkylen -CH₂- ist;
R₁ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ ist -(CH₂)ₚC(OH)(CH₂)_{q}-;
p ist 0;
q ist 0; und
R₂ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F}.

22. Eine Verbindung gemäß Anspruch 1, worin
L₁ Alkylen ist;
R₁ ist Aryl;
L₂ -(CH₂)ₚCH=NO(CH₂)_{q}-; und
R₂ ist Aryl,
vorzugsweise worin
L₁ Alkylen ist;
R₁ ist Aryl, worin Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Ethylendioxy, Nitro, Oxo, -NR_{E}R_{F}, (NR_{E}R_{F}) carbonyl, -N (R_{A})C(O)NR_{B}R_{C} und -CH=NOR_{EE};
L₂ ist -(CH₂)ₚCH=NO(CH₂)_{q}-; und
R₂ ist Aryl, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Ethylendioxy, Nitro, Oxo, -NR_{E}R_{F}, (NR_{E}R_{F})carbonyl, -N (R_{A})C(O)NR_{B}R_{C} und -CH=NOR_{EE};
noch bevorzugter, worin
A₁, A₂ und A₃ jeweils Carboxy sind;
A₄, A₅, A₆, A₇, A₈, A₉, A₁₀ und A₁₁ jeweils Wasserstoff sind;
L₁ ist Alkylen, worin das Alkylen -CH₂- ist;
R₁ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ ist -(CH₂)ₚCH=NO(CH₂)_{q}-;
p ist 0;
q ist eine ganze Zahl von 0-1; und
R₂ ist Aryl, worin Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F}.

23. Eine Verbindung gemäß Anspruch 1, worin
L₁ Alkylen ist;
R₁ ist Aryl;
L₂ ist -(CH₂)ₚCH=NO(CH₂)_{q}-; und
R₂ ist Heterocyclus,
vorzugsweise, worin
A₁, A₂ und A₃ sind jeweils Carboxy;
A₄, A₅, A₆, A₇, A₈, A₉, A₁₀ und A₁₁ sind jeweils Wasserstoff;
L₁ ist Alkylen, worin das Alkylen -CH₂- ist;
R₁ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ ist -(CH₂)ₚCH=NO(CH₂)_{q}-;
p ist 0;
q ist 0; und
R₂ ist Heterocyclus, worin der Heterocyclus Pyranyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Nitro, -N(R_{A})C (O)NR_{B}R_{C} und -NR_{E}R_{F}.

24. Eine Verbindung gemäß Anspruch 1, worin
L₁ Alkylen ist;
R₁ ist Cycloalkyl;
L₂ ist nicht vorhanden; und
R₂ ist nicht vorhanden,
vorzugsweise, worin
A₁, A₂ und A₃ jeweils Carboxy sind;
A₄, A₅, A₆, A₇, A₈, A₉, A₁₀ und A₁₁ jeweils Wasserstoff sind;
L₁ ist -CH₂-;
R₁ ist Cycloalkyl;
L₂ ist nicht vorhanden; und
R₂ ist nicht vorhanden.

25. Eine Verbindung gemäß Anspruch 1, worin
L₁ -(CH₂)ₘO(CH₂)ₙ- ist;
R₁ ist Aryl;
L₂ ist nicht vorhanden; und
R₂ ist nicht vorhanden,
vorzugsweise worin
L₁ -(CH₂)ₘO(CH₂)ₙ- ist;
R₁ ist Aryl, worin das Aryl Phenyl ist, substituiert mit 0, 1, 2, 3, 4 oder 5 Substituenten, jeweils unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkinyl, Alkinyloxy, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F}) carbonyl, -N (R_{A}) C (O)NR_{B}R_{C} und -CH=NOR_{EE};
L₂ ist nicht vorhanden; und
R₂ ist nicht vorhanden,
noch bevorzugter, worin
A₁, A₂, A₃ jeweils Carboxy sind;
A₄, A₅, A₆, A₇, A₈, A₉, A₁₀ und A₁₁ jeweils Wasserstoff sind;
L₁ ist -(CH₂)ₘO(CH₂)ₙ-;
m ist eine ganze Zahl von 2-4;
n ist 0;
R₁ ist Aryl, worin das Aryl Phenyl ist, substituiert mit 0, 1 oder 2 Substituenten gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Methylendioxy, Nitro, -CH=NOR_{EE} und -NR_{E}R_{F};
L₂ ist nicht vorhanden; und
R₂ ist nicht vorhanden.

26. Eine Verbindung gemäß Anspruch 1, worin
L₁ Alkylen ist,
R₁ ist Heterocyclus;
L₂ ist nicht vorhanden; und
R₂ ist nicht vorhanden,
vorzugsweise, worin
L₁ Alkylen ist;
R₁ ist Heterocyclus, worin der Heterocyclus gewählt ist aus der Gruppe bestehend aus Furyl, Pyrazinyl, Pyridazinyl, Pyridinyl, Pyrimidinyl und Thienyl, worin der Heterocyclus substituiert ist mit 0, 1, 2 oder 3 Substituenten unabhängig gewählt aus der Gruppe bestehend aus Alkenyl, Alkenyloxy, Alkoxy, Alkoxyalkoxy, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfonyl, Alkylthio, Alkylthioalkyl, Alkenyl, Alkenyloxy, Arylalkoxycarbonyl, Carboxy, Carboxyalkyl, Cyano, Cyanoalkyl, Ethylendioxy, Formyl, Haloalkoxy, Haloalkyl, Haloalkylthio, Halogen, Hydroxy, Hydroxyalkyl, Mercapto, Methylendioxy, Nitro, Oxo, -NR_{E}R_{F}, (NR_{E}R_{F}) carbonyl, -N(R_{A})C(O)NR_{B}R_{C} und -CH=NOR_{EE};
L₂ ist nicht vorhanden; und
R₂ ist nicht vorhanden,
noch bevorzugter worin
A₁, A₂ und A₃ jeweils Carboxy sind;
A₄, A₅, A₆, A₇, A₈, A₉, A₁₀ und A₁₁ jeweils Wasserstoff sind;
L₁ ist Alkylen, worin Alkylen -CH₂- ist;
R₁ ist Heterocyclus, worin der Heterocyclus Thienyl ist, substituiert mit 0, 1 oder 2 Substituenten, gewählt aus der Gruppe bestehend aus Alkoxy, Alkoxycarbonyl, Alkenyl, Alkenyloxy, Alkyl, Alkylthio, Cyano, Formyl, Haloalkoxy, Haloalkyl, Haloalkylthio, Halogen, Hydroxyalkyl, Nitro, -N(R_{A})C (O)NR_{B}R_{C} und -NR_{E}R_{F};
L₂ ist nicht vorhanden; und
R₂ ist nicht vorhanden.

27. Eine Verbindung gemäß Anspruch 6, gewählt aus der Gruppe bestehend aus:
5-({(4-Chlorbenzyl)[(1S)-1,2,3,4-tetrahydro-1-napthalenyl] amino}carbonyl)-1,2,4-benzentricarbonsäure;
5-({(4-Brombenzyl)[(1S)-1,2,3,4-tetrahydro-1-napthalenyl] amino}carbonyl)-1,2,4-benzentricarbonsäure;
5-({(3-Brombenzyl)[(1S)-1,2,3,4-tetrahydro-1-napthalenyl] amino}carbonyl)-1,2,4-benzentricarbonsäure;
5-({(3,4-Dichlorbenzyl)[(1S)-1,2,3,4-tetrahydro-1-napthalenyl]amino}carbonyl)-1,2,4-benzentricarbonsäure;
5-({(4-Cyanobenzyl)[(1S)-1,2,3,4-tetrahydro-1-napthalenyl] amino}carbonyl)-1,2,4-benzentricarbonsäure;
5-({(4-Chlor-3-nitrobenzyl)[(1S)-1,2,3,4-tetrahydro-1-napthalenyl]amino}carbonyl)-1,2,4-benzentricarbonsäure;
5-({(4-(Dimethylamino)benzyl][(1S)-1,2,3,4-tetrahydro-1-napthalenyl]amino}carbonyl)-1,2,4-benzentricarbonsäure;
5-({(3-Chlorbenzyl)[(1S)-1,2,3,4-tetrahydro-1-napthalenyl] amino}carbonyl)-1,2,4-benzentricarbonsäure;
5-({(3-Cyanobenzyl)[(1S)-1,2,3,4-tetrahydro-1-napthalenyl] amino}carbonyl)-1,2,4-benzentricarbonsäure;
5-({(2-Phenylpropyl)[(1S)-1,2,3,4-tetrahydro-1-napthalenyl] amino}carbonyl)-1,2,4-benzentricarbonsäure;
5-({(2-Phenylethyl)[(1S)-1,2,3,4-tetrahydro-1-napthalenyl] amino}carbonyl)-1,2,4-benzentricarbonsäure;
5-({Benzyl[(1S)-1,2,3,4-tetrahydro-1-napthalenyl]amino} carbonyl)-1,2,4-benzentricarbonsäure;
5-({(3-Methoxybenzyl)[(1S)-1,2,3,4-tetrahydro-1-napthalenyl]amino}carbonyl)-1,2,4-benzentricarbonsäure;
5-({(3-Nitrobenzyl)[(1S)-1,2,3,4-tetrahydro-1-napthalenyl] amino}carbonyl)-1,2,4-benzentricarbonsäure;
5-({[Spiro[1,3-Benzodioxol-5-yl-2,1'-cyclohexan]methyl] [(1S)-1,2,3,4-tetrahydro-1-napthalenyl]amino}carbonyl)-1,2,4-benzentricarbonsäure;
5-({(3-(Allyloxybenzyl][(1S)-1,2,3,4-tetrahydro-1-napthalenyl]amino}carbonyl)-1,2,4-benzentricarbonsäure;
5-({(3,5-bis(Trifluormethyl)benzyl][(1S)-1,2,3,4-tetrahydro-1-napthalenyl]amino}carbonyl)-1,2,4-benzentricarbonsäure;
5-({(1S)-1,2,3,4-tetrahydro-1-napthalenyl[3-(trifluormethyl)benzyl]amino}carbonyl)-1,2,4-benzentricarbonsäure;
5-({(3,5-Difluorbenzyl)[(1S)-1,2,3,4-tetrahydro-1-napthalenyl]amino}carbonyl)-1,2,4-benzentricarbonsäure;
5-({[4-Fluor-3-(trifluormethyl)benzyl][(1S)-1,2,3,4-tetrahydro-1-napthalenyl]amino}carbonyl)-1,2,4-benzentricarbonsäure;
5-({(3,5-Dibrombenzyl)[(1S)-1,2,3,4-tetrahydro-1-napthalenyl]amino}carbonyl)-1,2,4-benzentricarbonsäure;
5-({[3-Fluor-5-(trifluormethyl)benzyl][(1S)-1,2,3,4-tetrahydro-1-napthalenyl]amino}carbonyl)-1,2,4-benzentricarbonsäure;
5-({(3,5-Dimethoxybenzyl)[(1S)-1,2,3,4-tetrahydro-1-napthalenyl]amino}carbonyl)-1,2,4-benzentricarbonsäure;
5-({(2,3-Dichlorbenzyl)[(1S)-1,2,3,4-tetrahydro-1-napthalenyl]amino}carbonyl)-1,2,4-benzentricarbonsäure;
5-({(2,4-Dichlorbenzyl)[(1S)-1,2,3,4-tetrahydro-1-napthalenyl]amino}carbonyl)-1,2,4-benzentricarbonsäure;
5-({(3,5-Dimethylbenzyl)[(1S)-1,2,3,4-tetrahydro-1-napthalenyl]amino}carbonyl)-1,2,4-benzentricarbonsäure;
5-[((1S)-1,2,3,4-tetrahydro-1-napthalenyl{3-[(trifluormethyl)sulfanyl]benzyl}amino)carbonyl)-1,2,4-benzentricarbonsäure;
5-({[3-(Methylsulfanyl)benzyl][(1S)-1,2,3,4-tetrahydro-1-napthalenyl]amino}carbonyl)-1,2,4-benzentricarbonsäure;
5-({{3-(Methoxyimino)methyl]benzyl}[(1S)-1,2,3,4-tetrahydro-1-napthalenyl]amino}carbonyl)-1,2,4-benzentricarbonsäure;
5-({[3-(Hydroxymethyl)benzyl][(1S)-1,2,3,4-tetrahydro-1-napthalenyl]amino}carbonyl)-1,2,4-benzentricarbonsäure;
5-({(3-Formylbenzyl)[(1S)-1,2,3,4-tetrahydro-1-napthalenyl] amino}carbonyl)-1,2,4-benzentricarbonsäure;
5-({{3-[(tert-Butoxyimino)methyl]benzyl}[(1S)-1,2,3,4-tetrahydro-1-napthalenyl]amino}carbonyl)-1,2,4-benzentricarbonsäure; und
5-({{3-[(Isopropoxyimino)methyl]benzyl}[(1S)-1,2,3,4-tetrahydro-1-napthalenyl]amino}carbonyl)-1,2,4-benzentricarbonsäure;
5-({(9H-Fluoren-2-ylmethyl)[(1S)-1,2,3,4-tetrahydro-1-napthalenyl]amino}carbonyl)-1,2,4-benzentricarbonsäure;
5-({[(9-Hydroxy-9H-fluoren-2-yl)methyl][(1S)-1,2,3,4-tetrahydro-1-napthalenyl]amino}carbonyl)-1,2,4-benzentricarbonsäure; und
5-({[(9-Oxo-9H-fluoren-2-yl)methyl][(1S)-1,2,3,4-tetrahydro-1-napthalenyl]amino}carbonyl)-1,2,4-benzentricarbonsäure;
5-({([1,1'-Biphenyl]-4-ylmethyl)[(1S)-1,2,3,4-tetrahydro-1-napthalenyl]amino}carbonyl)-1,2,4-benzentricarbonsäure;
5-({[(2'-Chlor[1,1'-biphenyl]-4-yl)methyl][(1S)-1,2,3,4-tetrahydro-1-napthalenyl]amino}carbonyl)-1,2,4-benzentricarbonsäure;
5-({[(3',5'-Dichlor[1,1'-biphenyl]-4-yl)methyl][(1S)-1,2,3,4-tetrahydro-1-napthalenyl]amino}carbonyl)-1,2,4-benzentricarbonsäure;
5-({[(2'-Methoxy[1,1'-biphenyl]-4-yl)methyl][(1S)-1,2,3,4-tetrahydro-1-napthalenyl]amino}carbonyl)-1,2,4-benzentricarbonsäure;
5-({[(4'-Chlor[1,1'-biphenyl]-4-yl)methyl][(1S)-1,2,3,4-tetrahydro-1-napthalenyl]amino}carbonyl)-1,2,4-benzentricarbonsäure;
5-({[(4'-Fluor[1,1'-biphenyl]-4-yl)methyl][(1S)-1,2,3,4-tetrahydro-1-napthalenyl]amino}carbonyl)-1,2,4-benzentricarbonsäure;
5-({[(4'-Methoxy[1,1'-biphenyl]-4-yl)methyl][(1S)-1,2,3,4-tetrahydro-1-napthalenyl]amino}carbonyl)-1,2,4-benzentricarbonsäure;
5-[((1S)-1,2,3,4-tetrahydro-1-napthalenyl{[2'-(trifluormethyl)[1,1'-biphenyl]-4-yl]methyl}amino)carbonyl]-1,2,4-benzentricarbonsäure;
5-({[(2'-Methyl[1,1'-biphenyl]-4-yl)methyl][(1S)-1,2,3,4-tetrahydro-1-napthalenyl]amino}carbonyl)-1,2,4-benzentricarbonsäure;
5-[((1S)-1,2,3,4-tetrahydro-1-napthalenyl{[4'-(trifluormethyl)[1,1'-biphenyl]-2-yl]methyl}amino)carbonyl]-1,2,4-benzentricarbonsäure;
5-({[(4'-Fluor[1,1'-biphenyl]-2-yl)methyl][(1S)-1,2,3,4-tetrahydro-1-napthalenyl]amino}carbonyl)-1,2,4-benzentricarbonsäure;
5-({[(4'-Fluor[1,1'-biphenyl]-3-yl)methyl][(1S)-1,2,3,4-tetrahydro-1-napthalenyl]amino}carbonyl)-1,2,4-benzentricarbonsäure;
5-[((1S)-1,2,3,4-Tetrahydro-1-napthalenyl{[4'-(trifluormethyl)[1,1'-biphenyl]-4-yl]methyl}amino)carbonyl]-1,2,4-benzentricarbonsäure;
5-({([1,1'-Biphenyl]-2-ylmethyl)[(1S)-1,2,3,4-tetrahydro-1-napthalenyl]amino}carbonyl)-1,2,4-benzentricarbonsäure;
5-({([1,1'-Biphenyl]-3-ylmethyl)[(1S)-1,2,3,4-tetrahydro-1-napthalenyl]amino}carbonyl)-1,2,4-benzentricarbonsäure;
5-({[(2-Methyl[1,1'-biphenyl]-3-yl)methyl][(1S)-1,2,3,4-tetrahydro-1-napthalenyl]amino}carbonyl)-1,2,4-benzentricarbonsäure;
5-[((1S)-1,2,3,4-Tetrahydro-1-napthalenyl{[4'-(trifluormethyl)[1,1'-biphenyl]-3-yl]methyl}amino)carbonyl]-1,2,4-benzentricarbonsäure;
5-({[(5'-Fluor-2'-methoxy[1,1'-biphenyl]-3-yl)methyl][(1S)-1,2,3,4-tetrahydro-1-napthalenyl]amino}carbonyl)-1,2,4-benzentricarbonsäure;
5-({[(5'-Chlor-2'-methoxy[1,1'-biphenyl]-3-yl)methyl][(1S)-1,2,3,4-tetrahydro-1-napthalenyl]amino}carbonyl)-1,2,4-benzentricarbonsäure;
5-({[(3',5'-Dichlor[1,1'-biphenyl]-3-yl)methyl][(1S)-1,2,3,4-tetrahydro-1-napthalenyl]amino}carbonyl)-1,2,4-benzentricarbonsäure;
5-({[(2'-Methoxy[1,1'-biphenyl]-3-yl)methyl][(1S)-1,2,3,4-tetrahydro-1-napthalenyl]amino}carbonyl)-1,2,4-benzentricarbonsäure;
5-({[(2'-Chlor[1,1'-biphenyl]-3-yl)methyl][(1S)-1,2,3,4-tetrahydro-1-napthalenyl]amino}carbonyl)-1,2,4-benzentricarbonsäure;
5-({[(2',5'-Dimethoxy[1,1'-biphenyl]-3-yl)methyl][(1S)-1,2,3,4-tetrahydro-1-napthalenyl]amino}carbonyl)-1,2,4-benzentricarbonsäure;
5-({[(3'-Chlor[1,1'-biphenyl]-3-yl)methyl][(1S)-1,2,3,4-tetrahydro-1-napthalenyl]amino}carbonyl)-1,2,4-benzentricarbonsäure;
5-({[(3',4'-Dichlor[1,1'-biphenyl]-3-yl)methyl][(1S)-1,2,3,4-tetrahydro-1-napthalenyl]amino}carbonyl)-1,2,4-benzentricarbonsäure;
5-({[(3'-Chlor-4'-fluor[1,1'-biphenyl]-3-yl)methyl][(1S)-1,2,3,4-tetrahydro-1-napthalenyl]amino}carbonyl)-1,2,4-benzentricarbonsäure;
5-({[(3'-Nitro[1,1'-biphenyl]-3-yl)methyl][(1S)-1,2,3,4-tetrahydro-1-napthalenyl]amino}carbonyl)-1,2,4-benzentricarbonsäure;
5-({[(3'-Methoxy[1,1'-biphenyl]-3-yl)methyl][(1S)-1,2,3,4-tetrahydro-1-napthalenyl]amino}carbonyl)-1,2,4-benzentricarbonsäure;
5-({[(4'-Chlor[1,1'-biphenyl]-3-yl)methyl][(1S)-1,2,3,4-tetrahydro-1-napthalenyl]amino}carbonyl)-1,2,4-benzentricarbonsäure; und
5-({[(1S)-1,2,3,4-Tetrahydro-1-napthalenyl [(3',4',5'-trimethoxy[1,1'-biphenyl]-3-yl)methyl]amino} carbonyl)-1,2,4-benzentricarbonsäure;
6-({[(3',4'-Dichlor-1,1'-biphenyl-3-yl)methyl][(1S)-1,2,3,4-tetrahydro-1-napthalenyl]amino}carbonyl)-1,3-dioxo-1,3-dihydro-2-benzothiophen-5-carbonsäure;
5-({[4-(1-Pyrrolidinyl)benzyl][(1S)-1,2,3,4-tetrahydro-1-napthalenyl]amino}carbonyl)-1,2,4-benzentricarbonsäure;
5-({[4-(3-Pyridinyl)benzyl][(1S)-1,2,3,4-tetrahydro-1-napthalenyl]amino}carbonyl)-1,2,4-benzentricarbonsäure;
5-({(1S)-1,2,3,4-Tetrahydro-1-napthalenyl[3-(3-thienyl) benzyl]amino}carbonyl)-1,2,4-benzentricarbonsäure; und
5-({(1S)-1,2,3,4-Tetrahydro-1-napthalenyl[3-(2-thienyl) benzyl]amino}carbonyl)-1,2.,4-benzentricarbonsäure;
5-({(3-Benzylbenzyl)[(1S)-1,2,3,4-tetrahydro-1-napthalenyl] amino}carbonyl)-1,2,4-benzentricarbonsäure;
5-({[3-(4-Chlorphenoxy)benzyl][(1S)-1,2,3,4-tetrahydro-1-napthalenyl]amino}carbonyl)-1,2,4-benzentricarbonsäure;
5-[((1S)-1,2,3,4-Tetrahydro-1-napthalenyl{3-[3-(trifluormethyl)phenoxy]benzyl}amino)carbonyl]-1,2,4-benzentricarbonsäure;
5-({[3-(4-Methoxyphenoxy)benzyl][(1S)-1,2,3,4-tetrahydro-1-napthalenyl]amino}carbonyl)-1,2,4-benzentricarbonsäure;
5-({[3-(3,4-Dichlorphenoxy)benzyl][(1S)-1,2,3,4-tetrahydro-1-napthalenyl]amino}carbonyl)-1,2,4-benzentricarbonsäure;
5-({[3-(4-Chlorphenoxy)benzyl][(1S)-1,2,3,4-tetrahydro-1-napthalenyl]amino}carbonyl)-1,2,4-benzentricarbonsäure;
5-({[3-(4-tert-Butylphenoxy)benzyl][(1S)-1,2,3,4-tetrahydro-1-napthalenyl]amino}carbonyl)-1,2,4-benzentricarbonsäure;
5-({[3-(4-Methylphenoxy)benzyl][(1S)-1,2,3,4-tetrahydro-1-napthalenyl]amino}carbonyl)-1,2,4-benzentricarbonsäure;
5-({[3-(3,5-Dichlorphenoxy)benzyl][(1S)-1,2,3,4-tetrahydro-1-napthalenyl]amino}carbonyl)-1,2,4-benzentricarbonsäure;
5-({[3-(4-Nitrophenoxy)benzyl][(1S)-1,2,3,4-tetrahydro-1-napthalenyl]amino}carbonyl)-1,2,4-benzentricarbonsäure;
5-({[3-(4-Cyanophenoxy)benzyl][(1S)-1,2,3,4-tetrahydro-1-napthalenyl]amino}carbonyl)-1,2,4-benzentricarbonsäure; und
5-({[3-(4-Fluorphenoxy)benzyl][(1S)-1,2,3,4-tetrahydro-1-napthalenyl]amino}carbonyl)-1,2,4-benzentricarbonsäure;
5-({(3-Phenoxybenzyl)[(1S)-1,2,3,4-tetrahydro-1-napthalenyl]amino}carbonyl)-1,2,4-benzentricarbonsäure;
2-[(Methylamino)carbonyl]-5-({(3-phenoxybenzyl)[(1S)-1,2,3,4-tetrahydro-1-napthalenyl]amino}carbonyl)terephthalsäure;
4-[(Methylamino)carbonyl]-6-({(3-phenoxybenzyl)[(1S)-1,2,3,4-tetrahydro-1-napthalenyl]amino}carbonyl)isophthalsäure;
2-[(Cyanoamino)carbonyl]-5-({(3-phenoxybenzyl)[(1S)-1,2,3,4-tetrahydro-1-napthalenyl]amino}carbonyl)terephthalsäure;
4-[(Cyanoamino)carbonyl]-6-({(3-phenoxybenzyl)[(1S)-1,2,3,4-tetrahydro-1-napthalenyl]amino}carbonyl)isophthalsäure;
2-(Aminocarbonyl)-5-({(3-phenoxybenzyl)[(1S)-1,2,3,4-tetrahydro-1-napthalenyl]amino}carbonyl)terephthalsäure;
2-(Aminocarbonyl)-6-({(3-phenoxybenzyl)[(1S)-1,2,3,4-tetrahydro-1-napthalenyl]amino}carbonyl)isophthalsäure;
2-(Methoxycarbonyl)-5-({(3-phenoxybenzyl)[(1S)-1,2,3,4-tetrahydro-1-napthalenyl]amino}carbonyl)terephthalsäure;
4-(Methoxycarbonyl)-6-({(3-phenoxybenzyl)[(1S)-1,2,3,4-tetrahydro-1-napthalenyl]amino}carbonyl)isophthalsäure;
2-Hydroxy-5-({(3-phenoxybenzyl)[(1S)-1,2,3,4-tetrahydro-1-napthalenyl]amino}carbonyl)terephthalsäure;
4-Hydroxy-6-({(3-phenoxybenzyl)[(1S)-1,2,3,4-tetrahydro-1-napthalenyl]amino}carbonyl)isophthalsäure;
2-({(3-Phenoxybenzyl)[(1S)-1,2,3,4-tetrahydro-1-napthalenyl]amino}carbonyl)-5-sulfoterephthalsäure; und
4-({(3-Phenoxybenzyl)[(1S)-1,2,3,4-tetrahydro-1-napthalenyl]amino}carbonyl)-6-sulfoisophthalsäure;
4-({[(4-Methoxyphenyl)sulfonyl]amino}carbonyl)-6-({(3-phenoxybenzyl)[(1S)-1,2,3,4-tetrahydro-1-napthalenyl]amino} carbonyl)isophthalsäure;
2-({[(4-Methoxyphenyl)sulfonyl]amino}carbonyl-5-({(3-phenoxybenzyl)[(1S)-1,2,3,4-tetrahydro-1-napthalenyl]amino} carbonyl)terephthalsäure;
4-({[(3-Chlor-4-methylphenyl)sulfonyl]amino}carbonyl)-6-({(3-phenoxybenzyl)[(1S)-2,2,3,4-tetrahydro-1-napthalenyl]amino} carbonyl)isophthalsäure;
2-({[(3-Chlor-4-methylphenyl)sulfonyl]amino}carbonyl-5-({(3-phenoxybenzyl)[(1S)-1,2,3,4-tetrahydro-1-napthalenyl]amino} carbonyl)terephthalsäure;
4-({[(2-Chlorphenyl)sulfonyl]amino}carbonyl)-6-({(3-phenoxybenzyl)[(1S)-1,2,3,4-tetrahydro-1-napthalenyl]amino} carbonyl)isophthalsäure;
2-({[(2-Chlorphenyl)sulfonyl]amino}carbonyl)-5-({(3-phenoxybenzyl)[(1S)-1,2,3,4-tetrahydro-1-napthalenyl]amino} carbonyl)terephthalsäure;
4-({[(3-Chlorphenyl)sulfonyl]amino}carbonyl)-6-({(3-phenoxybenzyl)[(1S)-1,2,3,4-tetrahydro-1-napthalenyl]amino} carbonyl)isophthalsäure;
2-({[(3-Chlorphenyl)sulfonyl]amino}carbonyl)-5-({(3-phenoxybenzyl)[(1S)-1,2,3,4-tetrahydro-1-napthalenyl]amino} carbonyl)terephthalsäure;
4-({[(4-Chlorphenyl)sulfonyl]amino}carbonyl)-6-({(3-phenoxybenzyl)[(1S)-1,2,3,4-tetrahydro-1-napthalenyl]amino} carbonyl)isophthalsäure;
2-({[(4-Chlorphenyl)sulfonyl]amino}carbonyl)-5-({(3-phenoxybenzyl)[(1S)-1,2,3,4-tetrahydro-1-napthalenyl]amino} carbonyl)terephthalsäure;
4-({[(4-Nitrophenyl)sulfonyl]amino}carbonyl)-6-([(3-phenoxybenzyl)[(1S)-1,2,3,4-tetrahydro-1-napthalenyl]amino} carbonyl)isophthalsäure;
2-({[(4-Nitrophenyl)sulfonyl]amino}carbonyl)-5-({(3-phenoxybenzyl)[(1S)-1,2,3,4-tetrahydro-1-napthalenyl]amino} carbonyl)terephthalsäure;
4-({[(4-Bromphenyl)sulfonyl]amino}carbonyl)-6-({(3-phenoxybenzyl)[(1S)-1,2,3,4-tetrahydro-1-napthalenyl]amino} carbonyl)isophthalsäure;
2-({[(4-Bromphenyl)sulfonyl]amino}carbonyl)-5-({(3-phenoxybenzyl)[(1S)-1,2,3,4-tetrahydro-1-napthalenyl]amino} carbonyl)terephthalsäure;
4-({[(3-Nitrophenyl)sulfonyl]amino}carbonyl)-6-({(3-phenoxybenzyl)[(1S)-1,2,3,4-tetrahydro-1-napthalenyl]amino} carbonyl)isophthalsäure;
2-({[(3-Nitrophenyl)sulfonyl]amino}carbonyl)-5-({(3-phenoxybenzyl)[(1S)-1,2,3,4-tetrahydro-1-napthalenyl]amino} carbonyl)terephthalsäure;
4-({[(Phenyl)sulfonyl]amino}carbonyl)-6-({(3-phenoxybenzyl) [(1S)-1,2,3,4-tetrahydro-1-napthalenyl]amino}carbonyl) isophthalsäure;
2-({[(Phenyl)sulfonyl]amino}carbonyl)-5-({(3-phenoxybenzyl) [(1S)-1,2,3,4-tetrahydro-1-napthalenyl]amino}carbonyl) terephthalsäure;
4-({[(2-Nitrophenyl)sulfonyl]amino}carbonyl)-6-({(3-phenoxybenzyl)[(1S)-1,2,3,4-tetrahydro-1-napthalenyl]amino} carbonyl)isophthalsäure;
2-({[(2-Nitrophenyl)sulfonyl]amino}carbonyl)-5-({(3-phenoxybenzyl)[(1S)-1,2,3,4-tetrahydro-1-napthalenyl]amino} carbonyl)terephthalsäure;
4-({[(2-Methylphenyl)sulfonyl]amino}carbonyl)-6-({(3-phenoxybenzyl)[(1S)-1,2,3,4-tetrahydro-1-napthalenyl]amino} carbonyl)isophthalsäure;
2-({[(2-Methylphenyl)sulfonyl]amino}carbonyl)-5-({(3-phenoxybenzyl)[(1S)-1,2,3,4-tetrahydro-1-napthalenyl]amino} carbonyl)terephthalsäure;
4-({[(4-[(2,3-Dihydroxypropyl)amino]phenyl}sulfonyl)amino] carbonyl}-6-({(3-phenoxybenzyl)[(1S)-1,2,3,4-tetrahydro-1-napthalenyl]amino}carbonyl)isophthalsäure;
2-({[(4-[(2,3-Dihydroxyphenyl)amino]phenyl}sulfonyl)amino] carbonyl}-5-({(3-phenoxybenzyl)[(1S)-1,2,3,4-tetrahydro-1-napthalenyl]amino}carbonyl)terephthalsäure;
2-({[(4-{[(Methylamino)carbonyl]amino}phenyl)sulfonyl] amino}carbonyl)-5-({(3-phenoxybenzyl)[(1S)-1,2,3,4-tetrahydro-1-napthalenyl]amino}carbonyl)terephthalsäure;
4-({[(4-{[(Methylamino)carbonyl]amino}phenyl)sulfonyl] amino}carbonyl)-6-({(3-phenoxybenzyl)[(1S)-1,2,3,4-tetrahydro-1-napthalenyl]amino}carbonyl)isophthalsäure;
4-[({[4-(Butyrylamino)phenyl]sulfonyl}amino)carbonyl]-6-({(3-phenoxybenzyl)[(1S)-1,2,3,4-tetrahydro-1-napthalenyl]amino} carbonyl)isophthalsäure;
2-[({[(4-(Butyrylamino)phenyl]sulfonyl}amino)carbonyl]-5-({(3-phenoxybenzyl)[(1S)-1,2,3,4-tetrahydro-1-napthalenyl]amino} carbonyl)terephthalsäure;
4-({[(3,4-Difluorphenyl)sulfonyl]amino}carbonyl)-6-({(3-phenoxybenzyl)[(1S)-1,2,3,4-tetrahydro-1-napthalenyl]amino} carbonyl)isophthalsäure;
2-({[(3,4-Difluorphenyl)sulfonyl]amino}carbonyl)-5-({(3-phenoxybenzyl)[(1S)-1,2,3,4-tetrahydro-1-napthalenyl]amino} carbonyl)terephthalsäure;
4-({[(2,5-Difluorphenyl)sulfonyl]amino}carbonyl)-6-({(3-phenoxybenzyl)[(1S)-1,2,3,4-tetrahydro-1-napthalenyl]amino} carbonyl)isophthalsäure;
2-({[(2,5-Difluorphenyl)sulfonyl]amino}carbonyl)-5-({(3-phenoxybenzyl)[(1S)-1,2,3,4-tetrahydro-1-napthalenyl]amino} carbonyl)terephthalsäure;
4-({[(2-Methoxy-4-methylphenyl)sulfonyl]amino}carbonyl)-6-({(3-phenoxybenzyl)[(1S)-1,2,3,4-tetrahydro-1-napthalenyl]amino} carbonyl)isophthalsäure;
2-({[(2-Methoxy-4-methylphenyl)sulfonyl]amino}carbonyl)-5-({(3-phenoxybenzyl)[(1S)-1,2,3,4-tetrahydro-1-napthalenyl]amino} carbonyl)terephthalsäure;
4-({[(4-Methylphenyl)sulfonyl]amino}carbonyl)-6-({(3-phenoxybenzyl)[(1S)-1,2,3,4-tetrahydro-1-napthalenyl]amino} carbonyl)isophthalsäure;
2-({[(4-Methylphenyl)sulfonyl]amino}carbonyl)-5-({(3-phenoxybenzyl)[(1S)-1,2,3,4-tetrahydro-2-napthalenyl]amino} carbonyl)terephthalsäure;
2-({[(2-Fluorphenyl)sulfonyl]amino}carbonyl)-5-({(3-phenoxybenzyl)[(1S)-1,2,3,4-tetrahydro-1-napthalenyl]amino} carbonyl)terephthalsäure;
4-({[(2-Fluorphenyl)sulfonyl]amino}carbonyl)-6-({(3-phenoxybenzyl)[(1S)-1,2,3,4-tetrahydro-1-napthalenyl]amino} carbonyl)isophthalsäure;
2-({[(4-Hydroxyphenyl)sulfonyl]amino}carbonyl-5-({(3-phenoxybenzyl)[(1S)-1,2,3,4-tetrahydro-1-napthalenyl]amino} carbonyl)terephthalsäure; und
4-({[(4-Hydroxyphenyl)sulfonyl]amino}carbonyl-6-({(3-phenoxybenzyl)[(1S)-1,2,3,4-tetrahydro-1-napthalenyl]amino} carbonyl)isophthalsäure;
4-({[(5-Methyl-2-pyridinyl)sulfonyl]amino}carbonyl)-6-({(3-phenoxybenzyl)[(1S)-1,2,3,4-tetrahydro-1-napthalenyl]amino} carbonyl)isophthalsäure;
2-({[(5-Methyl-2-pyridinyl)sulfonyl]amino}carbonyl)-5-({(3-phenoxybenzyl)[(1S)-1,2,3,4-tetrahydro-1-naphthalenyl]amino} carbonyl)terephthalsäure;
4-({[(5-Nitro-2-thienyl)sulfonyl]amino}carbonyl)-6-({(3-phenoxybenzyl)[(1S)-1,2,3,4-tetrahydro-1-napthalenyl]amino} carbonyl)isophthalsäure;
2-({[(5-Nitro-2-thienyl)sulfonyl]amino}carbonyl)-5-({(3-phenoxybenzyl)[(1S)-1,2,3,4-tetrahydro-1-napthalenyl]amino} carbonyl)terephthalsäure;
2-({[(5-Chlor-2-thienyl)sulfonyl]amino}carbonyl)-5-({(3-phenoxybenzyl)[(1S)-1,2,3,4-tetrahydro-1-napthalenyl]amino} carbonyl)terephthalsäure; und
4-({[(5-Chlor-2-thienyl)sulfonyl]amino}carbonyl)-6-({(3-phenoxybenzyl)[(1S)-1,2,3,4-tetrahydro-1-napthalenyl]amino} carbonyl)isophthalsäure;
4-{[(Benzylsulfonyl)amino]carbonyl}-6-({(3-phenoxybenzyl) [(1S)-1,2,3,4-tetrahydro-1-napthalenyl]amino}carbonyl) isophthalsäure; und
2-{[(Benzylsulfonyl)amino]carbonyl}-5-({(3-phenoxybenzyl) [(1S)-1,2,3,4-tetrahydro-1-napthalenyl]amino}carbonyl) terephthalsäure;
5-({[3-(Benzyloxy)benzyl][(1S)-1,2,3,4-tetrahydro-1-napthalenyl]amino}carbonyl)-1,2,4-benzentricarbonsäure;
5-({[3-(Cyclohexyloxy)benzyl][(1S)-1,2,3,4-tetrahydro-1-napthalenyl]amino}carbonyl)-1,2,4-benzentricarbonsäure;
5-({[3-[Exo-bicyclo[2.2.1]hept-2-yloxy]benzyl}[(1S)-1,2,3,4-tetrahydro-1-napthalenyl]amino}carbonyl)-1,2,4-benzentricarbonsäure;
5-({[3-(2-Cyclohexen-1-yloxy)benzyl][(1S)-1,2,3,4-tetrahydro-1-napthalenyl]amino}carbonyl)-1,2,4-benzentricarbonsäure;
5-({[3-(4-Pyridinyloxy)benzyl][(1S)-1,2,3,4-tetrahydro-1-napthalenyl]amino}carbonyl)-1,2,4-benzentricarbonsäure; und
5-({[3-(2-Pyrimidinyloxy)benzyl][(1S)-1,2,3,4-tetrahydro-1-napthalenyl]amino}carbonyl)-1,2,4-benzentricarbonsäure;
5-({[3-(Phenylsulfanyl)benzyl][(1S)-1,2,3,4-tetrahydro-1-napthalenyl]amino}carbonyl)-1,2,4-benzentricarbonsäure;
5-({{[3-[(4-Methoxyphenyl)sulfanyl]benzyl}[(1S)-1,2,3,4-tetrahydro-1-napthalenyl]amino}carbonyl)-1,2,4-benzentricarbonsäure;
5-({{3-[(4-Nitrophenyl)sulfanyl]benzyl}[(1S)-1,2,3,4-tetrahydro-1-napthalenyl]amino}carbonyl)-1,2,4-benzentricarbonsäure; und
5-({{3-[(4-Chlorphenyl)sulfanyl]benzyl}[(1S)-1,2,3,4-tetrahydro-1-napthalenyl]amino}carbonyl)-1,2,4-benzentricarbonsäure;
5-({(3-Benzoylbenzyl)[(1S)-1,2,3,4-tetrahydro-1-napthalenyl]amino}carbonyl)-1,2,4-benzentricarbonsäure;
5-({{3-[Hydroxy(phenyl)methyl]benzyl}[(1S)-1,2,3,4-tetrahydro-1-napthalenyl]amino}carbonyl)-1,2,4-benzentricarbonsäure;
5-({{3-[(Phenoxyimino)methyl]benzyl}[(1S)-1,2,3,4-tetrahydro-1-napthalenyl]amino}carbonyl)-1,2,4-benzentricarbonsäure;
5-({{3-{[(Benzyloxy)imino]methyl}benzyl)[(1S)-1,2,3,4-tetrahydro-1-napthalenyl]amino}carbonyl)-1,2,4-benzentricarbonsäure;
5-({[3-({[(4-Nitrobenzyl)oxy]imino}methyl)benzyl][(1S)-1,2,3,4-tetrahydro-1-napthalenyl]amino}carbonyl)-1,2,4-benzentricarbonsäure; und
5-({[3-({[(2,3,9,5,6-Pentafluorbenzyl)oxy]imino}methyl) benzyl][(1S)-1,2,3,4-tetrahydro-1-napthalenyl]amino}carbonyl)-1,2,4-benzentricarbonsäure;
5-{[(1S)-1,2,3,4-Tetrahydro-1-napthalenyl(3-{[(tetrahydro-2H-pyran-2-yloxy)imino]methyl}benzyl)amino]carbonyl}-1,2,4-benzentricarbonsäure;
5-({(Cyclohexylmethyl)[(1S)-1,2,3,4-tetrahydro-1-napthalenyl]amino}carbonyl)-1,2,4-benzentricarbonsäure;
5-({(4-Phenoxybutyl)[(1S)-1,2,3,4-tetrahydro-1-napthalenyl] amino}carbonyl)-1,2,4-benzentricarbonsäure;
5-({{[5-(Ethoxycarbonyl)-2-thienyl]methyl}[(1S)-1,2,3,4-tetrahydro-1-napthalenyl]amino}carbonyl)-1,2,4-benzentricarbonsäure.

28. Eine pharmazeutische Zusammensetzung, die eine therapeutisch wirksame Menge einer Verbindung gemäß Anspruch 1 oder ein pharmazeutisch verträgliches Salz davon in Kombination mit einem pharmazeutisch verträglichen Träger umfaßt.

29. Verwendung einer Verbindung gemäß Anspruch 1 oder ein pharmazeutisch verträgliches Salz davon zur Herstellung eines Medikaments zur Behandlung von Blasenüberaktivität oder Harninkontinenz oder Schmerzen in einem Wirtssäugetier, das eine solche Behandlung benötigt.

## Revendications

1. Composé de formule (I) 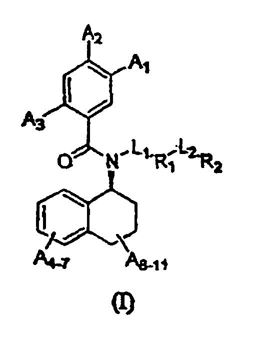 ou un sel pharmaceutiquement acceptable de celui-ci, dans lequel,
A₁ et A₂ sont chacun indépendamment choisis dans le groupe constitué par les groupes alcoxycarbonyle, alkylcarbonyloxy, carboxy, hydroxy, hydroxyalkyle, (NR_{A}R_{B})carbonyle, (NR_{C}S(O)₂R_{D})carbonyle, -S(O)₂OH et tétrazolyle ; ou
A₁ et A₂ conjointement avec les atomes de carbone auxquels ils sont liés forment un hétérocycle à cinq chaînons contenant un atome de soufre dans lequel l'hétérocycle à cinq chaînons est facultativement substitué par 1 ou 2 substituants choisis dans le groupe constitué par les groupes mercapto et oxo ;
A₃ est choisi dans le groupe constitué par les groupes alcoxycarbonyle, alkylcarbonyloxy, carboxy, hydroxy, hydroxyalkyle, (NR_{A}R_{B})carbonyle, (NR_{C}S(O)₂R_{D})carbonyle, -S(O)₂OH et tétrazolyle ;
A₄, A₅, A₆ et A₇ sont chacun indépendamment choisis dans le groupe constitué par un atome d'hydrogène, un groupe alcoxy, alcoxycarbonyle, alcényle, alkyle, alkylcarbonyle, alkylcarbonyloxy, alcynyle, carboxy, cyano, haloalcoxy, haloalkyle, halogène, hydroxy, hydroxyalkyle, nitro, -NR_{E}R_{F} et (NR_{E}R_{F}) carbonyle ;
A₈, A₉, A₁₀ et A₁₁ sont chacun indépendamment choisis dans le groupe constitué par un atome d'hydrogène, un groupe alcoxy, alcoxycarbonyle, alcényle, alkyle, alkylcarbonyle, alkylcarbonyloxy, alcynyle, carboxy, haloalcoxy, haloalkyle, halogène, hydroxy, hydroxyalkyle, -NR_{E}R_{F} et (NR_{E}R_{F})carbonyle et oxo ;
R_{A} et R_{B} sont chacun indépendamment choisis dans le groupe constitué par un atome d'hydrogène, un groupe alkyle et un groupe cyano ;
R_{C} est choisi dans le groupe constitué par un atome d'hydrogène et un groupe alkyle ;
R_{D} est choisi dans le groupe constitué par les groupes alcoxy, alkyle, aryle, arylalcoxy, arylalkyle, haloalcoxy et haloalkyle ;
R_{E} et R_{F} sont chacun indépendamment choisis dans le groupe constitué par un atome d'hydrogène, les groupes alkyle, alkylcarbonyle, formyle, et hydroxyalkyle ;
L₁ est choisi dans le groupe constitué par les groupes alcénylène, alkylène, alcynylène, -(CH₂)ₘO(CH₂)ₙ-, - (CH₂)ₘS(CH₂)ₙ- et - (CH₂)ₚC(O)(CH₂)_{q}- dans lequel chaque groupe est pris avec l'extrémité gauche liée à N et l'extrémité droite liée à R₁ ;
m est un nombre entier de 0 à 10 ;
n est un nombre entier de 0 à 10 ;
R₁ est choisi dans le groupe constitué par les groupes aryle, cycloalcényle, cycloalkyle et hétérocyclique ;
dans lequel le groupe aryle est substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH=NOR_{EE} dans lequel R_{EE} est choisi parmi un atome d'hydrogène et un groupe alkyle, tel que défini ci-dedans ; les groupes cycloalcényle et cycloalkyle sont substitués par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F}) carbonyle et -CH=NOR_{EE} dans lequel R_{EE} est choisi parmi un atome d'hydrogène et un groupe alkyle, tel que défini ci-dedans ; et le groupe hétérocyclique est substitué par 0, 1, 2, ou 3 substituants indépendamment choisis parmi les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, arylalcoxycarbonyl, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F}) carbonyle, -N(R_{A})C(O)NR_{B}R_{c}, et -CH=NOR_{EE} dans lequel R_{EE} est choisi parmi un atome d'hydrogène et un groupe alkyle, tel que défini ci-dedans ;
L₂ est absent ou choisi dans le groupe constitué par une liaison covalente, un groupe alcénylène, alkylène, alcynylène, -(CH₂)ₚO(CH₂)_{q}, -(CH₂)ₚS(CH₂)_{q}, -(CH₂)ₚC(O)(CH₂)_{q}, -(CH₂)ₚC(OH)(CH₂)_{q} et - (CH₂)ₚCH=H(CH₂)_{q}- dans lequel chaque groupe est pris avec l'extrémité gauche liée à R₁ et l'extrémité droite liée à R₂ ;
p est un nombre entier de 0 à 10 ;
q est un nombre entier de 0 à 10 ; et
R₂ est absent ou choisi dans le groupe constitué par les groupes aryle, cycloalcényle, cycloalkyle et hétérocyclique ;
dans lequel à toute occurrence le groupe aryle est substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH=NOR_{EE} dans lequel R_{EE} est choisi parmi un atome d'hydrogène et un groupe alkyle, tel que défini ci-dedans ; les groupes cycloalcényle et cycloalkyle sont substitués par 0, 1, 2, 3, 4 ou 5 substituants chacun indépendamment choisis parmi les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle et -CH=NOR_{EE} dans lequel R_{EE} est choisi parmi un atome d'hydrogène et un groupe alkyle, tel que défini ci-dedans ; et le groupe hétérocyclique peut être substitué par 0, 1, 2, ou 3 substituants indépendamment choisis parmi les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyl, alcynyloxy, arylalcoxycarbonyl, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F}) carbonyle, -N(R_{A})C(O)NR_{E}R_{c}, et -CH=NOR_{EE} dans lequel R_{EE} est choisi parmi un atome d'hydrogène et un groupe alkyle, tel que défini ci-dedans.

2. Composé selon la revendication 1, dans lequel
A₁ et A₂ sont chacun indépendamment choisis dans le groupe constitué par un groupe alcoxycarbonyle, carboxy, hydroxy, (NR_{A}R_{B}) carbonyle, (NR_{c}S(O)₂R_{D}) carbonyle, -S(O)₂OH ;
A₃ est un groupe carboxy ;
A₄, A₅, A₆, A₇, A₈, A₉, A₁₀ et A₁₁ sont chacun un atome d'hydrogène ;
R_{A} et R_{B} sont chacun indépendamment choisis dans le groupe constitué par un atome d'hydrogène, un groupe alkyle et un groupe cyano ;
L₁ est choisi dans le groupe constitué par les groupes alkylène et -(CH₂)ₘO(CH₂)ₙ-;
R₁ est choisi dans le groupe constitué par les groupes aryle, cycloalkyle et hétérocyclique ;
L₂ est absent ou choisi dans le groupe constitué par une liaison covalente, un groupe alkylène, -(CH₂)ₚO(CH₂)_{q}-, -(CH₂)ₚC(O)(CH₂)_{q}-, -(CH₂)ₚC(OH)(CH₂)_{q}-, -(CH₂)ₚS(CH₂)_{q}-, et - (CH₂)ₚCH=NO(CH₂)_{q}-;
p est 0 ;
q est un nombre entier de 0 à 1 ; et
R₂ est absent ou dans le groupe constitué par les groupes aryle, cycloalcényle, cycloalkyle et hétérocyclique.

3. Composé selon la revendication 1, dans lequel A₁ et A₂ conjointement avec les atomes de carbone auxquels ils sont liés forment un hétérocycle à 5 chaînons contenant un atome de soufre dans lequel l'hétérocycle à 5 chaînons est substitué par 0, 1, ou 2 substituants choisis dans le groupe constitué par les groupes mercapto et oxo.

4. Composé selon la revendication 1, dans lequel
L₁ est un groupe alkylène ;
R₁ est un groupe aryle ;
L₂ est absent ; et
R₂ est absent.

5. Composé selon la revendication 1, dans lequel
L₁ est un groupe alkylène ;
R₁ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F}) carbonyle, -N(R_{A})C(O)NR_{B}R_{c}, et -CH=NOR_{EE} ;
L₂ est absent ; et
R₂ est absent,
de préférence dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est choisi dans le groupe constitué par -CH₂-, -CH₂CH₂- et -CH₂CH(CH₃)- ;
R₁ est un groupe aryle dans lequel ledit groupe aryle est un groupe phényle substitué par 0, 1 ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est absent ; et
R₂ est absent.

6. Composé selon la revendication 1, dans lequel
L₁ est un groupe alkylène ;
R₁ est un groupe aryle dans lequel ledit groupe aryle est un groupe fluorényle substitué par 0, 1 ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxy, hydroxyalkyle, nitro, oxo, et -NR_{E}R_{F} ;
L₂ est absent ; et
R₂ est absent ;
de préférence dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe fluorényle substitué par 0 ou 1 substituant choisi dans le groupe constitué par les groupes hydroxy et oxo ;
L₂ est absent ; et
R₂ est absent.

7. Composé selon la revendication 1, dans lequel
L₁ est un groupe alkylène ;
R₁ est un groupe aryle ;
L₂ est une liaison covalente ; et
R₂ est un groupe aryle ;
de préférence dans lequel
L₁ est un groupe alkylène ;
R₁ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH=NOR_{EE} ;
L₂ est une liaison covalente ; et
R₂ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH=NOR_{EE},
de manière davantage préférée dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est une liaison covalente ; et
R₂ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F}.

8. Composé selon la revendication 1, dans lequel
A₁ et A₂ conjointement avec les atomes de carbone auxquels ils sont liés forment un hétérocycle à 5 chaînons contenant un atome de soufre dans lequel l'hétérocycle est substitué par 0, 1, ou 2 substituants choisis dans le groupe constitué par les groupes mercapto et oxo ;
A₃ est un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est une liaison covalente ; et
R₂ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} .

9. Composé selon la revendication 1, dans lequel
L₁ est un groupe alkylène ;
R₁ est un groupe aryle ;
L₂ est une liaison covalente ; et
R₂ est un hétérocycle ;
de préférence dans lequel
L₁ est un groupe alkylène ;
R₁ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH-NOR_{EE} ;
L₂ est une liaison covalente ; et
R₂ est un hétérocycle dans lequel ledit hétérocycle est choisi dans le groupe constitué par les groupes azétidinyle, azépanyle, aziridinyle, furyle, morpholinyle, pipérazinyle, pipéridinyle, pyrazinyle, pyridazinyle, pyridinyle, pyrimidinyle, pyrrolidinyle et thiényle dans lequel ledit hétérocycle est substitué par 0, 1, 2 ou 3 substituants indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, arylalcoxycarbonyl, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F}) carbonyle, -N(R_{A})C(O)NR_{B}R_{c}, et -CH=NOR_{EE} ;
de manière davantage préférée dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est une liaison covalente ; et
R₂ est un hétérocycle dans lequel ledit hétérocycle est choisi dans le groupe constitué par les groupes pyridinyle, pyrrolidinyle et thiényle dans lequel ledit hétérocycle est substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, nitro, et -NR_{E}R_{F} .

10. Composé selon la revendication 1, dans lequel,
L₁ est un groupe alkylène ;
R₁ est un groupe aryle ;
L₂ est un groupe alkylène ; et
R₂ est un groupe aryle ;
de préférence dans lequel
L₁ est un groupe alkylène ;
R₁ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH=NOR_{EE} ;
L₂ est un groupe alkylène ; et
R₂ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH=NOR_{EE},
de manière davantage préférée dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est un groupe alkylène dans lequel ledit groupe alkylène est -CH₂- ; et
R₂ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} .

11. Composé selon la revendication 1, dans lequel,
L₁ est un groupe alkylène ;
R₁ est un groupe aryle ;
L₂ est (CH₂)ₚO(CH₂)_{q}- ; et
R₂ est un groupe aryle,
de préférence dans lequel
L₁ est un groupe alkylène ;
R₁ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F}) carbonyle, -N(R_{A})C(O)NR_{B}R_{c}, et -CH=NOR_{EE} ;
L₂ est -(CH₂)ₚO(CH₂)_{q}- ; et
R₂ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F}) carbonyle, -N(R_{A})C(O) NR_{E}R_{C}, et -CH=NOR_{EE} ;
ou dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est -(CH₂)ₚO(CH₂)_{q}- ;
p est 0 ;
q est 0 ; et
R₂ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
ou dans lequel
A₁ et A₂ sont chacun indépendamment choisis dans le groupe constitué par un groupe alcoxycarbonyle, carboxy, hydroxy, (NR_{A}R_{B}) carbonyle, et -S(O)₂OH ;
A₃ est un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est -(CH₂)ₚO(CH₂)_{q}- ;
p est 0 ;
q est 0 ; et
R₂ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F}.

12. Composé selon la revendication 1, dans lequel,
A₁ et A₂ sont chacun indépendamment choisis dans le groupe constitué par les groupes carboxy et (NR_{C}S(O)₂R_{D})carbonyle, dans lequel un de A₁ ou A₂ est (NR_{C}S(O)₂R_{D})carbonyle ;
A₃ est un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F}) carbonyle, -N(R_{A})C(O)NR_{B}R_{c}, et -CH=NOR_{EE} ;
L₂ est -(CH₂)ₚO(CH₂)_{q}- ;
p est 0 ;
q est 0 ; et
R₂ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH=NOR_{EE} ;
de préférence dans lequel
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
R₂ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ; et
R_{D} est choisi dans le groupe constitué par les groupes alkyle et aryle, dans lequel de préférence ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, -NR_{E}R_{F}, et -N(R_{A})C(O)NR_{B}R_{c}.

13. Composé selon la revendication 1, dans lequel,
A₁ et A₂ sont chacun indépendamment choisis dans le groupe constitué par les groupes carboxy et (NR_{C}S(O)₂R_{D}) carbonyle, dans lequel un de A₁ et A₂ est (NR_{C}S(O)₂R_{D})carbonyle ;
A₃ est un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F}) carbonyle, -N(R_{A})C(O)NR_{B}R_{c}, et -CH=NOR_{EE} ;
L₂ est - (CH₂)ₚO(CH₂)_{q}- ;
p est 0 ;
q est 0 ;
R₂ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH=NOR_{EE} ; et
R_{D} est un hétérocycle,
de préférence dans lequel
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
R₂ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ; et
R_{D} est un hétérocycle dans lequel ledit hétérocycle est un groupe pyridinyle et un groupe thiényle dans lequel ledit hétérocycle est substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, nitro, -N(R_{A})C(O)NR_{B}R_{c}, et -NR_{E}R_{F}.

14. Composé selon la revendication 1, dans lequel,
A₁ et A₂ sont chacun indépendamment choisis dans le groupe constitué par les groupes carboxy et (NR_{C}S(O)₂R_{D})carbonyle dans lequel un de A₁ et A₂ est (NR_{C}S(O)₂R_{D})carbonyle ;
A₃ est un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{C}, et CH=NOR_{EE} ;
L₂ est -(CH₂)ₚO(CH₂)_{q}- ;
p est 0 ;
q est 0 ;
R₂ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènèdioxy, nitro, oxo, -NR_{E}R_{F}, (NR_{E}R_{F})carbonyle, -N(R_{A})C(O)NR_{B}R_{c}, et -CH=NOR_{EE} ; et
R_{D} est un groupe arylalkyle,
de préférence dans lequel
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
R₂ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ; et
R_{D} est un groupe arylalkyle dans lequel le groupe aryle du groupe arylalkyle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F}.

15. Composé selon la revendication 1, dans lequel,
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est - (CH₂)ₚO(CH₂)_{q}- ;
p est 0 ;
q est 1 ; et
R₂ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F}.

16. Composé selon la revendication 1, dans lequel
L₁ est un groupe alkylène ;
R₁ est un groupe aryle ;
L₂ est -(CH₂)ₚO(CH₂)_{q}- ; et
R₂ est un groupe cycloalkyle,
de préférence dans lequel
L₁ est un groupe alkylène ;
R₁ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH=NOR_{EE} ;
L₂ est - (CH₂)ₚO(CH₂)_{q}- ; et
R₂ est un groupe cycloalkyle,
de manière davantage préférée dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est - (CH₂)ₚO(CH₂)_{q}- ;
p est 0 ;
q est 0 ; et
R₂ est un groupe cycloalkyle.

17. Composé selon la revendication 1, dans lequel,
L₁ est un groupe alkylène ;
R₁ est un groupe aryle ;
L₂ est -(CH₂)ₚO(CH₂)_{q}- ; et
R₂ est un groupe cycloalcényle,
de préférence dans lequel
L₁ est un groupe alkylène ;
R₁ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F}) carbonyle, -N(R_{A})C(O)NR_{B}R_{c}, et -CH=NOR_{EE} ;
L₂ est - (CH₂)ₚO(CH₂)_{q}- ; et
R₂ est un groupe cycloalcényle,
de manière davantage préférée dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est - (CH₂)ₚO(CH₂)_{q}- ;
p est 0 ;
q est 0 ; et
R₂ est un groupe cycloalcényle.

18. Composé selon la revendication 1, dans lequel,
L₁ est un groupe alkylène ;
R₁ est un groupe aryle ;
L₂ est -(CH₂)ₚO(CH₂)_{q}- ; et
R₂ est un hétérocycle,
de préférence dans lequel
L₁ est un groupe alkylène ;
R₁ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH=NOR_{EE} ;
L₂ est -(CH₂)ₚO(CH₂)_{q}- ; et
R₂ est un hétérocycle dans lequel ledit hétérocycle est choisi dans le groupe constitué par les groupes furyle, pyrazinyle, pyridazinyle, pyridinyle, pyrimidinyle, et thiényle dans lequel ledit hétérocycle est substitué par 0, 1, 2 ou 3 substituants indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, arylalcoxycarbonyl, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F}) carbonyle, *-*N(R_{A})C(O)NR_{E}R_{C}, et -CH=NOR_{EE},
de manière davantage préférée dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est - (CH₂)ₚO(CH₂)_{q}- ;
p est 0 ;
q est 0 ; et
R₂ est un hétérocycle dans lequel ledit hétérocycle est choisi dans le groupe constitué par les groupes pyridinyle et pyrimidinyle dans lequel ledit hétérocycle est substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, nitro, -N(R_{A})C(O) NR_{E}R_{C}, et -NR_{E}R_{F}.

19. Composé selon la revendication 1, dans lequel,
L₁ est un groupe alkylène ;
R₁ est un groupe aryle ;
L₂ est - (CH₂)ₚS(CH₂)_{q}- ; et
R₂ est un groupe aryle,
de préférence dans lequel
L₁ est un groupe alkylène ;
R₁ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH=NOR_{EE} ;
L₂ est - (CH₂)ₚS(CH₂)_{q}- ; et
R₂ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F}) carbonyle, -N(R_{A})C(O)NR_{B}R_{c}, et -CH=NOR_{EE},
de manière davantage préférée dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est - (CH₂)ₚS(CH₂)_{q}- ;
p est 0 ;
q est 0 ; et
R₂ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F}.

20. Composé selon la revendication 1, dans lequel
L₁ est un groupe alkylène ;
R₁ est un groupe aryle ;
L₂ est -(CH₂)ₚC(O)(CH₂)_{q}- ; et
R₂ est un groupe aryle,
de préférence dans lequel
L₁ est un groupe alkylène ;
R₁ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH=NOR_{EE} ;
L₂ est - (CH₂)ₚC(O)(CH₂)_{q}- ; et
R₂ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH=NOR_{EE},
de manière davantage préférée dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est -(CH₂)ₚC(O)(CH₂)_{q}- ;
p est 0 ;
q est 0 ; et
R₂ est un groupe aryle dans lequel ledit groupe aryle est un groupe phényle substitué par 0, 1, ou 2 substituants choisis dans le groupe constitué par les groupés alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F}.

21. Composé selon la revendication 1, dans lequel,
L₁ est un groupe alkylène ;
R₁ est un groupe aryle ;
L₂ est -(CH₂)ₚC(OH)(CH₂)_{q}- ; et
R₂ est un groupe aryle ;
de préférence dans lequel
L₁ est un groupe alkylène ;
R₁ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH=NOR_{EE} ;
L₂ est - (CH₂)ₚC(OH)(CH₂)_{q}- ; et
R₂ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH-NOR_{EE} ;
de manière davantage préférée dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est - (CH₂)ₚC(OH)(CH₂)_{q}- ;
p est 0 ;
q est 0 ; et
R₂ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F}.

22. Composé selon la revendication 1, dans lequel,
L₁ est un groupe alkylène ;
R₁ est un groupe aryle ;
L₂ est - (CH₂)ₚCH=NO(CH₂)_{q}- ; et
R₂ est un groupe aryle,
de préférence dans lequel
L₁ est un groupe alkylène ;
R₁ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O)NR_{B}R_{c}, et -CH=NOR_{EE} ;
L₂ est -(CH₂)ₚCH=NO(CH₂)_{q}- ; et
R₂ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle, -N(R_{A})C(O) NR_{E}R_{C}, et -CH=NOR_{EE},
de manière davantage préférée dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est -(CH₂)ₚCH=NO(CH₂)_{q}- ;
p est 0 ;
q est un nombre entier de 0 à 1 ; et
R₂ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F}.

23. Composé selon la revendication 1, dans lequel,
L₁ est un groupe alkylène ;
R₁ est un groupe aryle ;
L₂ est - (CH₂)ₚCH=NO(CH₂)_{q}- et
R₂ est un hétérocycle,
de préférence dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est un groupe alkylène dans lequel ledit groupe alkylène est -CH₂- ;
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est -(CH₂)ₚCH=NO(CH₂)_{q}- ;
p est 0 ;
q est 0 ; et
R₂ est un hétérocycle dans lequel ledit hétérocycle est un groupe pyranyle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, nitro, -N(R_{A})C(O)NR_{B}R_{c}, et -NR_{E}R_{F}.

24. Composé selon la revendication 1, dans lequel,
L₁ est un groupe alkylène ;
R₁ est un groupe cycloalkyle ;
L₂ est absent ; et
R₂ est absent,
de préférence dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est -CH₂- ;
R₁ est un groupe cycloalkyle ;
L₂ est absent ; et
R₂ est absent.

25. Composé selon la revendication 1, dans lequel,
L₁ est - (CH₂)ₘO(CH₂)ₙ- ;
R₁ est un groupe aryle ;
L₂ est absent ; et
R₂ est absent,
de préférence dans lequel
L₁ est - (CH₂)ₘO(CH₂)ₙ- ;
R₁ est un groupe aryle dans lequel ledit groupe aryle est un groupe phényle substitué par 0, 1, 2, 3, 4 ou 5 substituants chacun indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F}) carbonyle, -N(R_{A})C(O)NR_{B}R_{c}, et -CH=NOR_{EE} ;
L₂ est absent ; et
R₂ est absent,
de manière davantage préférée dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est -(CH₂)ₘO(CH₂)ₙ- ;
m est un nombre entier de 2 à 4 ;
n est 0 ;
R₁ est un groupe aryle dans lequel ledit groupe aryle est un groupe phényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, méthylènedioxy, nitro, -CH=NOR_{EE}, et -NR_{E}R_{F} ;
L₂ est absent ; et
R₂ est absent.

26. Composé selon la revendication 1, dans lequel,
L₁ est un groupe alkylène ;
R₁ est un hétérocycle ;
L₂ est absent ; et
R₂ est absent,
de préférence dans lequel
L₁ est un groupe alkylène ;
R₁ est un hétérocycle dans lequel ledit hétérocycle est choisi dans le groupe constitué par les groupes furyle, pyrazinyle, pyridazinyle, pyridinyle, pyrimidinyle, et thiényle dans lequel ledit hétérocycle est substitué par 0, 1, 2 ou 3 substituants indépendamment choisis dans le groupe constitué par les groupes alcényle, alcényloxy, alcoxy, alcoxyalcoxy, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfonyle, alkylthio, alkylthioalkyle, alcynyle, alcynyloxy, arylalcoxycarbonyl, carboxy, carboxyalkyle, cyano, cyanoalkyle, éthylènedioxy, formyle, haloalcoxy, haloalkyle, haloalkylthio, halogène, hydroxy, hydroxyalkyle, mercapto, méthylènedioxy, nitro, oxo, -NR_{E}R_{F}, (NR_{E}R_{F})carbonyle,-N(R_{A})C(O)NR_{B}R_{c}, et -CH=NOR_{EE} ;
L₂ est absent ; et
R₂ est absent,
de manière davantage préférée dans lequel
A₁, A₂ et A₃ sont chacun un groupe carboxy ;
A₄, A₅, A₆, A₇, A₈, A₉, A₁₀ et A₁₁ sont chacun un atome d'hydrogène ;
L₁ est un groupe alkylène dans lequel le groupe alkylène est -CH₂- ;
R₁ est un hétérocycle dans lequel ledit hétérocycle est un groupe thiényle substitué par 0, 1, ou 2 substituants choisis dans le groupe constitué par les groupes alcoxy, alcoxycarbonyle, alcényle, alcényloxy, alkyle, alkylthio, cyano, formyle, haloalcoxy, haloalkyle, haloalkylthio, halogène, hydroxyalkyle, nitro, -N(R_{A})C(O)NR_{B}R_{c}, et -NR_{E}R_{F} ;
L₂ est absent ; et
R₂ est absent.

27. Composé selon la revendication 6, choisi dans le groupe constitué par :
l'acide 5-({(4-chlorobenzyl)[(1S)-1,2,3,4-tétrahydro-1-naphtalényl]amino}carbonyl)-1,2,4-benzènetricarboxylique ;
l'acide 5-({(4-bromobenzyl)[(1S)-1,2,3,4-tétrahydro-1-naphtalényl]amino}carbonyl)-1,2,4-benzènetricarboxylique ;
l'acide 5-({(3-bromobenzyl)[(1S)-1,2,3,4-tétrahydro-1-naphtalényl]amino}carbonyl)-1,2,4-benzènetricarboxylique ;
l'acide 5-({(3,4-dichlorobenzyl)[(1S)-1,2,3,4-tétrahydro-1-naphtalényl]amino}carbonyl)-1,2,4-benzènetricarboxylique ;
l'acide 5-({(4-cyanobenzyl)[(1S)-1,2,3,4-tétrahydro-1-naphtalényl]amino}carbonyl)-1,2,4-benzènetricarboxylique ;
l'acide 5-({(4-chloro-3-nitrobenzyl)[(1S)-1,2,3,4-tétrahydro-1-naphtalényl]amino}carbonyl)-1,2,4-benzènetricarboxylique ;
l'acide 5-({[4-(diméthylamino}benzyl][(1S)-1,2,3,4-tétrahydro-1-naphtalényl]amino}carbonyl)-1,2,4-benzènetricarboxylique ;
l'acide 5-({(3-chlorobenzyl)[(1S)-1,2,3,4-tétrahydro-1-naphtalényl]amino}carbonyl)-1,2,4-benzènetricarboxylique ;
l'acide 5-({(3-cyanobenzyl)[(1S)-1,2,3,4-tétrahydro-1-naphtalényl]amino}carbonyl)-1,2,4-benzènetricarboxylique ;
l'acide 5-({(2-phénylpropyl)[(1S)-1,2,3,4-tétrahydro-1-naphtalényl]amino}carbonyl)-1,2,4-benzènetricarboxylique ;
l'acide 5-({(2-phényléthyl)[(1S)-1,2,3,4-tétrahydro-1-naphtalényl]amino}carbonyl)-1,2,4-benzènetricarboxylique ;
l'acide 5-({benzyl[(1S)-1,2,3,4-tétrahydro-1-naphtalényl]amino}carbonyl)-1,2,4-benzènetricarboxylique ;
l'acide 5-({(3-méthoxybenzyl)[(1S)-1,2,3,4-tétrahydro-1-naphtalényl]amino}carbonyl)-1,2,4-benzènetricarboxylique ;
l'acide 5-({(3-nitrobenzyl)[(1S)-1,2,3,4-tétrahydro-1-naphtalényl]amino}carbonyl)-1,2,4-benzènetricarboxylique ;
l'acide 5-({[spiro[1,3-benzodioxol-5-yl-2,1'-cyclohexane]méthyl][(1S)-1,2,3,4-tétrahydro-1-naphtalényl]amino}-carbonyl)-1,2,4-benzènetricarboxylique ;
l'acide 5-({[3-(allyloxy)benzyl][(1S)-1,2,3,4-tétrahydro-1-naphtalényl]amino}carbonyl)-1,2,4-benzènetricarboxylique ;
l'acide 5-({[3,5-bis(trifluorométhyl)benzyl][(1S)-1,2,3,4-tétrahydro-1-naphtalényl]amino}carbonyl)-1,2,4-benzènetricarboxylique ;
l'acide 5-({(1S)-1,2,3,4-tétrahydro-1-naphtalényl[3(trifluorométhyl)benzyl]amino}carbonyl)-1,2,4-benzènetricarboxylique ;
l'acide 5-({(3,5-difluorobenzyl)[(1S)-1,2,3,4-tétrahydro-1-naphtalényl]amino}carbonyl)-1,2,4-benzènetricarboxylique ;
l'acide 5-({[4-fluoro-3-(trifluorométhyl)benzyl][(1S)-1,2,3,4-tétrahydro-1-naphtalényl]amino}carbonyl)-1,2,4-benzènetricarboxylique ;
l'acide 5-({(3,5-dibromobenzyl)[(1S)-1,2,3,4-tétrahydro-1-naphtalényl]amino}carbonyl)-1,2,4-benzènetricarboxylique ;
l'acide 5-({[3-fluoro-5-(trifluorométhyl)benzyl][(1S)-1,2,3,4-tétrahydro-1-naphtalényl]amino}carbonyl)-1,2,4-benzènetricarboxylique ;
l'acide 5-({(3,5-diméthoxybenzyl)[(1S)-1,2,3,4-tétrahydro-1-naphtalényl]amino}carbonyl)-1,2,4-benzènetricarboxylique ;
l'acide 5-({(2,3-dichlorobenzyl)[(1S)-1,2,3,4-tétrahydro-1-naphtalényl]amino}carbonyl)-1,2,4-benzènetricarboxylique ;
l'acide 5-({(2,4-dichlorobenzyl)[(1S)-1,2,3,4-tétrahydro-1-naphtalényl]amino}carbonyl)-1,2,4-benzènetricarboxylique ;
l'acide 5-({(3,5-diméthylbenzyl)[(1S)-1,2,3,4-tétrahydro-1-naphtalényl]amino}carbonyl)-1,2,4-benzènetricarboxylique ;
l'acide 5-[((1S)-1,2,3,4-tétrahydro-1-naphtalényl{3[(trifluorométhyl)sulfanyl]benzyl}amino)carbonyl]-1,2,4-benzènetricarboxylique ;
l'acide 5-({[3-(méthylsulfanyl)benzyl][(1S)-1,2,3,4-tétrahydro-1-naphtalényl]amino}carbonyl)-1,2,4-benzènetricarboxylique ;
l'acide 5-({{3-[(méthoxyimino)méthyl]benzyl}[(1S)-1,2,3,4-tétrahydro-1-naphtalényl]amino}carbonyl)-1,2,4-benzènetricaxboxylique ;
l'acide 5-({[3-(hydroxyméthyl)benzyl][(1S)-1,2,3,4-tétrahydro-1-naphtalényl]amino}carbonyl)-1,2,4-benzènetricarboxylique ;
l'acide 5-({(3-formylbenzyl)[(1S)-1,2,3,4-tétrahydro-1-naphtalényl]amino}carbonyl)-1,2,4-benzènetricaxboxylique ;
l'acide 5-({{3-[(tert-butoxyimino)méthyl]benzyl}[(1S)-1,2,3,4-tétrahydro-1-naphtalényl]amino}carbonyl)-1,2,4-benzènetricarboxylique ; et
l'acide 5-({{3-[(isopropoxyimino)méthyl]benzyl][(1S)-1,2,3,4-tétrahydro-1-naphtalényl]amino}carbonyl)-1,2,4-benzènetricarboxylique ;
l'acide 5-({(9H-fluoren-2-ylméthyl)[(1S)-1,2,3,4-tétrahydro-1-naphtalényl]amino}carbonyl)-1,2,4-benzènetricarboxylique ;
l'acide 5-({[(9-hydroxy-9H-fluoren-2-yl)méthyl][(1S)-1,2,3,4-tétrahydro-1-naphtalényl]amino}carbonyl)-1,2,4-benzènetricarboxylique ; et
l'acide 5-({[(9-oxo-9H-fluoren-2-yl)méthyl][(1S)-1,2,3,4-tétrahydro-1-naphtalényl]amino}carbonyl)-1,2,4-benzènetricarboxylique ;
l'acide 5-({([1,1'-biphényl]-4-ylméthyl)[(1S)-1,2,3,4-tétrahydro-1-naphtalényl]amino}carbonyl)-1,2,4-benzènetricarboxylique ;
l'acide 5-({[(2'-chloro [1,1'-biphényl]-4-yl)méthyl][(1S)-1,2,3,4-tétrahydro-1-naphtalényl]amino}carbonyl)-1,2,4-benzènetricarboxylique ;
l'acide 5-({[(3',5'-dichloro[1,1'-biphényl]-4-yl)méthyl]-[(1S)-1,2,3,4-tétrahydro-1-naphtalényl]amino}carbonyl)-1,2,4-benzènetricarboxylique ;
l'acide 5-({[(2'-méthoxy[1,1'-biphényl]-4-yl)méthyl]-[(1S)-1,2,3,4-tétrahydro-1-naphtalényl]amino}carbonyl)-1,2,4-benzènetricarboxylique ;
l'acide 5-({[(4'-chloro[1,1'-biphényl]-4-yl)méthyl][(1S)-1,2,3,4-tétrahydro-1-naphtalényl]amino}carbonyl)-1,2,4-benzènetricarboxylique ;
l'acide 5-({[(4'-fluoro[1,1'-biphényl]-4-yl)méthyl][(1S)-1,2,3,4-tétrahydro-1-naphtalényl]amino}carbonyl)-1,2,4-benzènetricarboxylique ;
l'acide 5-({[(4'-méthoxy[1,1'-biphényl]-4-yl)méthyl]-[(1S)-1,2,3,4-tétrahydro-1-naphtalényl]amino}carbonyl)-1,2,4-benzènetricarboxylique ;
l'acide 5-[((1S)-1,2,3,4-tétrahydro-1-naphtalényl{[2'-(trifluorométhyl)[1,1'-biphényl]-4-yl]méthyl}amino)carbonyl]-1,2,4-benzènetricarboxylique ;
l'acide 5-({[(2'-méthyl[1,1'-biphényl]-4-yl)méthyl][(1S)-1,2,3,4-tétrahydro-1-naphtalényl]amino}carbonyl)-1,2,4-benzènetricarboxylique ;
l'acide 5-[((1S)-1,2,3,4-tétrahydro-1-naphtalényl{[4'-(trifluorométhyl)[1,1'-biphényl]-2-yl]méthyl}amino)carbonyl]-1,2,4-benzènetricarboxylique ;
l'acide 5- ({[(4'-fluoro[1,1'-biphényl]-2-yl)méthyl][(1S)-1,2,3,4-tétrahydro-1-naphtalényl]amino}carbonyl)-1,2,4-benzènetricarboxylique ;
l'acide 5-({[(4'-fluoro[1,1'-biphényl]-3-yl)méthyl][(1S)-1,2,3,4-tétrahydro-1-naphtalényl]amino}carbonyl)-1,2,4-benzènetricarboxylique ;
l'acide 5-[((1S)-1,2,3,4-tétrahydro-1-naphtalényl{[4'-(trifluorométhyl)[1,1'-biphényl]-4-yl]méthyl}amino)carbonyl]-1,2,4-benzènetricarboxylique ;
l'acide 5-({([1,1'-biphényl]-2-ylméthyl)[(1S)-1,2,3,4-tétrahydro-1-naphtalényl]amino}carbonyl)-1,2,4-benzènetricarboxylique ;
l'acide 5-({([1,1'-biphényl]-3-ylméthyl)[(1S)-1,2,3,4-tétrahydro-1-naphtalényl]amino}carbonyl)-1,2,4-benzènetricarboxylique ;
l'acide 5-({[(2-méthyl[1,1'-biphényl]-3-yl)méthyl][(1S)-1,2,3,4-tétrahydro-1-naphtalényl]amino}carbonyl)-1,2,4-benzènetricarboxylique ;
l'acide 5-[((1S)-1,2,3,4-tétrahydro-1-naphtalényl{[4'-(trifluorométhyl)[1,1'-biphényl]-3-yl]méthyl}amino)carbonyl]-1,2,4-benzènetricarboxylique ;
l'acide 5-({[(5'-fluoro-2'-méthoxy[1,1'-biphényl]-3-yl)méthyl][(1S)-1,2,3,4-tétrahydro-1-naphtalényl]amino}carbonyl)-1,2,4-benzènetricarboxylique ;
l'acide 5-({[(5'-chloro-2'-méthoxy[1,1'-biphényl]-3-yl)méthyl][(1S)-1,2,3,4-tétrahydro-1-naphtalényl]amino}-carbonyl)-1,2,4-benzènetricarboxylique ;
l'acide 5-({[(3',5'-dichloro[1,1'-biphényl]-3-yl)méthyl]-[(1S)-1,2,3,4-tétrahydro-1-naphtalényl]amino}carbonyl)-1,2,4-benzènetricarboxylique ;
l'acide 5-({[(2'-méthoxy[1,1'-biphényl]-3-yl)méthyl]-[(1S)-1,2,3,4-tétrahydro-1-naphtalényl]amino}carbonyl)-1,2,4-benzènetricarboxylique ;
l'acide 5-({[(2'-chloro[1,1'-biphényl]-3-yl)méthyl][(1S)-1,2,3,4-tétrahydro-1-naphtalényl]amino}carbonyl)-1,2,4-benzènetricarboxylique ;
l'acide 5-({[(2',5'-diméthoxy[1,1'-biphényl]-3-yl)-méthyl][(1S)-1,2,3,4-tétrahydro-1-naphtalényl]amino}carbonyl)-1,2,4-benzènetricarboxylique ;
l'acide 5-({ [(3'-chloro[1,1'-biphényl] -3-yl)méthyl] [(1S)-1,2,3,4-tétrahydro-1-naphtalényl]amino}carbonyl)-1,2,4-benzènetricarboxylique ;
l'acide 5-({[(3',4'-dichloro[1,1'-biphényl]-3-yl)méthyl][(1S)-1,2,3,4-tétrahydro-1-naphtalényl]amino}-carbonyl)-1,2,4-benzènetricarboxylique ;
l'acide 5-({[(3'-chloro-4'-fluoro[1,1'-biphényl]-3-yl)-méthyl][(1S)-1,2,3,4-tétrahydro-1-naphtalényl]amino}carbonyl)-1,2,4-benzènetricarboxylique ;
l'acide 5-({[(3'-nitro[1,1'-biphényl]-3-yl)méthyl][(1S)-1,2,3,4-tétrahydro-1-naphtalényl]amino}carbonyl)-1,2,4-benzènetricarboxylique ;
l'acide 5-({[(3'-méthoxy[1,1'-biphényl]-3-yl)méthyl]-[(1S)-1,2,3,4-tétrahydro-1-naphtalényl]amino}carbonyl)-1,2,4-benzènetricarboxylique ;
l'acide 5-({[(4'-chloro[1,1'-biphényl]-3-yl)méthyl][(1S)-1,2,3,4-tétrahydro-1-naphtalényl]amino}carbonyl)-1,2,4-benzènetricarboxylique ;
l'acide 5-({(1S)-1,2,3,4-tétrahydro-1-naphtalényl-[(3',4',5'-triméthoxy[1,1'-biphényl]3-yl)méthyl]amino}-carbonyl)-1,2,4-benzènetricarboxylique ;
l'acide 6-({[(3',4'-dichloro-1,1'-biphényl-3-yl)méthyl]-[(1S)-1,2,3,4-tétrahydro-1-naphtalényl]amino}carbonyl)-1,3-dioxo-1,3-dihydro-2-benzothiophéne-5-carboxylique ;
l'acide 5-({[4-(1-pyrrolidinyl)benzyl][(1S)-1,2,3,4-tétrahydro-1-naphtalényl]amino}carbonyl)-1,2,4-benzènetricarboxylique ;
l'acide 5-({[4-(3-pyridinyl)benzyl][(1S)-1,2,3,4-tétrahydro-1-naphtalényl]amino}carbonyl)-1,2,4-benzènetricarboxylique ;
l'acide 5-({(1S)-1,2,3,4-tétrahydro-1-naphtalényl[3-(3-thiényl)benzyl]amino}carbonyl)-1,2,4-benzènetricarboxylique ; et
l'acide 5-({(1S)-1,2,3,4-tétrahydro-1-naphtalényl[3-(2-thiényl)bénzyl]amino}carbonyl)-1,2,4-benzènetricarboxylique ;
l'acide 5-({(3-benzylbenzyl)[(1S)-1,2,3,4-tétrahydro-1-naphtalényl]amino}carbonyl)-1,2,4-benzènetricarboxylique ;
l'acide 5-({[3-(4-chlorophénoxy)benzyl][(1S)-1,2,3,4-tétrahydro-1-naphtalényl]amino}carbonyl)-1,2,4-benzènetricarboxylique ;
l'acide 5-[((1S)-1,2,3,4-tétrahydro-1-naphtalényl{3-[3(trifluorométhyl)phénoxy]benzyl}amino)carbonyl]-1,2,4-benzènetricarboxylique ;
l'acide 5-({[3-(4-méthoxyphénoxy)benzyl][(1S)-1,2,3,4 tétrahydro-1-naphtalényl]amino}carbonyl)-1,2,4-benzènetricarboxylique ;
l'acide 5-({[3-(3,4-dichlorophénoxy)benzyl][(1S)-1,2,3,4-tétrahydro-1-naphtalényl]amino}carbonyl)-1,2,4-benzènetricarboxylique ;
l'acide 5-({[3-(4-chlorophénoxy)benzyl][(1S)-1,2,3,4-tétrahydro-1-naphtalényl]amino}carbonyl)-1,2,4-benzènetricarboxylique ;
l'acide 5-({[3-(4-tert-butylphénoxy)benzyl][(1S)-1,2,3,4-tétrahydro-1-naphtalényl]amino}carbonyl)-1,2,4-benzènetricarboxylique ;
l'acide 5-({[3-(4-méthylphénoxy)benzyl][(1S)-1,2,3,4-tétrahydro-1-naphtalényl]amino}carbonyl)-1,2,4-benzènetricarboxylique ;
l'acide 5-({[3-(3,5-dichlorophénoxy)benzyl][(1S)-1,2,3,4-tétrahydro-1-naphtalényl]amino}carbonyl)-1,2,4-benzènetricarboxylique ;
l'acide 5-({[3-(4-nitrophénoxy)benzyl][(1S)-1,2,3,4-tétrahydro-1-naphtalényl]amino}carbonyl)-1,2,4-benzènetricarboxylique ;
l'acide 5-({[3-(4-cyanophénoxy)benzyl][(1S)-1,2,3,4-tétrahydro-1-naphtalényl]amino}carbonyl)-1,2,4-benzènetricarboxylique ; et
l'acide 5-({[3-(4-fluorophénoxy)benzyl][(1S)-1,2,3,4-tétrahydro-1-naphtalényl]amino}carbonyl)-1,2,4-benzènetricarboxylique ;
l'acide 5-({(3-phénoxybenzyl)[(1S)-1,2,3,4-tétrahydro-1-naphtalényl]amino}carbonyl)-1,2,4-benzènetricarboxylique ;
l'acide 2-[(méthylamino)carbonyl]-5-({(3-phénoxybenzyl)[(1S)-1,2,3,4-tétrahydro-1-naphtalényl]amino}carbonyl)-téréphtalique ;
l'acide 4-[(méthylamino)carbonyl]-6-({(3-phénoxybenzyl)[(1S)-1,2,3,4-tétrahydro-1-naphtalényl]amino}carbonyl)-isophtalique ;
l'acide 2-[(cyanoamino)carbonyl]-5-({(3-phénoxybenzyl)-[(1S)-1,2,3,4-tétrahydro-1-naphtalényl]amino}carbonyl)téréphtalique ;
l'acide 4-[(cyanoamino)carbonyl]-6-({(3-phénoxybenzyl)[(1S)-1,2,3,4-tétrahydro-1-naphtalényl]amino}carbonyl)-isophtalique ;
l'acide 2-(aminocarbonyl)-5-({(3-phénoxybenzyl)[(1S)-1,2,3,4-tétrahydro-1-naphtalényl]amino}carbonyl)téréphtalique ;
l'acide 4-(aminocarbonyl)-6-({(3-phénoxybenzyl)[(1S)-1,2,3,4-tétrahydro-1-naphtalényl]amino}carbonyl)isophtalique ;
l'acide 2-(méthoxycarbonyl)-5-({(3-phénoxybenzyl)[(1S)-1,2,3,4-tétrahydro-1-naphtalényl]amino}carbonyl)téréphtalique ;
l'acide 4-(méthoxycarbonyl)-6-({(3-phénoxybenzyl)[(1S)-1,2,3,4-tétrahydro-1-naphtalényl]amino}carbonyl)isophtalique ;
l'acide- 2-hydroxy-5-({(3-phénoxybenzyl)[(1S)-1,2,3,4-tétrahydro-1-naphtalényl]amino}carbonyl)téréphtalique ;
l'acide 4-hydroxy-6-({(3-phénoxybenzyl)[(1S)-1,2,3,4-tétrahydro-1-naphtalényl]amino}carbonyl)isophtalique ;
l'acide 2-({(3-phénoxybenzyl)[(1S)-1,2,3,4-tétrahydro-1-naphtalényl]amino}carbonyl)-5-sulfotéréphtalique ; et
l'acide 4-({(3-phénoxybenzyl)[(1S)-1,2,3,4-tétrahydro-1-naphtalényl]amino}carbonyl)-6-sulfoisophtalique ;
l'acide 4-({[(4-méthoxyphényl)sulfonyl]amino}carbonyl)-6-({(3-phénoxybenzyl)[(1S)-1,2,3,4-tétrahydro-1-naphtalényl]-amino}carbonyl)isophtalique ;
l'acide 2-({[(4-méthoxyphényl)sulfonyl]amino}carbonyl)-5-({(3-phénoxybenzyl)[(1S)-1,2,3,4-tétrahydro-1-naphtalényl]-amino}carbonyl)téréphtalique ;
l'acide 4-({[(3-chloro-4-méthylphényl)sulfonyl]amino}-carbonyl)-6-({(3-phénoxybenzyl)[(1S)-1,2,3,4-tétrahydro-1-naphtalényl]amino}carbonyl)isophtalique ;
l'acide 2-({[(3-chloro-4-méthylphényl)sulfonyl]amino}-carbonyl)-5-({(3-phénoxybenzyl)[(1S)-1,2,3,4-tétrahydro-1-naphtalényl]amino}carbonyl)téréphtalique ;
l'acide 4-({[(2-chlorophényl)sulfonyl]amino}carbonyl)-6-({(3-phénoxybenzyl)[(1S)-1,2,3,4-tétrahydro-1-naphtalényl]-amino}carbonyl)isophtalique ;
l'acide 2-({[(2-chlorophényl)sulfonyl]amino}carbonyl)-5-({(3-phénoxybenzyl)[(1S)-1,2,3,4-tétrahydro-1-naphtalényl]-amino}carbonyl)téréphtalique ;
l'acide 4-({[(3-chlorophényl)sulfonyl]amino}carbonyl)-6-({(3-phénoxybenzyl)[(1S)-1,2,3,4-tétrahydro-1-naphtalényl]-amino}carbonyl)isophtalique ;
l'acide 2-({[(3-chlorophényl)sulfonyl]amino}carbonyl)-5-({(3-phénoxybenzyl)[(1S)-1,2,3,4-tétrahydro-1-naphtalényl]-amino}carbonyl)téréphtalique ;
l'acide 4-({[(4-chlorophényl)sulfonyl]amino}carbonyl)-6-({(3-phénoxybenzyl)[(1S)-1,2,3,4-tétrahydro-1-naphtalényl]-amino}carbonyl)isophtalique ;
l'acide 2-({[(4-chlorophényl)sulfonyl]amino}carbonyl)-5-({(3-phénoxybenzyl)[(1S)-1,2,3,4-tétrahydro-1-naphtalényl]-amino}carbonyl)téréphtalique ;
l'acide 4-({[(4-nitrophényl)sulfonyl]amino}carbonyl)-6-({(3-phénoxybenzyl)[(1S)-1,2,3,4-tétrahydro-1-naphtalényl]-amino}carbonyl)isophtalique ;
l'acide 2-({[(4-nitrophényl)sulfonyl]amino}carbonyl)-5-({(3-phénoxybenzyl)[(1S)-1,2,3,4-tétrahydro-1-naphtalényl]-amino}carbonyl)téréphtalique ;
l'acide 4-({[(4-bromophényl)sulfonyl]amino}carbonyl)-6-({(3-phénoxybenzyl)[(1S)-1,2,3,4-tétrahydro-1-naphtalényl]-amino}carbonyl)isophtalique ;
l'acide 2-({[(4-bromophényl)sulfonyl]amino}carbonyl)-5-({(3-phénoxybenzyl)[(1S)-1,2,3,4-tétrahydro-1-naphtalényl]-amino}carbonyl)téréphtalique ;
l'acide 4-({[(3-nitrophényl)sulfonyl]amino}carbonyl)-6-({(3-phénoxybenzyl)[(1S)-1,2,3,4-tétrahydro-1-naphtalényl]-amino}carbonyl)isophtalique ;
l'acide 2-({[(3-nitrophényl)sulfonyl]amino}carbonyl)-5-({(3-phénoxybenzyl)[(1S)-1,2,3,4-tétrahydro-1-naphtalényl]-amino}carbonyl)téréphtalique ;
l'acide 4-({[(phényl)sulfonyl]amino}carbonyl)-6-({(3-phénoxybenzyl)[(1S)-1,2,3,4-tétrahydro-1-naphtalényl]amino}-carbonyl)isophtalique ;
l'acide 2-({[(phényl)sulfonyl]amino}carbonyl)-5-({(3-phénoxybenzyl)[(1S)-1,2,3,4-tétrahydro-1-naphtalényl]amino}-carbonyl)téréphtalique ;
l'acide 4-({[(2-nitrophényl)sulfonyl]amino}carbonyl)-6-({(3-phénoxybenzyl)[(1S)-1,2,3,4-tétrahydro-1-naphtalényl]-amino}carbonyl)isophtalique ;
l'acide 2-({[(2-nitrophényl)sulfonyl]amino}carbonyl)-5-({(3-phénoxybenzyl)[(1S)-1,2,3,4-tétrahydro-1-naphtalényl]-amino}carbonyl)téréphtalique ;
l'acide 4-({[(2-méthylphényl)sulfonyl]amino}carbonyl)-6-({(3-phénoxybenzyl)[(1S)-1,2,3,4-tétrahydro-1-naphtalényl]-amino}carbonyl)isophtalique ;
l'acide 2-({[(2-méthylphényl)sulfonyl]amino}carbonyl)-5-({(3-phénoxybenzyl)[(1S)-1,2,3,4-tétrahydro-1-naphtalényl]-amino}carbonyl)téréphtalique ;
l'acide 4-{[({4-[(2,3-dihydroxypropyl)amino]phényl}-sulfonyl)amino]carbonyl}-6-({(3-phénoxybenzyl)[(1S)-1,2,3,4-tétrahydro-1-naphtalényl]amino}carbonyl)isophtalique ;
l'acide 2-{[({4-[(2,3-dihydroxypropyl)amino]phényl}-sulfonyl)amino]carbonyl}-5-({(3-phénoxybenzyl)[(1S)-1,2,3,4-tétrahydro-1-naphtalényl]amino}carbonyl)téréphtalique ;
l'acide 2-({[(4-{[(méthylamino)carbonyl]amino}phényl)-sulfonyl]amino}carbonyl)-5-({(3-phénoxybenzyl)[(1S)-1,2,3,4-tétrahydro-1-naphtalényl]amino}carbonyl)téréphtalique ;
l'acide 4-({[(4-{[(méthylamino)carbonyl]amino}phényl)-sulfonyl]amino}carbonyl)-6-({(3-phénoxybenzyl)[(1S)-1,2,3,4-tétrahydro-1-naphtalényl]amino}carbonyl)isophtalique ;
l'acide 4-[({[4-(butyrylamino)phényl]sulfonyl}-amino}carbonyl]-6-({(3-phénoxybenzyl)[(1S)-1,2,3,4-tétrahydro-1-naphtalényl]amino}carbonyl)isophtalique ;
l'acide 2-[({[4-(butyrylamino)phényl]sulfonyl}amino}-carbonyl]-5-({(3-phénoxybenzyl)[(1S)-1,2,3,4-tétrahydro-1-naphtalényl]amino}carbonyl)téréphtalique ;
l'acide 4-({[(3,4-difluorophényl)sulfonyl]amino}-carbonyl)-6-({(3-phénoxybenzyl)[(1S)-1,2,3,4-tétrahydro-1-naphtalényl]amino}carbonyl)isophtalique ;
l'acide 2-({[(3,4-difluorophényl)sulfonyl]amino}-carbonyl)-5-({(3-phénoxybenzyl)[(1S)-1,2,3,4-tétrahydro-1-naphtalényl]amino}carbonyl)téréphtalique ;
l'acide 4-({[(2,5-difluorophényl)sulfonyl]amino}-carbonyl)-6-({(3-phénoxybenzyl)[(1S)-1,2,3,4-tétrahydro-1-naphtalényl]amino}carbonyl)isophtalique ;
l'acide 2-({[(2,5-difluorophényl)sulfonyl]amino}carbonyl)-5-({(3-phénoxybenzyl)[(1S)-1,2,3,4-tétrahydro-1-naphtalényl]amino}carbonyl)téréphtalique ;
l'acide 4-({[(2-méthoxy-4-méthylphényl)sulfonyl]amino}-carbonyl)-6-({(3-phénoxybenzyl)[(1S)-1,2,3,4-tétrahydro-1-naphtalényl]amino}carbonyl)isophtalique ;
l'acide 2-({[(2-méthoxy-4-méthylphényl)sulfonyl]amino}-carbonyl)-5-({(3-phénoxybenzyl)[(1S)-1,2,3,4-tétrahydro-1-naphtalényl]amino}carbonyl)téréphtalique ;
l'acide 4-({[(4-méthylphényl)sulfonyl]amino}carbonyl)-6-({(3-phénoxybenzyl)[(1S)-1,2,3,4-tétrahydro-1-naphtalényl]amino}carbonyl)isophtalique ;
l'acide 2-({[(4-méthylphényl)sulfonyl]amino}carbonyl)-5-({(3-phénoxybenzyl)[(1S)-1,2,3,4-tétrahydro-1-naphtalényl]-amino}carbonyl)téréphtalique ;
l'acide 2-({[(2-fluorophényl)sulfonyl]amino}carbonyl)-5-({(3-phénoxybenzyl)[(1S)-1,2,3,4-tétrahydro-1-naphtalényl]-amino}carbonyl)téréphtalique ;
l'acide 4-({[(2-fluorophényl)sulfonyl]amino}carbonyl)-6-({(3-phénoxybenzyl)[(1S)-1,2,3,4-tétrahydro-1-naphtalényl]-amino}carbonyl)isophtalique ;
l'acide 2-({[(4-hydroxyphényl)sulfonyl]amino}carbonyl)-5-({(3-phénoxybenzyl)[(1S)-1,2,3,4-tétrahydro-1-naphtalényl]-amino}carbonyl)téréphtalique ; et
l'acide 4-({[(4-hydroxyphényl)sulfonyl]amino}carbonyl)-6-({(3-phénoxybenzyl)[(1S)-1,2,3,4-tétrahydro-1-naphtalényl]-amino}carbonyl)isophtalique ;
l'acide 4-({[(5-méthyl-2-pyridinyl)sulfonyl]amino}-carbonyl)-6-({(3-phénoxybenzyl)[(1S)-1,2,3,4-tétrahydro-1-naphtalényl]amino}carbonyl)isophtalique ;
l'acide 2-({[(5-méthyl-2-pyridinyl)sulfonyl]amino}-carbonyl)-5-({(3-phénoxybenzyl)[(1S)-1,2,3,4-tétrahydro-1-naphtalényl]amino}carbonyl)téréphtalique ;
l'acide 4-({[(5-nitro-2-thiényl)sulfonyl]amino}carbonyl)-6-({(3-phénoxybenzyl)[(1S)-1,2,3,4-tétrahydro-1-naphtalényl]-amino}carbonyl)isophtalique ;
l'acide 2-({[(5-nitro-2-thiényl)sulfonyl]amino}carbonyl)-5-({(3-phénoxybenzyl)[(1S)-1,2,3,4-tétrahydro-1-naphtalényl]-amino}carbonyl)téréphtalique ;
l'acide 2-({[(5-chloro-2-thiényl)sulfonyl]amino}-carbonyl)-5-({(3-phénoxybenzyl)[(1S)-1,2,3,4-tétrahydro-1-naphtalényl]amino}carbonyl)téréphtalique ; et
l'acide 4-({[(5-chloro-2-thiényl)sulfonyl]amino}-carbonyl)-6-({(3-phénoxybenzyl)[(1S)-1,2,3,4-tétrahydro-1-naphtalényl]amino}carbonyl)isophtalique ;
l'acide 4-{[(benzylsulfonyl)amino]carbonyl}-6-({(3-phénoxybenzyl)[(1S)-1,2,3,4-tétrahydro-1-naphtalényl]amino}-carbonyl)isophtalique ; et
l'acide 2-{[(benzylsulfonyl)amino]carbonyl}-5-({(3-phénoxybenzyl)[(1S)-1,2,3,4-tétrahydro-1-naphtalényl]amino}-carbonyl)téréphtalique ;
l'acide 5-({[3-(benzoyloxy)benzyl][(1S)-1,2,3,4-tétrahydro-1-naphtalényl]amino}carbonyl)-1,2,4-benzènetricarboxylique ;
l'acide 5-({[3-(cyclohexylamine)benzyl][(1S)-1,2,3,4-tétrahydro-1-naphtalényl]amino}carbonyl)-1,2,4-benzènetricarboxylique ;
l'acide 5-({3-[exo-bicyclo[2. 2. 1]hept-2-yloxy]benzyl}-[(1S)-1,2,3,4-tétrahydro-1-naphtalényl]amino}carbonyl)-1,2,4-benzènetricarboxylique ;
l'acide 5-({[3-(2-cyclohexen-1-yloxy)benzyl][(1S)-1,2,3,4-tétrahydro-1-naphtalényl]amino}carbonyl)-1,2,4-benzènetricarboxylique ;
l'acide 5-({[3-(4-pyridinyloxy)benzyl][(1S)-1,2,3,4-tétrahydro-1-naphtalényl]amino}carbonyl)-1,2,4-benzènetricarboxylique ; et
l'acide 5-({[3-(2-pyrimidinyloxy)benzyl][(1S)-1,2,3,4-tétrahydro-1-naphtalényl]amino}carbonyl)-1,2,4-benzènetricarboxylique ;
l'acide 5-({[3-(phénylsulfanyl)benzyl][(1S)-1,2,3,4-tétrahydro-1-naphtalényl]amino}carbonyl)-1,2,4-benzènetricarboxylique ;
l'acide 5-({{3-[(4-méthoxyphényl)sulfanyl]benzyl}[(1S)-1,2,3,4-tétrahydro-1-naphtalényl]amino}carbonyl)-1,2,4-benzènetricarboxylique ;
l'acide 5-({{3-[(4-nitrophényl)sulfanyl]benzyl}[(1S)-1,2,3,4-tétrahydro-1-naphtalényl]amino}carbonyl)-1,2,4-benzènetricarboxylique ; et
l'acide 5-({{3-[(4-chlorophényl)sulfanyl]benzyl}[(1S)-1,2,3,4-tétrahydro-1-naphtalényl]amino}carbonyl)-1,2,4-benzènetricarboxylique ;
l'acide 5-({(3-benzoylbenzyl)[(1S)-1,2,3,4-tétrahydro-1-naphtalényl]amino}carbonyl)-1,2,4-benzènetricarboxylique ;
l'acide 5-({{3[hydroxy(phényl)méthyl]benzyl}[(1S)-1,2,3,4-tétrahydro-1-naphtalényl]amino}carbonyl)-1,2,4-benzènetricarboxylique ;
l'acide 5-({{3-[(phénoxyimino)méthyl]benzyl}[(1S)-1,2,3,4-tétrahydro-1-naphtalényl]amino}carbonyl)-1,2,4-benzènetricarboxylique ;
l'acide 5-({(3-{[(benzyloxy)imino]méthyl}benzyl)[(1S)-1,2,3,4-tétrahydro-1-naphtalényl]amino}carbonyl)-1,2,4-benzènetricarboxylique ;
l'acide 5-({[3-({[(4-nitrobenzyl)oxy]imino}méthyl)-benzyl][(1S)-1,2,3,4-tétrahydro-1-naphtalényl]amino}carbonyl)-1,2,4-benzènetricarboxylique ; et
l'acide 5-({[3-({[(2,3,4,5,6-pentafluorobenzyl)oxy]-imino}méthyl)benzyl][(1S)-1,2,3,4-tétrahydro-1-naphtalényl]-amino}carbonyl)-1,2,4-benzènetricarboxylique ;
l'acide 5-{[(1S)-1,2,3,4-tétrahydro-1-naphtalényl(3-{[(tétrahydro-2H-pyran-2-yloxy)imino]méthyl}benzyl)amino]-carbonyl}-1,2,4-benzènetricarboxylique ;
l'acide 5-({(cyclohexylméthyl)[(1S)-1,2,3,4-tétrahydro-1-naphtalényl]amino}carbonyl)-1,2,4-benzènetricarboxylique ;
l'acide 5-({(4-phénoxybutyl)[(1S)-1,2,3,4-tétrahydro-1-naphtalényl]amino}carbonyl)-1,2,4-benzènetricarboxylique ;
l'acide 5-({{[5-(éthoxycarbonyl)-2-thiényl]méthyl}[(1S)-1,2,3,4-tétrahydro-1-naphtalényl]amino}carbonyl)-1,2,4-benzènetricarboxylique.

28. Composition pharmaceutique comprenant une quantité thérapeutiquement efficace d'un composé selon la revendication 1, ou d'un sel pharmaceutiquement acceptable de celui-ci en combinaison avec un vecteur pharmaceutiquement acceptable.

29. Utilisation d'un composé selon la revendication 1, ou d'un sel pharmaceutiquement acceptable de celui-ci pour la fabrication d'un médicament destiné au traitement de la suractivité de la vessie ou de l'incontinence urinaire ou de la douleur chez un mammifère hôte nécessitant un tel traitement.
